# EUROPEAN PATENT APPLICATION

(11) **EP 1 696 028 A2**
(43) Date of publication of application: **30.08.2006**
(21) Application number: 06005998.7
(22) Date of filing: 22.05.2001
(51) Int. Cl.: C12N 15/12, C12N 15/62, C12N 5/10, C12Q 1/68, C07K 14/47, C07K 16/18, A61K 38/00, G01N 33/50

(54) **Compositions and methods for the therapy and diagnosis of breast cancer**

(30) Priority: 24.05.2000 US 577505; 08.06.2000 US 590583; 26.10.2000 US 699295; 16.03.2001 US 810936
(62) Divisional of application: 01939366.9
(71) Applicant: CORIXA CORPORATION, Seattle, WA 98101 (US)
(72) Inventor: Frudakis, Tony, Seattle, WA 98101 (US); Reed, Steven, Seattle, WA 98101 (US); Smith, John, Seattle, WA 98101 (US); Misher, Lynda, Seattle, WA 98101 (US); Dillon, Davin, Seattle, WA 98101 (US); Retter, Marc, Seattle, WA 98101 (US); Wang, Aijun, Seattle, WA 98101 (US); Skeiky, Yasir, Seattle, WA 98101 (US); Harlocker, Susan, Seattle, WA 98101 (US); Day, Craig, Seattle, WA 98101 (US)
(74) Representative: Thornton, Neil

(57) **Abstract**

Compositions and methods for the therapy and diagnosis of cancer, particularly breast cancer, are disclosed. Illustrative compositions comprise one or more breast tumor polypeptides, immunogenic portions thereof, polynucleotides that encode such polypeptides, antigen presenting cell that expresses such polypeptides, and T cells that are specific for cells expressing such polypeptides. The disclosed compositions are useful, for example, in the diagnosis, prevention and/or treatment of diseases, particularly breast cancer.

## Description

### TECHNICAL FIELD OF THE INVENTION

The present invention relates generally to therapy and diagnosis of cancer, such as breast cancer. The invention is more specifically related to polypeptides, comprising at least a portion of a breast tumor protein, and to polynucleotides encoding such polypeptides. Such polypeptides and polynucleotides are useful in pharmaceutical compositions, e.g., vaccines, and other compositions for the diagnosis and treatment of breast cancer.

### BACKGROUND OF THE INVENTION

Breast cancer is a significant health problem for women in the United States and throughout the world. Although advances have been made in detection and treatment of the disease, breast cancer remains the second leading cause of cancer-related deaths in women, affecting more than 180,000 women in the United States each year. For women in North America, the life-time odds of getting breast cancer are now one in eight.

No vaccine or other universally successful method for the prevention or treatment of breast cancer is currently available. Management of the disease currently relies on a combination of early diagnosis (through routine breast screening procedures) and aggressive treatment, which may include one or more of a variety of treatments such as surgery, radiotherapy, chemotherapy and hormone therapy. The course of treatment for a particular breast cancer is often selected based on a variety of prognostic parameters, including an analysis of specific tumor markers. *See, e.g.,* Porter-Jordan and Lippman, *Breast Cancer* 8:73-100 (1994). However, the use of established markers often leads to a result that is difficult to interpret, and the high mortality observed in breast cancer patients indicates that improvements are needed in the treatment, diagnosis and prevention of the disease.

Accordingly, there is a need in the art for improved methods for therapy and diagnosis of breast cancer. The present invention fulfills these needs and further provides other related advantages.

### SUMMARY OF THE INVENTION

In one aspect, the present invention provides polynucleotide compositions comprising a sequence selected from the group consisting of:
(a) sequences provided in SEQ ID NO: 1, 3-86, 142-298, 301-303, 307, 313, 314, 316, 317, 325 and 327-330;
(b) complements of the sequences provided in SEQ ID NO: 1, 3-86, 142-298, 301-303, 307, 313, 314, 316, 317, 325 and 327-330;
(c) sequences consisting of at least 20 contiguous residues of a sequence provided in SEQ ID NO: 1, 3-86, 142-298, 301-303, 307, 313, 314, 316, 317, 325 and 327-330;
(d) sequences that hybridize to a sequence provided in SEQ ID NO: 1, 3-86, 142-298, 301-303, 307, 313, 314, 316, 317, 325 and 327-330, under moderately stringent conditions;
(e) sequences having at least 75% identity to a sequence of SEQ ID NO: 1, 3-86, 142-298, 301-303, 307, 313, 314, 316, 317, 325 and 327-330;
(f) sequences having at least 90% identity to a sequence of SEQ ID NO: 1, 3-86, 142-298, 301-303, 307, 313, 314, 316, 317, 325 and 327-330; and
(g) degenerate variants of a sequence provided in SEQ ID NO: 1, 3-86, 142-298, 301-303, 307, 313, 314, 316, 317, 325 and 327-330.

In one preferred embodiment, the polynucleotide compositions of the invention are expressed in at least about 20%, more preferably in at least about 30%, and most preferably in at least about 50% of breast tumors samples tested, at a level that is at least about 2-fold, preferably at least about 5-fold, and most preferably at least about 10-fold higher than that for normal tissues.

The present invention, in another aspect, provides polypeptide compositions comprising an amino acid sequence that is encoded by a polynucleotide sequence described above.

The present invention further provides polypeptide compositions comprising an amino acid sequence selected from the group consisting of sequences recited in SEQ ID NO: 299, 300, 304-306, 308-312, 314, 326 and 331-334.

In certain preferred embodiments, the polypeptides and/or polynucleotides of the present invention are immunogenic, i.e., they are capable of eliciting an immune response, particularly a humoral and/or cellular immune response, as further described herein.

The present invention further provides fragments, variants and/or derivatives of the disclosed polypeptide and/or polynucleotide sequences, wherein the fragments, variants and/or derivatives preferably have a level of immunogenic activity of at least about 50%, preferably at least about 70% and more preferably at least about 90% of the level of immunogenic activity of a polypeptide sequence set forth in SEQ ID NOs: 299, 300, 304-306, 308-312, 314, 326 and 331-334 or a polypeptide sequence encoded by a polynucleotide sequence set forth in SEQ ID NOs: 1, 3-86, 142-298, 301-303, 307, 313, 314, 316, 317, 325 and 327-330.

The present invention further provides polynucleotides that encode a polypeptide described above, expression vectors comprising such polynucleotides and host cells transformed or transfected with such expression vectors.

Within other aspects, the present invention provides pharmaceutical compositions comprising a polypeptide or polynucleotide as described above and a physiologically acceptable carrier.

Within a related aspect of the present invention, the pharmaceutical compositions, e.g., vaccine compositions, are provided for prophylactic or therapeutic applications. Such compositions generally comprise an immunogenic polypeptide or polynucleotide of the invention and an immunostimulant, such as an adjuvant.

The present invention further provides pharmaceutical compositions that comprise: (a) an antibody or antigen-binding fragment thereof that specifically binds to a polypeptide of the present invention, or a fragment thereof; and (b) a physiologically acceptable carrier.

Within further aspects, the present invention provides pharmaceutical compositions comprising: (a) an antigen presenting cell that expresses a polypeptide as described above and (b) a pharmaceutically acceptable carrier or excipient. Illustrative antigen presenting cells include dendritic cells, macrophages, monocytes, fibroblasts and B cells.

Within related aspects, pharmaceutical compositions are provided that comprise: (a) an antigen presenting cell that expresses a polypeptide as described above and (b) an immunostimulant.

The present invention further provides, in other aspects, fusion proteins that comprise at least one polypeptide as described above, as well as polynucleotides encoding such fusion proteins, typically in the form of pharmaceutical compositions, e.g., vaccine compositions, comprising a physiologically acceptable carrier and/or an immunostimulant. The fusions proteins may comprise multiple immunogenic polypeptides or portions/variants thereof, as described herein, and may further comprise one or more polypeptide segments for facilitating the expression, purification and/or immunogenicity of the polypeptide(s).

Within further aspects, the present invention provides methods for stimulating an immune response in a patient, preferably a T cell response in a human patient, comprising administering a pharmaceutical composition described herein. The patient may be afflicted with breast cancer, in which case the methods provide treatment for the disease, or patient considered at risk for such a disease may be treated prophylactically.

Within further aspects, the present invention provides methods for inhibiting the development of a cancer in a patient, comprising administering to a patient a pharmaceutical composition as recited above. The patient may be afflicted with breast cancer, in which case the methods provide treatment for the disease, or patient considered at risk for such a disease may be treated prophylactically.

The present invention further provides, within other aspects, methods for removing tumor cells from a biological sample, comprising contacting a biological sample with T cells that specifically react with a polypeptide of the present invention, wherein the step of contacting is performed under conditions and for a time sufficient to permit the removal of cells expressing the protein from the sample.

Within related aspects, methods are provided for inhibiting the development of a cancer in a patient, comprising administering to a patient a biological sample treated as described above.

Methods are further provided, within other aspects, for stimulating and/or expanding T cells specific for a polypeptide of the present invention, comprising contacting T cells with one or more of: (i) a polypeptide as described above; (ii) a polynucleotide encoding such a polypeptide; and/or (iii) an antigen presenting cell that expresses such a polypeptide; under conditions and for a time sufficient to permit the stimulation and/or expansion of T cells. Isolated T cell populations comprising T cells prepared as described above are also provided.

Within further aspects, the present invention provides methods for inhibiting the development of a cancer in a patient, comprising administering to a patient an effective amount of a T cell population as described above.

The present invention further provides methods for inhibiting the development of a cancer in a patient, comprising the steps of: (a) incubating CD4⁺ and/or CD8⁺ T cells isolated from a patient with one or more of: (i) a polypeptide comprising at least an immunogenic portion of polypeptide disclosed herein; (ii) a polynucleotide encoding such a polypeptide; and (iii) an antigen-presenting cell that expressed such a polypeptide; and (b) administering to the patient an effective amount of the proliferated T cells, and thereby inhibiting the development of a cancer in the patient. Proliferated cells may, but need not, be cloned prior to administration to the patient.

Within further aspects, the present invention provides methods for determining the presence or absence of a cancer, preferably a breast cancer, in a patient comprising: (a) contacting a biological sample obtained from a patient with a binding agent that binds to a polypeptide as recited above; (b) detecting in the sample an amount of polypeptide that binds to the binding agent; and (c) comparing the amount of polypeptide with a predetermined cut-off value, and therefrom determining the presence or absence of a cancer in the patient. Within preferred embodiments, the binding agent is an antibody, more preferably a monoclonal antibody.

The present invention also provides, within other aspects, methods for monitoring the progression of a cancer in a patient. Such methods comprise the steps of: (a) contacting a biological sample obtained from a patient at a first point in time with a binding agent that binds to a polypeptide as recited above; (b) detecting in the sample an amount of polypeptide that binds to the binding agent; (c) repeating steps (a) and (b) using a biological sample obtained from the patient at a subsequent point in time; and (d) comparing the amount of polypeptide detected in step (c) with the amount detected in step (b) and therefrom monitoring the progression of the cancer in the patient.

The present invention further provides, within other aspects, methods for determining the presence or absence of a cancer in a patient, comprising the steps of: (a) contacting a biological sample obtained from a patient with an oligonucleotide that hybridizes to a polynucleotide that encodes a polypeptide of the present invention; (b) detecting in the sample a level of a polynucleotide, preferably mRNA, that hybridizes to the oligonucleotide; and (c) comparing the level of polynucleotide that hybridizes to the oligonucleotide with a predetermined cut-off value, and therefrom determining the presence or absence of a cancer in the patient. Within certain embodiments, the amount of mRNA is detected via polymerase chain reaction using, for example, at least one oligonucleotide primer that hybridizes to a polynucleotide encoding a polypeptide as recited above, or a complement of such a polynucleotide. Within other embodiments, the amount of mRNA is detected using a hybridization technique, employing an oligonucleotide probe that hybridizes to a polynucleotide that encodes a polypeptide as recited above, or a complement of such a polynucleotide.

In related aspects, methods are provided for monitoring the progression of a cancer in a patient, comprising the steps of: (a) contacting a biological sample obtained from a patient with an oligonucleotide that hybridizes to a polynucleotide that encodes a polypeptide of the present invention; (b) detecting in the sample an amount of a polynucleotide that hybridizes to the oligonucleotide; (c) repeating steps (a) and (b) using a biological sample obtained from the patient at a subsequent point in time; and (d) comparing the amount of polynucleotide detected in step (c) with the amount detected in step (b) and therefrom monitoring the progression of the cancer in the patient.

Within further aspects, the present invention provides antibodies, such as monoclonal antibodies, that bind to a polypeptide as described above, as well as diagnostic kits comprising such antibodies. Diagnostic kits comprising one or more oligonucleotide probes or primers as described above are also provided.

These and other aspects of the present invention will become apparent upon reference to the following detailed description and attached drawings. All references disclosed herein are hereby incorporated by reference in their entirety as if each was incorporated individually.

### BRIEF DESCRIPTION OF THE DRAWINGS

Figure 1 shows the differential display PCR products, separated by gel electrophoresis, obtained from cDNA prepared from normal breast tissue (lanes 1 and 2) and from cDNA prepared from breast tumor tissue from the same patient (lanes 3 and 4). The arrow indicates the band corresponding to B18Ag1.
Figure 2 is a northern blot comparing the level of B18Ag1 mRNA in breast tumor tissue (lane 1) with the level in normal breast tissue.
Figure 3 shows the level of B18Ag1 mRNA in breast tumor tissue compared to that in various normal and non-breast tumor tissues as determined by RNase protection assays.
Figure 4 is a genomic clone map showing the location of additional retroviral sequences obtained from ends of XbaI restriction digests (provided in SEQ ID NO:3 - SEQ ID NO:10) relative to B18Ag1.
Figures 5A and 5B show the sequencing strategy, genomic organization and predicted open reading frame for the retroviral element containing B18Ag1.
Figure 6 shows the nucleotide sequence of the representative breast tumor-specific cDNA B18Ag1.
Figure 7 shows the nucleotide sequence of the representative breast tumor-specific cDNA B17Ag1.
Figure 8 shows the nucleotide sequence of the representative breast tumor-specific cDNA B17Ag2.
Figure 9 shows the nucleotide sequence of the representative breast tumor-specific cDNA B13Ag2a.
Figure 10 shows the nucleotide sequence of the representative breast tumor-specific cDNA B13Ag1b.
Figure 11 shows the nucleotide sequence of the representative breast tumor-specific cDNA B13Ag1a.
Figure 12 shows the nucleotide sequence of the representative breast tumor-specific cDNA B11Ag1.
Figure 13 shows the nucleotide sequence of the representative breast tumor-specific cDNA B3CA3c.
Figure 14 shows the nucleotide sequence of the representative breast tumor-specific cDNA B9CG1.
Figure 15 shows the nucleotide sequence of the representative breast tumor-specific cDNA B9CG3.
Figure 16 shows the nucleotide sequence of the representative breast tumor-specific cDNA B2CA2.
Figure 17 shows the nucleotide sequence of the representative breast tumor-specific cDNA B3CA1.
Figure 18 shows the nucleotide sequence of the representative breast tumor-specific cDNA B3CA2.
Figure 19 shows the nucleotide sequence of the representative breast tumor-specific cDNA B3CA3.
Figure 20 shows the nucleotide sequence of the representative breast tumor-specific cDNA B4CA1.
Figure 21A depicts RT-PCR analysis of breast tumor genes in breast tumor tissues (lanes 1-8) and normal breast tissues (lanes 9-13) and H₂O (lane 14).
Figure 21B depicts RT-PCR analysis of breast tumor genes in prostate tumors (lane 1, 2), colon tumors (lane 3), lung tumor (lane 4), normal prostate (lane 5), normal colon (lane 6), normal kidney (lane 7), normal liver (lane 8), normal lung (lane 9), normal ovary (lanes 10, 18), normal pancreases (lanes 11, 12), normal skeletal muscle (lane 13), normal skin (lane 14), normal stomach (lane 15), normal testes (lane 16), normal small intestine (lane 17), HBL-100 (lane 19), MCF-12A (lane 20), breast tumors (lanes 21-23), H₂O (lane 24), and colon tumor (lane 25).
Figure 22 shows the recognition of a B11Ag1 peptide (referred to as B11-8) by an anti-B11-8 CTL line.
Figure 23 shows the recognition of a cell line transduced with the antigen B11Ag1 by the B 11-8 specific clone A1.
Figure 24 shows recognition of a lung adenocarcinoma line (LT-140-22) and a breast adenocarcinoma line (CAMA-1) by the B11-8 specific clone A1.

### DETAILED DESCRIPTION OF THE INVENTION

The present invention is directed generally to compositions and their use in the therapy and diagnosis of cancer, particularly breast cancer. As described further below, illustrative compositions of the present invention include, but are not restricted to, polypeptides, particularly immunogenic polypeptides, polynucleotides encoding such polypeptides, antibodies and other binding agents, antigen presenting cells (APCs) and immune system cells (*e.g*., T cells).

The practice of the present invention will employ, unless indicated specifically to the contrary, conventional methods of virology, immunology, microbiology, molecular biology and recombinant DNA techniques within the skill of the art, many of which are described below for the purpose of illustration. Such techniques are explained fully in the literature. See, *e.g*., Sambrook, et al. Molecular Cloning: A Laboratory Manual (2nd Edition, 1989); Maniatis et al. Molecular Cloning: A Laboratory Manual (1982); DNA Cloning: A Practical Approach, vol. I & II (D. Glover, ed.); Oligonucleotide Synthesis (N. Gait, ed., 1984); Nucleic Acid Hybridization (B. Hames & S. Higgins, eds., 1985); Transcription and Translation (B. Hames & S. Higgins, eds., 1984); Animal Cell Culture (R. Freshney, ed., 1986); Perbal, A Practical Guide to Molecular Cloning (1984).

All publications, patents and patent applications cited herein, whether supra or infra, are hereby incorporated by reference in their entirety.

As used in this specification and the appended claims, the singular forms "a," "an" and "the" include plural references unless the content clearly dictates otherwise.

### Polypeptide Compositions

As used herein, the term "polypeptide" " is used in its conventional meaning, i.e., as a sequence of amino acids. The polypeptides are not limited to a specific length of the product; thus, peptides, oligopeptides, and proteins are included within the definition of polypeptide, and such terms may be used interchangeably herein unless specifically indicated otherwise. This term also does not refer to or exclude post-expression modifications of the polypeptide, for example, glycosylations, acetylations, phosphorylations and the like, as well as other modifications known in the art, both naturally occurring and non-naturally occurring. A polypeptide may be an entire protein, or a subsequence thereof. Particular polypeptides of interest in the context of this invention are amino acid subsequences comprising epitopes, i.e., antigenic determinants substantially responsible for the immunogenic properties of a polypeptide and being capable of evoking an immune response.

Particularly illustrative polypeptides of the present invention comprise those encoded by a polynucleotide sequence set forth in any one of SEQ ID NOs: 1, 3-86, 142-298, 301-303, 307, 313, 314, 316, 317, 325 and 327-330, or a sequence that hybridizes under moderately stringent conditions, or, alternatively, under highly stringent conditions, to a polynucleotide sequence set forth in any one of SEQ ID NOs: 1, 3-86, 142-298, 301-303, 307, 313, 314, 316, 317, 325 and 327-330. Certain other illustrative polypeptides of the invention comprise amino acid sequences as set forth in any one of SEQ ID NOs: 299, 300, 304-306, 308-312, 314, 326 and 331-334.

The polypeptides of the present invention are sometimes herein referred to as breast tumor proteins or breast tumor polypeptides, as an indication that their identification has been based at least in part upon their increased levels of expression in breast tumor samples. Thus, a " breast tumor polypeptide" or "breast tumor protein," refers generally to a polypeptide sequence of the present invention, or a polynucleotide sequence encoding such a polypeptide, that is expressed in a substantial proportion of breast tumor samples, for example preferably greater than about 20%, more preferably greater than about 30%, and most preferably greater than about 50% or more of breast tumor samples tested, at a level that is at least two fold, and preferably at least five fold, greater than the level of expression in normal tissues, as determined using a representative assay provided herein. A breast tumor polypeptide sequence of the invention, based upon its increased level of expression in tumor cells, has particular utility both as a diagnostic marker as well as a therapeutic target, as further described below.

In certain preferred embodiments, the polypeptides of the invention are immunogenic, *i.e*., they react detectably within an immunoassay (such as an ELISA or T-cell stimulation assay) with antisera and/or T-cells from a patient with breast cancer. Screening for immunogenic activity can be performed using techniques well known to the skilled artisan. For example, such screens can be performed using methods such as those described in Harlow and Lane, *Antibodies: A Laboratory Manual,* Cold Spring Harbor Laboratory, 1988. In one illustrative example, a polypeptide may be immobilized on a solid support and contacted with patient sera to allow binding of antibodies within the sera to the immobilized polypeptide. Unbound sera may then be removed and bound antibodies detected using, for example, ¹²⁵I-labeled Protein A.

As would be recognized by the skilled artisan, immunogenic portions of the polypeptides disclosed herein are also encompassed by the present invention. An "immunogenic portion," as used herein, is a fragment of an immunogenic polypeptide of the invention that itself is immunologically reactive (*i. e.*, specifically binds) with the B-cells and/or T-cell surface antigen receptors that recognize the polypeptide. Immunogenic portions may generally be identified using well known techniques, such as those summarized in Paul, *Fundamental Immunology,* 3rd ed., 243-247 (Raven Press, 1993) and references cited therein. Such techniques include screening polypeptides for the ability to react with antigen-specific antibodies, antisera and/or T-cell lines or clones. As used herein, antisera and antibodies are "antigen-specific" if they specifically bind to an antigen (*i.e*., they react with the protein in an ELISA or other immunoassay, and do not react detectably with unrelated proteins). Such antisera and antibodies may be prepared as described herein, and using well-known techniques.

In one preferred embodiment, an immunogenic portion of a polypeptide of the present invention is a portion that reacts with antisera and/or T-cells at a level that is not substantially less than the reactivity of the full-length polypeptide (*e.g*., in an ELISA and/or T-cell reactivity assay). Preferably, the level of immunogenic activity of the immunogenic portion is at least about 50%, preferably at least about 70% and most preferably greater than about 90% of the immunogenicity for the full-length polypeptide. In some instances, preferred immunogenic portions will be identified that have a level of immunogenic activity greater than that of the corresponding full-length polypeptide, *e.g*., having greater than about 100% or 150% or more immunogenic activity.

In certain other embodiments, illustrative immunogenic portions may include peptides in which an N-terminal leader sequence and/or transmembrane domain have been deleted. Other illustrative immunogenic portions will contain a small N-and/or C-terminal deletion (*e.g*., 1-30 amino acids, preferably 5-15 amino acids), relative to the mature protein.

In another embodiment, a polypeptide composition of the invention may also comprise one or more polypeptides that are immunologically reactive with T cells and/or antibodies generated against a polypeptide of the invention, particularly a polypeptide having an amino acid sequence disclosed herein, or to an immunogenic fragment or variant thereof.

In another embodiment of the invention, polypeptides are provided that comprise one or more polypeptides that are capable of eliciting T cells and/or antibodies that are immunologically reactive with one or more polypeptides described herein, or one or more polypeptides encoded by contiguous nucleic acid sequences contained in the polynucleotide sequences disclosed herein, or immunogenic fragments or variants thereof, or to one or more nucleic acid sequences which hybridize to one or more of these sequences under conditions of moderate to high stringency.

The present invention, in another aspect, provides polypeptide fragments comprising at least about 5, 10, 15, 20, 25, 50, or 100 contiguous amino acids, or more, including all intermediate lengths, of a polypeptide compositions set forth herein, such as those set forth in SEQ ID NOs: 299, 300, 304-306, 308-312, 314, 326 and 331-334, or those encoded by a polynucleotide sequence set forth in a sequence of SEQ ID NOs: 1, 3-86, 142-298, 301-303, 307, 313, 314, 316, 317, 325 and 327-330.

In another aspect, the present invention provides variants of the polypeptide compositions described herein. Polypeptide variants generally encompassed by the present invention will typically exhibit at least about 70%, 75%, 80%, 85%, 90%, 91%, 92%, 93%, 94%, 95%, 96%, 97%, 98%, or 99% or more identity (determined as described below), along its length, to a polypeptide sequences set forth herein.

In one preferred embodiment, the polypeptide fragments and variants provide by the present invention are immunologically reactive with an antibody and/or T-cell that reacts with a full-length polypeptide specifically set for the herein.

In another preferred embodiment, the polypeptide fragments and variants provided by the present invention exhibit a level of immunogenic activity of at least about 50%, preferably at least about 70%, and most preferably at least about 90% or more of that exhibited by a full-length polypeptide sequence specifically set forth herein.

A polypeptide "variant," as the term is used herein, is a polypeptide that typically differs from a polypeptide specifically disclosed herein in one or more substitutions, deletions, additions and/or insertions. Such variants may be naturally occurring or may be synthetically generated, for example, by modifying one or more of the above polypeptide sequences of the invention and evaluating their immunogenic activity as described herein and/or using any of a number of techniques well known in the art.

For example, certain illustrative variants of the polypeptides of the invention include those in which one or more portions, such as an N-terminal leader sequence or transmembrane domain, have been removed. Other illustrative variants include variants in which a small portion (*e.g*., 1-30 amino acids, preferably 5-15 amino acids) has been removed from the N- and/or C-terminal of the mature protein.

In many instances, a variant will contain conservative substitutions. A "conservative substitution" is one in which an amino acid is substituted for another amino acid that has similar properties, such that one skilled in the art of peptide chemistry would expect the secondary structure and hydropathic nature of the polypeptide to be substantially unchanged. As described above, modifications may be made in the structure of the polynucleotides and polypeptides of the present invention and still obtain a functional molecule that encodes a variant or derivative polypeptide with desirable characteristics, e.g., with immunogenic characteristics. When it is desired to alter the amino acid sequence of a polypeptide to create an equivalent, or even an improved, immunogenic variant or portion of a polypeptide of the invention, one skilled in the art will typically change one or more of the codons of the encoding DNA sequence according to Table 1.

For example, certain amino acids may be substituted for other amino acids in a protein structure without appreciable loss of interactive binding capacity with structures such as, for example, antigen-binding regions of antibodies or binding sites on substrate molecules. Since it is the interactive capacity and nature of a protein that defines that protein's biological functional activity, certain amino acid sequence substitutions can be made in a protein sequence, and, of course, its underlying DNA coding sequence, and nevertheless obtain a protein with like properties. It is thus contemplated that various changes may be made in the peptide sequences of the disclosed compositions, or corresponding DNA sequences which encode said peptides without appreciable loss of their biological utility or activity.

**TABLE 1**

| **Amino Acids** | | | **Codons** | | | | | |
|---|---|---|---|---|---|---|---|---|
| Alanine | Ala | A | GCA | GCC | GCG | GCU | | |
| Cysteine | Cys | C | UGC | UGU | | | | |
| Aspartic acid | Asp | D | GAC | GAU | | | | |
| Glutamic acid | Glu | E | GAA | GAG | | | | |
| Phenylalanine | Phe | F | UUC | UUU | | | | |
| Glycine | Gly | G | GGA | GGC | GGG | GGU | | |
| Histidine | His | H | CAC | CAU | | | | |
| Isoleucine | Ile | I | AUA | AUC | AUU | | | |
| Lysine | Lys | K | AAA | AAG | | | | |
| Leucine | Leu | L | UUA | UUG | CUA | CUC | CUG | CUU |
| Methionine | Met | M | AUG | | | | | |
| Asparagine | Asn | N | AAC | AAU | | | | |
| Proline | Pro | P | CCA | CCC | CCG | CCU | | |
| Glutamine | Gln | Q | CAA | CAG | | | | |
| Arginine | Arg | R | AGA | AGG | CGA | CGC | CGG | CGU |
| Serine | Ser | S | AGC | AGU | UCA | UCC | UCG | UCU |
| Threonine | Thr | T | ACA | ACC | ACG | ACU | | |
| Valine | Val | V | GUA | GUC | GUG | GUU | | |
| Tryptophan | Trp | W | UGG | | | | | |
| Tyrosine | Tyr | Y | UAC | UAU | | | | |

In making such changes, the hydropathic index of amino acids may be considered. The importance of the hydropathic amino acid index in conferring interactive biologic function on a protein is generally understood in the art (Kyte and Doolittle, 1982, incorporated herein by reference). It is accepted that the relative hydropathic character of the amino acid contributes to the secondary structure of the resultant protein, which in turn defines the interaction of the protein with other molecules, for example, enzymes, substrates, receptors, DNA, antibodies, antigens, and the like. Each amino acid has been assigned a hydropathic index on the basis of its hydrophobicity and charge characteristics (Kyte and Doolittle, 1982). These values are: isoleucine (+4.5); valine (+4.2); leucine (+3.8); phenylalanine (+2.8); cysteine/cystine (+2.5); methionine (+1.9); alanine (+1.8); glycine (-0.4); threonine (-0.7); serine (-0.8); tryptophan (-0.9); tyrosine (-1.3); proline (-1.6); histidine (-3.2); glutamate (-3.5); glutamine (-3.5); aspartate (-3.5); asparagine (-3.5); lysine (-3.9); and arginine (-4.5).

It is known in the art that certain amino acids may be substituted by other amino acids having a similar hydropathic index or score and still result in a protein with similar biological activity, *i.e.* still obtain a biological functionally equivalent protein. In making such changes, the substitution of amino acids whose hydropathic indices are within ±2 is preferred, those within ±1 are particularly preferred, and those within ±0.5 are even more particularly preferred. It is also understood in the art that the substitution of like amino acids can be made effectively on the basis of hydrophilicity. U. S. Patent 4,554,101 (specifically incorporated herein by reference in its entirety), states that the greatest local average hydrophilicity of a protein, as governed by the hydrophilicity of its adjacent amino acids, correlates with a biological property of the protein.

As detailed in U. S. Patent 4,554,101, the following hydrophilicity values have been assigned to amino acid residues: arginine (+3.0); lysine (+3.0); aspartate (+3.0 ± 1); glutamate (+3.0 ± 1); serine (+0.3); asparagine (+0.2); glutamine (+0.2); glycine (0); threonine (-0.4); proline (-0.5 ± 1); alanine (-0.5); histidine (-0.5); cysteine (-1.0); methionine (-1.3); valine (-1.5); leucine (-1.8); isoleucine (-1.8); tyrosine (-2.3); phenylalanine (-2.5); tryptophan (-3.4). It is understood that an amino acid can be substituted for another having a similar hydrophilicity value and still obtain a biologically equivalent, and in particular, an immunologically equivalent protein. In such changes, the substitution of amino acids whose hydrophilicity values are within ±2 is preferred, those within ±1 are particularly preferred, and those within ±0.5 are even more particularly preferred.

As outlined above, amino acid substitutions are generally therefore based on the relative similarity of the amino acid side-chain substituents, for example, their hydrophobicity, hydrophilicity, charge, size, and the like. Exemplary substitutions that take various of the foregoing characteristics into consideration are well known to those of skill in the art and include: arginine and lysine; glutamate and aspartate; serine and threonine; glutamine and asparagine; and valine, leucine and isoleucine.

In addition, any polynucleotide may be further modified to increase stability *in vivo.* Possible modifications include, but are not limited to, the addition of flanking sequences at the 5' and/or 3' ends; the use of phosphorothioate or 2' O-methyl rather than phosphodiesterase linkages in the backbone; and/or the inclusion of nontraditional bases such as inosine, queosine and wybutosine, as well as acetyl-methyl-, thio- and other modified forms of adenine, cytidine, guanine, thymine and uridine.

Amino acid substitutions may further be made on the basis of similarity in polarity, charge, solubility, hydrophobicity, hydrophilicity and/or the amphipathic nature of the residues. For example, negatively charged amino acids include aspartic acid and glutamic acid; positively charged amino acids include lysine and arginine; and amino acids with uncharged polar head groups having similar hydrophilicity values include leucine, isoleucine and valine; glycine and alanine; asparagine and glutamine; and serine, threonine, phenylalanine and tyrosine. Other groups of amino acids that may represent conservative changes include: (1) ala, pro, gly, glu, asp, gln, asn, ser, thr; (2) cys, ser, tyr, thr; (3) val, ile, leu, met, ala, phe; (4) lys, arg, his; and (5) phe, tyr, trp, his. A variant may also, or alternatively, contain nonconservative changes. In a preferred embodiment, variant polypeptides differ from a native sequence by substitution, deletion or addition of five amino acids or fewer. Variants may also (or alternatively) be modified by, for example, the deletion or addition of amino acids that have minimal influence on the immunogenicity, secondary structure and hydropathic nature of the polypeptide.

As noted above, polypeptides may comprise a signal (or leader) sequence at the N-terminal end of the protein, which co-translationally or post-translationally directs transfer of the protein. The polypeptide may also be conjugated to a linker or other sequence for ease of synthesis, purification or identification of the polypeptide *(e.g.,* poly-His), or to enhance binding of the polypeptide to a solid support. For example, a polypeptide may be conjugated to an immunoglobulin Fc region.

When comparing polypeptide sequences, two sequences are said to be "identical" if the sequence of amino acids in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins - Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) *CABIOS 5*:151-153; Myers, E.W. and Muller W. (1988) *CABIOS 4*:11-17; Robinson, E.D. (1971) *Comb. Theor 11:*105*;* Santou, N. Nes, M. (1987) *Mol. Biol. Evol.* 4:406-425; Sneath, P.H.A. and Sokal, R.R. (1973) *Numerical Taxonomy - the Principles and Practice of Numerical Taxonomy,* Freeman Press, San Francisco, CA; Wilbur, W.J. and Lipman, D.J. (1983) *Proc. Natl. Acad., Sci. USA 80*:726-730.

Alternatively, optimal alignment of sequences for comparison may be conducted by the local identity algorithm of Smith and Waterman (1981) *Add. APL. Math* 2:482, by the identity alignment algorithm of Needleman and Wunsch (1970) *J. Mol. Biol.* 48:443, by the search for similarity methods of Pearson and Lipman (1988) *Proc. Natl. Acad. Sci. USA* 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) *Nucl. Acids Res.* 25:3389-3402 and Altschul et al. (1990) *J. Mol. Biol.* 215:403-410, respectively. BLAST and BLAST 2.0 can be used, for example with the parameters described herein, to determine percent sequence identity for the polynucleotides and polypeptides of the invention. Software for performing BLAST analyses is publicly available through the National Center for Biotechnology Information. For amino acid sequences, a scoring matrix can be used to calculate the cumulative score. Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment.

In one preferred approach, the "percentage of sequence identity" is determined by comparing two optimally aligned sequences over a window of comparison of at least 20 positions, wherein the portion of the polypeptide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical amino acid residue occurs in both sequences to yield the number of matched positions, dividing the number of matched positions by the total number of positions in the reference sequence (*i.e*., the window size) and multiplying the results by 100 to yield the percentage of sequence identity.

Within other illustrative embodiments, a polypeptide may be a fusion polypeptide that comprises multiple polypeptides as described herein, or that comprises at least one polypeptide as described herein and an unrelated sequence, such as a known tumor protein. A fusion partner may, for example, assist in providing T helper epitopes (an immunological fusion partner), preferably T helper epitopes recognized by humans, or may assist in expressing the protein (an expression enhancer) at higher yields than the native recombinant protein. Certain preferred fusion partners are both immunological and expression enhancing fusion partners. Other fusion partners may be selected so as to increase the solubility of the polypeptide or to enable the polypeptide to be targeted to desired intracellular compartments. Still further fusion partners include affinity tags, which facilitate purification of the polypeptide.

Fusion polypeptides may generally be prepared using standard techniques, including chemical conjugation. Preferably, a fusion polypeptide is expressed as a recombinant polypeptide, allowing the production of increased levels, relative to a non-fused polypeptide, in an expression system. Briefly, DNA sequences encoding the polypeptide components may be assembled separately, and ligated into an appropriate expression vector. The 3' end of the DNA sequence encoding one polypeptide component is ligated, with or without a peptide linker, to the 5' end of a DNA sequence encoding the second polypeptide component so that the reading frames of the sequences are in phase. This permits translation into a single fusion polypeptide that retains the biological activity of both component polypeptides.

A peptide linker sequence may be employed to separate the first and second polypeptide components by a distance sufficient to ensure that each polypeptide folds into its secondary and tertiary structures. Such a peptide linker sequence is incorporated into the fusion polypeptide using standard techniques well known in the art. Suitable peptide linker sequences may be chosen based on the following factors: (1) their ability to adopt a flexible extended conformation; (2) their inability to adopt a secondary structure that could interact with functional epitopes on the first and second polypeptides; and (3) the lack of hydrophobic or charged residues that might react with the polypeptide functional epitopes. Preferred peptide linker sequences contain Gly, Asn and Ser residues. Other near neutral amino acids, such as Thr and Ala may also be used in the linker sequence. Amino acid sequences which may be usefully employed as linkers include those disclosed in Maratea et al., *Gene 40*:39-46, 1985; Murphy et al., *Proc. Natl. Acad. Sci. USA 83*:8258-8262, 1986; U.S. Patent No. 4,935,233 and U.S. Patent No. 4,751,180. The linker sequence may generally be from 1 to about 50 amino acids in length. Linker sequences are not required when the first and second polypeptides have non-essential N-terminal amino acid regions that can be used to separate the functional domains and prevent steric interference.

The ligated DNA sequences are operably linked to suitable transcriptional or translational regulatory elements. The regulatory elements responsible for expression of DNA are located only 5' to the DNA sequence encoding the first polypeptides. Similarly, stop codons required to end translation and transcription termination signals are only present 3' to the DNA sequence encoding the second polypeptide.

The fusion polypeptide can comprise a polypeptide as described herein together with an unrelated immunogenic protein, such as an immunogenic protein capable of eliciting a recall response. Examples of such proteins include tetanus, tuberculosis and hepatitis proteins *(see,* for example, Stoute et al. *New Engl. J Med., 336*:86-91, 1997).

In one preferred embodiment, the immunological fusion partner is derived from a Mycobacterium sp., such as a Mycobacterium tuberculosis-derived Ra12 fragment. Ra12 compositions and methods for their use in enhancing the expression and/or immunogenicity of heterologous polynucleotide/polypeptide sequences is described in U.S. Patent Application 60/158,585, the disclosure of which is incorporated herein by reference in its entirety. Briefly, Ra12 refers to a polynucleotide region that is a subsequence of a *Mycobacterium tuberculosis* MTB32A nucleic acid. MTB32A is a serine protease of 32 KD molecular weight encoded by a gene in virulent and avirulent strains of *M. tuberculosis.* The nucleotide sequence and amino acid sequence of MTB32A have been described (for example, U.S. Patent Application 60/158,585; see also, *Skeiky et al., Infection and Immun.* (1999) 67:3998-4007, incorporated herein by reference). C-terminal fragments of the MTB32A coding sequence express at high levels and remain as a soluble polypeptides throughout the purification process. Moreover, Ra12 may enhance the immunogenicity of heterologous immunogenic polypeptides with which it is fused. One preferred Ra12 fusion polypeptide comprises a 14 KD C-terminal fragment corresponding to amino acid residues 192 to 323 of MTB32A. Other preferred Ra12 polynucleotides generally comprise at least about 15 consecutive nucleotides, at least about 30 nucleotides, at least about 60 nucleotides, at least about 100 nucleotides, at least about 200 nucleotides, or at least about 300 nucleotides that encode a portion of a Ra12 polypeptide. Ra12 polynucleotides may comprise a native sequence *(i.e.,* an endogenous sequence that encodes a Ra12 polypeptide or a portion thereof) or may comprise a variant of such a sequence. Ra12 polynucleotide variants may contain one or more substitutions, additions, deletions and/or insertions such that the biological activity of the encoded fusion polypeptide is not substantially diminished, relative to a fusion polypeptide comprising a native Ra12 polypeptide. Variants preferably exhibit at least about 70% identity, more preferably at least about 80% identity and most preferably at least about 90% identity to a polynucleotide sequence that encodes a native Ra12 polypeptide or a portion thereof.

Within other preferred embodiments, an immunological fusion partner is derived from protein D, a surface protein of the gram-negative bacterium Haemophilus influenza B (WO 91/18926). Preferably, a protein D derivative comprises approximately the first third of the protein (*e.g*., the first N-terminal 100-110 amino acids), and a protein D derivative may be lipidated. Within certain preferred embodiments, the first 109 residues of a Lipoprotein D fusion partner is included on the N-terminus to provide the polypeptide with additional exogenous T-cell epitopes and to increase the expression level in *E*. *coli* (thus functioning as an expression enhancer). The lipid tail ensures optimal presentation of the antigen to antigen presenting cells. Other fusion partners include the non-structural protein from influenzae virus, NS1 (hemaglutinin). Typically, the N-terminal 81 amino acids are used, although different fragments that include T-helper epitopes may be used.

In another embodiment, the immunological fusion partner is the protein known as LYTA, or a portion thereof (preferably a C-terminal portion). LYTA is derived from *Streptococcus pneumoniae,* which synthesizes an N-acetyl-L-alanine amidase known as amidase LYTA (encoded by the LytA gene; *Gene 43*:265-292, 1986). LYTA is an autolysin that specifically degrades certain bonds in the peptidoglycan backbone. The C-terminal domain of the LYTA protein is responsible for the affinity to the choline or to some choline analogues such as DEAE. This property has been exploited for the development of *E. coli* C-LYTA expressing plasmids useful for expression of fusion proteins. Purification of hybrid proteins containing the C-LYTA fragment at the amino terminus has been described (*see Biotechnology 10*:795-798, 1992). Within a preferred embodiment, a repeat portion of LYTA may be incorporated into a fusion polypeptide. A repeat portion is found in the C-terminal region starting at residue 178. A particularly preferred repeat portion incorporates residues 188-305.

Yet another illustrative embodiment involves fusion polypeptides, and the polynucleotides encoding them, wherein the fusion partner comprises a targeting signal capable of directing a polypeptide to the endosomal/lysosomal compartment, as described in U.S. Patent No. 5,633,234. An immunogenic polypeptide of the invention, when fused with this targeting signal, will associate more efficiently with MHC class II molecules and thereby provide enhanced in vivo stimulation of CD4⁺ T-cells specific for the polypeptide.

Polypeptides of the invention are prepared using any of a variety of well known synthetic and/or recombinant techniques, the latter of which are further described below. Polypeptides, portions and other variants generally less than about 150 amino acids can be generated by synthetic means, using techniques well known to those of ordinary skill in the art. In one illustrative example, such polypeptides are synthesized using any of the commercially available solid-phase techniques, such as the Merrifield solid-phase synthesis method, where amino acids are sequentially added to a growing amino acid chain. *See* Merrifield, *J. Am. Chem. Soc. 85*:2149-2146, 1963. Equipment for automated synthesis of polypeptides is commercially available from suppliers such as Perkin Elmer/Applied BioSystems Division (Foster City, CA), and may be operated according to the manufacturer's instructions.

In general, polypeptide compositions (including fusion polypeptides) of the invention are isolated. An "isolated" polypeptide is one that is removed from its original environment. For example, a naturally-occurring protein or polypeptide is isolated if it is separated from some or all of the coexisting materials in the natural system. Preferably, such polypeptides are also purified, e.g., are at least about 90% pure, more preferably at least about 95% pure and most preferably at least about 99% pure.

### Polynucleotide Compositions

The present invention, in other aspects, provides polynucleotide compositions. The terms "DNA" and "polynucleotide" are used essentially interchangeably herein to refer to a DNA molecule that has been isolated free of total genomic DNA of a particular species. "Isolated," as used herein, means that a polynucleotide is substantially away from other coding sequences, and that the DNA molecule does not contain large portions of unrelated coding DNA, such as large chromosomal fragments or other functional genes or polypeptide coding regions. Of course, this refers to the DNA molecule as originally isolated, and does not exclude genes or coding regions later added to the segment by the hand of man.

As will be understood by those skilled in the art, the polynucleotide compositions of this invention can include genomic sequences, extra-genomic and plasmid-encoded sequences and smaller engineered gene segments that express, or may be adapted to express, proteins, polypeptides, peptides and the like. Such segments may be naturally isolated, or modified synthetically by the hand of man.

As will be also recognized by the skilled artisan, polynucleotides of the invention may be single-stranded (coding or antisense) or double-stranded, and may be DNA (genomic, cDNA or synthetic) or RNA molecules. RNA molecules may include HnRNA molecules, which contain introns and correspond to a DNA molecule in a one-to-one manner, and mRNA molecules, which do not contain introns. Additional coding or non-coding sequences may, but need not, be present within a polynucleotide of the present invention, and a polynucleotide may, but need not, be linked to other molecules and/or support materials.

Polynucleotides may comprise a native sequence (*i.e*., an endogenous sequence that encodes a polypeptide/protein of the invention or a portion thereof) or may comprise a sequence that encodes a variant or derivative, preferably and immunogenic variant or derivative, of such a sequence.

Therefore, according to another aspect of the present invention, polynucleotide compositions are provided that comprise some or all of a polynucleotide sequence set forth in any one of SEQ ID NOs: 1, 3-86, 142-298, 301-303, 307, 313, 314, 316, 317, 325 and 327-330, complements of a polynucleotide sequence set forth in any one of SEQ ID NOs: 1, 3-86, 142-298, 301-303, 307, 313, 314, 316, 317, 325 and 327-330, and degenerate variants of a polynucleotide sequence set forth in any one of SEQ ID NOs: 1, 3-86, 142-298, 301-303, 307, 313, 314, 316, 317, 325 and 327-330. In certain preferred embodiments, the polynucleotide sequences set forth herein encode immunogenic polypeptides, as described above.

In other related embodiments, the present invention provides polynucleotide variants having substantial identity to the sequences disclosed herein in SEQ ID NOs: 1, 3-86, 142-298, 301-303, 307, 313, 314, 316, 317, 325 and 327-330, for example those comprising at least 70% sequence identity, preferably at least 75%, 80%, 85%, 90%, 95%, 96%, 97%, 98%, or 99% or higher, sequence identity compared to a polynucleotide sequence of this invention using the methods described herein, (*e.g*., BLAST analysis using standard parameters, as described below). One skilled in this art will recognize that these values can be appropriately adjusted to determine corresponding identity of proteins encoded by two nucleotide sequences by taking into account codon degeneracy, amino acid similarity, reading frame positioning and the like.

Typically, polynucleotide variants will contain one or more substitutions, additions, deletions and/or insertions, preferably such that the immunogenicity of the polypeptide encoded by the variant polynucleotide is not substantially diminished relative to a polypeptide encoded by a polynucleotide sequence specifically set forth herein). The term "variants" should also be understood to encompasses homologous genes of xenogenic origin.

In additional embodiments, the present invention provides polynucleotide fragments comprising various lengths of contiguous stretches of sequence identical to or complementary to one or more of the sequences disclosed herein. For example, polynucleotides are provided by this invention that comprise at least about 10, 15, 20, 30, 40, 50, 75, 100, 150, 200, 300, 400, 500 or 1000 or more contiguous nucleotides of one or more of the sequences disclosed herein as well as all intermediate lengths there between. It will be readily understood that "intermediate lengths", in this context, means any length between the quoted values, such as 16, 17, 18, 19, *etc.;* 21, 22, 23, *etc.;* 30, 31, 32, *etc.;* 50, 51, 52, 53, *etc.;* 100, 101, 102, 103, *etc.;* 150, 151, 152, 153, *etc.;* including all integers through 200-500; 500-1,000, and the like.

In another embodiment of the invention, polynucleotide compositions are provided that are capable of hybridizing under moderate to high stringency conditions to a polynucleotide sequence provided herein, or a fragment thereof, or a complementary sequence thereof. Hybridization techniques are well known in the art of molecular biology. For purposes of illustration, suitable moderately stringent conditions for testing the hybridization of a polynucleotide of this invention with other polynucleotides include prewashing in a solution of 5 X SSC, 0.5% SDS, 1.0 mM EDTA (pH 8.0); hybridizing at 50°C-60°C, 5X SSC, overnight; followed by washing twice at 65°C for 20 minutes with each of 2X, 0.5X and 0.2X SSC containing 0.1% SDS. One skilled in the art will understand that the stringency of hybridization can be readily manipulated, such as by altering the salt content of the hybridization solution and/or the temperature at which the hybridization is performed. For example, in another embodiment, suitable highly stringent hybridization conditions include those described above, with the exception that the temperature of hybridization is increased, *e.g.,* to 60-65°C or 65-70°C.

In certain preferred embodiments, the polynucleotides described above, e.g., polynucleotide variants, fragments and hybridizing sequences, encode polypeptides that are immunologically cross-reactive with a polypeptide sequence specifically set forth herein. In other preferred embodiments, such polynucleotides encode polypeptides that have a level of immunogenic activity of at least about 50%, preferably at least about 70%, and more preferably at least about 90% of that for a polypeptide sequence specifically set forth herein.

The polynucleotides of the present invention, or fragments thereof, regardless of the length of the coding sequence itself, may be combined with other DNA sequences, such as promoters, polyadenylation signals, additional restriction enzyme sites, multiple cloning sites, other coding segments, and the like, such that their overall length may vary considerably. It is therefore contemplated that a nucleic acid fragment of almost any length may be employed, with the total length preferably being limited by the ease of preparation and use in the intended recombinant DNA protocol. For example, illustrative polynucleotide segments with total lengths of about 10,000, about 5000, about 3000, about 2,000, about 1,000, about 500, about 200, about 100, about 50 base pairs in length, and the like, (including all intermediate lengths) are contemplated to be useful in many implementations of this invention.

When comparing polynucleotide sequences, two sequences are said to be "identical" if the sequence of nucleotides in the two sequences is the same when aligned for maximum correspondence, as described below. Comparisons between two sequences are typically performed by comparing the sequences over a comparison window to identify and compare local regions of sequence similarity. A "comparison window" as used herein, refers to a segment of at least about 20 contiguous positions, usually 30 to about 75, 40 to about 50, in which a sequence may be compared to a reference sequence of the same number of contiguous positions after the two sequences are optimally aligned.

Optimal alignment of sequences for comparison may be conducted using the Megalign program in the Lasergene suite of bioinformatics software (DNASTAR, Inc., Madison, WI), using default parameters. This program embodies several alignment schemes described in the following references: Dayhoff, M.O. (1978) A model of evolutionary change in proteins- Matrices for detecting distant relationships. In Dayhoff, M.O. (ed.) Atlas of Protein Sequence and Structure, National Biomedical Research Foundation, Washington DC Vol. 5, Suppl. 3, pp. 345-358; Hein J. (1990) Unified Approach to Alignment and Phylogenes pp. 626-645 *Methods in Enzymology* vol. 183, Academic Press, Inc., San Diego, CA; Higgins, D.G. and Sharp, P.M. (1989) *CABIOS* 5:151-153; Myers, E.W. and Muller W. (1988) *CABIOS* 4:11-17; Robinson, E.D. (1971) *Comb. Theor* 11:105; Santou, N. Nes, M. (1987) *Mol. Biol. Evol.* 4:406-425; Sneath, P.H.A. and Sokal, R.R. (1973) *Numerical Taxonomy - the Principles and Practice of Numerical Taxonomy,* Freeman Press, San Francisco, CA; Wilbur, W.J. and Lipman, D.J. (1983) *Proc. Natl. Acad., Sci. USA 80*:726-730.

Alternatively, optimal alignment of sequences for comparison may be conducted by the local identity algorithm of Smith and Waterman (1981) *Add. APL. Math* 2:482, by the identity alignment algorithm of Needleman and Wunsch (1970) *J*. *Mol. Biol.* 48:443, by the search for similarity methods of Pearson and Lipman (1988) *Proc. Natl. Acad. Sci. USA* 85: 2444, by computerized implementations of these algorithms (GAP, BESTFIT, BLAST, FASTA, and TFASTA in the Wisconsin Genetics Software Package, Genetics Computer Group (GCG), 575 Science Dr., Madison, WI), or by inspection.

One preferred example of algorithms that are suitable for determining percent sequence identity and sequence similarity are the BLAST and BLAST 2.0 algorithms, which are described in Altschul et al. (1977) *Nucl. Acids Res.* 25:3389-3402 and Altschul et al. (1990) *J Mol. Biol.* 215:403-410, respectively. BLAST and BLAST 2.0 can be used, for example with the parameters described herein, to determine percent sequence identity for the polynucleotides of the invention. Software for performing BLAST analyses is publicly available through the National Center for Biotechnology Information. In one illustrative example, cumulative scores can be calculated using, for nucleotide sequences, the parameters M (reward score for a pair of matching residues; always >0) and N (penalty score for mismatching residues; always <0). Extension of the word hits in each direction are halted when: the cumulative alignment score falls off by the quantity X from its maximum achieved value; the cumulative score goes to zero or below, due to the accumulation of one or more negative-scoring residue alignments; or the end of either sequence is reached. The BLAST algorithm parameters W, T and X determine the sensitivity and speed of the alignment. The BLASTN program (for nucleotide sequences) uses as defaults a wordlength (W) of 11, and expectation (E) of 10, and the BLOSUM62 scoring matrix (see Henikoff and Henikoff (1989) *Proc. Natl. Acad. Sci. USA* 89:10915) alignments, (B) of 50, expectation (E) of 10, M=5, N=-4 and a comparison of both strands.

Preferably, the "percentage of sequence identity" is determined by comparing two optimally aligned sequences over a window of comparison of at least 20 positions, wherein the portion of the polynucleotide sequence in the comparison window may comprise additions or deletions (*i.e*., gaps) of 20 percent or less, usually 5 to 15 percent, or 10 to 12 percent, as compared to the reference sequences (which does not comprise additions or deletions) for optimal alignment of the two sequences. The percentage is calculated by determining the number of positions at which the identical nucleic acid bases occurs in both sequences to yield the number of matched positions, dividing the number of matched positions by the total number of positions in the reference sequence (*i.e*., the window size) and multiplying the results by 100 to yield the percentage of sequence identity.

It will be appreciated by those of ordinary skill in the art that, as a result of the degeneracy of the genetic code, there are many nucleotide sequences that encode a polypeptide as described herein. Some of these polynucleotides bear minimal homology to the nucleotide sequence of any native gene. Nonetheless, polynucleotides that vary due to differences in codon usage are specifically contemplated by the present invention. Further, alleles of the genes comprising the polynucleotide sequences provided herein are within the scope of the present invention. Alleles are endogenous genes that are altered as a result of one or more mutations, such as deletions, additions and/or substitutions of nucleotides. The resulting mRNA and protein may, but need not, have an altered structure or function. Alleles may be identified using standard techniques (such as hybridization, amplification and/or database sequence comparison).

Therefore, in another embodiment of the invention, a mutagenesis approach, such as site-specific mutagenesis, is employed for the preparation of immunogenic variants and/or derivatives of the polypeptides described herein. By this approach, specific modifications in a polypeptide sequence can be made through mutagenesis of the underlying polynucleotides that encode them. These techniques provides a straightforward approach to prepare and test sequence variants, for example, incorporating one or more of the foregoing considerations, by introducing one or more nucleotide sequence changes into the polynucleotide.

Site-specific mutagenesis allows the production of mutants through the use of specific oligonucleotide sequences which encode the DNA sequence of the desired mutation, as well as a sufficient number of adjacent nucleotides, to provide a primer sequence of sufficient size and sequence complexity to form a stable duplex on both sides of the deletion junction being traversed. Mutations may be employed in a selected polynucleotide sequence to improve, alter, decrease, modify, or otherwise change the properties of the polynucleotide itself, and/or alter the properties, activity, composition, stability, or primary sequence of the encoded polypeptide.

In certain embodiments of the present invention, the inventors contemplate the mutagenesis of the disclosed polynucleotide sequences to alter one or more properties of the encoded polypeptide, such as the immunogenicity of a polypeptide vaccine. The techniques of site-specific mutagenesis are well-known in the art, and are widely used to create variants of both polypeptides and polynucleotides. For example, site-specific mutagenesis is often used to alter a specific portion of a DNA molecule. In such embodiments, a primer comprising typically about 14 to about 25 nucleotides or so in length is employed, with about 5 to about 10 residues on both sides of the junction of the sequence being altered.

As will be appreciated by those of skill in the art, site-specific mutagenesis techniques have often employed a phage vector that exists in both a single stranded and double stranded form. Typical vectors useful in site-directed mutagenesis include vectors such as the M 13 phage. These phage are readily commercially-available and their use is generally well-known to those skilled in the art. Double-stranded plasmids are also routinely employed in site directed mutagenesis that eliminates the step of transferring the gene of interest from a plasmid to a phage.

In general, site-directed mutagenesis in accordance herewith is performed by first obtaining a single-stranded vector or melting apart of two strands of a double-stranded vector that includes within its sequence a DNA sequence that encodes the desired peptide. An oligonucleotide primer bearing the desired mutated sequence is prepared, generally synthetically. This primer is then annealed with the single-stranded vector, and subjected to DNA polymerizing enzymes such as E. *coli* polymerase I Klenow fragment, in order to complete the synthesis of the mutation-bearing strand. Thus, a heteroduplex is formed wherein one strand encodes the original non-mutated sequence and the second strand bears the desired mutation. This heteroduplex vector is then used to transform appropriate cells, such as *E. coli* cells, and clones are selected which include recombinant vectors bearing the mutated sequence arrangement.

The preparation of sequence variants of the selected peptide-encoding DNA segments using site-directed mutagenesis provides a means of producing potentially useful species and is not meant to be limiting as there are other ways in which sequence variants of peptides and the DNA sequences encoding them may be obtained. For example, recombinant vectors encoding the desired peptide sequence may be treated with mutagenic agents, such as hydroxylamine, to obtain sequence variants. Specific details regarding these methods and protocols are found in the teachings of Maloy *et al.,* 1994; Segal, 1976; Prokop and Bajpai, 1991; Kuby, 1994; and Maniatis *et al.,* 1982, each incorporated herein by reference, for that purpose.

As used herein, the term "oligonucleotide directed mutagenesis procedure" refers to template-dependent processes and vector-mediated propagation which result in an increase in the concentration of a specific nucleic acid molecule relative to its initial concentration, or in an increase in the concentration of a detectable signal, such as amplification. As used herein, the term "oligonucleotide directed mutagenesis procedure" is intended to refer to a process that involves the template-dependent extension of a primer molecule. The term template dependent process refers to nucleic acid synthesis of an RNA or a DNA molecule wherein the sequence of the newly synthesized strand of nucleic acid is dictated by the well-known rules of complementary base pairing (see, for example, Watson, 1987). Typically, vector mediated methodologies involve the introduction of the nucleic acid fragment into a DNA or RNA vector, the clonal amplification of the vector, and the recovery of the amplified nucleic acid fragment. Examples of such methodologies are provided by U. S. Patent No. 4,237,224, specifically incorporated herein by reference in its entirety.

In another approach for the production of polypeptide variants of the present invention, recursive sequence recombination, as described in U.S. Patent No. 5,837,458, may be employed. In this approach, iterative cycles of recombination and screening or selection are performed to "evolve" individual polynucleotide variants of the invention having, for example, enhanced immunogenic activity.

In other embodiments of the present invention, the polynucleotide sequences provided herein can be advantageously used as probes or primers for nucleic acid hybridization. As such, it is contemplated that nucleic acid segments that comprise a sequence region of at least about 15 nucleotide long contiguous sequence that has the same sequence as, or is complementary to, a 15 nucleotide long contiguous sequence disclosed herein will find particular utility. Longer contiguous identical or complementary sequences, *e.g*., those of about 20, 30, 40, 50, 100, 200, 500, 1000 (including all intermediate lengths) and even up to full length sequences will also be of use in certain embodiments.

The ability of such nucleic acid probes to specifically hybridize to a sequence of interest will enable them to be of use in detecting the presence of complementary sequences in a given sample. However, other uses are also envisioned, such as the use of the sequence information for the preparation of mutant species primers, or primers for use in preparing other genetic constructions.

Polynucleotide molecules having sequence regions consisting of contiguous nucleotide stretches of 10-14, 15-20, 30, 50, or even of 100-200 nucleotides or so (including intermediate lengths as well), identical or complementary to a polynucleotide sequence disclosed herein, are particularly contemplated as hybridization probes for use in, *e.g*., Southern and Northern blotting. This would allow a gene product, or fragment thereof, to be analyzed, both in diverse cell types and also in various bacterial cells. The total size of fragment, as well as the size of the complementary stretch(es), will ultimately depend on the intended use or application of the particular nucleic acid segment. Smaller fragments will generally find use in hybridization embodiments, wherein the length of the contiguous complementary region may be varied, such as between about 15 and about 100 nucleotides, but larger contiguous complementarity stretches may be used, according to the length complementary sequences one wishes to detect.

The use of a hybridization probe of about 15-25 nucleotides in length allows the formation of a duplex molecule that is both stable and selective. Molecules having contiguous complementary sequences over stretches greater than 15 bases in length are generally preferred, though, in order to increase stability and selectivity of the hybrid, and thereby improve the quality and degree of specific hybrid molecules obtained. One will generally prefer to design nucleic acid molecules having gene-complementary stretches of 15 to 25 contiguous nucleotides, or even longer where desired.

Hybridization probes may be selected from any portion of any of the sequences disclosed herein. All that is required is to review the sequences set forth herein, or to any continuous portion of the sequences, from about 15-25 nucleotides in length up to and including the full length sequence, that one wishes to utilize as a probe or primer. The choice of probe and primer sequences may be governed by various factors. For example, one may wish to employ primers from towards the termini of the total sequence.

Small polynucleotide segments or fragments may be readily prepared by, for example, directly synthesizing the fragment by chemical means, as is commonly practiced using an automated oligonucleotide synthesizer. Also, fragments may be obtained by application of nucleic acid reproduction technology, such as the PCR™ technology of U. S. Patent 4,683,202 (incorporated herein by reference), by introducing selected sequences into recombinant vectors for recombinant production, and by other recombinant DNA techniques generally known to those of skill in the art of molecular biology.

The nucleotide sequences of the invention may be used for their ability to selectively form duplex molecules with complementary stretches of the entire gene or gene fragments of interest. Depending on the application envisioned, one will typically desire to employ varying conditions of hybridization to achieve varying degrees of selectivity of probe towards target sequence. For applications requiring high selectivity, one will typically desire to employ relatively stringent conditions to form the hybrids, *e.g*., one will select relatively low salt and/or high temperature conditions, such as provided by a salt concentration of from about 0.02 M to about 0.15 M salt at temperatures of from about 50°C to about 70°C. Such selective conditions tolerate little, if any, mismatch between the probe and the template or target strand, and would be particularly suitable for isolating related sequences.

Of course, for some applications, for example, where one desires to prepare mutants employing a mutant primer strand hybridized to an underlying template, less stringent (reduced stringency) hybridization conditions will typically be needed in order to allow formation of the heteroduplex. In these circumstances, one may desire to employ salt conditions such as those of from about 0.15 M to about 0.9 M salt, at temperatures ranging from about 20°C to about 55°C. Cross-hybridizing species can thereby be readily identified as positively hybridizing signals with respect to control hybridizations. In any case, it is generally appreciated that conditions can be rendered more stringent by the addition of increasing amounts of formamide, which serves to destabilize the hybrid duplex in the same manner as increased temperature. Thus, hybridization conditions can be readily manipulated, and thus will generally be a method of choice depending on the desired results.

According to another embodiment of the present invention, polynucleotide compositions comprising antisense oligonucleotides are provided. Antisense oligonucleotides have been demonstrated to be effective and targeted inhibitors of protein synthesis, and, consequently, provide a therapeutic approach by which a disease can be treated by inhibiting the synthesis of proteins that contribute to the disease. The efficacy of antisense oligonucleotides for inhibiting protein synthesis is well established. For example, the synthesis of polygalactauronase and the muscarine type 2 acetylcholine receptor are inhibited by antisense oligonucleotides directed to their respective mRNA sequences (U. S. Patent 5,739,119 and U. S. Patent 5,759,829). Further, examples of antisense inhibition have been demonstrated with the nuclear protein cyclin, the multiple drug resistance gene (MDG1), ICAM-1, E-selectin, STK-1, striatal GABA_{A} receptor and human EGF (Jaskulski *et al.,* Science. 1988 Jun 10;240(4858):1544-6; Vasanthakumar and Ahmed, Cancer Commun. 1989;1(4):225-32; Peris *et al.,* Brain Res Mol Brain Res. 1998 Jun 15;57(2):310-20; U. S. Patent 5,801,154; U.S. Patent 5,789,573; U. S. Patent 5,718,709 and U.S. Patent 5,610,288). Antisense constructs have also been described that inhibit and can be used to treat a variety of abnormal cellular proliferations, *e.g*. cancer (U. S. Patent 5,747,470; U. S. Patent 5,591,317 and U. S. Patent 5,783,683).

Therefore, in certain embodiments, the present invention provides oligonucleotide sequences that comprise all, or a portion of, any sequence that is capable of specifically binding to polynucleotide sequence described herein, or a complement thereof. In one embodiment, the antisense oligonucleotides comprise DNA or derivatives thereof. In another embodiment, the oligonucleotides comprise RNA or derivatives thereof. In a third embodiment, the oligonucleotides are modified DNAs comprising a phosphorothioated modified backbone. In a fourth embodiment, the oligonucleotide sequences comprise peptide nucleic acids or derivatives thereof. In each case, preferred compositions comprise a sequence region that is complementary, and more preferably substantially-complementary, and even more preferably, completely complementary to one or more portions of polynucleotides disclosed herein. Selection of antisense compositions specific for a given gene sequence is based upon analysis of the chosen target sequence and determination of secondary structure, Tₘ, binding energy, and relative stability. Antisense compositions may be selected based upon their relative inability to form dimers, hairpins, or other secondary structures that would reduce or prohibit specific binding to the target mRNA in a host cell. Highly preferred target regions of the mRNA, are those which are at or near the AUG translation initiation codon, and those sequences which are substantially complementary to 5' regions of the mRNA. These secondary structure analyses and target site selection considerations can be performed, for example, using v.4 of the OLIGO primer analysis software and/or the BLASTN 2.0.5 algorithm software (Altschul *et al.,* Nucleic Acids Res. 1997, 25(17):3389-402).

The use of an antisense delivery method employing a short peptide vector, termed MPG (27 residues), is also contemplated. The MPG peptide contains a hydrophobic domain derived from the fusion sequence of HIV gp41 and a hydrophilic domain from the nuclear localization sequence of SV40 T-antigen (Morris *et al.,* Nucleic Acids Res. 1997 Jul 15;25(14):2730-6). It has been demonstrated that several molecules of the MPG peptide coat the antisense oligonucleotides and can be delivered into cultured mammalian cells in less than 1 hour with relatively high efficiency (90%). Further, the interaction with MPG strongly increases both the stability of the oligonucleotide to nuclease and the ability to cross the plasma membrane.

According to another embodiment of the invention, the polynucleotide compositions described herein are used in the design and preparation of ribozyme molecules for inhibiting expression of the tumor polypeptides and proteins of the present invention in tumor cells. Ribozymes are RNA-protein complexes that cleave nucleic acids in a site-specific fashion. Ribozymes have specific catalytic domains that possess endonuclease activity (Kim and Cech, Proc Natl Acad Sci U S A. 1987 Dec;84(24):8788-92; Forster and Symons, Cell. 1987 Apr 24;49(2):211-20). For example, a large number of ribozymes accelerate phosphoester transfer reactions with a high degree of specificity, often cleaving only one of several phosphoesters in an oligonucleotide substrate (Cech *et al.,* Cell. 1981 Dec;27(3 Pt 2):487-96; Michel and Westhof, J Mol Biol. 1990 Dec 5;216(3):585-610; Reinhold-Hurek and Shub, Nature. 1992 May 14;357(6374):173-6). This specificity has been attributed to the requirement that the substrate bind via specific base-pairing interactions to the internal guide sequence ("IGS") of the ribozyme prior to chemical reaction.

Six basic varieties of naturally-occurring enzymatic RNAs are known presently. Each can catalyze the hydrolysis of RNA phosphodiester bonds *in trans* (and thus can cleave other RNA molecules) under physiological conditions. In general, enzymatic nucleic acids act by first binding to a target RNA. Such binding occurs through the target binding portion of a enzymatic nucleic acid which is held in close proximity to an enzymatic portion of the molecule that acts to cleave the target RNA. Thus, the enzymatic nucleic acid first recognizes and then binds a target RNA through complementary base-pairing, and once bound to the correct site, acts enzymatically to cut the target RNA. Strategic cleavage of such a target RNA will destroy its ability to direct synthesis of an encoded protein. After an enzymatic nucleic acid has bound and cleaved its RNA target, it is released from that RNA to search for another target and can repeatedly bind and cleave new targets.

The enzymatic nature of a ribozyme is advantageous over many technologies, such as antisense technology (where a nucleic acid molecule simply binds to a nucleic acid target to block its translation) since the concentration of ribozyme necessary to affect a therapeutic treatment is lower than that of an antisense oligonucleotide. This advantage reflects the ability of the ribozyme to act enzymatically. Thus, a single ribozyme molecule is able to cleave many molecules of target RNA. In addition, the ribozyme is a highly specific inhibitor, with the specificity of inhibition depending not only on the base pairing mechanism of binding to the target RNA, but also on the mechanism of target RNA cleavage. Single mismatches, or base-substitutions, near the site of cleavage can completely eliminate catalytic activity of a ribozyme. Similar mismatches in antisense molecules do not prevent their action (Woolf *et al.,* Proc Natl Acad Sci U S A. 1992 Aug 15;89(16):7305-9). Thus, the specificity of action of a ribozyme is greater than that of an antisense oligonucleotide binding the same RNA site.

The enzymatic nucleic acid molecule may be formed in a hammerhead, hairpin, a hepatitis δ virus, group I intron or RNaseP RNA (in association with an RNA guide sequence) or Neurospora VS RNA motif. Examples of hammerhead motifs are described by Rossi *et al.* Nucleic Acids Res. 1992 Sep 11;20(17):4559-65. Examples of hairpin motifs are described by Hampel *et al.* (Eur. Pat. Appl. Publ. No. EP 0360257), Hampel and Tritz, Biochemistry 1989 Jun 13;28(12):4929-33; Hampel *et al.,* Nucleic Acids Res. 1990 Jan 25;18(2):299-304 and U. S. Patent 5,631,359. An example of the hepatitis δ virus motif is described by Perrotta and Been, Biochemistry. 1992 Dec 1;31(47):11843-52; an example of the RNaseP motif is described by Guerrier-Takada *et al.,* Cell. 1983 Dec;35(3 Pt 2):849-57; Neurospora VS RNA ribozyme motif is described by Collins (Saville and Collins, Cell. 1990 May 18;61(4):685-96; Saville and Collins, Proc Natl Acad Sci U S A. 1991 Oct 1;88(19):8826-30; Collins and Olive, Biochemistry. 1993 Mar 23;32(11):2795-9); and an example of the Group I intron is described in (U. S. Patent 4,987,071). All that is important in an enzymatic nucleic acid molecule of this invention is that it has a specific substrate binding site which is complementary to one or more of the target gene RNA regions, and that it have nucleotide sequences within or surrounding that substrate binding site which impart an RNA cleaving activity to the molecule. Thus the ribozyme constructs need not be limited to specific motifs mentioned herein.

Ribozymes may be designed as described in Int. Pat. Appl. Publ. No. WO 93/23569 and Int. Pat. Appl. Publ. No. WO 94/02595, each specifically incorporated herein by reference) and synthesized to be tested *in vitro* and *in vivo,* as described. Such ribozymes can also be optimized for delivery. While specific examples are provided, those in the art will recognize that equivalent RNA targets in other species can be utilized when necessary.

Ribozyme activity can be optimized by altering the length of the ribozyme binding arms, or chemically synthesizing ribozymes with modifications that prevent their degradation by serum ribonucleases (see *e.g*., Int. Pat. Appl. Publ. No. WO 92/07065; Int. Pat. Appl. Publ. No. WO 93/15187; Int. Pat. Appl. Publ. No. WO 91/03162; Eur. Pat. Appl. Publ. No. 92110298.4; U. S. Patent 5,334,711; and Int. Pat. Appl. Publ. No. WO 94/13688, which describe various chemical modifications that can be made to the sugar moieties of enzymatic RNA molecules), modifications which enhance their efficacy in cells, and removal of stem II bases to shorten RNA synthesis times and reduce chemical requirements.

Sullivan *et al.* (Int. Pat. Appl. Publ. No. WO 94/02595) describes the general methods for delivery of enzymatic RNA molecules. Ribozymes may be administered to cells by a variety of methods known to those familiar to the art, including, but not restricted to, encapsulation in liposomes, by iontophoresis, or by incorporation into other vehicles, such as hydrogels, cyclodextrins, biodegradable nanocapsules, and bioadhesive microspheres. For some indications, ribozymes may be directly delivered *ex vivo* to cells or tissues with or without the aforementioned vehicles. Alternatively, the RNA/vehicle combination may be locally delivered by direct inhalation, by direct injection or by use of a catheter, infusion pump or stent. Other routes of delivery include, but are not limited to, intravascular, intramuscular, subcutaneous or joint injection, aerosol inhalation, oral (tablet or pill form), topical, systemic, ocular, intraperitoneal and/or intrathecal delivery. More detailed descriptions of ribozyme delivery and administration are provided in Int. Pat. Appl. Publ. No. WO 94/02595 and Int. Pat. Appl. Publ. No. WO 93/23569, each specifically incorporated herein by reference.

Another means of accumulating high concentrations of a ribozyme(s) within cells is to incorporate the ribozyme-encoding sequences into a DNA expression vector. Transcription of the ribozyme sequences are driven from a promoter for eukaryotic RNA polymerase I (pol I), RNA polymerase II (pol II), or RNA polymerase III (pol III). Transcripts from pol II or pol III promoters will be expressed at high levels in all cells; the levels of a given pol II promoter in a given cell type will depend on the nature of the gene regulatory sequences (enhancers, silencers, *etc.)* present nearby. Prokaryotic RNA polymerase promoters may also be used, providing that the prokaryotic RNA polymerase enzyme is expressed in the appropriate cells Ribozymes expressed from such promoters have been shown to function in mammalian cells. Such transcription units can be incorporated into a variety of vectors for introduction into mammalian cells, including but not restricted to, plasmid DNA vectors, viral DNA vectors (such as adenovirus or adeno-associated vectors), or viral RNA vectors (such as retroviral, semliki forest virus, sindbis virus vectors).

In another embodiment of the invention, peptide nucleic acids (PNAs) compositions are provided. PNA is a DNA mimic in which the nucleobases are attached to a pseudopeptide backbone (Good and Nielsen, Antisense Nucleic Acid Drug Dev. 1997 7(4) 431-37). PNA is able to be utilized in a number methods that traditionally have used RNA or DNA. Often PNA sequences perform better in techniques than the corresponding RNA or DNA sequences and have utilities that are not inherent to RNA or DNA. A review of PNA including methods of making, characteristics of, and methods of using, is provided by Corey *(Trends Biotechnol* 1997 Jun;15(6):224-9). As such, in certain embodiments, one may prepare PNA sequences that are complementary to one or more portions of the ACE mRNA sequence, and such PNA compositions may be used to regulate, alter, decrease, or reduce the translation of ACE-specific mRNA, and thereby alter the level of ACE activity in a host cell to which such PNA compositions have been administered.

PNAs have 2-aminoethyl-glycine linkages replacing the normal phosphodiester backbone of DNA (Nielsen *et al., Science* 1991 Dec 6;254(5037):1497-500; Hanvey *et al.,* Science. 1992 Nov 27;258(5087):1481-5; Hyrup and Nielsen, Bioorg Med Chem. 1996 Jan;4(1):5-23). This chemistry has three important consequences: firstly, in contrast to DNA or phosphorothioate oligonucleotides, PNAs are neutral molecules; secondly, PNAs are achiral, which avoids the need to develop a stereoselective synthesis; and thirdly, PNA synthesis uses standard Boc or Fmoc protocols for solid-phase peptide synthesis, although other methods, including a modified Merrifield method, have been used.

PNA monomers or ready-made oligomers are commercially available from PerSeptive Biosystems (Framingham, MA). PNA syntheses by either Boc or Fmoc protocols are straightforward using manual or automated protocols (Norton *et al.,* Bioorg Med Chem. 1995 Apr;3(4):437-45). The manual protocol lends itself to the production of chemically modified PNAs or the simultaneous synthesis of families of closely related PNAs.

As with peptide synthesis, the success of a particular PNA synthesis will depend on the properties of the chosen sequence. For example, while in theory PNAs can incorporate any combination of nucleotide bases, the presence of adjacent purines can lead to deletions of one or more residues in the product. In expectation of this difficulty, it is suggested that, in producing PNAs with adjacent purines, one should repeat the coupling of residues likely to be added inefficiently. This should be followed by the purification of PNAs by reverse-phase high-pressure liquid chromatography, providing yields and purity of product similar to those observed during the synthesis of peptides.

Modifications of PNAs for a given application may be accomplished by coupling amino acids during solid-phase synthesis or by attaching compounds that contain a carboxylic acid group to the exposed N-terminal amine. Alternatively, PNAs can be modified after synthesis by coupling to an introduced lysine or cysteine. The ease with which PNAs can be modified facilitates optimization for better solubility or for specific functional requirements. Once synthesized, the identity of PNAs and their derivatives can be confirmed by mass spectrometry. Several studies have made and utilized modifications of PNAs (for example, Norton *et al.,* Bioorg Med Chem. 1995 Apr;3(4):437-45; Petersen *et al.,* J Pept Sci. 1995 May-Jun;1(3):175-83; Orum *et al.,* Biotechniques. 1995 Sep;19(3):472-80; Footer *et al.,* Biochemistry. 1996 Aug 20;35(33):10673-9; Griffith *et al.,* Nucleic Acids Res. 1995 Aug 11;23(15):3003-8; Pardridge *et al.,* Proc Natl Acad Sci U S A. 1995 Jun 6;92(12):5592-6; Boffa *et al.,* Proc Natl Acad Sci U S A. 1995 Mar 14;92(6):1901-5; Gambacorti-Passerini *et al.,* Blood. 1996 Aug 15;88(4):1411-7; Armitage *et al.,* Proc Natl Acad Sci U S A. 1997 Nov 11;94(23):12320-5; Seeger *et al.,* Biotechniques. 1997 Sep;23(3):512-7). U.S. Patent No. 5,700,922 discusses PNA-DNA-PNA chimeric molecules and their uses in diagnostics, modulating protein in organisms, and treatment of conditions susceptible to therapeutics.

Methods of characterizing the antisense binding properties of PNAs are discussed in Rose (Anal Chem. 1993 Dec 15;65(24):3545-9) and Jensen *et al.* (Biochemistry. 1997 Apr 22;36(16):5072-7). Rose uses capillary gel electrophoresis to determine binding of PNAs to their complementary oligonucleotide, measuring the relative binding kinetics and stoichiometry. Similar types of measurements were made by Jensen *et al.* using BIAcore™ technology.

Other applications of PNAs that have been described and will be apparent to the skilled artisan include use in DNA strand invasion, antisense inhibition, mutational analysis, enhancers of transcription, nucleic acid purification, isolation of transcriptionally active genes, blocking of transcription factor binding, genome cleavage, biosensors*, in situ* hybridization, and the like.

### Polynucleotide Identification, Characterization and Expression

Polynucleotides compositions of the present invention may be identified, prepared and/or manipulated using any of a variety of well established techniques (see generally, Sambrook et al., *Molecular Cloning: A Laboratory Manual,* Cold Spring Harbor Laboratories, Cold Spring Harbor, NY, 1989, and other like references). For example, a polynucleotide may be identified by screening a microarray of cDNAs for tumor-associated expression (i.e., expression that is at least two fold greater in a tumor than in normal tissue, as determined using a representative assay provided herein). Such screens may be performed, for example, using the microarray technology of Affymetrix, Inc. (Santa Clara, CA) according to the manufacturer's instructions (and essentially as described by Schena et al., *Proc. Natl. Acad. Sci. USA 93:*10614-10619*,* 1996 and Heller et al., *Proc. Natl. Acad. Sci. USA 94*:2150-2155, 1997). Alternatively, polynucleotides may be amplified from cDNA prepared from cells expressing the proteins described herein, such as tumor cells.

Many template dependent processes are available to amplify a target sequences of interest present in a sample. One of the best known amplification methods is the polymerase chain reaction (PCR^{TM}) which is described in detail in U.S. Patent Nos. 4,683,195, 4,683,202 and 4,800,159, each of which is incorporated herein by reference in its entirety. Briefly, in PCR™, two primer sequences are prepared which are complementary to regions on opposite complementary strands of the target sequence. An excess of deoxynucleoside triphosphates is added to a reaction mixture along with a DNA polymerase *(e.g., Taq* polymerase). If the target sequence is present in a sample, the primers will bind to the target and the polymerase will cause the primers to be extended along the target sequence by adding on nucleotides. By raising and lowering the temperature of the reaction mixture, the extended primers will dissociate from the target to form reaction products, excess primers will bind to the target and to the reaction product and the process is repeated. Preferably reverse transcription and PCR™ amplification procedure may be performed in order to quantify the amount of mRNA amplified. Polymerase chain reaction methodologies are well known in the art.

Any of a number of other template dependent processes, many of which are variations of the PCR™ amplification technique, are readily known and available in the art. Illustratively, some such methods include the ligase chain reaction (referred to as LCR), described, for example, in Eur. Pat. Appl. Publ. No. 320,308 and U.S. Patent No. 4,883,750; Qbeta Replicase, described in PCT Intl. Pat. Appl. Publ. No. PCT/US87/00880; Strand Displacement Amplification (SDA) and Repair Chain Reaction (RCR). Still other amplification methods are described in Great Britain Pat. Appl. No. 2 202 328, and in PCT Intl. Pat. Appl. Publ. No. PCT/US89/01025. Other nucleic acid amplification procedures include transcription-based amplification systems (TAS) (PCT Intl. Pat. Appl. Publ. No. WO 88/10315), including nucleic acid sequence based amplification (NASBA) and 3SR. Eur. Pat. Appl. Publ. No. 329,822 describes a nucleic acid amplification process involving cyclically synthesizing single-stranded RNA ("ssRNA"), ssDNA, and double-stranded DNA (dsDNA). PCT Intl. Pat. Appl. Publ. No. WO 89/06700 describes a nucleic acid sequence amplification scheme based on the hybridization of a promoter/primer sequence to a target single-stranded DNA ("ssDNA") followed by transcription of many RNA copies of the sequence. Other amplification methods such as "RACE" (Frohman, 1990), and "one-sided PCR" (Ohara, 1989) are also well-known to those of skill in the art.

An amplified portion of a polynucleotide of the present invention may be used to isolate a full length gene from a suitable library (e.g., a tumor cDNA library) using well known techniques. Within such techniques, a library (cDNA or genomic) is screened using one or more polynucleotide probes or primers suitable for amplification. Preferably, a library is size-selected to include larger molecules. Random primed libraries may also be preferred for identifying 5' and upstream regions of genes. Genomic libraries are preferred for obtaining introns and extending 5' sequences.

For hybridization techniques, a partial sequence may be labeled (e.g., by nick-translation or end-labeling with ³²P) using well known techniques. A bacterial or bacteriophage library is then generally screened by hybridizing filters containing denatured bacterial colonies (or lawns containing phage plaques) with the labeled probe *(see* Sambrook et al., *Molecular Cloning: A Laboratory Manual,* Cold Spring Harbor Laboratories, Cold Spring Harbor, NY, 1989). Hybridizing colonies or plaques are selected and expanded, and the DNA is isolated for further analysis. cDNA clones may be analyzed to determine the amount of additional sequence by, for example, PCR using a primer from the partial sequence and a primer from the vector. Restriction maps and partial sequences may be generated to identify one or more overlapping clones. The complete sequence may then be determined using standard techniques, which may involve generating a series of deletion clones. The resulting overlapping sequences can then assembled into a single contiguous sequence. A full length cDNA molecule can be generated by ligating suitable fragments, using well known techniques.

Alternatively, amplification techniques, such as those described above, can be useful for obtaining a full length coding sequence from a partial cDNA sequence. One such amplification technique is inverse PCR *(see* Triglia et al., *Nucl. Acids Res. 16*:8186, 1988), which uses restriction enzymes to generate a fragment in the known region of the gene. The fragment is then circularized by intramolecular ligation and used as a template for PCR with divergent primers derived from the known region. Within an alternative approach, sequences adjacent to a partial sequence may be retrieved by amplification with a primer to a linker sequence and a primer specific to a known region. The amplified sequences are typically subjected to a second round of amplification with the same linker primer and a second primer specific to the known region. A variation on this procedure, which employs two primers that initiate extension in opposite directions from the known sequence, is described in WO 96/38591. Another such technique is known as "rapid amplification of cDNA ends" or RACE. This technique involves the use of an internal primer and an external primer, which hybridizes to a polyA region or vector sequence, to identify sequences that are 5' and 3' of a known sequence. Additional techniques include capture PCR (Lagerstrom et al., *PCR Methods Applic. 1*:111-19, 1991) and walking PCR (Parker et al., *Nucl. Acids. Res. 19*:3055-60, 1991). Other methods employing amplification may also be employed to obtain a full length cDNA sequence.

In certain instances, it is possible to obtain a full length cDNA sequence by analysis of sequences provided in an expressed sequence tag (EST) database, such as that available from GenBank. Searches for overlapping ESTs may generally be performed using well known programs (e.g., NCBI BLAST searches), and such ESTs may be used to generate a contiguous full length sequence. Full length DNA sequences may also be obtained by analysis of genomic fragments.

In other embodiments of the invention, polynucleotide sequences or fragments thereof which encode polypeptides of the invention, or fusion proteins or functional equivalents thereof, may be used in recombinant DNA molecules to direct expression of a polypeptide in appropriate host cells. Due to the inherent degeneracy of the genetic code, other DNA sequences that encode substantially the same or a functionally equivalent amino acid sequence may be produced and these sequences may be used to clone and express a given polypeptide.

As will be understood by those of skill in the art, it may be advantageous in some instances to produce polypeptide-encoding nucleotide sequences possessing non-naturally occurring codons. For example, codons preferred by a particular prokaryotic or eukaryotic host can be selected to increase the rate of protein expression or to produce a recombinant RNA transcript having desirable properties, such as a half-life which is longer than that of a transcript generated from the naturally occurring sequence.

Moreover, the polynucleotide sequences of the present invention can be engineered using methods generally known in the art in order to alter polypeptide encoding sequences for a variety of reasons, including but not limited to, alterations which modify the cloning, processing, and/or expression of the gene product. For example, DNA shuffling by random fragmentation and PCR reassembly of gene fragments and synthetic oligonucleotides may be used to engineer the nucleotide sequences. In addition, site-directed mutagenesis may be used to insert new restriction sites, alter glycosylation patterns, change codon preference, produce splice variants, or introduce mutations, and so forth.

In another embodiment of the invention, natural, modified, or recombinant nucleic acid sequences may be ligated to a heterologous sequence to encode a fusion protein. For example, to screen peptide libraries for inhibitors of polypeptide activity, it may be useful to encode a chimeric protein that can be recognized by a commercially available antibody. A fusion protein may also be engineered to contain a cleavage site located between the polypeptide-encoding sequence and the heterologous protein sequence, so that the polypeptide may be cleaved and purified away from the heterologous moiety.

Sequences encoding a desired polypeptide may be synthesized, in whole or in part, using chemical methods well known in the art (see Caruthers, M. H. et al. (1980) *Nucl. Acids Res. Symp. Ser.* 215-223, Horn, T. et al. (1980) *Nucl. Acids Res. Symp. Ser.* 225-232). Alternatively, the protein itself may be produced using chemical methods to synthesize the amino acid sequence of a polypeptide, or a portion thereof. For example, peptide synthesis can be performed using various solid-phase techniques (Roberge, J. Y. et al. (1995) *Science 269:*202-204) and automated synthesis may be achieved, for example, using the ABI 431 A Peptide Synthesizer (Perkin Elmer, Palo Alto, CA).

A newly synthesized peptide may be substantially purified by preparative high performance liquid chromatography (*e.g*., Creighton, T. (1983) Proteins, Structures and Molecular Principles, WH Freeman and Co., New York, N.Y.) or other comparable techniques available in the art. The composition of the synthetic peptides may be confirmed by amino acid analysis or sequencing (*e.g*., the Edman degradation procedure). Additionally, the amino acid sequence of a polypeptide, or any part thereof, may be altered during direct synthesis and/or combined using chemical methods with sequences from other proteins, or any part thereof, to produce a variant polypeptide.

In order to express a desired polypeptide, the nucleotide sequences encoding the polypeptide, or functional equivalents, may be inserted into appropriate expression vector, *i.e*., a vector which contains the necessary elements for the transcription and translation of the inserted coding sequence. Methods which are well known to those skilled in the art may be used to construct expression vectors containing sequences encoding a polypeptide of interest and appropriate transcriptional and translational control elements. These methods include *in vitro* recombinant DNA techniques, synthetic techniques, and *in vivo* genetic recombination. Such techniques are described, for example, in Sambrook, J. et al. (1989) Molecular Cloning, A Laboratory Manual, Cold Spring Harbor Press, Plainview, N.Y., and Ausubel, F. M. et al. (1989) Current Protocols in Molecular Biology, John Wiley & Sons, New York. N.Y.

A variety of expression vector/host systems may be utilized to contain and express polynucleotide sequences. These include, but are not limited to, microorganisms such as bacteria transformed with recombinant bacteriophage, plasmid, or cosmid DNA expression vectors; yeast transformed with yeast expression vectors; insect cell systems infected with virus expression vectors (*e.g*., baculovirus); plant cell systems transformed with virus expression vectors (*e.g*., cauliflower mosaic virus, CaMV; tobacco mosaic virus, TMV) or with bacterial expression vectors (*e.g*., Ti or pBR322 plasmids); or animal cell systems.

The "control elements" or "regulatory sequences" present in an expression vector are those non-translated regions of the vector--enhancers, promoters, 5' and 3' untranslated regions--which interact with host cellular proteins to carry out transcription and translation. Such elements may vary in their strength and specificity. Depending on the vector system and host utilized, any number of suitable transcription and translation elements, including constitutive and inducible promoters, may be used. For example, when cloning in bacterial systems, inducible promoters such as the hybrid lacZ promoter of the PBLUESCRIPT phagemid (Stratagene, La Jolla, Calif.) or PSPORT1 plasmid (Gibco BRL, Gaithersburg, MD) and the like may be used. In mammalian cell systems, promoters from mammalian genes or from mammalian viruses are generally preferred. If it is necessary to generate a cell line that contains multiple copies of the sequence encoding a polypeptide, vectors based on SV40 or EBV may be advantageously used with an appropriate selectable marker.

In bacterial systems, any of a number of expression vectors may be selected depending upon the use intended for the expressed polypeptide. For example, when large quantities are needed, for example for the induction of antibodies, vectors which direct high level expression of fusion proteins that are readily purified may be used. Such vectors include, but are not limited to, the multifunctional *E. coli* cloning and expression vectors such as BLUESCRIPT (Stratagene), in which the sequence encoding the polypeptide of interest may be ligated into the vector in frame with sequences for the amino-terminal Met and the subsequent 7 residues of .beta.-galactosidase so that a hybrid protein is produced; pIN vectors (Van Heeke, G. and S. M. Schuster (1989) *J Biol. Chem. 264*:5503-5509); and the like. pGEX Vectors (Promega, Madison, Wis.) may also be used to express foreign polypeptides as fusion proteins with glutathione S-transferase (GST). In general, such fusion proteins are soluble and can easily be purified from lysed cells by adsorption to glutathione-agarose beads followed by elution in the presence of free glutathione. Proteins made in such systems may be designed to include heparin, thrombin, or factor XA protease cleavage sites so that the cloned polypeptide of interest can be released from the GST moiety at will.

In the yeast, Saccharomyces cerevisiae, a number of vectors containing constitutive or inducible promoters such as alpha factor, alcohol oxidase, and PGH may be used. For reviews, see Ausubel et al. (supra) and Grant et al. (1987) *Methods Enzymol.* 153 :516-544.

In cases where plant expression vectors are used, the expression of sequences encoding polypeptides may be driven by any of a number of promoters. For example, viral promoters such as the 35S and 19S promoters of CaMV may be used alone or in combination with the omega leader sequence from TMV (Takamatsu, N. (1987) *EMBO J. 6*:307-311. Alternatively, plant promoters such as the small subunit of RUBISCO or heat shock promoters may be used (Coruzzi, G. et al. (1984) *EMBO J. 3*:1671-1680; Broglie, R. et al. (1984) *Science 224*:838-843; and Winter, J. et al. (1991) *Results Probl. Cell Differ. 17*:85-105). These constructs can be introduced into plant cells by direct DNA transformation or pathogen-mediated transfection. Such techniques are described in a number of generally available reviews (see, for example, Hobbs, S. or Murry, L. E. in McGraw Hill Yearbook of Science and Technology (1992) McGraw Hill, New York, N.Y.; pp. 191-196).

An insect system may also be used to express a polypeptide of interest. For example, in one such system, Autographa californica nuclear polyhedrosis virus (AcNPV) is used as a vector to express foreign genes in Spodoptera frugiperda cells or in Trichoplusia larvae. The sequences encoding the polypeptide may be cloned into a non-essential region of the virus, such as the polyhedrin gene, and placed under control of the polyhedrin promoter. Successful insertion of the polypeptide-encoding sequence will render the polyhedrin gene inactive and produce recombinant virus lacking coat protein. The recombinant viruses may then be used to infect, for example, S. frugiperda cells or Trichoplusia larvae in which the polypeptide of interest may be expressed (Engelhard, E. K. et al. (1994) *Proc. Natl. Acad. Sci. 91* :3224-3227).

In mammalian host cells, a number of viral-based expression systems are generally available. For example, in cases where an adenovirus is used as an expression vector, sequences encoding a polypeptide of interest may be ligated into an adenovirus transcription/translation complex consisting of the late promoter and tripartite leader sequence. Insertion in a non-essential E1 or E3 region of the viral genome may be used to obtain a viable virus which is capable of expressing the polypeptide in infected host cells (Logan, J. and Shenk, T. (1984) *Proc. Natl. Acad. Sci.* 81:3655-3659). In addition, transcription enhancers, such as the Rous sarcoma virus (RSV) enhancer, may be used to increase expression in mammalian host cells.

Specific initiation signals may also be used to achieve more efficient translation of sequences encoding a polypeptide of interest. Such signals include the ATG initiation codon and adjacent sequences. In cases where sequences encoding the polypeptide, its initiation codon, and upstream sequences are inserted into the appropriate expression vector, no additional transcriptional or translational control signals may be needed. However, in cases where only coding sequence, or a portion thereof, is inserted, exogenous translational control signals including the ATG initiation codon should be provided. Furthermore, the initiation codon should be in the correct reading frame to ensure translation of the entire insert. Exogenous translational elements and initiation codons may be of various origins, both natural and synthetic. The efficiency of expression may be enhanced by the inclusion of enhancers which are appropriate for the particular cell system which is used, such as those described in the literature (Scharf, D. et al. (1994) *Results Probl. Cell Differ. 20:*125-162)*.*

In addition, a host cell strain may be chosen for its ability to modulate the expression of the inserted sequences or to process the expressed protein in the desired fashion. Such modifications of the polypeptide include, but are not limited to, acetylation, carboxylation. glycosylation, phosphorylation, lipidation, and acylation. Post-translational processing which cleaves a "prepro" form of the protein may also be used to facilitate correct insertion, folding and/or function. Different host cells such as CHO, COS, HeLa, MDCK, HEK293, and WI38, which have specific cellular machinery and characteristic mechanisms for such post-translational activities, may be chosen to ensure the correct modification and processing of the foreign protein.

For long-term, high-yield production of recombinant proteins, stable expression is generally preferred. For example, cell lines which stably express a polynucleotide of interest may be transformed using expression vectors which may contain viral origins of replication and/or endogenous expression elements and a selectable marker gene on the same or on a separate vector. Following the introduction of the vector, cells may be allowed to grow for 1-2 days in an enriched media before they are switched to selective media. The purpose of the selectable marker is to confer resistance to selection, and its presence allows growth and recovery of cells which successfully express the introduced sequences. Resistant clones of stably transformed cells may be proliferated using tissue culture techniques appropriate to the cell type.

Any number of selection systems may be used to recover transformed cell lines. These include, but are not limited to, the herpes simplex virus thymidine kinase (Wigler, M. et al. (1977) *Cell 11*:223-32) and adenine phosphoribosyltransferase (Lowy, I. et al. (1990) *Cell 22*:817-23) genes which can be employed in tk.sup.- or aprt.sup.- cells, respectively. Also, antimetabolite, antibiotic or herbicide resistance can be used as the basis for selection; for example, dhfr which confers resistance to methotrexate (Wigler, M. et al. (1980) *Proc. Natl. Acad. Sci. 77*:3567-70); npt, which confers resistance to the aminoglycosides, neomycin and G-418 (Colbere-Garapin, F. et al (1981) *J. Mol. Biol. 150:*1-14)*;* and als or pat, which confer resistance to chlorsulfuron and phosphinotricin acetyltransferase, respectively (Murry, *supra).* Additional selectable genes have been described, for example, trpB, which allows cells to utilize indole in place of tryptophan, or hisD, which allows cells to utilize histinol in place of histidine (Hartman, S. C. and R. C. Mulligan (1988) *Proc. Natl. Acad. Sci. 85*:8047-51). The use of visible markers has gained popularity with such markers as anthocyanins, beta-glucuronidase and its substrate GUS, and luciferase and its substrate luciferin, being widely used not only to identify transformants, but also to quantify the amount of transient or stable protein expression attributable to a specific vector system (Rhodes, C. A. et al. (1995) *Methods Mol. Biol. 55*:121-131).

Although the presence/absence of marker gene expression suggests that the gene of interest is also present, its presence and expression may need to be confirmed. For example, if the sequence encoding a polypeptide is inserted within a marker gene sequence, recombinant cells containing sequences can be identified by the absence of marker gene function. Alternatively, a marker gene can be placed in tandem with a polypeptide-encoding sequence under the control of a single promoter. Expression of the marker gene in response to induction or selection usually indicates expression of the tandem gene as well.

Alternatively, host cells that contain and express a desired polynucleotide sequence may be identified by a variety of procedures known to those of skill in the art. These procedures include, but are not limited to, DNA-DNA or DNA-RNA hybridizations and protein bioassay or immunoassay techniques which include, for example, membrane, solution, or chip based technologies for the detection and/or quantification of nucleic acid or protein.

A variety of protocols for detecting and measuring the expression of polynucleotide-encoded products, using either polyclonal or monoclonal antibodies specific for the product are known in the art. Examples include enzyme-linked immunosorbent assay (ELISA), radioimmunoassay (RIA), and fluorescence activated cell sorting (FACS). A two-site, monoclonal-based immunoassay utilizing monoclonal antibodies reactive to two non-interfering epitopes on a given polypeptide may be preferred for some applications, but a competitive binding assay may also be employed. These and other assays are described, among other places, in Hampton, R. et al. (1990; Serological Methods, a Laboratory Manual, APS Press, St Paul. Minn.) and Maddox, D. E. et al. (1983; *J. Exp. Med. 158*:1211-1216).

A wide variety of labels and conjugation techniques are known by those skilled in the art and may be used in various nucleic acid and amino acid assays. Means for producing labeled hybridization or PCR probes for detecting sequences related to polynucleotides include oligolabeling, nick translation, end-labeling or PCR amplification using a labeled nucleotide. Alternatively, the sequences, or any portions thereof may be cloned into a vector for the production of an mRNA probe. Such vectors are known in the art, are commercially available, and may be used to synthesize RNA probes in vitro by addition of an appropriate RNA polymerase such as T7, T3, or SP6 and labeled nucleotides. These procedures may be conducted using a variety of commercially available kits. Suitable reporter molecules or labels, which may be used include radionuclides, enzymes, fluorescent, chemiluminescent, or chromogenic agents as well as substrates, cofactors, inhibitors, magnetic particles, and the like.

Host cells transformed with a polynucleotide sequence of interest may be cultured under conditions suitable for the expression and recovery of the protein from cell culture. The protein produced by a recombinant cell may be secreted or contained intracellularly depending on the sequence and/or the vector used. As will be understood by those of skill in the art, expression vectors containing polynucleotides of the invention may be designed to contain signal sequences which direct secretion of the encoded polypeptide through a prokaryotic or eukaryotic cell membrane. Other recombinant constructions may be used to join sequences encoding a polypeptide of interest to nucleotide sequence encoding a polypeptide domain which will facilitate purification of soluble proteins. Such purification facilitating domains include, but are not limited to, metal chelating peptides such as histidine-tryptophan modules that allow purification on immobilized metals, protein A domains that allow purification on immobilized immunoglobulin, and the domain utilized in the FLAGS extension/affinity purification system (Immunex Corp., Seattle, Wash.). The inclusion of cleavable linker sequences such as those specific for Factor XA or enterokinase (Invitrogen. San Diego, Calif.) between the purification domain and the encoded polypeptide may be used to facilitate purification. One such expression vector provides for expression of a fusion protein containing a polypeptide of interest and a nucleic acid encoding 6 histidine residues preceding a thioredoxin or an enterokinase cleavage site. The histidine residues facilitate purification on IMIAC (immobilized metal ion affinity chromatography) as described in Porath, J. et al. (1992, *Prot. Exp. Purif. 3*:263-281) while the enterokinase cleavage site provides a means for purifying the desired polypeptide from the fusion protein. A discussion of vectors which contain fusion proteins is provided in Kroll, D. J. et al. (1993; *DNA Cell Biol. 12:*441-453).

In addition to recombinant production methods, polypeptides of the invention, and fragments thereof, may be produced by direct peptide synthesis using solid-phase techniques (Merrifield J. (1963) *J. Am. Chem. Soc. 85*:2149-2154). Protein synthesis may be performed using manual techniques or by automation. Automated synthesis may be achieved, for example, using Applied Biosystems 431A Peptide Synthesizer (Perkin Elmer). Alternatively, various fragments may be chemically synthesized separately and combined using chemical methods to produce the full length molecule.

### Antibody Compositions, Fragments Thereof and Other Binding Agents

According to another aspect, the present invention further provides binding agents, such as antibodies and antigen-binding fragments thereof, that exhibit immunological binding to a tumor polypeptide disclosed herein, or to a portion, variant or derivative thereof. An antibody, or antigen-binding fragment thereof, is said to "specifically bind," "immunogically bind," and/or is "immunologically reactive" to a polypeptide of the invention if it reacts at a detectable level (within, for example, an ELISA assay) with the polypeptide, and does not react detectably with unrelated polypeptides under similar conditions.

Immunological binding, as used in this context, generally refers to the non-covalent interactions of the type which occur between an immunoglobulin molecule and an antigen for which the immunoglobulin is specific. The strength, or affinity of immunological binding interactions can be expressed in terms of the dissociation constant (K_{d}) of the interaction, wherein a smaller K_{d} represents a greater affinity. Immunological binding properties of selected polypeptides can be quantified using methods well known in the art. One such method entails measuring the rates of antigen-binding site/antigen complex formation and dissociation, wherein those rates depend on the concentrations of the complex partners, the affinity of the interaction, and on geometric parameters that equally influence the rate in both directions. Thus, both the "on rate constant" (Kₒₙ) and the "off rate constant" (K_{off}) can be determined by calculation of the concentrations and the actual rates of association and dissociation. The ratio of K_{off} /Kₒₙ enables cancellation of all parameters not related to affinity, and is thus equal to the dissociation constant K_{d}. See, generally, Davies et al. (1990) Annual Rev. Biochem. 59:439-473.

An "antigen-binding site," or "binding portion" of an antibody refers to the part of the immunoglobulin molecule that participates in antigen binding. The antigen binding site is formed by amino acid residues of the N-terminal variable ("V") regions of the heavy ("H") and light ("L") chains. Three highly divergent stretches within the V regions of the heavy and light chains are referred to as "hypervariable regions" which are interposed between more conserved flanking stretches known as "framework regions," or "FRs". Thus the term "FR" refers to amino acid sequences which are naturally found between and adjacent to hypervariable regions in immunoglobulins. In an antibody molecule, the three hypervariable regions of a light chain and the three hypervariable regions of a heavy chain are disposed relative to each other in three dimensional space to form an antigen-binding surface. The antigen-binding surface is complementary to the three-dimensional surface of a bound antigen, and the three hypervariable regions of each of the heavy and light chains are referred to as "complementarity-determining regions," or "CDRs."

Binding agents may be further capable of differentiating between patients with and without a cancer, such as breast cancer, using the representative assays provided herein. For example, antibodies or other binding agents that bind to a tumor protein will preferably generate a signal indicating the presence of a cancer in at least about 20% of patients with the disease, more preferably at least about 30% of patients. Alternatively, or in addition, the antibody will generate a negative signal indicating the absence of the disease in at least about 90% of individuals without the cancer. To determine whether a binding agent satisfies this requirement, biological samples (e.g., blood, sera, sputum, urine and/or tumor biopsies) from patients with and without a cancer (as determined using standard clinical tests) may be assayed as described herein for the presence of polypeptides that bind to the binding agent. Preferably, a statistically significant number of samples with and without the disease will be assayed. Each binding agent should satisfy the above criteria; however, those of ordinary skill in the art will recognize that binding agents may be used in combination to improve sensitivity.

Any agent that satisfies the above requirements may be a binding agent. For example, a binding agent may be a ribosome, with or without a peptide component, an RNA molecule or a polypeptide. In a preferred embodiment, a binding agent is an antibody or an antigen-binding fragment thereof. Antibodies may be prepared by any of a variety of techniques known to those of ordinary skill in the art. *See, e.g.,* Harlow and Lane, *Antibodies: A Laboratory Manual,* Cold Spring Harbor Laboratory, 1988. In general, antibodies can be produced by cell culture techniques, including the generation of monoclonal antibodies as described herein, or via transfection of antibody genes into suitable bacterial or mammalian cell hosts, in order to allow for the production of recombinant antibodies. In one technique, an immunogen comprising the polypeptide is initially injected into any of a wide variety of mammals (*e.g*., mice, rats, rabbits, sheep or goats). In this step, the polypeptides of this invention may serve as the immunogen without modification. Alternatively, particularly for relatively short polypeptides, a superior immune response may be elicited if the polypeptide is joined to a carrier protein, such as bovine serum albumin or keyhole limpet hemocyanin. The immunogen is injected into the animal host, preferably according to a predetermined schedule incorporating one or more booster immunizations, and the animals are bled periodically. Polyclonal antibodies specific for the polypeptide may then be purified from such antisera by, for example, affinity chromatography using the polypeptide coupled to a suitable solid support.

Monoclonal antibodies specific for an antigenic polypeptide of interest may be prepared, for example, using the technique of Kohler and Milstein, *Eur. J. Immunol. 6*:511-519, 1976, and improvements thereto. Briefly, these methods involve the preparation of immortal cell lines capable of producing antibodies having the desired specificity (i.e., reactivity with the polypeptide of interest). Such cell lines may be produced, for example, from spleen cells obtained from an animal immunized as described above. The spleen cells are then immortalized by, for example, fusion with a myeloma cell fusion partner, preferably one that is syngeneic with the immunized animal. A variety of fusion techniques may be employed. For example, the spleen cells and myeloma cells may be combined with a nonionic detergent for a few minutes and then plated at low density on a selective medium that supports the growth of hybrid cells, but not myeloma cells. A preferred selection technique uses HAT (hypoxanthine, aminopterin, thymidine) selection. After a sufficient time, usually about 1 to 2 weeks, colonies of hybrids are observed. Single colonies are selected and their culture supernatants tested for binding activity against the polypeptide. Hybridomas having high reactivity and specificity are preferred.

Monoclonal antibodies may be isolated from the supernatants of growing hybridoma colonies. In addition, various techniques may be employed to enhance the yield, such as injection of the hybridoma cell line into the peritoneal cavity of a suitable vertebrate host, such as a mouse. Monoclonal antibodies may then be harvested from the ascites fluid or the blood. Contaminants may be removed from the antibodies by conventional techniques, such as chromatography, gel filtration, precipitation, and extraction. The polypeptides of this invention may be used in the purification process in, for example, an affinity chromatography step.

A number of therapeutically useful molecules are known in the art which comprise antigen-binding sites that are capable of exhibiting immunological binding properties of an antibody molecule. The proteolytic enzyme papain preferentially cleaves IgG molecules to yield several fragments, two of which (the "F(ab)" fragments) each comprise a covalent heterodimer that includes an intact antigen-binding site. The enzyme pepsin is able to cleave IgG molecules to provide several fragments, including the "F(ab')₂ " fragment which comprises both antigen-binding sites. An "Fv" fragment can be produced by preferential proteolytic cleavage of an IgM, and on rare occasions IgG or IgA immunoglobulin molecule. Fv fragments are, however, more commonly derived using recombinant techniques known in the art. The Fv fragment includes a non-covalent V_{H}::V_{L} heterodimer including an antigen-binding site which retains much of the antigen recognition and binding capabilities of the native antibody molecule. Inbar et al. (1972) Proc. Nat. Acad. Sci. USA 69:2659-2662; Hochman et al. (1976) Biochem 15:2706-2710; and Ehrlich et al. (1980) Biochem 19:4091-4096.

A single chain Fv ("sFv") polypeptide is a covalently linked V_{H}::V_{L} heterodimer which is expressed from a gene fusion including V_{H}- and V_{L}-encoding genes linked by a peptide-encoding linker. Huston et al. (1988) Proc. Nat. Acad. Sci. USA 85(16):5879-5883. A number of methods have been described to discern chemical structures for converting the naturally aggregated--but chemically separated--light and heavy polypeptide chains from an antibody V region into an sFv molecule which will fold into a three dimensional structure substantially similar to the structure of an antigen-binding site. See, *e.g.,* U.S. Pat. Nos. 5,091,513 and 5,132,405, to Huston et al.; and U.S. Pat. No. 4,946,778, to Ladner et al.

Each of the above-described molecules includes a heavy chain and a light chain CDR set, respectively interposed between a heavy chain and a light chain FR set which provide support to the CDRS and define the spatial relationship of the CDRs relative to each other. As used herein, the term "CDR set" refers to the three hypervariable regions of a heavy or light chain V region. Proceeding from the N-terminus of a heavy or light chain, these regions are denoted as "CDR1," "CDR2," and "CDR3" respectively. An antigen-binding site, therefore, includes six CDRs, comprising the CDR set from each of a heavy and a light chain V region. A polypeptide comprising a single CDR, (*e.g*., a CDR1, CDR2 or CDR3) is referred to herein as a "molecular recognition unit." Crystallographic analysis of a number of antigen-antibody complexes has demonstrated that the amino acid residues of CDRs form extensive contact with bound antigen, wherein the most extensive antigen contact is with the heavy chain CDR3. Thus, the molecular recognition units are primarily responsible for the specificity of an antigen-binding site.

As used herein, the term "FR set" refers to the four flanking amino acid sequences which frame the CDRs of a CDR set of a heavy or light chain V region. Some FR residues may contact bound antigen; however, FRs are primarily responsible for folding the V region into the antigen-binding site, particularly the FR residues directly adjacent to the CDRS. Within FRs, certain amino residues and certain structural features are very highly conserved. In this regard, all V region sequences contain an internal disulfide loop of around 90 amino acid residues. When the V regions fold into a binding-site, the CDRs are displayed as projecting loop motifs which form an antigen-binding surface. It is generally recognized that there are conserved structural regions of FRs which influence the folded shape of the CDR loops into certain "canonical" structures--regardless of the precise CDR amino acid sequence. Further, certain FR residues are known to participate in non-covalent interdomain contacts which stabilize the interaction of the antibody heavy and light chains.

A number of "humanized" antibody molecules comprising an antigen-binding site derived from a non-human immunoglobulin have been described, including chimeric antibodies having rodent V regions and their associated CDRs fused to human constant domains (Winter et al. (1991) Nature 349:293-299; Lobuglio et al. (1989) Proc. Nat. Acad. Sci. USA 86:4220-4224; Shaw et al. (1987) J Immunol. 138:4534-4538; and Brown et al. (1987) Cancer Res. 47:3577-3583), rodent CDRs grafted into a human supporting FR prior to fusion with an appropriate human antibody constant domain (Riechmann et al. (1988) Nature 332:323-327; Verhoeyen et al. (1988) Science 239:1534-1536; and Jones et al. (1986) Nature 321:522-525), and rodent CDRs supported by recombinantly veneered rodent FRs (European Patent Publication No. 519,596, published Dec. 23, 1992). These "humanized" molecules are designed to minimize unwanted immunological response toward rodent antihuman antibody molecules which limits the duration and effectiveness of therapeutic applications of those moieties in human recipients.

As used herein, the terms "veneered FRs" and "recombinantly veneered FRs" refer to the selective replacement of FR residues from, e.g., a rodent heavy or light chain V region, with human FR residues in order to provide a xenogeneic molecule comprising an antigen-binding site which retains substantially all of the native FR polypeptide folding structure. Veneering techniques are based on the understanding that the ligand binding characteristics of an antigen-binding site are determined primarily by the structure and relative disposition of the heavy and light chain CDR sets within the antigen-binding surface. Davies et al. (1990) Ann. Rev. Biochem. 59:439-473. Thus, antigen binding specificity can be preserved in a humanized antibody only wherein the CDR structures, their interaction with each other, and their interaction with the rest of the V region domains are carefully maintained. By using veneering techniques, exterior (*e.g*., solvent-accessible) FR residues which are readily encountered by the immune system are selectively replaced with human residues to provide a hybrid molecule that comprises either a weakly immunogenic, or substantially non-immunogenic veneered surface.

The process of veneering makes use of the available sequence data for human antibody variable domains compiled by Kabat et al., in Sequences of Proteins of Immunological Interest, 4th ed., (U.S. Dept. of Health and Human Services, U.S. Government Printing Office, 1987), updates to the Kabat database, and other accessible U.S. and foreign databases (both nucleic acid and protein). Solvent accessibilities of V region amino acids can be deduced from the known three-dimensional structure for human and murine antibody fragments. There are two general steps in veneering a murine antigen-binding site. Initially, the FRs of the variable domains of an antibody molecule of interest are compared with corresponding FR sequences of human variable domains obtained from the above-identified sources. The most homologous human V regions are then compared residue by residue to corresponding murine amino acids. The residues in the murine FR which differ from the human counterpart are replaced by the residues present in the human moiety using recombinant techniques well known in the art. Residue switching is only carried out with moieties which are at least partially exposed (solvent accessible), and care is exercised in the replacement of amino acid residues which may have a significant effect on the tertiary structure of V region domains, such as proline, glycine and charged amino acids.

In this manner, the resultant "veneered" murine antigen-binding sites are thus designed to retain the murine CDR residues, the residues substantially adjacent to the CDRs, the residues identified as buried or mostly buried (solvent inaccessible), the residues believed to participate in non-covalent (*e.g*., electrostatic and hydrophobic) contacts between heavy and light chain domains, and the residues from conserved structural regions of the FRs which are believed to influence the "canonical" tertiary structures of the CDR loops. These design criteria are then used to prepare recombinant nucleotide sequences which combine the CDRs of both the heavy and light chain of a murine antigen-binding site into human-appearing FRs that can be used to transfect mammalian cells for the expression of recombinant human antibodies which exhibit the antigen specificity of the murine antibody molecule.

In another embodiment of the invention, monoclonal antibodies of the present invention may be coupled to one or more therapeutic agents. Suitable agents in this regard include radionuclides, differentiation inducers, drugs, toxins, and derivatives thereof. Preferred radionuclides include ⁹⁰Y, ¹²³I, ¹²⁵I, ¹³¹I, ¹⁸⁶Re, ¹⁸⁸Re, ²¹¹At, and ²¹²Bi. Preferred drugs include methotrexate, and pyrimidine and purine analogs. Preferred differentiation inducers include phorbol esters and butyric acid. Preferred toxins include ricin, abrin, diptheria toxin, cholera toxin, gelonin, Pseudomonas exotoxin, Shigella toxin, and pokeweed antiviral protein.

A therapeutic agent may be coupled (*e.g*., covalently bonded) to a suitable monoclonal antibody either directly or indirectly (*e.g*., via a linker group). A direct reaction between an agent and an antibody is possible when each possesses a substituent capable of reacting with the other. For example, a nucleophilic group, such as an amino or sulfhydryl group, on one may be capable of reacting with a carbonyl-containing group, such as an anhydride or an acid halide, or with an alkyl group containing a good leaving group (*e.g*., a halide) on the other.

Alternatively, it may be desirable to couple a therapeutic agent and an antibody via a linker group. A linker group can function as a spacer to distance an antibody from an agent in order to avoid interference with binding capabilities. A linker group can also serve to increase the chemical reactivity of a substituent on an agent or an antibody, and thus increase the coupling efficiency. An increase in chemical reactivity may also facilitate the use of agents, or functional groups on agents, which otherwise would not be possible.

It will be evident to those skilled in the art that a variety of bifunctional or polyfunctional reagents, both homo- and hetero-functional (such as those described in the catalog of the Pierce Chemical Co., Rockford, IL), may be employed as the linker group. Coupling may be effected, for example, through amino groups, carboxyl groups, sulfhydryl groups or oxidized carbohydrate residues. There are numerous references describing such methodology, *e.g*., U.S. Patent No. 4,671,958, to Rodwell et al.

Where a therapeutic agent is more potent when free from the antibody portion of the immunoconjugates of the present invention, it may be desirable to use a linker group which is cleavable during or upon internalization into a cell. A number of different cleavable linker groups have been described. The mechanisms for the intracellular release of an agent from these linker groups include cleavage by reduction of a disulfide bond (*e.g*., U.S. Patent No. 4,489,710, to Spitler), by irradiation of a photolabile bond *(e.g.,* U.S. Patent No. 4,625,014, to Senter et al.), by hydrolysis of derivatized amino acid side chains (*e.g*., U.S. Patent No. 4,638,045, to Kohn et al.), by serum complement-mediated hydrolysis (*e.g*., U.S. Patent No. 4,671,958, to Rodwell et al.), and acid-catalyzed hydrolysis (*e.g*., U.S. Patent No. 4,569,789, to Blattler et al.).

It may be desirable to couple more than one agent to an antibody. In one embodiment, multiple molecules of an agent are coupled to one antibody molecule. In another embodiment, more than one type of agent may be coupled to one antibody. Regardless of the particular embodiment, immunoconjugates with more than one agent may be prepared in a variety of ways. For example, more than one agent may be coupled directly to an antibody molecule, or linkers that provide multiple sites for attachment can be used. Alternatively, a carrier can be used.

A carrier may bear the agents in a variety of ways, including covalent bonding either directly or via a linker group. Suitable carriers include proteins such as albumins (*e.g*., U.S. Patent No. 4,507,234, to Kato et al.), peptides and polysaccharides such as aminodextran (*e.g*., U.S. Patent No. 4,699,784, to Shih et al.). A carrier may also bear an agent by noncovalent bonding or by encapsulation, such as within a liposome vesicle (*e.g*., U.S. Patent Nos. 4,429,008 and 4,873,088). Carriers specific for radionuclide agents include radiohalogenated small molecules and chelating compounds. For example, U.S. Patent No. 4,735,792 discloses representative radiohalogenated small molecules and their synthesis. A radionuclide chelate may be formed from chelating compounds that include those containing nitrogen and sulfur atoms as the donor atoms for binding the metal, or metal oxide, radionuclide. For example, U.S. Patent No. 4,673,562, to Davison et al. discloses representative chelating compounds and their synthesis.

### T Cell Compositions

The present invention, in another aspect, provides T cells specific for a tumor polypeptide disclosed herein, or for a variant or derivative thereof. Such cells may generally be prepared *in vitro* or *ex vivo,* using standard procedures. For example, T cells may be isolated from bone marrow, peripheral blood, or a fraction of bone marrow or peripheral blood of a patient, using a commercially available cell separation system, such as the Isolex™ System, available from Nexell Therapeutics, Inc. (Irvine, CA; see also U.S. Patent No. 5,240,856; U.S. Patent No. 5,215,926; WO 89/06280; WO 91/16116 and WO 92/07243). Alternatively, T cells may be derived from related or unrelated humans, non-human mammals, cell lines or cultures.

T cells may be stimulated with a polypeptide, polynucleotide encoding a polypeptide and/or an antigen presenting cell (APC) that expresses such a polypeptide. Such stimulation is performed under conditions and for a time sufficient to permit the generation of T cells that are specific for the polypeptide of interest. Preferably, a tumor polypeptide or polynucleotide of the invention is present within a delivery vehicle, such as a microsphere, to facilitate the generation of specific T cells.

T cells are considered to be specific for a polypeptide of the present invention if the T cells specifically proliferate, secrete cytokines or kill target cells coated with the polypeptide or expressing a gene encoding the polypeptide. T cell specificity may be evaluated using any of a variety of standard techniques. For example, within a chromium release assay or proliferation assay, a stimulation index of more than two fold increase in lysis and/or proliferation, compared to negative controls, indicates T cell specificity. Such assays may be performed, for example, as described in Chen et al., *Cancer Res.* 54:1065-1070, 1994. Alternatively, detection of the proliferation of T cells may be accomplished by a variety of known techniques. For example, T cell proliferation can be detected by measuring an increased rate of DNA synthesis (*e.g*., by pulse-labeling cultures of T cells with tritiated thymidine and measuring the amount of tritiated thymidine incorporated into DNA). Contact with a tumor polypeptide (100 ng/ml - 100 µg/ml, preferably 200 ng/ml - 25 µg/ml) for 3 - 7 days will typically result in at least a two fold increase in proliferation of the T cells. Contact as described above for 2-3 hours should result in activation of the T cells, as measured using standard cytokine assays in which a two fold increase in the level of cytokine release *(e.g.,* TNF or IFN-γ) is indicative of T cell activation *(see* Coligan et al., Current Protocols in Immunology, vol. 1, Wiley Interscience (Greene 1998)). T cells that have been activated in response to a tumor polypeptide, polynucleotide or polypeptide-expressing APC may be CD4⁺ and/or CD8⁺. Tumor polypeptide-specific T cells may be expanded using standard techniques. Within preferred embodiments, the T cells are derived from a patient, a related donor or an unrelated donor, and are administered to the patient following stimulation and expansion.

For therapeutic purposes, CD4⁺ or CD8⁺ T cells that proliferate in response to a tumor polypeptide, polynucleotide or APC can be expanded in number either *in vitro* or *in vivo.* Proliferation of such T cells *in vitro* may be accomplished in a variety of ways. For example, the T cells can be re-exposed to a tumor polypeptide, or a short peptide corresponding to an immunogenic portion of such a polypeptide, with or without the addition of T cell growth factors, such as interleukin-2, and/or stimulator cells that synthesize a tumor polypeptide. Alternatively, one or more T cells that proliferate in the presence of the tumor polypeptide can be expanded in number by cloning. Methods for cloning cells are well known in the art, and include limiting dilution.

### Pharmaceutical Compositions

In additional embodiments, the present invention concerns formulation of one or more of the polynucleotide, polypeptide, T-cell and/or antibody compositions disclosed herein in pharmaceutically-acceptable carriers for administration to a cell or an animal, either alone, or in combination with one or more other modalities of therapy.

It will be understood that, if desired, a composition as disclosed herein may be administered in combination with other agents as well, such as, e.g., other proteins or polypeptides or various pharmaceutically-active agents. In fact, there is virtually no limit to other components that may also be included, given that the additional agents do not cause a significant adverse effect upon contact with the target cells or host tissues. The compositions may thus be delivered along with various other agents as required in the particular instance. Such compositions may be purified from host cells or other biological sources, or alternatively may be chemically synthesized as described herein. Likewise, such compositions may further comprise substituted or derivatized RNA or DNA compositions.

Therefore, in another aspect of the present invention, pharmaceutical compositions are provided comprising one or more of the polynucleotide, polypeptide, antibody, and/or T-cell compositions described herein in combination with a physiologically acceptable carrier. In certain preferred embodiments, the pharmaceutical compositions of the invention comprise immunogenic polynucleotide and/or polypeptide compositions of the invention for use in prophylactic and theraputic vaccine applications. Vaccine preparation is generally described in, for example, M.F. Powell and M.J. Newman, eds., "Vaccine Design (the subunit and adjuvant approach)," Plenum Press (NY, 1995). Generally, such compositions will comprise one or more polynucleotide and/or polypeptide compositions of the present invention in combination with one or more immunostimulants.

It will be apparent that any of the pharmaceutical compositions described herein can contain pharmaceutically acceptable salts of the polynucleotides and polypeptides of the invention. Such salts can be prepared, for example, from pharmaceutically acceptable non-toxic bases, including organic bases (*e.g*., salts of primary, secondary and tertiary amines and basic amino acids) and inorganic bases *(e.g.,* sodium, potassium, lithium, ammonium, calcium and magnesium salts).

In another embodiment, illustrative immunogenic compositions, e.g., vaccine compositions, of the present invention comprise DNA encoding one or more of the polypeptides as described above, such that the polypeptide is generated *in situ.* As noted above, the polynucleotide may be administered within any of a variety of delivery systems known to those of ordinary skill in the art. Indeed, numerous gene delivery techniques are well known in the art, such as those described by Rolland, *Crit. Rev. Therap. Drug Carrier Systems 15*:143-198, 1998, and references cited therein. Appropriate polynucleotide expression systems will, of course, contain the necessary regulatory DNA regulatory sequences for expression in a patient (such as a suitable promoter and terminating signal). Alternatively, bacterial delivery systems may involve the administration of a bacterium (such as *Bacillus-Calmette-Guerrin)* that expresses an immunogenic portion of the polypeptide on its cell surface or secretes such an epitope.

Therefore, in certain embodiments, polynucleotides encoding immunogenic polypeptides described herein are introduced into suitable mammalian host cells for expression using any of a number of known viral-based systems. In one illustrative embodiment, retroviruses provide a convenient and effective platform for gene delivery systems. A selected nucleotide sequence encoding a polypeptide of the present invention can be inserted into a vector and packaged in retroviral particles using techniques known in the art. The recombinant virus can then be isolated and delivered to a subject. A number of illustrative retroviral systems have been described (*e.g*., U.S. Pat. No. 5,219,740; Miller and Rosman (1989) BioTechniques 7:980-990; Miller, A. D. (1990) Human Gene Therapy 1:5-14; Scarpa et al. (1991) Virology 180:849-852; Bums et al. (1993) Proc. Natl. Acad. Sci. USA 90:8033-8037; and Boris-Lawrie and Temin (1993) Cur. Opin. Genet. Develop. 3:102-109.

In addition, a number of illustrative adenovirus-based systems have also been described. Unlike retroviruses which integrate into the host genome, adenoviruses persist extrachromosomally thus minimizing the risks associated with insertional mutagenesis (Haj-Ahmad and Graham (1986) J. Virol. 57:267-274; Bett et al. (1993) J. Virol. 67:5911-5921; Mittereder et al. (1994) Human Gene Therapy 5:717-729; Seth et al. (1994) J. Virol. 68:933-940; Barr et al. (1994) Gene Therapy 1:51-58; Berkner, K. L. (1988) BioTechniques 6:616-629; and Rich et al. (1993) Human Gene Therapy 4:461-476).

Various adeno-associated virus (AAV) vector systems have also been developed for polynucleotide delivery. AAV vectors can be readily constructed using techniques well known in the art. See, *e.g*., U.S. Pat. Nos. 5,173,414 and 5,139,941; International Publication Nos. WO 92/01070 and WO 93/03769; Lebkowski et al. (1988) Molec. Cell. Biol. 8:3988-3996; Vincent et al. (1990) Vaccines 90 (Cold Spring Harbor Laboratory Press); Carter, B. J. (1992) Current Opinion in Biotechnology 3:533-539; Muzyczka, N. (1992) Current Topics in Microbiol. and Immunol. 158:97-129; Kotin, R. M. (1994) Human Gene Therapy 5:793-801; Shelling and Smith (1994) Gene Therapy 1:165-169; and Zhou et al. (1994) J. Exp. Med. 179:1867-1875.

Additional viral vectors useful for delivering the polynucleotides encoding polypeptides of the present invention by gene transfer include those derived from the pox family of viruses, such as vaccinia virus and avian poxvirus. By way of example, vaccinia virus recombinants expressing the novel molecules can be constructed as follows. The DNA encoding a polypeptide is first inserted into an appropriate vector so that it is adjacent to a vaccinia promoter and flanking vaccinia DNA sequences, such as the sequence encoding thymidine kinase (TK). This vector is then used to transfect cells which are simultaneously infected with vaccinia. Homologous recombination serves to insert the vaccinia promoter plus the gene encoding the polypeptide of interest into the viral genome. The resulting TK.sup.(-) recombinant can be selected by culturing the cells in the presence of 5-bromodeoxyuridine and picking viral plaques resistant thereto.

A vaccinia-based infection/transfection system can be conveniently used to provide for inducible, transient expression or coexpression of one or more polypeptides described herein in host cells of an organism. In this particular system, cells are first infected in vitro with a vaccinia virus recombinant that encodes the bacteriophage T7 RNA polymerase. This polymerase displays exquisite specificity in that it only transcribes templates bearing T7 promoters. Following infection, cells are transfected with the polynucleotide or polynucleotides of interest, driven by a T7 promoter. The polymerase expressed in the cytoplasm from the vaccinia virus recombinant transcribes the transfected DNA into RNA which is then translated into polypeptide by the host translational machinery. The method provides for high level, transient, cytoplasmic production of large quantities of RNA and its translation products. See, *e.g.,* Elroy-Stein and Moss, Proc. Natl. Acad. Sci. USA (1990) 87:6743-6747; Fuerst et al. Proc. Natl. Acad. Sci. USA (1986) 83:8122-8126.

Alternatively, avipoxviruses, such as the fowlpox and canarypox viruses, can also be used to deliver the coding sequences of interest. Recombinant avipox viruses, expressing immunogens from mammalian pathogens, are known to confer protective immunity when administered to non-avian species. The use of an Avipox vector is particularly desirable in human and other mammalian species since members of the Avipox genus can only productively replicate in susceptible avian species and therefore are not infective in mammalian cells. Methods for producing recombinant Avipoxviruses are known in the art and employ genetic recombination, as described above with respect to the production of vaccinia viruses. See, *e.g*., WO 91/12882; WO 89/03429; and WO 92/03545.

Any of a number of alphavirus vectors can also be used for delivery of polynucleotide compositions of the present invention, such as those vectors described in U.S. Patent Nos. 5,843,723; 6,015,686; 6,008,035 and 6,015,694. Certain vectors based on Venezuelan Equine Encephalitis (VEE) can also be used, illustrative examples of which can be found in U.S. Patent Nos. 5,505,947 and 5,643,576.

Moreover, molecular conjugate vectors, such as the adenovirus chimeric vectors described in Michael et al. J. Biol. Chem. (1993) 268:6866-6869 and Wagner et al. Proc. Natl. Acad. Sci. USA (1992) 89:6099-6103, can also be used for gene delivery under the invention.

Additional illustrative information on these and other known viral-based delivery systems can be found, for example, in Fisher-Hoch et al., *Proc. Natl. Acad. Sci. USA* 86:317-321, 1989; Flexner et al., *Ann. N.Y. Acad. Sci. 569*:86-103, 1989; Flexner et al., *Vaccine* 8:17-21, 1990; U.S. Patent Nos. 4,603,112, 4,769,330, and 5,017,487; WO 89/01973; U.S. Patent No. 4,777,127; GB 2,200,651; EP 0,345,242; WO 91/02805; Berkner, *Biotechniques* 6:616-627, 1988; Rosenfeld et al., *Science 252*:431-434, 1991; Kolls et al., *Proc. Natl. Acad. Sci. USA* 91:215-219, 1994; Kass-Eisler et al., *Proc. Natl. Acad. Sci. USA 90:*11498-11502, 1993; Guzman et al., *Circulation* 88:2838-2848, 1993; and Guzman et al., *Cir. Res.* 73:1202-1207, 1993.

In certain embodiments, a polynucleotide may be integrated into the genome of a target cell. This integration may be in the specific location and orientation *via* homologous recombination (gene replacement) or it may be integrated in a random, non-specific location (gene augmentation). In yet further embodiments, the polynucleotide may be stably maintained in the cell as a separate, episomal segment of DNA. Such polynucleotide segments or "episomes" encode sequences sufficient to permit maintenance and replication independent of or in synchronization with the host cell cycle. The manner in which the expression construct is delivered to a cell and where in the cell the polynucleotide remains is dependent on the type of expression construct employed.

In another embodiment of the invention, a polynucleotide is administered/delivered as "naked" DNA, for example as described in Ulmer et al., *Science 259*:1745-1749, 1993 and reviewed by Cohen, *Science* 259:1691-1692, 1993. The uptake of naked DNA may be increased by coating the DNA onto biodegradable beads, which are efficiently transported into the cells.

In still another embodiment, a composition of the present invention can be delivered via a particle bombardment approach, many of which have been described. In one illustrative example, gas-driven particle acceleration can be achieved with devices such as those manufactured by Powderject Pharmaceuticals PLC (Oxford, UK) and Powderject Vaccines Inc. (Madison, WI), some examples of which are described in U.S. Patent Nos. 5,846,796; 6,010,478; 5,865,796; 5,584,807; and EP Patent No. 0500 799. This approach offers a needle-free delivery approach wherein a dry powder formulation of microscopic particles, such as polynucleotide or polypeptide particles, are accelerated to high speed within a helium gas jet generated by a hand held device, propelling the particles into a target tissue of interest.

In a related embodiment, other devices and methods that may be useful for gas-driven needle-less injection of compositions of the present invention include those provided by Bioject, Inc. (Portland, OR), some examples of which are described in U.S. Patent Nos. 4,790,824; 5,064,413; 5,312,335; 5,383,851; 5,399,163; 5,520,639 and 5,993,412.

According to another embodiment, the pharmaceutical compositions described herein will comprise one or more immunostimulants in addition to the immunogenic polynucleotide, polypeptide, antibody, T-cell and/or APC compositions of this invention. An immunostimulant refers to essentially any substance that enhances or potentiates an immune response (antibody and/or cell-mediated) to an exogenous antigen. One preferred type of immunostimulant comprises an adjuvant. Many adjuvants contain a substance designed to protect the antigen from rapid catabolism, such as aluminum hydroxide or mineral oil, and a stimulator of immune responses, such as lipid A, *Bortadella pertussis* or *Mycobacterium tuberculosis* derived proteins. Certain adjuvants are commercially available as, for example, Freund's Incomplete Adjuvant and Complete Adjuvant (Difco Laboratories, Detroit, MI); Merck Adjuvant 65 (Merck and Company, Inc., Rahway, NJ); AS-2 (SmithKline Beecham, Philadelphia, PA); aluminum salts such as aluminum hydroxide gel (alum) or aluminum phosphate; salts of calcium, iron or zinc; an insoluble suspension of acylated tyrosine; acylated sugars; cationically or anionically derivatized polysaccharides; polyphosphazenes; biodegradable microspheres; monophosphoryl lipid A and quil A. Cytokines, such as GM-CSF, interleukin-2, -7, -12, and other like growth factors, may also be used as adjuvants.

Within certain embodiments of the invention, the adjuvant composition is preferably one that induces an immune response predominantly of the Th1 type. High levels of Th1-type cytokines (*e.g*., IFN-γ, TNFα, IL-2 and IL-12) tend to favor the induction of cell mediated immune responses to an administered antigen. In contrast, high levels of Th2-type cytokines (*e.g*., IL-4, IL-5, IL-6 and IL-10) tend to favor the induction of humoral immune responses. Following application of a vaccine as provided herein, a patient will support an immune response that includes Th1- and Th2- type responses. Within a preferred embodiment, in which a response is predominantly Th1-type, the level of Th1-type cytokines will increase to a greater extent than the level of Th2-type cytokines. The levels of these cytokines may be readily assessed using standard assays. For a review of the families of cytokines, see Mosmann and Coffman, *Ann. Rev. Immunol.* 7:145-173, 1989.

Certain preferred adjuvants for eliciting a predominantly Th1-type response include, for example, a combination of monophosphoryl lipid A, preferably 3-de-O-acylated monophosphoryl lipid A, together with an aluminum salt. MPL® adjuvants are available from Corixa Corporation (Seattle, WA; *see,* for example, US Patent Nos. 4,436,727; 4,877,611; 4,866,034 and 4,912,094). CpG-containing oligonucleotides (in which the CpG dinucleotide is unmethylated) also induce a predominantly Th1 response. Such oligonucleotides are well known and are described, for example, in WO 96/02555, WO 99/33488 and U.S. Patent Nos. 6,008,200 and 5,856,462. Immunostimulatory DNA sequences are also described, for example, by Sato et al., *Science* 273:352, 1996. Another preferred adjuvant comprises a saponin, such as Quil A, or derivatives thereof, including QS21 and QS7 (Aquila Biopharmaceuticals Inc., Framingham, MA); Escin; Digitonin; or *Gypsophila* or *Chenopodium quinoa* saponins . Other preferred formulations include more than one saponin in the adjuvant combinations of the present invention, for example combinations of at least two of the following group comprising QS21, QS7, Quil A, β-escin, or digitonin.

Alternatively the saponin formulations may be combined with vaccine vehicles composed of chitosan or other polycationic polymers, polylactide and polylactide-co-glycolide particles, poly-N-acetyl glucosamine-based polymer matrix, particles composed of polysaccharides or chemically modified polysaccharides, liposomes and lipid-based particles, particles composed of glycerol monoesters, etc. The saponins may also be formulated in the presence of cholesterol to form particulate structures such as liposomes or ISCOMs. Furthermore, the saponins may be formulated together with a polyoxyethylene ether or ester, in either a non-particulate solution or suspension, or in a particulate structure such as a paucilamelar liposome or ISCOM. The saponins may also be formulated with excipients such as Carbopol^{R} to increase viscosity, or may be formulated in a dry powder form with a powder excipient such as lactose.

In one preferred embodiment, the adjuvant system includes the combination of a monophosphoryl lipid A and a saponin derivative, such as the combination of QS21 and 3D-MPL® adjuvant, as described in WO 94/00153, or a less reactogenic composition where the QS21 is quenched with cholesterol, as described in WO 96/33739. Other preferred formulations comprise an oil-in-water emulsion and tocopherol. Another particularly preferred adjuvant formulation employing QS21, 3D-MPL® adjuvant and tocopherol in an oil-in-water emulsion is described in WO 95/17210.

Another enhanced adjuvant system involves the combination of a CpG-containing oligonucleotide and a saponin derivative particularly the combination of CpG and QS21 is disclosed in WO 00/09159. Preferably the formulation additionally comprises an oil in water emulsion and tocopherol.

Additional illustrative adjuvants for use in the pharmaceutical compositions of the invention include Montanide ISA 720 (Seppic, France), SAF (Chiron, California, United States), ISCOMS (CSL), MF-59 (Chiron), the SBAS series of adjuvants (*e.g*., SBAS-2 or SBAS-4, available from SmithKline Beecham, Rixensart, Belgium), Detox (Enhanzyn® ) (Corixa, Hamilton, MT), RC-529 (Corixa, Hamilton, MT) and other aminoalkyl glucosaminide 4-phosphates (AGPs), such as those described in pending U.S. Patent Application Serial Nos. 08/853,826 and 09/074,720, the disclosures of which are incorporated herein by reference in their entireties, and polyoxyethylene ether adjuvants such as those described in WO 99/52549A1.

Other preferred adjuvants include adjuvant molecules of the general formula

(I): HO(CH₂CH₂O)ₙ-A-R,

wherein, n is 1-50, A is a bond or-C(O)-, R is C₁₋₅₀ alkyl or Phenyl C₁₋₅₀ alkyl.

One embodiment of the present invention consists of a vaccine formulation comprising a polyoxyethylene ether of general formula (I), wherein n is between 1 and 50, preferably 4-24, most preferably 9; the *R* component is C₁₋₅₀, preferably C₄-C₂₀ alkyl and most preferably C₁₂ alkyl, and *A* is a bond. The concentration of the polyoxyethylene ethers should be in the range 0.1-20%, preferably from 0.1-10%, and most preferably in the range 0.1-1%. Preferred polyoxyethylene ethers are selected from the following group: polyoxyethylene-9-lauryl ether, polyoxyethylene-9-steoryl ether, polyoxyethylene-8-steoryl ether, polyoxyethylene-4-lauryl ether, polyoxyethylene-35-lauryl ether, and polyoxyethylene-23-lauryl ether. Polyoxyethylene ethers such as polyoxyethylene lauryl ether are described in the Merck index (12^{th} edition: entry 7717). These adjuvant molecules are described in WO 99/52549.

The polyoxyethylene ether according to the general formula (I) above may, if desired, be combined with another adjuvant. For example, a preferred adjuvant combination is preferably with CpG as described in the pending UK patent application GB 9820956.2.

According to another embodiment of this invention, an immunogenic composition described herein is delivered to a host via antigen presenting cells (APCs), such as dendritic cells, macrophages, B cells, monocytes and other cells that may be engineered to be efficient APCs. Such cells may, but need not, be genetically modified to increase the capacity for presenting the antigen, to improve activation and/or maintenance of the T cell response, to have anti-tumor effects *per se* and/or to be immunologically compatible with the receiver *(i.e.,* matched HLA haplotype). APCs may generally be isolated from any of a variety of biological fluids and organs, including tumor and peritumoral tissues, and may be autologous, allogeneic, syngeneic or xenogeneic cells.

Certain preferred embodiments of the present invention use dendritic cells or progenitors thereof as antigen-presenting cells. Dendritic cells are highly potent APCs (Banchereau and Steinman, *Nature 392*:245-251, 1998) and have been shown to be effective as a physiological adjuvant for eliciting prophylactic or therapeutic antitumor immunity *(see* Timmerman and Levy, *Ann. Rev. Med.* 50:507-529, 1999). In general, dendritic cells may be identified based on their typical shape (stellate *in situ,* with marked cytoplasmic processes (dendrites) visible *in vitro),* their ability to take up, process and present antigens with high efficiency and their ability to activate naïve T cell responses. Dendritic cells may, of course, be engineered to express specific cell-surface receptors or ligands that are not commonly found on dendritic cells *in vivo* or *ex vivo,* and such modified dendritic cells are contemplated by the present invention. As an alternative to dendritic cells, secreted vesicles antigen-loaded dendritic cells (called exosomes) may be used within a vaccine *(see* Zitvogel et al., *Nature Med. 4*:594-600, 1998).

Dendritic cells and progenitors may be obtained from peripheral blood, bone marrow, tumor-infiltrating cells, peritumoral tissues-infiltrating cells, lymph nodes, spleen, skin, umbilical cord blood or any other suitable tissue or fluid. For example, dendritic cells may be differentiated *ex vivo* by adding a combination of cytokines such as GM-CSF, IL-4, IL-13 and/or TNFα to cultures of monocytes harvested from peripheral blood. Alternatively, CD34 positive cells harvested from peripheral blood, umbilical cord blood or bone marrow may be differentiated into dendritic cells by adding to the culture medium combinations of GM-CSF, IL-3, TNFα, CD40 ligand, LPS, flt3 ligand and/or other compound(s) that induce differentiation, maturation and proliferation of dendritic cells.

Dendritic cells are conveniently categorized as "immature" and "mature" cells, which allows a simple way to discriminate between two well characterized phenotypes. However, this nomenclature should not be construed to exclude all possible intermediate stages of differentiation. Immature dendritic cells are characterized as APC with a high capacity for antigen uptake and processing, which correlates with the high expression of Fcγ receptor and mannose receptor. The mature phenotype is typically characterized by a lower expression of these markers, but a high expression of cell surface molecules responsible for T cell activation such as class I and class II MHC, adhesion molecules (*e.g*., CD54 and CD 11) and costimulatory molecules (*e.g*., CD40, CD80, CD86 and 4-1BB).

APCs may generally be transfected with a polynucleotide of the invention (or portion or other variant thereof) such that the encoded polypeptide, or an immunogenic portion thereof, is expressed on the cell surface. Such transfection may take place *ex vivo,* and a pharmaceutical composition comprising such transfected cells may then be used for therapeutic purposes, as described herein. Alternatively, a gene delivery vehicle that targets a dendritic or other antigen presenting cell may be administered to a patient, resulting in transfection that occurs *in vivo. In vivo* and *ex vivo* transfection of dendritic cells, for example, may generally be performed using any methods known in the art, such as those described in WO 97/24447, or the gene gun approach described by Mahvi et al., *Immunology and cell Biology* 75:456-460, 1997. Antigen loading of dendritic cells may be achieved by incubating dendritic cells or progenitor cells with the tumor polypeptide, DNA (naked or within a plasmid vector) or RNA; or with antigen-expressing recombinant bacterium or viruses (*e.g*., vaccinia, fowlpox, adenovirus or lentivirus vectors). Prior to loading, the polypeptide may be covalently conjugated to an immunological partner that provides T cell help *(e.g.,* a carrier molecule). Alternatively, a dendritic cell may be pulsed with a non-conjugated immunological partner, separately or in the presence of the polypeptide.

While any suitable carrier known to those of ordinary skill in the art may be employed in the pharmaceutical compositions of this invention, the type of carrier will typically vary depending on the mode of administration. Compositions of the present invention may be formulated for any appropriate manner of administration, including for example, topical, oral, nasal, mucosal, intravenous, intracranial, intraperitoneal, subcutaneous and intramuscular administration.

Carriers for use within such pharmaceutical compositions are biocompatible, and may also be biodegradable. In certain embodiments, the formulation preferably provides a relatively constant level of active component release. In other embodiments, however, a more rapid rate of release immediately upon administration may be desired. The formulation of such compositions is well within the level of ordinary skill in the art using known techniques. Illustrative carriers useful in this regard include microparticles of poly(lactide-co-glycolide), polyacrylate, latex, starch, cellulose, dextran and the like. Other illustrative delayed-release carriers include supramolecular biovectors, which comprise a non-liquid hydrophilic core (e.g., a cross-linked polysaccharide or oligosaccharide) and, optionally, an external layer comprising an amphiphilic compound, such as a phospholipid *(see e.g.,* U.S. Patent No. 5,151,254 and PCT applications WO 94/20078, WO/94/23701 and WO 96/06638). The amount of active compound contained within a sustained release formulation depends upon the site of implantation, the rate and expected duration of release and the nature of the condition to be treated or prevented.

In another illustrative embodiment, biodegradable microspheres (*e.g*., polylactate polyglycolate) are employed as carriers for the compositions of this invention. Suitable biodegradable microspheres are disclosed, for example, in U.S. Patent Nos. 4,897,268; 5,075,109; 5,928,647; 5,811,128; 5,820,883; 5,853,763; 5,814,344, 5,407,609 and 5,942,252. Modified hepatitis B core protein carrier systems. such as described in WO/99 40934, and references cited therein, will also be useful for many applications. Another illustrative carrier/delivery system employs a carrier comprising particulate-protein complexes, such as those described in U.S. Patent No. 5,928,647, which are capable of inducing a class I-restricted cytotoxic T lymphocyte responses in a host.

The pharmaceutical compositions of the invention will often further comprise one or more buffers (*e.g*., neutral buffered saline or phosphate buffered saline), carbohydrates (*e.g*., glucose, mannose, sucrose or dextrans), mannitol, proteins, polypeptides or amino acids such as glycine, antioxidants, bacteriostats, chelating agents such as EDTA or glutathione, adjuvants (*e.g*., aluminum hydroxide), solutes that render the formulation isotonic, hypotonic or weakly hypertonic with the blood of a recipient, suspending agents, thickening agents and/or preservatives. Alternatively, compositions of the present invention may be formulated as a lyophilizate.

The pharmaceutical compositions described herein may be presented in unit-dose or multi-dose containers, such as sealed ampoules or vials. Such containers are typically sealed in such a way to preserve the sterility and stability of the formulation until use. In general, formulations may be stored as suspensions, solutions or emulsions in oily or aqueous vehicles. Alternatively, a pharmaceutical composition may be stored in a freeze-dried condition requiring only the addition of a sterile liquid carrier immediately prior to use.

The development of suitable dosing and treatment regimens for using the particular compositions described herein in a variety of treatment regimens, including *e.g*., oral, parenteral, intravenous, intranasal, and intramuscular administration and formulation, is well known in the art, some of which are briefly discussed below for general purposes of illustration.

In certain applications, the pharmaceutical compositions disclosed herein may be delivered *via* oral administration to an animal. As such, these compositions may be formulated with an inert diluent or with an assimilable edible carrier, or they may be enclosed in hard- or soft-shell gelatin capsule, or they may be compressed into tablets, or they may be incorporated directly with the food of the diet.

The active compounds may even be incorporated with excipients and used in the form of ingestible tablets, buccal tables, troches, capsules, elixirs, suspensions, syrups, wafers, and the like (see, for example, Mathiowitz *et al.,* Nature 1997 Mar 27;386(6623):410-4; Hwang *et al.,* Crit Rev Ther Drug Carrier Syst 1998;15(3):243-84; U. S. Patent 5,641,515; U. S. Patent 5,580,579 and U. S. Patent 5,792,451). Tablets, troches, pills, capsules and the like may also contain any of a variety of additional components, for example, a binder, such as gum tragacanth, acacia, cornstarch, or gelatin; excipients, such as dicalcium phosphate; a disintegrating agent, such as corn starch, potato starch, alginic acid and the like; a lubricant, such as magnesium stearate; and a sweetening agent, such as sucrose, lactose or saccharin may be added or a flavoring agent, such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar, or both. Of course, any material used in preparing any dosage unit form should be pharmaceutically pure and substantially non-toxic in the amounts employed. In addition, the active compounds may be incorporated into sustained-release preparation and formulations.

Typically, these formulations will contain at least about 0.1% of the active compound or more, although the percentage of the active ingredient(s) may, of course, be varied and may conveniently be between about 1 or 2% and about 60% or 70% or more of the weight or volume of the total formulation. Naturally, the amount of active compound(s) in each therapeutically useful composition may be prepared is such a way that a suitable dosage will be obtained in any given unit dose of the compound. Factors such as solubility, bioavailability, biological half-life, route of administration, product shelf life, as well as other pharmacological considerations will be contemplated by one skilled in the art of preparing such pharmaceutical formulations, and as such, a variety of dosages and treatment regimens may be desirable.

For oral administration the compositions of the present invention may alternatively be incorporated with one or more excipients in the form of a mouthwash, dentifrice, buccal tablet, oral spray, or sublingual orally-administered formulation. Alternatively, the active ingredient may be incorporated into an oral solution such as one containing sodium borate, glycerin and potassium bicarbonate, or dispersed in a dentifrice, or added in a therapeutically-effective amount to a composition that may include water, binders, abrasives, flavoring agents, foaming agents, and humectants. Alternatively the compositions may be fashioned into a tablet or solution form that may be placed under the tongue or otherwise dissolved in the mouth.

In certain circumstances it will be desirable to deliver the pharmaceutical compositions disclosed herein parenterally, intravenously, intramuscularly, or even intraperitoneally. Such approaches are well known to the skilled artisan, some of which are further described, for example, in U. S. Patent 5,543,158; U. S. Patent 5,641,515 and U. S. Patent 5,399,363. In certain embodiments, solutions of the active compounds as free base or pharmacologically acceptable salts may be prepared in water suitably mixed with a surfactant, such as hydroxypropylcellulose. Dispersions may also be prepared in glycerol, liquid polyethylene glycols, and mixtures thereof and in oils. Under ordinary conditions of storage and use, these preparations generally will contain a preservative to prevent the growth of microorganisms.

Illustrative pharmaceutical forms suitable for injectable use include sterile aqueous solutions or dispersions and sterile powders for the extemporaneous preparation of sterile injectable solutions or dispersions (for example, see U. S. Patent 5,466,468). In all cases the form must be sterile and must be fluid to the extent that easy syringability exists. It must be stable under the conditions of manufacture and storage and must be preserved against the contaminating action of microorganisms, such as bacteria and fungi. The carrier can be a solvent or dispersion medium containing, for example, water, ethanol, polyol (*e.g*., glycerol, propylene glycol, and liquid polyethylene glycol, and the like), suitable mixtures thereof, and/or vegetable oils. Proper fluidity may be maintained, for example, by the use of a coating, such as lecithin, by the maintenance of the required particle size in the case of dispersion and/or by the use of surfactants. The prevention of the action of microorganisms can be facilitated by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

In one embodiment, for parenteral administration in an aqueous solution, the solution should be suitably buffered if necessary and the liquid diluent first rendered isotonic with sufficient saline or glucose. These particular aqueous solutions are especially suitable for intravenous, intramuscular, subcutaneous and intraperitoneal administration. In this connection, a sterile aqueous medium that can be employed will be known to those of skill in the art in light of the present disclosure. For example, one dosage may be dissolved in 1 ml of isotonic NaCl solution and either added to 1000 ml of hypodermoclysis fluid or injected at the proposed site of infusion, (see for example, "Remington's Pharmaceutical Sciences" 15th Edition, pages 1035-1038 and 1570-1580). Some variation in dosage will necessarily occur depending on the condition of the subject being treated. Moreover, for human administration, preparations will of course preferably meet sterility, pyrogenicity, and the general safety and purity standards as required by FDA Office of Biologics standards.

In another embodiment of the invention, the compositions disclosed herein may be formulated in a neutral or salt form. Illustrative pharmaceutically-acceptable salts include the acid addition salts (formed with the free amino groups of the protein) and which are formed with inorganic acids such as, for example, hydrochloric or phosphoric acids, or such organic acids as acetic, oxalic, tartaric, mandelic, and the like. Salts formed with the free carboxyl groups can also be derived from inorganic bases such as, for example, sodium, potassium, ammonium, calcium, or ferric hydroxides, and such organic bases as isopropylamine, trimethylamine, histidine, procaine and the like. Upon formulation, solutions will be administered in a manner compatible with the dosage formulation and in such amount as is therapeutically effective.

The carriers can further comprise any and all solvents, dispersion media, vehicles, coatings, diluents, antibacterial and antifungal agents, isotonic and absorption delaying agents, buffers, carrier solutions, suspensions, colloids, and the like. The use of such media and agents for pharmaceutical active substances is well known in the art. Except insofar as any conventional media or agent is incompatible with the active ingredient, its use in the therapeutic compositions is contemplated. Supplementary active ingredients can also be incorporated into the compositions. The phrase "pharmaceutically-acceptable" refers to molecular entities and compositions that do not produce an allergic or similar untoward reaction when administered to a human.

In certain embodiments, the pharmaceutical compositions may be delivered by intranasal sprays, inhalation, and/or other aerosol delivery vehicles. Methods for delivering genes, nucleic acids, and peptide compositions directly to the lungs *via* nasal aerosol sprays has been described, *e.g.,* in U. S. Patent 5,756,353 and U. S. Patent 5,804,212. Likewise, the delivery of drugs using intranasal microparticle resins (Takenaga *et al.,* J Controlled Release 1998 Mar 2;52(1-2):81-7) and lysophosphatidyl-glycerol compounds (U. S. Patent 5,725,871) are also well-known in the pharmaceutical arts. Likewise, illustrative transmucosal drug delivery in the form of a polytetrafluoroetheylene support matrix is described in U. S. Patent 5,780,045.

In certain embodiments, liposomes, nanocapsules, microparticles, lipid particles, vesicles, and the like, are used for the introduction of the compositions of the present invention into suitable host cells/organisms. In particular, the compositions of the present invention may be formulated for delivery either encapsulated in a lipid particle, a liposome, a vesicle, a nanosphere, or a nanoparticle or the like. Alternatively, compositions of the present invention can be bound, either covalently or non-covalently, to the surface of such carrier vehicles.

The formation and use of liposome and liposome-like preparations as potential drug carriers is generally known to those of skill in the art (see for example, Lasic, Trends Biotechnol 1998 Jul;16(7):307-21; Takakura, Nippon Rinsho 1998 Mar;56(3):691-5; Chandran *et al.,* Indian J Exp Biol. 1997 Aug;35(8):801-9; Margalit, Crit Rev Ther Drug Carrier Syst. 1995;12(2-3):233-61; U.S. Patent 5,567,434; U.S. Patent 5,552,157; U.S. Patent 5,565,213; U.S. Patent 5,738,868 and U.S. Patent 5,795,587, each specifically incorporated herein by reference in its entirety).

Liposomes have been used successfully with a number of cell types that are normally difficult to transfect by other procedures, including T cell suspensions, primary hepatocyte cultures and PC 12 cells (Renneisen *et al.,* J Biol Chem. 1990 Sep 25;265(27):16337-42; Muller *et al.,* DNA Cell Biol. 1990 Apr;9(3):221-9). In addition, liposomes are free of the DNA length constraints that are typical of viral-based delivery systems. Liposomes have been used effectively to introduce genes, various drugs, radiotherapeutic agents, enzymes, viruses, transcription factors, allosteric effectors and the like, into a variety of cultured cell lines and animals. Furthermore, he use of liposomes does not appear to be associated with autoimmune responses or unacceptable toxicity after systemic delivery.

In certain embodiments, liposomes are formed from phospholipids that are dispersed in an aqueous medium and spontaneously form multilamellar concentric bilayer vesicles (also termed multilamellar vesicles (MLVs).

Alternatively, in other embodiments, the invention provides for pharmaceutically-acceptable nanocapsule formulations of the compositions of the present invention. Nanocapsules can generally entrap compounds in a stable and reproducible way (see, for example, Quintanar-Guerrero *et al.,* Drug Dev Ind Pharm. 1998 Dec;24(12):1113-28). To avoid side effects due to intracellular polymeric overloading, such ultrafine particles (sized around 0.1 µm) may be designed using polymers able to be degraded *in vivo.* Such particles can be made as described, for example, by Couvreur *et al.,* Crit Rev Ther Drug Carrier Syst. 1988;5(1):1-20; zur Muhlen *et al.,* Eur J Pharm Biopharm. 1998 Mar;45(2):149-55; Zambaux *et al.* J Controlled Release. 1998 Jan 2;50(1-3):31-40; and U. S. Patent 5,145,684.

### Cancer Therapeutic Methods

In further aspects of the present invention, the pharmaceutical compositions described herein may be used for the treatment of cancer, particularly for the immunotherapy of breast cancer. Within such methods, the pharmaceutical compositions described herein are administered to a patient, typically a warm-blooded animal, preferably a human. A patient may or may not be afflicted with cancer. Accordingly, the above pharmaceutical compositions may be used to prevent the development of a cancer or to treat a patient afflicted with a cancer. Pharmaceutical compositions and vaccines may be administered either prior to or following surgical removal of primary tumors and/or treatment such as administration of radiotherapy or conventional chemotherapeutic drugs. As discussed above, administration of the pharmaceutical compositions may be by any suitable method, including administration by intravenous, intraperitoneal, intramuscular, subcutaneous, intranasal, intradermal, anal, vaginal, topical and oral routes.

Within certain embodiments, immunotherapy may be active immunotherapy, in which treatment relies on the *in vivo* stimulation of the endogenous host immune system to react against tumors with the administration of immune response-modifying agents (such as polypeptides and polynucleotides as provided herein).

Within other embodiments, immunotherapy may be passive immunotherapy, in which treatment involves the delivery of agents with established tumor-immune reactivity (such as effector cells or antibodies) that can directly or indirectly mediate antitumor effects and does not necessarily depend on an intact host immune system. Examples of effector cells include T cells as discussed above, T lymphocytes (such as CD8⁺ cytotoxic T lymphocytes and CD4⁺ T-helper tumor-infiltrating lymphocytes), killer cells (such as Natural Killer cells and lymphokine-activated killer cells), B cells and antigen-presenting cells (such as dendritic cells and macrophages) expressing a polypeptide provided herein. T cell receptors and antibody receptors specific for the polypeptides recited herein may be cloned, expressed and transferred into other vectors or effector cells for adoptive immunotherapy. The polypeptides provided herein may also be used to generate antibodies or anti-idiotypic antibodies (as described above and in U.S. Patent No. 4,918,164) for passive immunotherapy.

Effector cells may generally be obtained in sufficient quantities for adoptive immunotherapy by growth *in vitro,* as described herein. Culture conditions for expanding single antigen-specific effector cells to several billion in number with retention of antigen recognition *in vivo* are well known in the art. Such *in vitro* culture conditions typically use intermittent stimulation with antigen, often in the presence of cytokines (such as IL-2) and non-dividing feeder cells. As noted above, immunoreactive polypeptides as provided herein may be used to rapidly expand antigen-specific T cell cultures in order to generate a sufficient number of cells for immunotherapy. In particular, antigen-presenting cells, such as dendritic, macrophage, monocyte, fibroblast and/or B cells, may be pulsed with immunoreactive polypeptides or transfected with one or more polynucleotides using standard techniques well known in the art. For example, antigen-presenting cells can be transfected with a polynucleotide having a promoter appropriate for increasing expression in a recombinant virus or other expression system. Cultured effector cells for use in therapy must be able to grow and distribute widely, and to survive long term *in vivo.* Studies have shown that cultured effector cells can be induced to grow in vivo and to survive long term in substantial numbers by repeated stimulation with antigen supplemented with IL-2 (*see,* for example, Cheever et al., *Immunological Reviews 157:*177*,* 1997).

Alternatively, a vector expressing a polypeptide recited herein may be introduced into antigen presenting cells taken from a patient and clonally propagated *ex vivo* for transplant back into the same patient. Transfected cells may be reintroduced into the patient using any means known in the art, preferably in sterile form by intravenous, intracavitary, intraperitoneal or intratumor administration.

Routes and frequency of administration of the therapeutic compositions described herein, as well as dosage, will vary from individual to individual, and may be readily established using standard techniques. In general, the pharmaceutical compositions and vaccines may be administered by injection (*e.g*., intracutaneous, intramuscular, intravenous or subcutaneous), intranasally (*e.g*., by aspiration) or orally. Preferably, between 1 and 10 doses may be administered over a 52 week period. Preferably, 6 doses are administered, at intervals of 1 month, and booster vaccinations may be given periodically thereafter. Alternate protocols may be appropriate for individual patients. A suitable dose is an amount of a compound that, when administered as described above, is capable of promoting an anti-tumor immune response, and is at least 10-50% above the basal *(i. e.*, untreated) level. Such response can be monitored by measuring the anti-tumor antibodies in a patient or by vaccine-dependent generation of cytolytic effector cells capable of killing the patient's tumor cells *in vitro.* Such vaccines should also be capable of causing an immune response that leads to an improved clinical outcome (*e.g*., more frequent remissions, complete or partial or longer disease-free survival) in vaccinated patients as compared to non-vaccinated patients. In general, for pharmaceutical compositions and vaccines comprising one or more polypeptides, the amount of each polypeptide present in a dose ranges from about 25 µg to 5 mg per kg of host. Suitable dose sizes will vary with the size of the patient, but will typically range from about 0.1 mL to about 5 mL.

In general, an appropriate dosage and treatment regimen provides the active compound(s) in an amount sufficient to provide therapeutic and/or prophylactic benefit. Such a response can be monitored by establishing an improved clinical outcome (e.g., more frequent remissions, complete or partial, or longer disease-free survival) in treated patients as compared to non-treated patients. Increases in preexisting immune responses to a tumor protein generally correlate with an improved clinical outcome. Such immune responses may generally be evaluated using standard proliferation, cytotoxicity or cytokine assays, which may be performed using samples obtained from a patient before and after treatment.

### Cancer Detection and Diagnostic Compositions, Methods and Kits

In general, a cancer may be detected in a patient based on the presence of one or more breast tumor proteins and/or polynucleotides encoding such proteins in a biological sample (for example, blood, sera, sputum urine and/or tumor biopsies) obtained from the patient. In other words, such proteins may be used as markers to indicate the presence or absence of a cancer such as breast cancer. In addition, such proteins may be useful for the detection of other cancers. The binding agents provided herein generally permit detection of the level of antigen that binds to the agent in the biological sample. Polynucleotide primers and probes may be used to detect the level of mRNA encoding a tumor protein, which is also indicative of the presence or absence of a cancer. In general, a breast tumor sequence should be present at a level that is at least three fold higher in tumor tissue than in normal tissue

There are a variety of assay formats known to those of ordinary skill in the art for using a binding agent to detect polypeptide markers in a sample. *See, e.g.,* Harlow and Lane, *Antibodies: A Laboratory Manual,* Cold Spring Harbor Laboratory, 1988. In general, the presence or absence of a cancer in a patient may be determined by (a) contacting a biological sample obtained from a patient with a binding agent; (b) detecting in the sample a level of polypeptide that binds to the binding agent; and (c) comparing the level of polypeptide with a predetermined cut-off value.

In a preferred embodiment, the assay involves the use of binding agent immobilized on a solid support to bind to and remove the polypeptide from the remainder of the sample. The bound polypeptide may then be detected using a detection reagent that contains a reporter group and specifically binds to the binding agent/polypeptide complex. Such detection reagents may comprise, for example, a binding agent that specifically binds to the polypeptide or an antibody or other agent that specifically binds to the binding agent, such as an anti-immunoglobulin, protein G, protein A or a lectin. Alternatively, a competitive assay may be utilized, in which a polypeptide is labeled with a reporter group and allowed to bind to the immobilized binding agent after incubation of the binding agent with the sample. The extent to which components of the sample inhibit the binding of the labeled polypeptide to the binding agent is indicative of the reactivity of the sample with the immobilized binding agent. Suitable polypeptides for use within such assays include full length breast tumor proteins and polypeptide portions thereof to which the binding agent binds, as described above.

The solid support may be any material known to those of ordinary skill in the art to which the tumor protein may be attached. For example, the solid support may be a test well in a microtiter plate or a nitrocellulose or other suitable membrane. Alternatively, the support may be a bead or disc, such as glass, fiberglass, latex or a plastic material such as polystyrene or polyvinylchloride. The support may also be a magnetic particle or a fiber optic sensor, such as those disclosed, for example, in U.S. Patent No. 5,359,681. The binding agent may be immobilized on the solid support using a variety of techniques known to those of skill in the art, which are amply described in the patent and scientific literature. In the context of the present invention, the term "immobilization" refers to both noncovalent association, such as adsorption, and covalent attachment (which may be a direct linkage between the agent and functional groups on the support or may be a linkage by way of a cross-linking agent). Immobilization by adsorption to a well in a microtiter plate or to a membrane is preferred. In such cases, adsorption may be achieved by contacting the binding agent, in a suitable buffer, with the solid support for a suitable amount of time. The contact time varies with temperature, but is typically between about 1 hour and about 1 day. In general, contacting a well of a plastic microtiter plate (such as polystyrene or polyvinylchloride) with an amount of binding agent ranging from about 10 ng to about 10 µg, and preferably about 100 ng to about 1 µg, is sufficient to immobilize an adequate amount of binding agent.

Covalent attachment of binding agent to a solid support may generally be achieved by first reacting the support with a bifunctional reagent that will react with both the support and a functional group, such as a hydroxyl or amino group, on the binding agent. For example, the binding agent may be covalently attached to supports having an appropriate polymer coating using benzoquinone or by condensation of an aldehyde group on the support with an amine and an active hydrogen on the binding partner *(see, e.g.,* Pierce Immunotechnology Catalog and Handbook, 1991, at A12-A13).

In certain embodiments, the assay is a two-antibody sandwich assay. This assay may be performed by first contacting an antibody that has been immobilized on a solid support, commonly the well of a microtiter plate, with the sample, such that polypeptides within the sample are allowed to bind to the immobilized antibody. Unbound sample is then removed from the immobilized polypeptide-antibody complexes and a detection reagent (preferably a second antibody capable of binding to a different site on the polypeptide) containing a reporter group is added. The amount of detection reagent that remains bound to the solid support is then determined using a method appropriate for the specific reporter group.

More specifically, once the antibody is immobilized on the support as described above, the remaining protein binding sites on the support are typically blocked. Any suitable blocking agent known to those of ordinary skill in the art, such as bovine serum albumin or Tween 20™ (Sigma Chemical Co., St. Louis, MO). The immobilized antibody is then incubated with the sample, and polypeptide is allowed to bind to the antibody. The sample may be diluted with a suitable diluent, such as phosphate-buffered saline (PBS) prior to incubation. In general, an appropriate contact time (i.e., incubation time) is a period of time that is sufficient to detect the presence of polypeptide within a sample obtained from an individual with breast cancer. Preferably, the contact time is sufficient to achieve a level of binding that is at least about 95% of that achieved at equilibrium between bound and unbound polypeptide. Those of ordinary skill in the art will recognize that the time necessary to achieve equilibrium may be readily determined by assaying the level of binding that occurs over a period of time. At room temperature, an incubation time of about 30 minutes is generally sufficient.

Unbound sample may then be removed by washing the solid support with an appropriate buffer, such as PBS containing 0.1% Tween 20™. The second antibody, which contains a reporter group, may then be added to the solid support. Preferred reporter groups include those groups recited above.

The detection reagent is then incubated with the immobilized antibody-polypeptide complex for an amount of time sufficient to detect the bound polypeptide. An appropriate amount of time may generally be determined by assaying the level of binding that occurs over a period of time. Unbound detection reagent is then removed and bound detection reagent is detected using the reporter group. The method employed for detecting the reporter group depends upon the nature of the reporter group. For radioactive groups, scintillation counting or autoradiographic methods are generally appropriate. Spectroscopic methods may be used to detect dyes, luminescent groups and fluorescent groups. Biotin may be detected using avidin, coupled to a different reporter group (commonly a radioactive or fluorescent group or an enzyme). Enzyme reporter groups may generally be detected by the addition of substrate (generally for a specific period of time), followed by spectroscopic or other analysis of the reaction products.

To determine the presence or absence of a cancer, such as breast cancer, the signal detected from the reporter group that remains bound to the solid support is generally compared to a signal that corresponds to a predetermined cut-off value. In one preferred embodiment, the cut-off value for the detection of a cancer is the average mean signal obtained when the immobilized antibody is incubated with samples from patients without the cancer. In general, a sample generating a signal that is three standard deviations above the predetermined cut-off value is considered positive for the cancer. In an alternate preferred embodiment, the cut-off value is determined using a Receiver Operator Curve, according to the method of Sackett et al., *Clinical Epidemiology: A Basic Science for Clinical Medicine,* Little Brown and Co., 1985, p. 106-7. Briefly, in this embodiment, the cut-off value may be determined from a plot of pairs of true positive rates (*i.e*., sensitivity) and false positive rates (100%-specificity) that correspond to each possible cut-off value for the diagnostic test result. The cut-off value on the plot that is the closest to the upper left-hand corner (*i.e*., the value that encloses the largest area) is the most accurate cut-off value, and a sample generating a signal that is higher than the cut-off value determined by this method may be considered positive. Alternatively, the cut-off value may be shifted to the left along the plot, to minimize the false positive rate, or to the right, to minimize the false negative rate. In general, a sample generating a signal that is higher than the cut-off value determined by this method is considered positive for a cancer.

In a related embodiment, the assay is performed in a flow-through or strip test format, wherein the binding agent is immobilized on a membrane, such as nitrocellulose. In the flow-through test, polypeptides within the sample bind to the immobilized binding agent as the sample passes through the membrane. A second, labeled binding agent then binds to the binding agent-polypeptide complex as a solution containing the second binding agent flows through the membrane. The detection of bound second binding agent may then be performed as described above. In the strip test format, one end of the membrane to which binding agent is bound is immersed in a solution containing the sample. The sample migrates along the membrane through a region containing second binding agent and to the area of immobilized binding agent. Concentration of second binding agent at the area of immobilized antibody indicates the presence of a cancer. Typically, the concentration of second binding agent at that site generates a pattern, such as a line, that can be read visually. The absence of such a pattern indicates a negative result. In general, the amount of binding agent immobilized on the membrane is selected to generate a visually discernible pattern when the biological sample contains a level of polypeptide that would be sufficient to generate a positive signal in the two-antibody sandwich assay, in the format discussed above. Preferred binding agents for use in such assays are antibodies and antigen-binding fragments thereof. Preferably, the amount of antibody immobilized on the membrane ranges from about 25 ng to about 1µg, and more preferably from about 50 ng to about 500 ng. Such tests can typically be performed with a very small amount of biological sample.

Of course, numerous other assay protocols exist that are suitable for use with the tumor proteins or binding agents of the present invention. The above descriptions are intended to be exemplary only. For example, it will be apparent to those of ordinary skill in the art that the above protocols may be readily modified to use tumor polypeptides to detect antibodies that bind to such polypeptides in a biological sample. The detection of such tumor protein specific antibodies may correlate with the presence of a cancer.

A cancer may also, or alternatively, be detected based on the presence of T cells that specifically react with a tumor protein in a biological sample. Within certain methods, a biological sample comprising CD4⁺ and/or CD8⁺ T cells isolated from a patient is incubated with a tumor polypeptide, a polynucleotide encoding such a polypeptide and/or an APC that expresses at least an immunogenic portion of such a polypeptide, and the presence or absence of specific activation of the T cells is detected. Suitable biological samples include, but are not limited to, isolated T cells. For example, T cells may be isolated from a patient by routine techniques (such as by Ficoll/Hypaque density gradient centrifugation of peripheral blood lymphocytes). T cells may be incubated *in vitro* for 2-9 days (typically 4 days) at 37°C with polypeptide *(e.g.,* 5 - 25 µg/ml). It may be desirable to incubate another aliquot of a T cell sample in the absence of tumor polypeptide to serve as a control. For CD4⁺ T cells, activation is preferably detected by evaluating proliferation of the T cells. For CD8⁺ T cells, activation is preferably detected by evaluating cytolytic activity. A level of proliferation that is at least two fold greater and/or a level of cytolytic activity that is at least 20% greater than in disease-free patients indicates the presence of a cancer in the patient.

As noted above, a cancer may also, or alternatively, be detected based on the level of mRNA encoding a tumor protein in a biological sample. For example, at least two oligonucleotide primers may be employed in a polymerase chain reaction (PCR) based assay to amplify a portion of a tumor cDNA derived from a biological sample, wherein at least one of the oligonucleotide primers is specific for (*i.e*., hybridizes to) a polynucleotide encoding the tumor protein. The amplified cDNA is then separated and detected using techniques well known in the art, such as gel electrophoresis. Similarly, oligonucleotide probes that specifically hybridize to a polynucleotide encoding a tumor protein may be used in a hybridization assay to detect the presence of polynucleotide encoding the tumor protein in a biological sample.

To permit hybridization under assay conditions, oligonucleotide primers and probes should comprise an oligonucleotide sequence that has at least about 60%, preferably at least about 75% and more preferably at least about 90%, identity to a portion of a polynucleotide encoding a tumor protein of the invention that is at least 10 nucleotides, and preferably at least 20 nucleotides, in length. Preferably, oligonucleotide primers and/or probes hybridize to a polynucleotide encoding a polypeptide described herein under moderately stringent conditions, as defined above. Oligonucleotide primers and/or probes which may be usefully employed in the diagnostic methods described herein preferably are at least 10-40 nucleotides in length. In a preferred embodiment, the oligonucleotide primers comprise at least 10 contiguous nucleotides, more preferably at least 15 contiguous nucleotides, of a DNA molecule having a sequence as disclosed herein. Techniques for both PCR based assays and hybridization assays are well known in the art *(see,* for example, Mullis et al., *Cold Spring Harbor Symp. Quant. Biol.,* 51:263, 1987; Erlich ed., *PCR Technology,* Stockton Press, NY, 1989).

One preferred assay employs RT-PCR, in which PCR is applied in conjunction with reverse transcription. Typically, RNA is extracted from a biological sample, such as biopsy tissue, and is reverse transcribed to produce cDNA molecules. PCR amplification using at least one specific primer generates a cDNA molecule, which may be separated and visualized using, for example, gel electrophoresis. Amplification may be performed on biological samples taken from a test patient and from an individual who is not afflicted with a cancer. The amplification reaction may be performed on several dilutions of cDNA spanning two orders of magnitude. A two-fold or greater increase in expression in several dilutions of the test patient sample as compared to the same dilutions of the non-cancerous sample is typically considered positive.

In another embodiment, the compositions described herein may be used as markers for the progression of cancer. In this embodiment, assays as described above for the diagnosis of a cancer may be performed over time, and the change in the level of reactive polypeptide(s) or polynucleotide(s) evaluated. For example, the assays may be performed every 24-72 hours for a period of 6 months to 1 year, and thereafter performed as needed. In general, a cancer is progressing in those patients in whom the level of polypeptide or polynucleotide detected increases over time. In contrast, the cancer is not progressing when the level of reactive polypeptide or polynucleotide either remains constant or decreases with time.

Certain *in vivo* diagnostic assays may be performed directly on a tumor. One such assay involves contacting tumor cells with a binding agent. The bound binding agent may then be detected directly or indirectly via a reporter group. Such binding agents may also be used in histological applications. Alternatively, polynucleotide probes may be used within such applications.

As noted above, to improve sensitivity, multiple tumor protein markers may be assayed within a given sample. It will be apparent that binding agents specific for different proteins provided herein may be combined within a single assay. Further, multiple primers or probes may be used concurrently. The selection of tumor protein markers may be based on routine experiments to determine combinations that results in optimal sensitivity. In addition, or alternatively, assays for tumor proteins provided herein may be combined with assays for other known tumor antigens.

The present invention further provides kits for use within any of the above diagnostic methods. Such kits typically comprise two or more components necessary for performing a diagnostic assay. Components may be compounds, reagents, containers and/or equipment. For example, one container within a kit may contain a monoclonal antibody or fragment thereof that specifically binds to a tumor protein. Such antibodies or fragments may be provided attached to a support material, as described above. One or more additional containers may enclose elements, such as reagents or buffers, to be used in the assay. Such kits may also, or alternatively, contain a detection reagent as described above that contains a reporter group suitable for direct or indirect detection of antibody binding.

Alternatively, a kit may be designed to detect the level of mRNA encoding a tumor protein in a biological sample. Such kits generally comprise at least one oligonucleotide probe or primer, as described above, that hybridizes to a polynucleotide encoding a tumor protein. Such an oligonucleotide may be used, for example, within a PCR or hybridization assay. Additional components that may be present within such kits include a second oligonucleotide and/or a diagnostic reagent or container to facilitate the detection of a polynucleotide encoding a tumor protein.

The following Examples are offered by way of illustration and not by way of limitation.

### EXAMPLE 1

### PREPARATION OF BREAST TUMOR-SPECIFIC CDNAS USING DIFFERENTIAL DISPLAY RT-PCR

This Example illustrates the preparation of cDNA molecules encoding breast tumor-specific polypeptides using a differential display screen.

### A. Preparation of B18Ag1 cDNA and Characterization of mRNA Expression

Tissue samples were prepared from breast tumor and normal tissue of a patient with breast cancer that was confirmed by pathology after removal from the patient. Normal RNA and tumor RNA was extracted from the samples and mRNA was isolated and converted into cDNA using a (dT)₁₂AG (SEQ ID NO:130) anchored 3' primer. Differential display PCR was then executed using a randomly chosen primer (CTTCAACCTC) (SEQ ID NO:103). Amplification conditions were standard buffer containing 1.5 mM MgCl₂, 20 pmol of primer, 500 pmol dNTP, and 1 unit of *Taq* DNA polymerase (Perkin-Elmer, Branchburg, NJ). Forty cycles of amplification were performed using 94°C denaturation for 30 seconds, 42°C annealing for 1 minute, and 72 °C extension for 30 seconds. An RNA fingerprint containing 76 amplified products was obtained. Although the RNA fingerprint of breast tumor tissue was over 98% identical to that of the normal breast tissue, a band was repeatedly observed to be specific to the RNA fingerprint pattern of the tumor. This band was cut out of a silver stained gel, subcloned into the T-vector (Novagen, Madison, WI) and sequenced.

The sequence of the cDNA, referred to as B18Ag1, is provided in SEQ ID NO:1. A database search of GENBANK and EMBL revealed that the B18Ag1 fragment initially cloned is 77% identical to the endogenous human retroviral element S71, which is a truncated retroviral element homologous to the Simian Sarcoma Virus (SSV). S71 contains an incomplete *gag* gene, a portion of the *pol* gene and an LTR-like structure at the 3' terminus *(see* Werner et al., *Virology 174*:225-238 (1990)). B18Ag1 is also 64% identical to SSV in the region corresponding to the P30 (gag) locus. B18Ag1 contains three separate and incomplete reading frames covering a region which shares considerable homology to a wide variety of gag proteins of retroviruses which infect mammals. In addition, the homology to S71 is not just within the *gag* gene, but spans several kb of sequence including an LTR.

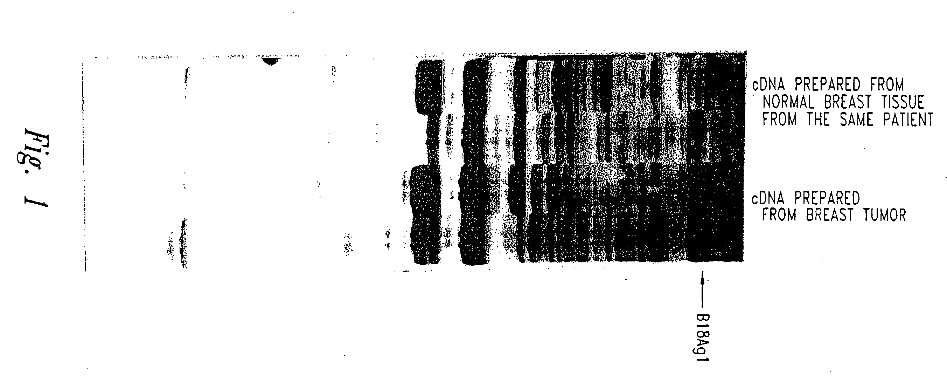

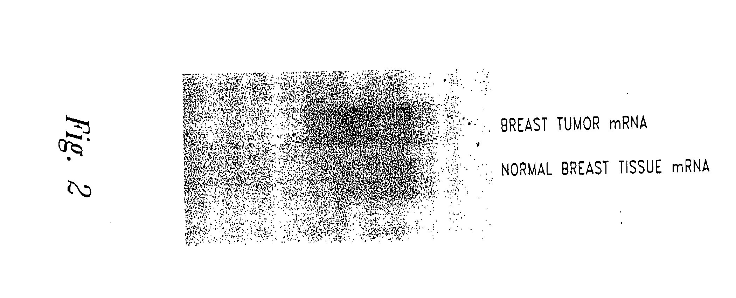

B18Ag1-specific PCR primers were synthesized using computer analysis guidelines. RT-PCR amplification (94°C, 30 seconds; 60°C→ 42°C, 30 seconds; 72°C, 30 seconds for 40 cycles) confirmed that B18Ag1 represents an actual mRNA sequence present at relatively high levels in the patient's breast tumor tissue. The primers used in amplification were B18Ag1-1 (CTG CCT GAG CCA CAA ATG) (SEQ ID NO:128) and B18Ag1-4 (CCG GAG GAG GAA GCT AGA GGA ATA) (SEQ ID NO:129) at a 3.5 mM magnesium concentration and a pH of 8.5, and B18Ag1-2 (ATG GCT ATT TTC GGG GCC TGA CA) (SEQ ID NO:126) and B18Ag1-3 (CCG GTA TCT CCT CGT GGG TAT T) (SEQ ID NO:127) at 2 mM magnesium at pH 9.5. The same experiments showed exceedingly low to nonexistent levels of expression in this patient's normal breast tissue *(see* Figure 1). RT-PCR experiments were then used to show that B18Ag1 mRNA is present in nine other breast tumor samples (from Brazilian and American patients) but absent in, or at exceedingly low levels in, the normal breast tissue corresponding to each cancer patient. RT-PCR analysis has also shown that the B18Ag1 transcript is not present in various normal tissues (including lymph node, myocardium and liver) and present at relatively low levels in PBMC and lung tissue. The presence of B18Ag1 mRNA in breast tumor samples, and its absence from normal breast tissue, has been confirmed by Northern blot analysis, as shown in Figure 2.

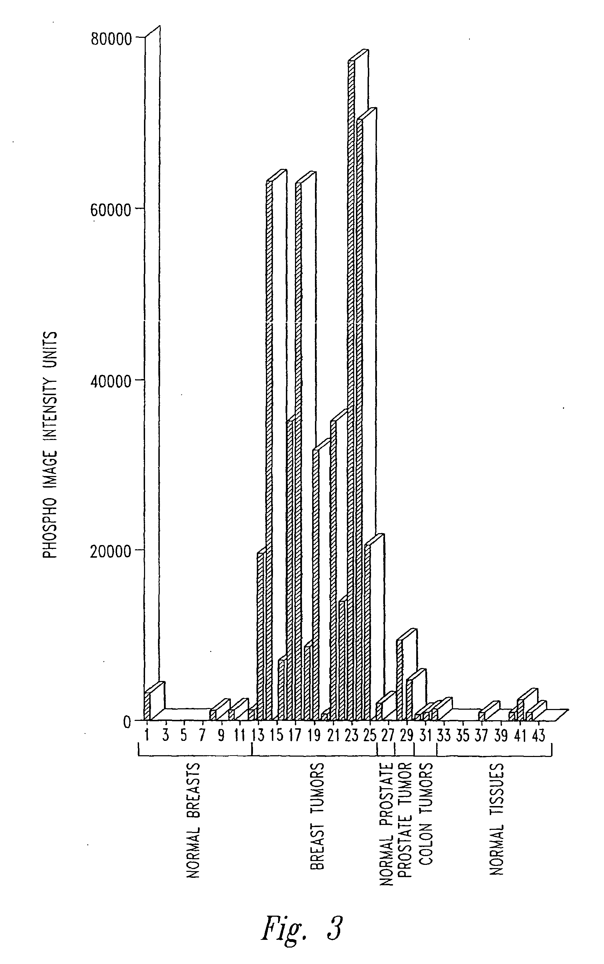

The differential expression of B18Ag1 in breast tumor tissue was also confirmed by RNase protection assays. Figure 3 shows the level of B18Ag1 mRNA in various tissue types as determined in four different RNase protection assays. Lanes 1-12 represent various normal breast tissue samples, lanes 13-25 represent various breast tumor samples; lanes 26-27 represent normal prostate samples; lanes 28-29 represent prostate tumor samples; lanes 30-32 represent colon tumor samples; lane 33 represents normal aorta; lane 34 represents normal small intestine; lane 35 represents normal skin, lane 36 represents normal lymph node; lane 37 represents normal ovary; lane 38 represents normal liver; lane 39 represents normal skeletal muscle; lane 40 represents a first normal stomach sample, lane 41 represents a second normal stomach sample; lane 42 represents a normal lung; lane 43 represents normal kidney; and lane 44 represents normal pancreas. Interexperimental comparison was facilitated by including a positive control RNA of known β-actin message abundance in each assay and normalizing the results of the different assays with respect to this positive control.

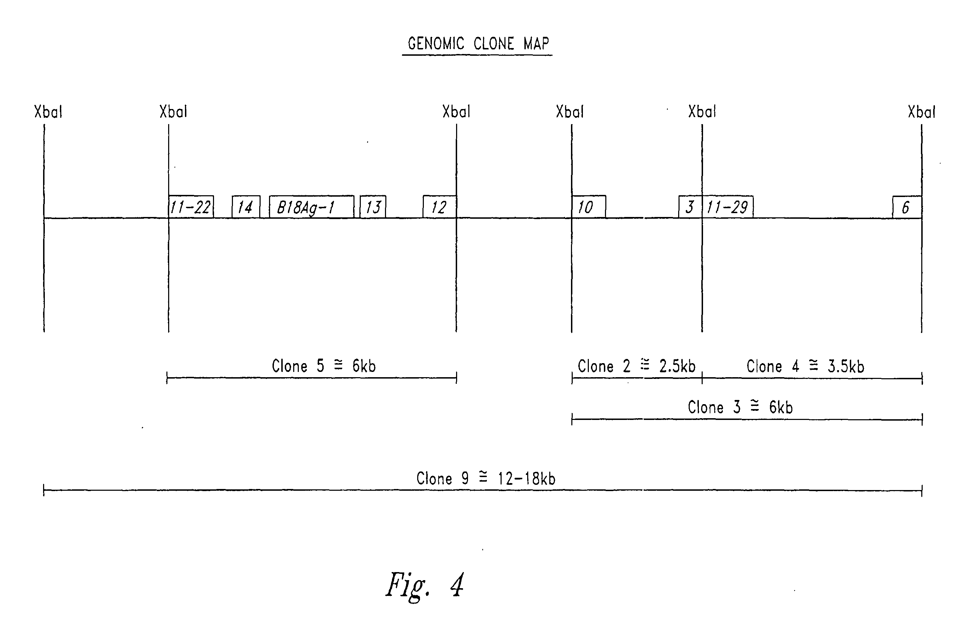

RT-PCR and Southern Blot analysis has shown the B18Ag1 locus to be present in human genomic DNA as a single copy endogenous retroviral element. A genomic clone of approximately 12-18 kb was isolated using the initial B18Ag1 sequence as a probe. Four additional subclones were also isolated by XbaI digestion. Additional retroviral sequences obtained from the ends of the XbaI digests of these clones (located as shown in Figure 4) are shown as SEQ ID NO:3 - SEQ ID NO: 10, where SEQ ID NO:3 shows the location of the sequence labeled 10 in Figure 4, SEQ ID NO:4 shows the location of the sequence labeled 11-29, SEQ ID NO:5 shows the location of the sequence labeled 3, SEQ ID NO:6 shows the location of the sequence labeled 6, SEQ ID NO:7 shows the location of the sequence labeled 12, SEQ ID NO:8 shows the location of the sequence labeled 13, SEQ ID NO:9 shows the location of the sequence labeled 14 and SEQ ID NO:10 shows the location of the sequence labeled 11-22.

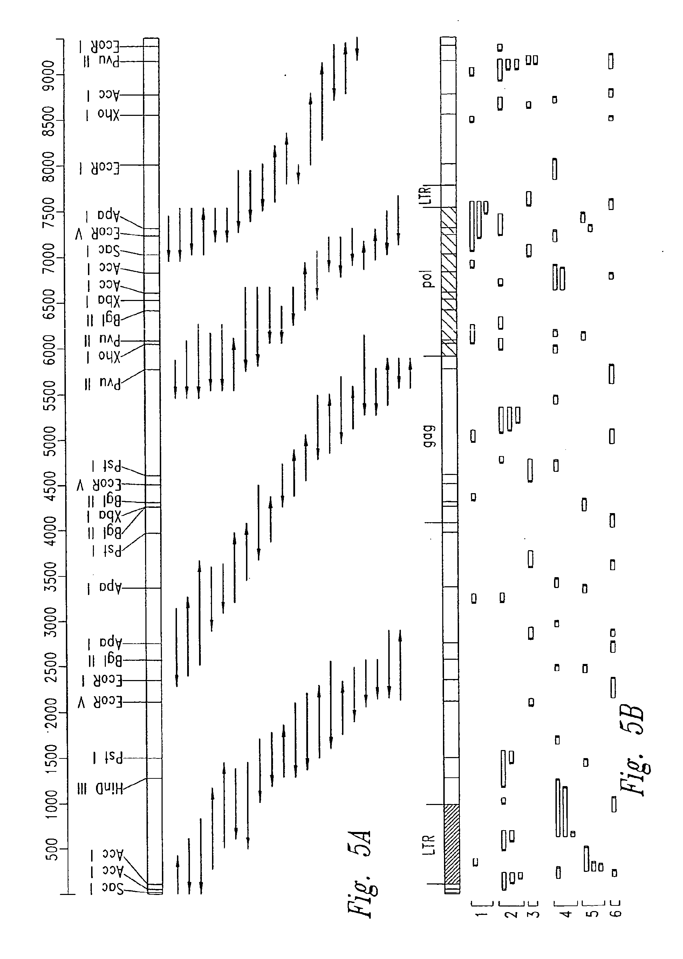

Subsequent studies demonstrated that the 12-18 kb genomic clone contains a retroviral element of about 7.75 kb, as shown in Figures 5A and 5B. The sequence of this retroviral element is shown in SEQ ID NO:141. The numbered line at the top of Figure 5A represents the sense strand sequence of the retroviral genomic clone. The box below this line shows the position of selected restriction sites. The arrows depict the different overlapping clones used to sequence the retroviral element. The direction of the arrow shows whether the single-pass subclone sequence corresponded to the sense or anti-sense strand. Figure 5B is a schematic diagram of the retroviral element containing B18Ag1 depicting the organization of viral genes within the element. The open boxes correspond to predicted reading frames, starting with a methionine, found throughout the element. Each of the six likely reading frames is shown, as indicated to the left of the boxes, with frames 1-3 corresponding to those found on the sense strand.

Using the cDNA of SEQ ID NO:1 as a probe, a longer cDNA was obtained (SEQ ID NO:227) which contains minor nucleotide differences (less than 1 %) compared to the genomic sequence shown in SEQ ID NO:141.

### B. Preparation of cDNA Molecules Encoding Other Breast Tumor-Specific Polypeptides

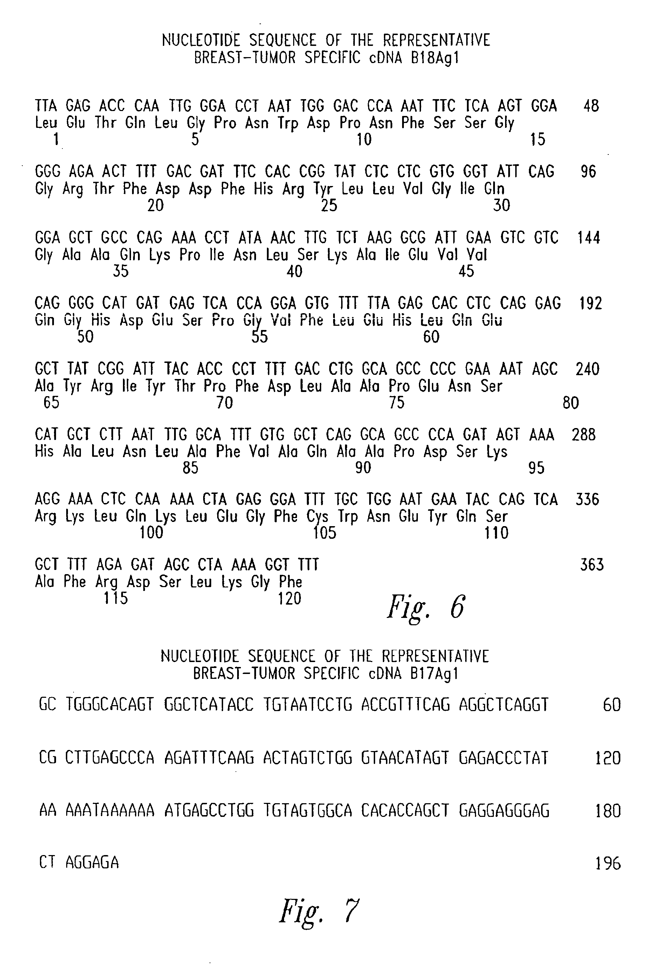

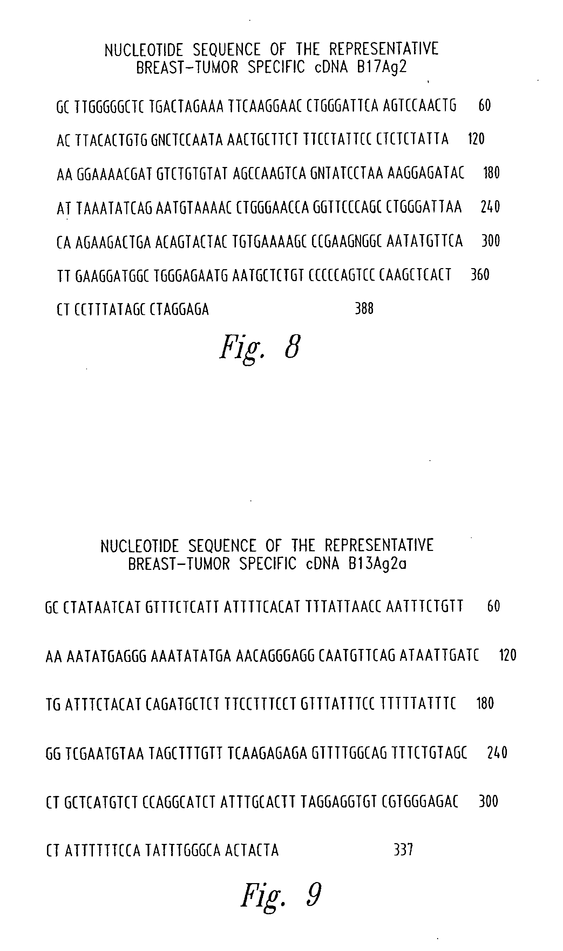

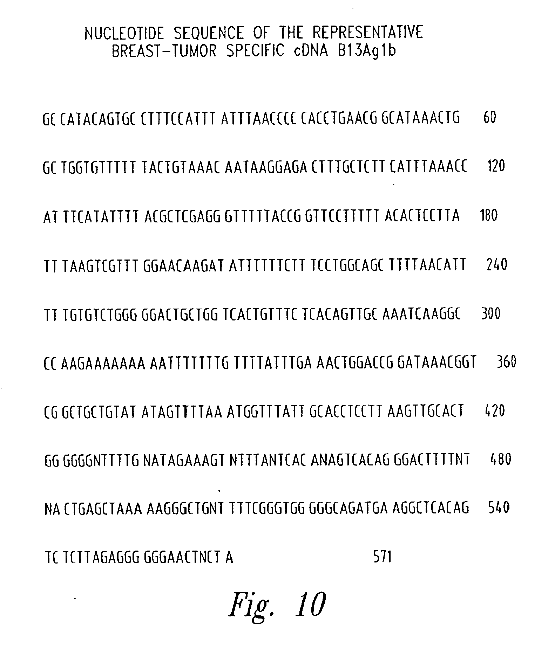

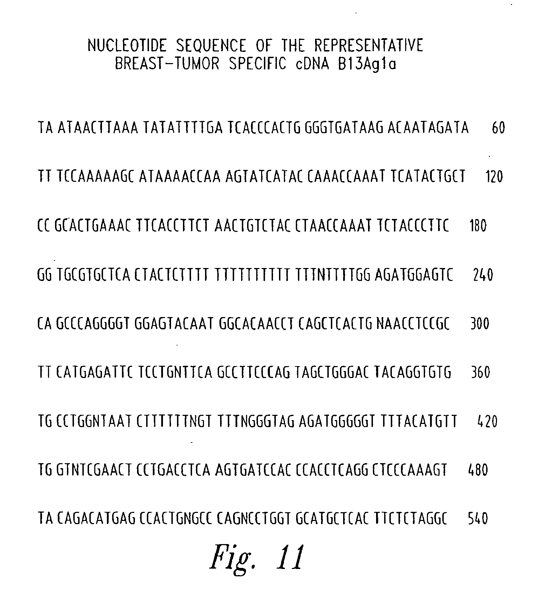

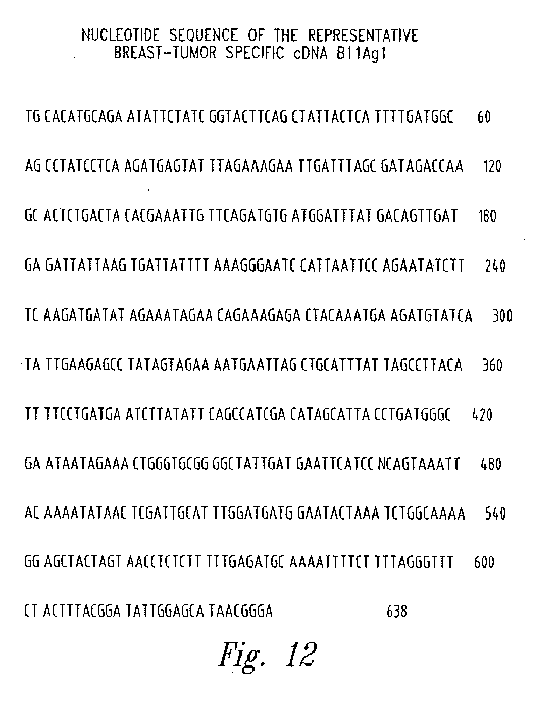

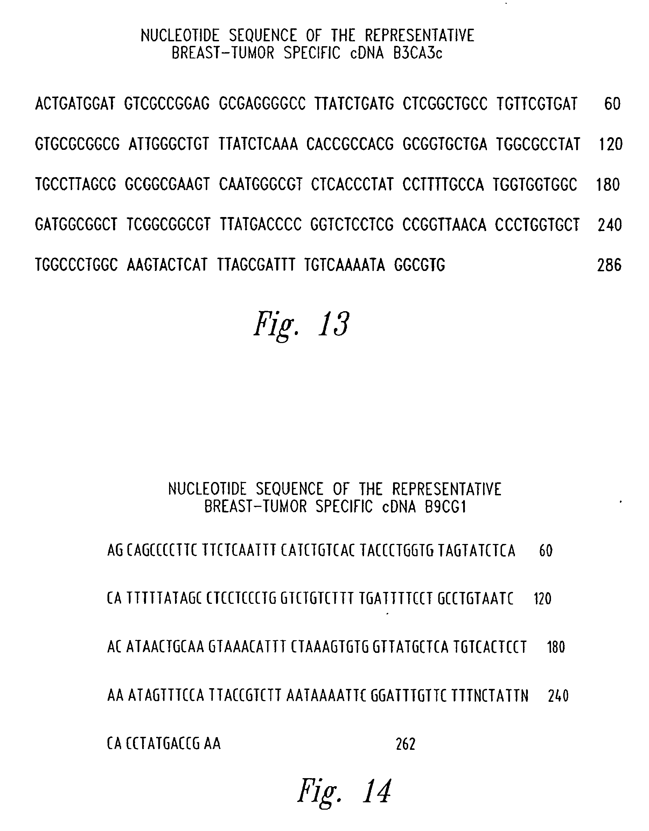

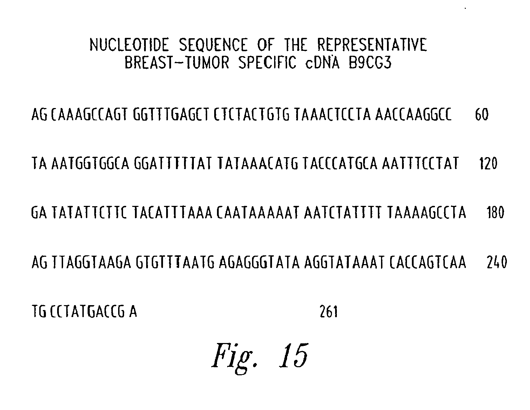

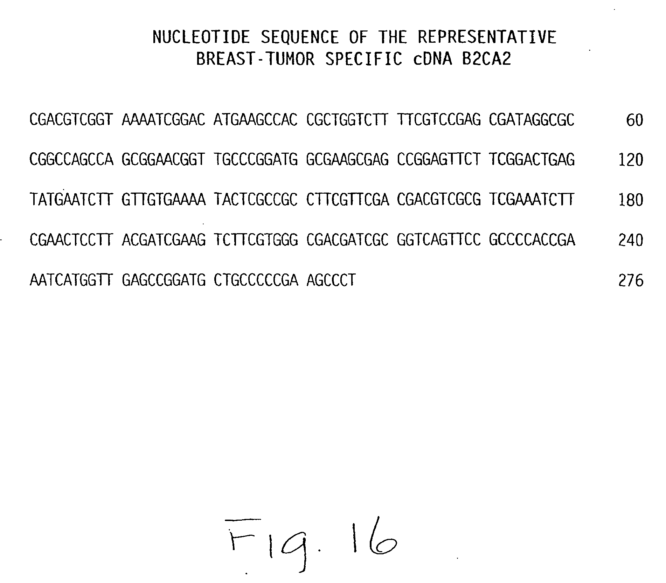

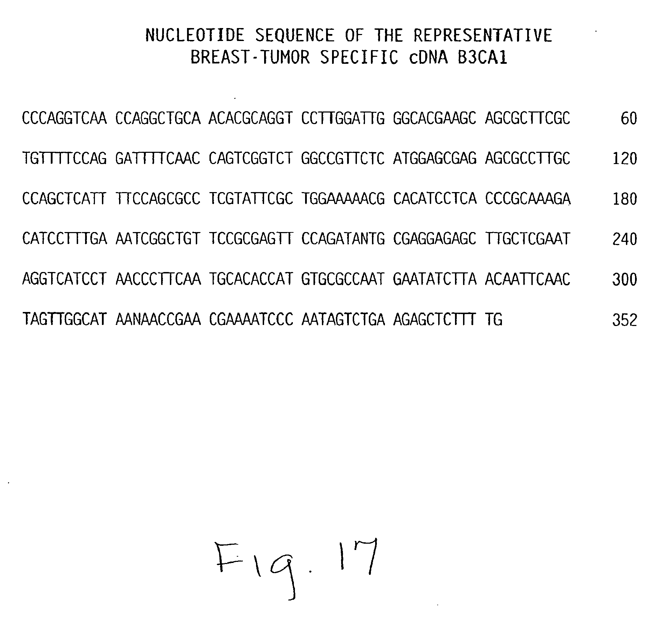

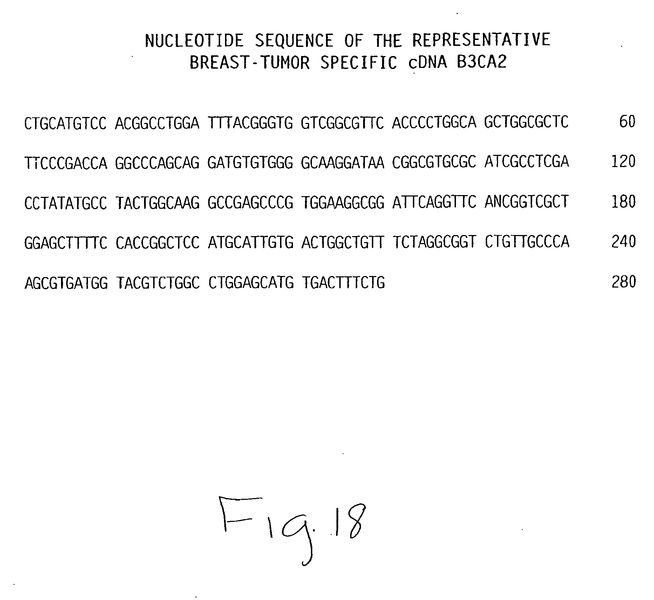

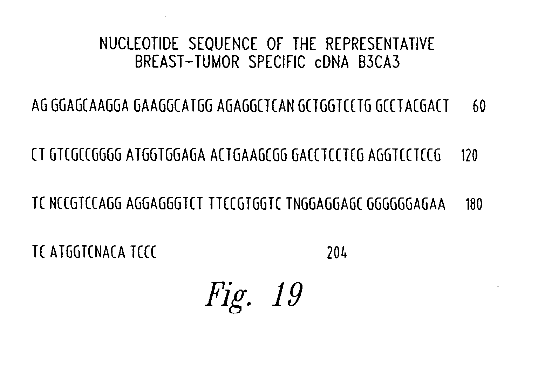

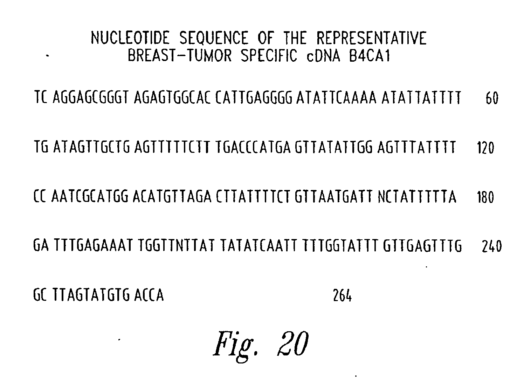

Normal RNA and tumor RNA was prepared and mRNA was isolated and converted into cDNA using a (dT)₁₂AG anchored 3' primer, as described above. Differential display PCR was then executed using the randomly chosen primers of SEQ ID NOs:87-125. Amplification conditions were as noted above, and bands observed to be specific to the RNA fingerprint pattern of the tumor were cut out of a silver stained gel, subcloned into either the T-vector (Novagen, Madison, WI) or the pCRII vector (Invitrogen, San Diego, CA) and sequenced. The sequences are provided in SEQ ID NO:11 - SEQ ID NO: 86. Of the 79 sequences isolated, 67 were found to be novel (SEQ ID NOs:11-26 and 28-77) *(see also* Figures 6-20).

An extended DNA sequence (SEQ ID NO:290) for the antigen B15Ag1 (originally identified partial sequence provided in SEQ ID NO:27) was obtained in further studies. Comparison of the sequence of SEQ ID NO:290 with those in the gene bank as described above, revealed homology to the known human β-A activin gene. Further studies led to the isolation of the full-length cDNA sequence for the antigen B21GT2 (also referred to as B311D; originally identified partial cDNA sequence provided in SEQ ID NOs:56). The full-length sequence is provided in SEQ ID NO:307, with the corresponding amino acid sequence being provided in SEQ ID NO:308. Further studies led to the isolation of a splice variant of B311D. The B311D clone of SEQ ID NO:316 was sequenced and a XhoI/NotI fragment from this clone was gel purified and 32P-cDTP labeled by random priming for use as a probe for further screening to obtain additional B311D gene sequence. Two fractions of a human breast tumor cDNA bacterial library were screened using standard techniques. One of the clones isolated in this manner yielded additional sequence which includes a poly A+ tail. The determined cDNA sequence of this clone (referred to as B311D_BT1_1A) is provided in SEQ ID NO:317. The sequences of SEQ ID NOs:316 and 317 were found to share identity over a 464 bp region, with the sequences diverging near the poly A+ sequence of SEQ ID NO:317.

Subsequent studies identified an additional 146 sequences (SEQ ID NOs:142-289), of which 115 appeared to be novel (SEQ ID NOs:142, 143, 146-152, 154-166, 168-176, 178-192, 194-198, 200-204, 206, 207, 209-214, 216, 218, 219, 221-240, 243-245, 247, 250, 251, 253, 255, 257-266, 268, 269, 271-273, 275, 276, 278, 280, 281, 284, 288 and 291). To the best of the inventors' knowledge none of the previously identified sequences have heretofore been shown to be expressed at a greater level in human breast tumor tissue than in normal breast tissue.

In further studies, several different splice forms of the antigen B11Ag1 (also referred to as B305D) were isolated, with each of the various splice forms containing slightly different versions of the B11Ag1 coding frame. Splice junction sequences define individual exons which, in various patterns and arrangements, make up the various splice forms. Primers were designed to examine the expression pattern of each of the exons using RT-PCR as described below. Each exon was found to show the same expression pattern as the original B11Ag1 clone, with expression being breast tumor-, normal prostate- and normal testis-specific. The determined cDNA sequences for the isolated protein coding exons are provided in SEQ ID NOs:292-298, respectively. The predicted amino acid sequences corresponding to the sequences of SEQ ID NOs:292 and 298 are provided in SEQ ID NOs:299 and 300. Additional studies using rapid amplification of cDNA ends (RACE), a 5' specific primer to one of the splice forms of B11Ag1 provided above and a breast adenocarcinoma, led to the isolation of three additional, related, splice forms referred to as isoforms B11C-15, B11C-8 and B11C-9,16. The determined cDNA sequences for these isoforms are provided in SEQ ID NO: 301-303, with the corresponding predicted amino acid sequences being provided in SEQ ID NOs:304-306.

The protein coding region of B11C-15 (SEQ ID NO: 301; also referred to as B305D isoform C) was used as a query sequence in a BLASTN search of the Genbank DNA database. A match was found to a genomic clone from chromosome 21 (Accessson no. AP001465). The pairwise alignments provided in the BLASTN output were used to identify the putative exon, or coding, sequence of the chromosome 21 sequence that corresponds to the B305D sequence. Based on the BlastN pairwise alignments, the following pieces of GenBank record AP001465 were put together: base pairs 67978-68499, 72870-72987, 73144-73335, 76085-76206, 77905-78085, 80520-80624, 87602-87633. This sequence was then aligned with the B305D isoform C sequence using the DNA Star Seqman program and excess sequence was deleted in such a way as to maintain the sequence most similar to B305D. The final edited form of the chromosome 21 sequence was 96.5% identical to B305D. This resulting edited sequence from chromosome 21 was then translated and found to contain no stop codons other than the final stop codon in the same position as that for B305D. As with B305D, the chromosome 21 sequence (provided in SEQ ID NO: 325) encoded a protein (SEQ ID NO: 326) with 384 amino acids. An alignment of this protein with the B305D isoform C protein (SEQ ID NO: 304)showed 90% amino acid identity.

The cDNA sequence of B305D isoform C (SEQ ID NO: 301) was used to identify homologs by searching the High Throughput Genome Sequencing (HTGS) database (NCBI, National Institutes for Health, Bethesda, MD). Homologs were identified on Chromosome 2 (Clone ID 9838181), Chromosome 10 (Clone ID 10933022), Chromosome 15 (Clone ID 11560284). These homologs shared greater than 90% identity with B305D isoform C at the nucleic acid level. All three of these homologs encode 384 amino acid ORFs that share greater than 90% identity with the amino acid sequence of SEQ ID NO: 304. Further searching of the GenBank database with the sequence of SEQ ID NO: 301 yielded a partial sequence homolog on Chromosome 22 (Clone ID 5931507). cDNA sequences for the Chromosome 2, 10, 15 and 22 homologs were constructed based on the homology with B305D isoform C and the conserved sequences at intron-exon junctions. The cDNA sequences for the Chromosome 22, 2, 15 and 10 homologs are provided in SEQ ID NO: 327-330, respectively, with the corresponding amino acid sequences being provided in SEQ ID NO: 331, 334, 333 and 332, respectively.

In subsequent studies on B305D isoform A (cDNA sequence provided in SEQ ID NO:292), the cDNA sequence (provided in SEQ ID NO:313) was found to contain an additional guanine residue at position 884, leading to a frameshift in the open reading frame. The determined DNA sequence of this ORF is provided in SEQ ID NO:314. This frameshift generates a protein sequence (provided in SEQ ID NO:315) of 293 amino acids that contains the C-terminal domain common to the other isoforms of B305D but that differs in the N-terminal region.

### EXAMPLE 2

### PREPARATION OF B18AG 1 DNA FROM HUMAN GENOMIC DNA

This Example illustrates the preparation of B18Ag1 DNA by amplification from human genomic DNA.

B18Ag 1 DNA may be prepared from 250 ng human genomic DNA using 20 pmol of B18Ag1 specific primers, 500 pmol dNTPS and 1 unit of *Taq* DNA polymerase (Perkin Elmer, Branchburg, NJ) using the following amplification parameters: 94°C for 30 seconds denaturing, 30 seconds 60°C to 42°C touchdown annealing in 2°C increments every two cycles and 72°C extension for 30 seconds. The last increment (a 42°C annealing temperature) should cycle 25 times. Primers were selected using computer analysis. Primers synthesized were B18Ag1-1, B18Ag1-2, B18Ag1-3, and B18Ag1-4. Primer pairs that may be used are 1+3, 1+4, 2+3, and 2+4.

Following gel electrophoresis, the band corresponding to B18Ag1 DNA may be excised and cloned into a suitable vector.

### EXAMPLE 3

### PREPARATION OF B18AG DNA FROM BREAST TUMOR CDNA

This Example illustrates the preparation of B18Ag1 DNA by amplification from human breast tumor cDNA.

First strand cDNA is synthesized from RNA prepared from human breast tumor tissue in a reaction mixture containing 500 ng poly A+ RNA, 200 pmol of the primer (T)₁₂AG *(i.e.,* TTT TTT TTT TTT AG) (SEQ ID NO:130), 1X first strand reverse transcriptase buffer, 6.7 mM DTT, 500 mmol dNTPs, and 1 unit AMV or MMLV reverse transcriptase (from any supplier, such as Gibco-BRL (Grand Island, NY)) in a final volume of 30 µl. After first strand synthesis, the cDNA is diluted approximately 25 fold and 1 µl is used for amplification as described in Example 2. While some primer pairs can result in a heterogeneous population of transcripts, the primers B18Ag1-2 (5'ATG GCT ATT TTC GGG GGC TGA CA) (SEQ ID NO:126) and B18Ag1-3 (5'CCG GTA TCT CCT CGT GGG TAT T) (SEQ ID NO:127) yield a single 151 bp amplification product.

### EXAMPLE 4

### IDENTIFICATION OF B-CELL AND T-CELL EPITOPES OF B 18AG 1

This Example illustrates the identification of B18Ag1 epitopes.

The B18Ag1 sequence can be screened using a variety of computer algorithms. To determine B-cell epitopes, the sequence can be screened for hydrophobicity and hydrophilicity values using the method of Hopp, *Prog. Clin. Biol. Res. 172B:*367-77 (1985) or, alternatively, Cease et al., *J*. *Exp*. *Med. 164*:1779-84 (1986) or Spouge et al., *J. Immunol. 138*:204-12 (1987). Additional Class II MHC (antibody or B-cell) epitopes can be predicted using programs such as AMPHI *(e.g.,* Margalit et al., *J. Immunol. 138*:2213 (1987)) or the methods of Rothbard and Taylor *(e.g., EMBO J.* 7:93 (1988)).

Once peptides (15-20 amino acids long) are identified using these techniques, individual peptides can be synthesized using automated peptide synthesis equipment (available from manufacturers such as Perkin Elmer/Applied Biosystems Division, Foster City, CA) and techniques such as Merrifield synthesis. Following synthesis, the peptides can used to screen sera harvested from either normal or breast cancer patients to determine whether patients with breast cancer possess antibodies reactive with the peptides. Presence of such antibodies in breast cancer patient would confirm the immunogenicity of the specific B-cell epitope in question. The peptides can also be tested for their ability to generate a serologic or humoral immune in animals (mice, rats, rabbits, chimps etc.) following immunization *in vivo.* Generation of a peptide-specific antiserum following such immunization further confirms the immunogenicity of the specific B-cell epitope in question.

To identify T-cell epitopes, the B18Ag1 sequence can be screened using different computer algorithms which are useful in identifying 8-10 amino acid motifs within the B18Ag1 sequence which are capable of binding to HLA Class I MHC molecules. *(see, e.g.,* Rammensee et al., *Immunogenetics 41*:178-228 (1995)). Following synthesis such peptides can be tested for their ability to bind to class I MHC using standard binding assays (*e.g*., Sette et al., *J Immunol.* 153:5586-92 (1994)) and more importantly can be tested for their ability to generate antigen reactive cytotoxic T-cells following *in vitro* stimulation of patient or normal peripheral mononuclear cells using, for example, the methods of Bakker et al., *Cancer Res.* 55:5330-34 (1995); Visseren et al., *J. Immunol. 154*:3991-98 (1995); Kawakami et al., *J. Immunol. 154*:3961-68 (1995); and Kast et al., *J. Immunol. 152*:3904-12 (1994). Successful *in vitro* generation of T-cells capable of killing autologous (bearing the same Class I MHC molecules) tumor cells following *in vitro* peptide stimulation further confirms the immunogenicity of the B18Ag1 antigen. Furthermore, such peptides may be used to generate murine peptide and B18Ag1 reactive cytotoxic T-cells following *in vivo* immunization in mice rendered transgenic for expression of a particular human MHC Class I haplotype (Vitiello et al., *J. Exp. Med. 173*:1007-15 (1991).

A representative list of predicted B18Ag1 B-cell and T-cell epitopes, broken down according to predicted HLA Class I MHC binding antigen, is shown below:
Predicted Th Motifs (B-cell epitopes) (SEQ ID NOS.: 131-133)
   SSGGRTFDDFHRYLLVGI
   QGAAQKPINLSKXIEVVQGHDE
   SPGVFLEHLQEAYRIYTPFDLSA
Predicted HLA A2.1 Motifs (T-cell epitopes) (SEQ ID NOS.: 134-140)
   YLLVGIQGA
   GAAQKPINL
   NLSKXIEVV
   EVVQGHDES
   HLQEAYRIY
   NLAFVAQAA
   FVAQAAPDS

### EXAMPLE 5

### IDENTIFICATION OF T-CELL EPITOPES OF B11AG1

This Example illustrates the identification of B11Ag1 (also referred to as B305D) epitopes. Four peptides, referred to as B11-8, B11-1, B11-5 and B11-12 (SEQ ID NOs:309-312, respectfully) were derived from the B11Ag1 gene.

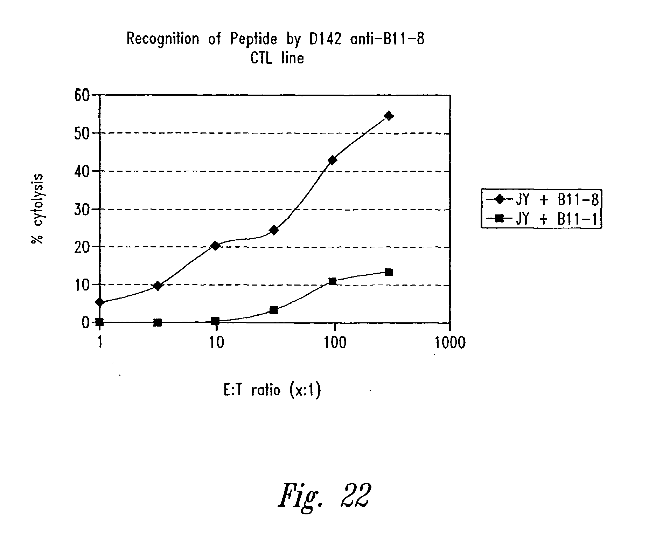

Human CD8 T cells were primed *in vitro* to the peptide B11-8 using dendritic cells according to the protocol of Van Tsai et al. *(Critical Reviews in Immunology 18*:65-75, 1998). The resulting CD8 T cell cultures were tested for their ability to recognize the B11-8 peptide or a negative control peptide, presented by the B-LCL line, JY. Briefly, T cells were incubated with autologous monocytes in the presence of 10 ug/ml peptide, 10 ng/ml IL-7 and 10 ug/ml IL-2, and assayed for their ability to specifically lyse target cells in a standard 51-Cr release assay. As shown in Fig. 22, the bulk culture line demonstrated strong recognition of the B 11-8 peptide with weaker recognition of the peptide B11-1.

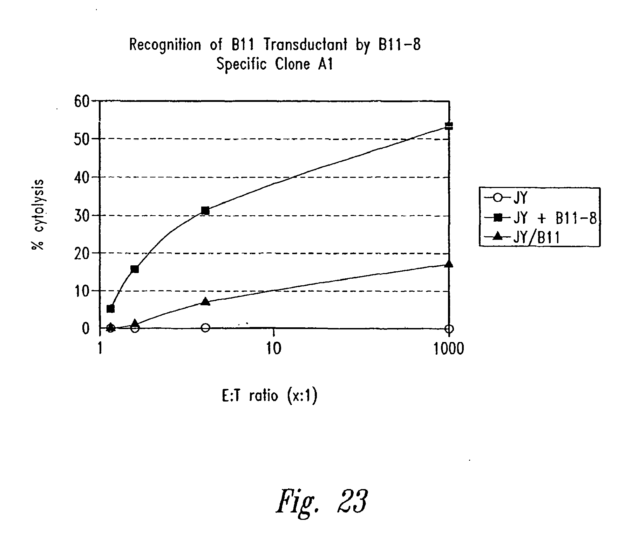

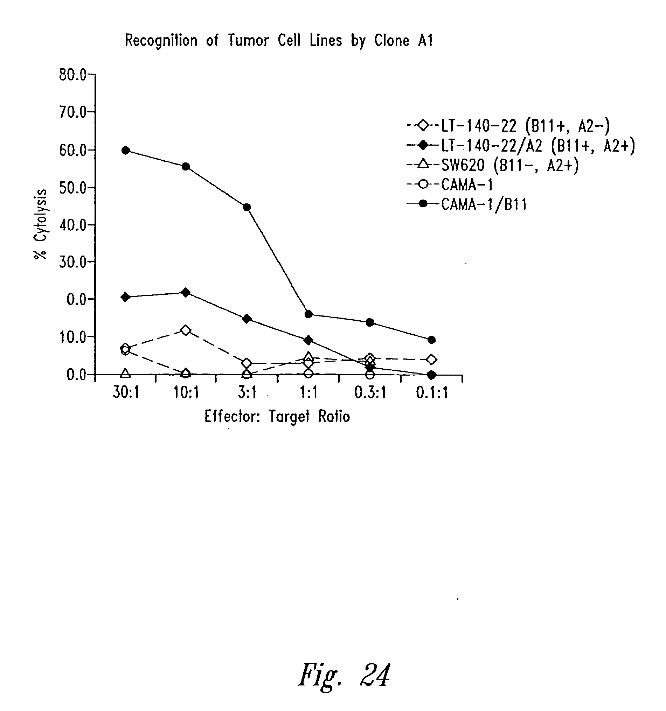

A clone from this CTL line was isolated following rapid expansion using the monoclonal antibody OKT3 and human IL-2. As shown in Fig. 23, this clone (referred to as A1), in addition to being able to recognize specific peptide, recognized JY LCL transduced with the B11Ag1 gene. This data demonstrates that B11-8 is a naturally processed epitope of the B11Ag1 gene. In addition these T cells were further found to recognize and lyse, in an HLA-A2 restricted manner, an established tumor cell line naturally expressing B11Ag1 (Fig. 24). The T cells strongly recognize a lung adenocarcinoma (LT-140-22) naturally expressing B11Ag1 transduced with HLA-A2, as well as an A2+ breast carcinoma (CAMA-1) transduced with B11Ag1, but not untransduced lines or another negative tumor line (SW620).

These data clearly demonstrate that these human T cells recognize not only B11-specific peptides but also transduced cells, as well as naturally expressing tumor lines.

CTL lines raised against the antigens B11-5 and B11-12, using the procedures described above, were found to recognize corresponding peptide-coated targets.

### 'EXAMPLE 6

### CHARACTERIZATION OF BREAST TUMOR GENES DISCOVERED BY DIFFERENTIAL DISPLAY PCR

The specificity and sensitivity of the breast tumor genes discovered by differential display PCR were determined using RT-PCR. This procedure enabled the rapid evaluation of breast tumor gene mRNA expression semiquantitatively without using large amounts of RNA. Using gene specific primers, mRNA expression levels in a variety of tissues were examined, including 8 breast tumors, 5 normal breasts, 2 prostate tumors, 2 colon tumors, 1 lung tumor, and 14 other normal adult human tissues, including normal prostate, colon, kidney, liver, lung, ovary, pancreas, skeletal muscle, skin, stomach and testes.

To ensure the semiquantitative nature of the RT-PCR, β-actin was used as internal control for each of the tissues examined. Serial dilutions of the first strand cDNAs were prepared and RT-PCR assays performed using β-actin specific primers. A dilution was then selected that enabled the linear range amplification of β -actin template, and which was sensitive enough to reflect the difference in the initial copy number. Using this condition, the β-actin levels were determined for each reverse transcription reaction from each tissue. DNA contamination was minimized by DNase treatment and by assuring a negative result when using first strand cDNA that was prepared without adding reverse transcriptase.

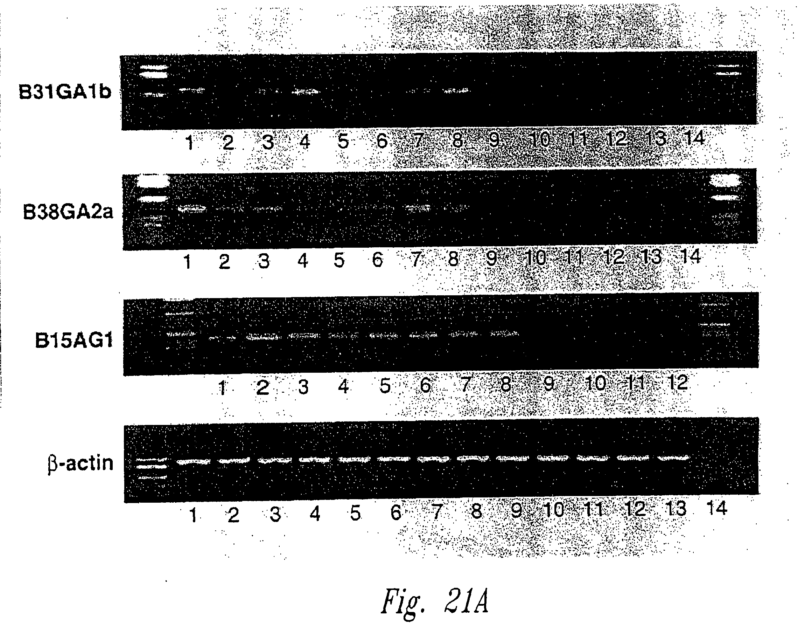

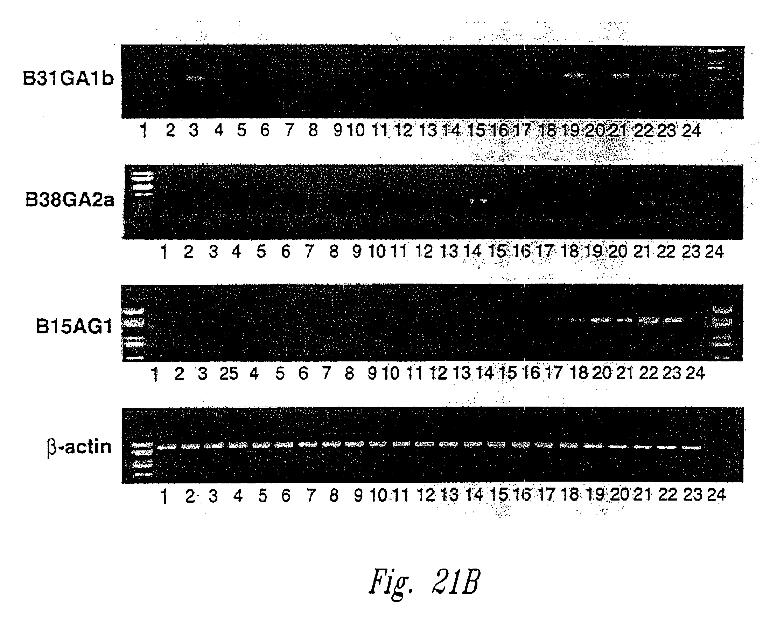

Using gene specific primers, the mRNA expression levels were determined in a variety of tissues. To date, 38 genes have been successfully examined by RT-PCR, five of which exhibit good specificity and sensitivity for breast tumors (B15AG-1, B31GA1b, B38GA2a, B11A1a and B18AG1a). Figures 21A and 21B depict the results for three of these genes: B15AG-1 (SEQ ID NO:27), B31GA1b (SEQ ID NO:148) and B38GA2a (SEQ ID NO:157). Table I summarizes the expression level of all the genes tested in normal breast tissue and breast tumors, and also in other tissues.

**TABLE I Percentage of Breast Cancer Antigens that are Expressed in Various Tissues**

| | | |
|---|---|---|
| Breast Tissues | Over-expressed in Breast Tumors | 84% |
| | Equally Expressed in Normals and Tumor | 16% |
| Other Tissues | Over-expressed in Breast Tumors but not in any Normal Tissues | 9% |
| | Over-expressed in Breast Tumors but Expressed in Some Normal Tissues | 30% |
| | Over-expressed in Breast Tumors but Equally Expressed in All Other Tissues | 61% |

### EXAMPLE 7

### PREPARATION AND CHARACTERIZATION OF ANTIBODIES AGAINST BREAST TUMOR POLYPEPTIDES

Polyclonal antibodies against the breast tumor antigen B305D were prepared as follows.

The breast tumor antigen expressed in an *E. coli* recombinant expression system was grown overnight in LB broth with the appropriate antibiotics at 37 °C in a shaking incubator. The next morning, 10 ml of the overnight culture was added to 500 ml to 2x YT plus appropriate antibiotics in a 2L-baffled Erlenmeyer flask. When the Optical Density (at 560 nm) of the culture reached 0.4-0.6, the cells were induced with IPTG (1 mM). Four hours after induction with IPTG, the cells were harvested by centrifugation. The cells were then washed with phosphate buffered saline and centrifuged again. The supernatant was discarded and the cells were either frozen for future use or immediately processed. Twenty ml of lysis buffer was added to the cell pellets and vortexed. To break open the *E*. *coli* cells, this mixture was then run through the French Press at a pressure of 16,000 psi. The cells were then centrifuged again and the supernatant and pellet were checked by SDS-PAGE for the partitioning of the recombinant protein. For proteins that localized to the cell pellet, the pellet was resuspended in 10 mM Tris pH 8.0, 1% CHAPS and the inclusion body pellet was washed and centrifuged again. This procedure was repeated twice more. The washed inclusion body pellet was solubilized with either 8 M urea or 6 M guanidine HCl containing 10 mM Tris pH 8.0 plus 10 mM imidazole. The solubilized protein was added to 5 ml of nickel-chelate resin (Qiagen) and incubated for 45 min to 1 hour at room temperature with continuous agitation. After incubation, the resin and protein mixture were poured through a disposable column and the flow through was collected. The column was then washed with 10-20 column volumes of the solubilization buffer. The antigen was then eluted from the column using 8M urea, 10 mM Tris pH 8.0 and 300 mM imidazole and collected in 3 ml fractions. A SDS-PAGE gel was run to determine which fractions to pool for further purification.

As a final purification step, a strong anion exchange resin such as HiPrepQ (Biorad) was equilibrated with the appropriate buffer and the pooled fractions from above were loaded onto the column. Antigen was eluted off the column with a increasing salt gradient. Fractions were collected as the column was run and another SDS-PAGE gel was run to determine which fractions from the column to pool. The pooled fractions were dialyzed against 10 mM Tris pH 8.0. The protein was then vialed after filtration through a 0.22 micron filter and the antigens were frozen until needed for immunization.

Four hundred micrograms of B305D antigen was combined with 100 micrograms of muramyldipeptide (MDP). Every four weeks rabbits were boosted with 100 micrograms mixed with an equal volume of Incomplete Freund's Adjuvant (IFA). Seven days following each boost, the animal was bled. Sera was generated by incubating the blood at 4 °C for 12-24 hours followed by centrifugation.

Ninety-six well plates were coated with B305D antigen by incubating with 50 microliters (typically 1 microgram) of recombinant protein at 4 °C for 20 hours. 250 microliters of BSA blocking buffer was added to the wells and incubated at room temperature for 2 hours. Plates were washed 6 times with PBS/0.01% Tween. Rabbit sera was diluted in PBS. Fifty microliters of diluted sera was added to each well and incubated at room temperature for 30 min. Plates were washed as described above before 50 microliters of goat anti-rabbit horse radish peroxidase (HRP) at a 1:10000 dilution was added and incubated at room temperature for 30 min. Plates were again washed as described above and 100 microliters of TMB microwell peroxidase substrate was added to each well. Following a 15 min incubation in the dark at room temperature, the colorimetric reaction was stopped with 100 microliters of 1N H₂SO₄ and read immediately at 450 nm. The polyclonal antibodies showed immunoreactivity to B305D.

Immunohistochemical (IHC) analysis of B305D expression in breast cancer and normal breast specimens was performed as follows. Paraffin-embedded formal fixed tissue was sliced into 8 micron sections. Steam heat induced epitope retrieval (SHIER) in 0.1 M sodium citrate buffer (pH 6.0) was used for optimal staining conditions. Sections were incubated with 10% serum/PBS for 5 minutes. Primary antibody was added to each section for 25 min at indicated concentrations followed by a 25 min incubation with either an anti-rabbit or anti-mouse biotinylated antibody. Endogenous peroxidase activity was blocked by three 1.5 min incubations with hydrogen peroxide. The avidin biotin complex/horseradish peroxidase (ABC/HRP) systems was used along with DAB chromagen to visualize antigen expression. Slides were counterstained with hematoxylin. B305D expression was detected in both breast tumor and normal breast tissue. However, the intensity of staining was much less in normal samples than in tumor samples and surface expression of B305D was observed only in breast tumor tissues.

A summary of real-time PCR and immunohistochemical analysis of B305D expression in an extensive panel of normal tissues is presented in Table II below. These results demonstrate minimal expression of B305D in testis, inconclusive results in gall bladder, and no detection in all other tissues tested.

**TABLE II**

| **mRNA** | **IHC staining** | **Tissue type** | **Summary** |
|---|---|---|---|
| Moderately positive | Positive | Testis | Nuclear staining of small minority of spermatids; spermatozoa negative; siminoma negative |
| Negative | Negative | Thymus | No expression |
| N/A | Negative | Artery | No expression |
| Negative | Negative | Skeletal muscle | No expression |
| Negative | Positive (weak staining) | Small bowel | No expression |
| Negative | Positive (weak staining) | Ovary | No expression |
| Negative | | Pituitary | No expression |
| Negative | Positive (weak staining) | Stomach | No expression |
| Negative | Negative | Spinal cord | No expression |
| Negative | Negative | Spleen | No expression |
| Negative | Negative | Ureter | No expression |
| N/A | Negative | Gall bladder | Inconclusive |
| N/A | Negative | Placenta | No expression |
| Negative | Negative | Thyroid | No expression |
| Negative | Negative | Heart | No expression |
| Negative | Negative | Kidney | No expression |
| Negative | Negative | Liver | No expression |
| Negative | Negative | Brain-cerebellum | No expression |
| Negative | Negative | Colon | No expression |
| Negative | Negative | Skin | No expression |
| Negative | Negative | Bone marrow | No expression |
| N/A | Negative | Parathyroid | No expression |
| Negative | Negative | Lung | No expression |
| Negative | Negative | Esophagus | No expression |
| Negative | Positive (weak staining) | Uterus | No expression |
| Negative | Negative | Adrenal | No expression |
| Negative | Negative | Pancreas | No expression |
| N/A | Negative | Lymph node | No expression |
| Negative | Negative | Brain-cortex | No expression |
| N/A | Negative | Fallopian tube | No expression |
| Negative | Positive (weak staining) | Bladder | No expression |
| Negative | N/A | Bone | No expression |
| Negative | N/A | Salivary gland | No expression |
| Negative | N/A | Activated PBMC | No expression |
| Negative | N/A | Resting PBMC | No expression |
| Negative | N/A | Trachea | No expression |
| Negative | N/A | Vena cava | No expression |
| Negative | N/A | Retina | No expression |
| Negative | N/A | Cartilage | No expression |

### EXAMPLE 8

### PROTEIN EXPRESSION OF BREAST TUMOR ANTIGENS

This example describes the expression and purification of the breast tumor antigen B305D in *E. coli* and in mammalian cells.

Expression of B305D isoform C-15 (SEQ ID NO:301; translated to 384 amino acids) in *E. coli* was achieved by cloning the open reading frame of B305D isoform C-15 downstream of the first 30 amino acids of the *M. tuberculosis* antigen Ra12 (SEQ ID NO:318) in pET17b. First, the internal EcoRI site in the B305D ORF was mutated without changing the protein sequence so that the gene could be cloned at the EcoRI site with Ra12. The PCR primers used for site-directed mutagenesis are shown in SEQ ID NO:319 (referred to as AW012) and SEQ ID NO:320 (referred to as AW013). The ORF of EcoRI site-modified B305D was then amplified by PCR using the primers AW014 (SEQ ID NO:321) and AW015 (SEQ ID NO:322). The PCR product was digested with EcoRI and ligated to the Ra12/pET17b vector at the EcoRI site. The sequence of the resulting fusion construct (referred to as Ra12mB11C) was confirmed by DNA sequencing. The determined cDNA sequence for the fusion construct is provided in SEQ ID NO:323, with the amino acid sequence being provided in SEQ ID NO:324.

The fusion construct was transformed into BL21 (DE3)CodonPlus-RIL *E. coli* (Stratagene) and grown overnight in LB broth with kanamycin. The resulting culture was induced with IPTG. Protein was transferred to PVDF membrane and blocked with 5% non-fat milk (in PBS-Tween buffer), washed three times and incubated with mouse anti-His tag antibody (Clontech) for 1 hour. The membrane was washed 3 times and probed with HRP-Protein A (Zymed) for 30 min. Finally, the membrane was washed 3 times and developed with ECL (Amersham). Expression was detected by Western blot.

For recombinant expression in mammalian cells, B305D isoform C-15 (SEQ ID NO:301; translated to 384 amino acids) was subcloned into the mammalian expression vectors pCEP4 and pcDNA3.1 (Invitrogen). These constructs were transfected into HEK293 cells (ATCC) using Fugene 6 reagent (Roche). Briefly, the HEK cells were plated at a density of 100,000 cells/ml in DMEM (Gibco) containing 10% FBS (Hyclone) and grown overnight. The following day, 2 ul of Fugene 6 was added to 100 ul of DMEM containing no FBS and incubated for 15 minutes at room temperature. The Fugene 6/DMEM mixture was added to 1 ug of B305D/pCEP4 or B305D/pcDNA plasmid DNA and incubated for 15 minutes at room temperature. The Fugene/DNA mix was then added to the HEK293 cells and incubated for 48-72 hours at 37 °C with 7% CO₂. Cells were rinsed with PBS, the collected and pelleted by centrifugation.

For Western blot analysis, whole cell lysates were generated by incubating the cells in Triton-X100 containing lysis buffer for 30 minutes on ice. Lysates were then cleared by centrifugation at 10,000 rpm for 5 minutes at 4 °C. Samples were diluted with SDS_PAGE loading buffer containing betamercaptoethanol, and boiled for 10 minutes prior to loading the SDS_PAGE gel. Proteins were transferred to nitrocellulose and probed using Protein A purified anti-B305D rabbit polyclonal sera (prepared as described above) at a concentration of 1 ug/ml. The blot was revealed with a goat anti-rabbit Ig coupled to HRP followed by incubation in ECL substrate. Expression of B305D was detected in the the HEK293 lysates transfected with B305D, but not in control HEK293 cells transfected with vector alone.

For FACS analysis, cells were washed further with ice cold staining buffer and then incubated with a 1:100 dilution of a goat anti-rabbit Ig (H+L)-FITC reagent (Southern Biotechnology) for 30 minutes on ice. Following 3 washes, the cells were resuspended in staining buffer containing Propidium Iodide (PI), a vital stain that allows for identification of permeable cells, and then analyzed by FACS. The FACS analysis showed surface expression of B305D protein.

From the foregoing it will be appreciated that, although specific embodiments of the invention have been described herein for purposes of illustration, various modifications may be made without deviating from the spirit and scope of the invention. Accordingly, the invention is not limited except as by the appended claims.

## Claims

1. An isolated polynucleotide comprising a sequence selected from the group consisting of:
(a) sequences provided in SEQ ID NO: 1, 3-86, 142-298, 301-303, 307, 313, 314, 316, 317, 325 and 327-330;
(b) complements of the sequences provided in SEQ ID NO: 1, 3-86, 142-298, 301-303, 307, 313, 314, 316, 317, 325 and 327-330;
(c) sequences consisting of at least 20 contiguous residues of a sequence provided in SEQ ID NO: 1, 3-86, 142-298, 301-303, 307, 313, 314, 316, 317, 325 and 327-330;
(d) sequences that hybridize to a sequence provided in SEQ ID NO: 1, 3-86, 142-298, 301-303, 307, 313, 314, 316, 317, 325 and 327-330, under moderately stringent conditions;
(e) sequences having at least 75% identity to a sequence of SEQ ID NO: 1, 3-86, 142-298, 301-303, 307, 313, 314, 316, 317, 325 and 327-330;
(f) sequences having at least 90% identity to a sequence of SEQ ID NO: 1, 3-86, 142-298, 301-303, 307, 313, 314, 316, 317, 325 and 327-330; and
(g) degenerate variants of a sequence provided in SEQ ID NO: 1, 3-86, 142-298, 301-303, 307, 313, 314, 316, 317, 325 and 327-330.

2. An isolated polypeptide comprising an amino acid sequence selected from the group consisting of:
(a) SEQ ID NO: 299, 300, 304-306, 308-312, 314, 326 and 331-334;
(b) sequences encoded by a polynucleotide of claim 1;
(c) sequences having at least 70% identity to a sequence encoded by a polynucleotide of claim 1; and
(d) sequences having at least 90% identity to a sequence encoded by a polynucleotide of claim 1.

3. An expression vector comprising a polynucleotide of claim 1 operably linked to an expression control sequence.

4. A host cell transformed or transfected with an expression vector according to claim 3.

5. An isolated antibody, or antigen-binding fragment thereof, that specifically binds to a polypeptide of claim 2.

6. A method for detecting the presence of a cancer in a patient, comprising the steps of:
(a) obtaining a biological sample from the patient;
(b) contacting the biological sample with a binding agent that binds to a polypeptide of claim 2;
(c) detecting in the sample an amount of polypeptide that binds to the binding agent; and
(d) comparing the amount of polypeptide to a predetermined cut-off value and therefrom determining the presence of a cancer in the patient.

7. A fusion protein comprising at least one polypeptide according to claim 2.

8. An oligonucleotide that hybridizes to a sequence recited in SEQ ID NO: 1, 3-86, 142-298, 301-303, 307, 313, 314, 316, 317, 325 and 327-330 under moderately stringent conditions.

9. A method for stimulating and/or expanding T cells specific for a tumor protein, comprising contacting T cells with at least one component selected from the group consisting of:
(a) polypeptides according to claim 2;
(b) polynucleotides according to claim 1; and
(c) antigen-presenting cells that express a polypeptide according to claim 2,
under conditions and for a time sufficient to permit the stimulation and/or expansion of T cells.

10. An isolated T cell population, comprising T cells prepared according to the method of claim 9.

11. A composition comprising a first component selected from the group consisting of physiologically acceptable carriers and immunostimulants, and a second component selected from the group consisting of:
(a) polypeptides according to claim 2;
(b) polynucleotides according to claim 1;
(c) antibodies according to claim 5;
(d) fusion proteins according to claim 7;
(e) T cell populations according to claim 10; and
(f) antigen presenting cells that express a polypeptide according to claim 2.

12. A method for stimulating an immune response in a patient, comprising administering to the patient a composition of claim 11.

13. A method for the treatment of a cancer in a patient, comprising administering to the patient a composition of claim 11.

14. A method for determining the presence of a cancer in a patient, comprising the steps of:
(a) obtaining a biological sample from the patient;
(b) contacting the biological sample with an oligonucleotide according to claim 8;
(c) detecting in the sample an amount of a polynucleotide that hybridizes to the oligonucleotide; and
(d) compare the amount of polynucleotide that hybridizes to the oligonucleotide to a predetermined cut-off value, and therefrom determining the presence of the cancer in the patient.

15. A diagnostic kit comprising at least one oligonucleotide according to claim 8.

16. A diagnostic kit comprising at least one antibody according to claim 5 and a detection reagent, wherein the detection reagent comprises a reporter group.

17. A method for inhibiting the development of a cancer in a patient, comprising the steps of:
(a) incubating CD4+ and/or CD8+ T cells isolated from a patient with at least one component selected from the group consisting of: (i) polypeptides according to claim 2; (ii) polynucleotides according to claim 1; and (iii) antigen presenting cells that express a polypeptide of claim 2, such that T cell proliferate;
(b) administering to the patient an effective amount of the proliferated T cells,
and thereby inhibiting the development of a cancer in the patient.


SEQUENCE LISTING

<110> Corixa Corporation
Frudakis, Tony N.
Reed, Steven G.
Smith, John M.
Misher, Linda E.
Dillon, Davin C.
Retter, Marc W.
Wang, Aijun
Skeiky, Yasir A.W.
Harlocker, Susan L.
Day, Craig H.

<120> COMPOSITIONS AND METHODS FOR THE
THERAPY AND DIAGNOSIS OF BREAST CANCER

<130> 210121.41930PC

<140> PCT
<141> 2001-05-22

<160> 334

<170> FastSEQ for Windows Version 3.0

<210> 1
<211> 363
<212> DNA
<213> Homo sapien

<400> 1
ttagagaccc aattgggacc taattgggac ccaaatttct caagtggagg gagaactttt 60
gacgatttcc accggtatct cctcgtgggt attcagggag ctgcccagaa acctataaac 120
ttgtctaagg cgattgaagt cgtccagggg catgatgagt caccaggagt gtttttagag 180
cacctccagg aggcttatcg gatttacacc ccttttgacc tggcagcccc cgaaaatagc 240
catgctctta atttggcatt tgtggctcag gcagccccag atagtaaaag gaaactccaa 300
aaactagagg gatttgctgg gaatgaatac cagtcagctt ttagagatag cctaaaaggt 360
ttt 363

<210> 2
<211> 121
<212> PRT
<213> Homo sapien

<400> 2

<210> 3
<211> 1080
<212> DNA
<213> Homo sapien

<220>
<221> misc_feature
<222> (1)...(1080)
<223> n = A,T,C or G

<400> 3
tcttagaatc ttcatacccc gaactcttgg gaaaacttta atcagtcacc tacagtctac 60
cacccattta ggaggagcaa agctacctca gctcctccgg agccgtttta agatccccca 120
tcttcaaagc ctaacagatc aagcagctct ccggtgcaca acctgcgccc aggtaaatgc 180
caaaaaaggt cctaaaccca gcccaggcca ccgtctccaa gaaaactcac caggagaaaa 240
gtgggaaatt gactttacag aagtaaaacc acaccgggct gggtacaaat accttctagt 300
actggtagac accttctctg gatggactga agcatttgct accaaaaacg aaactgtcaa 360
tatggtagtt aagtttttac tcaatgaaat catccctcga cgtgggctgc ctgttgccat 420
agggtctgat aatggaacgg ccttcgcctt gtctatagtt taatcagtca gtaaggcgtt 480
aaacattcaa tggaagctcc attgtgccta tcgacccaga gctctgggca agtagaacgc 540
atgaactgca ccctaaaaaa acactcttac aaaattaatc ttaaaaaccg gtgttaattg 600
tgttagtctc cttcccttag ccctacttag agttaaggtg caccccttac tgggctgggt 660
tctttacctt ttgaaatcat ntttnggaag gggctgccta tctttnctta actaaaaaan 720
gcccatttgg caaaaatttc ncaactaatt tntacgtncc tacgtctccc caacaggtan 780
aaaaatctnc tgcccttttc aaggaaccat cccatccatt cctnaacaaa aggcctgccn 840
ttcttccccc agttaactnt tttttnttaa aattcccaaa aaangaaccn cctgctggaa 900
aaacnccccc ctccaanccc cggccnaagn ggaaggttcc cttgaatccc ncccccncna 960
anggcccgga accnttaaan tngttccngg gggtnnggcc taaaagnccn atttggtaaa 1020
cctanaaatt ttttcttttn taaaaaccac nntttnnttt ttcttaaaca aaaccctntt 1080

<210> 4
<211> 1087
<212> DNA
<213> Homo sapien

<220>
<221> misc_feature
<222> (1)...(1087)
<223> n = A,T,C or G

<400> 4
tctagagctg cgcctggatc ccgccacagt gaggagacct gaagaccaga gaaaacacag 60
caagtaggcc ctttaaacta ctcacctgtg ttgtcttcta atttattctg ttttattttg 120
tttccatcat tttaaggggt taaaatcatc ttgttcagac ctcagcatat aaaatgaccc 180
atctgtagac ctcaggctcc aaccataccc caagagttgt ctggttttgt ttaaattact 240
gccaggtttc agctgcagat atccctggaa ggaatattcc agattccctg agtagtttcc 300
aggttaaaat cctataggct tcttctgttt tgaggaagag ttcctgtcag agaaaaacat 360
gattttggat ttttaacttt aatgcttgtg aaacgctata aaaaaaattt tctaccccta 420
gctttaaagt actgttagtg agaaattaaa attccttcag gaggattaaa ctgccatttc 480

agttacccta attccaaatg ttttggtggt tagaatcttc tttaatgttc ttgaagaagt 540
gttttatatt ttcccatcna gataaattct ctcncncctt nnttttntnt ctnntttttt 600
aaaacggant cttgctccgt tgtccangct gggaattttn ttttggccaa tctccgctnc 660
cttgcaanaa tnctgcntcc caaaattacc ncctttttcc cacctccacc ccnnggaatt 720
acctggaatt anaggccccc nccccccccc cggctaattt gttttgtttt ttagtaaaaa 780
acgggtttcc tgttttagtt aggatggccc anntctgacc ccntnatcnt ccccctcngc 840
cctcnaatnt tnggnntang gcttaccccc cccngnngtt tttcctccat tnaaattttc 900
tntggantct tgaatnncgg gttttccctt ttaaaccnat tttttttttn nnncccccan 960
ttttncctcc cccntntnta angggggttt cccaanccgg gtccnccccc angtccccaa 1020
tttttctccc cccccctctt ttttctttnc cccaaaantc ctatcttttc ctnnaaatat 1080
cnantnt 1087

<210> 5
<211> 1010
<212> DNA
<213> Homo sapien

<220>
<221> misc_feature
<222> (1)...(1010)
<223> n = A,T,C or G

<400> 5
tctagaccaa gaaatgggag gattttagag tgactgatga tttctctatc atctgcagtt 60
agtaaacatt ctccacagtt tatgcaaaaa gtaacaaaac cactgcagat gacaaacact 120
aggtaacaca catactatct cccaaatacc tacccacaag ctcaacaatt ttaaactgtt 180
aggatcactg gctctaatca ccatgacatg aggtcaccac caaaccatca agcgctaaac 240
agacagaatg tttccactcc tgatccactg tgtgggaaga agcaccgaac ttacccactg 300
gggggcctgc ntcanaanaa aagcccatgc ccccgggtnt ncctttnaac cggaacgaat 360
naacccacca tccccacanc tcctctgttc ntgggccctg catcttgtgg cctcntntnc 420
tttnggggan acntggggaa ggtaccccat ttcnttgacc ccncnanaaa accccngtgg 480
ccctttgccc tgattcncnt gggccttttc tcttttccct tttgggttgt ttaaattccc 540
aatgtccccn gaaccctctc cntnctgccc aaaacctacc taaattnctc nctangnntt 600
ttcttggtgt tncttttcaa aggtnacctt ncctgttcan ncccnacnaa aatttnttcc 660
ntatnntggn cccnnaaaaa nnnatcnncc cnaattgccc gaattggttn ggtttttcct 720
nctgggggaa accctttaaa tttccccctt ggccggcccc ccttttttcc cccctttnga 780
aggcaggngg ttcttcccga acttccaatt ncaacagccn tgcccattgn tgaaaccctt 840
ttcctaaaat taaaaaatan ccggttnngg nnggcctctt tcccctccng gngggnngng 900
aaantcctta ccccnaaaaa ggttgcttag cccccngtcc ccactccccc nggaaaaatn 960
aaccttttcn aaaaaaggaa tataantttn ccactccttn gttctcttcc 1010

<210> 6
<211> 950
<212> DNA
<213> Homo sapien

<220>
<221> misc_feature
<222> (1)...(950)
<223> n = A,T,C or G

<400> 6
tctagagctc gcggccgcga gctctaatac gactcactat agggcgtcga ctcgatctca 60
gctcactgca atctctgccc ccggggtcat gcgattctcc tgcctcagcc ttccaagtag 120
ctgggattac aggcgtgcaa caccacaccc ggctaatttt gtatttttaa tagagatggg 180
gttttccctt gttggccann atggtctcna acccctgacc tcnngtgatc cccccncccn 240
nganctcnna ctgctgggga tnnccgnnnn nnncctcccn ncncnnnnnn ncncnntccn 300
tnntccttnc tcnnnnnnnn cnntcnntcc nncttctcnc cnnntnttnt cnncnnccnn 360

cnnnccncnt ncccncnnnt tcncntncnn tntccnncnn nntcnncnnn cnnnncntnn 420
ccnntacntc ntnnncnnnt ccntctntnn cctcnncnnt cnctncncnt tntctcctcn 480
ntnnnnnnct ccnnnnntct cntcncnncn tncctcnntn nccncncccc ncctcncnnc 540
ctnntttnnn cnncnnntcc ntnccnttcn nntccnntnn cnncntcncn nncnttnttc 600
ccnccnnttc cttncncntn nnntntcnnn cncntcnntc ntttnctcct nnntcccnnc 660
tcnnttcncc cnnntccncc ccccncctnt ctctcncccn nntnnntntn nnncntccnc 720
tntcncnttc ntcnntncnt tnctntcnnc nncnntncnc tnccntntnt ctnnntcncn 780
tcncntntcn ccntccnttn ctntctcctn tntccttccc ctcncctnct cnttcnccnc 840
ccnntntntn tnncnccnnt nctnnncnnc cntcntttcn tctctnctnn nnntnncctc 900
nncccntncc ctnntncnct nctnntaccn tnctnctccn tcttccttcc 950

<210> 7
<211> 1086
<212> DNA
<213> Homo sapien

<220>
<221> misc_feature
<222> (1)...(1086)
<223> n = A,T,C or G

<400> 7
tctagagctc gcggccgcga gctcaattaa ccctcactaa agggagtcga ctcgatcaga 60
ctgttactgt gtctatgtag aaagaagtag acataagaga ttccattttg ttctgtacta 120
agaaaaattc ttctgccttg agatgctgtt aatctgtaac cctagcccca accctgtgct 180
cacagagaca tgtgctgtgt tgactcaagg ttcaatggat ttagggctat gctttgttaa 240
aaaagtgctt gaagataata tgcttgttaa aagtcatcac cattctctaa tctcaagtac 300
ccagggacac aatacactgc ggaaggccgc agggacctct gtctaggaaa gccaggtatt 360
gtccaagatt tctccccatg tgatagcctg agatatggcc tcatgggaag ggtaagacct 420
gactgtcccc cagcccgaca tcccccagcc cgacatcccc cagcccgaca cccgaaaagg 480
gtctgtgctg aggaagatta ntaaaagagg aaggctcttt gcattgaagt aagaagaagg 540
ctctgtctcc tgctcgtccc tgggcaataa aatgtcttgg tgttaaaccc gaatgtatgt 600
tctacttact gagaatagga gaaaacatcc ttagggctgg aggtgagaca ccctggcggc 660
atactgctct ttaatgcacg agatgtttgt ntaattgcca tccagggcca ncccctttcc 720
ttaacttttt atganacaaa aactttgttc ncttttcctg cgaacctctc cccctattan 780
cctattggcc tgcccatccc ctccccaaan ggtgaaaana tgttcntaaa tncgagggaa 840
tccaaaacnt tttcccgttg gtcccctttc caaccccgtc cctgggccnn tttcctcccc 900
aacntgtccc ggntccttcn ttcccncccc cttcccngan aaaaaacccc gtntganggn 960
gccccctcaa attataacct ttccnaaaca aannggttcn aaggtggttt gnttccggtg 1020
cggctggcct tgaggtcccc cctncacccc aatttggaan ccngtttttt ttattgcccn 1080
ntcccc 1086

<210> 8
<211> 1177
<212> DNA
<213> Homo sapien

<220>
<221> misc_feature
<222> (1)...(1177)
<223> n = A,T,C or G

<400> 8
nccntttaga tgttgacaan ntaaacaagc ngctcaggca gctgaaaaaa gccactgata 60
aagcatcctg gagtatcaga gtttactgtt agatcagcct catttgactt cccctcccac 120
atggtgttta aatccagcta cactacttcc tgactcaaac tccactattc ctgttcatga 180
ctgtcaggaa ctgttggaaa ctactgaaac tggccgacct gatcttcaaa atgtgcccct 240
aggaaaggtg gatgccaccg tgttcacaga cagtaccncc ttcctcgaga agggactacg 300

aggggccggt gcanctgtta ccaaggagac tnatgtgttg tgggctcagg ctttaccanc 360
aaacacctca ncncnnaagg ctgaattgat cgccctcact caggctctcg gatggggtaa 420
gggatattaa cgttaacact gacagcaggt acgcctttgc tactgtgcat gtacgtggag 480
ccatctacca ggagcgtggg ctactcactc ggcaggtggc tgtnatccac tgtaaangga 540
catcaaaagg aaaacnnggc tgttgcccgt ggtaaccana aanctgatcn ncagctcnaa 600
gatgctgtgt tgactttcac tcncncctct taaacttgct gcccacantc tcctttccca 660
accagatctg cctgacaatc cccatactca aaaaaaaaan aanactggcc ccgaacccna 720
accaataaaa acggggangg tnggtnganc nncctgaccc aaaaataatg gatcccccgg 780
gctgcaggaa ttcaattcan ccttatcnat acccccaacn nggngggggg ggccngtncc 840
cattncccct ntattnattc tttnnccccc cccccggcnt cctttttnaa ctcgtgaaag 900
ggaaaacctg ncttaccaan ttatcncctg gaccntcccc ttccncggtn gnttanaaaa 960
aaaagcccnc antcccntcc naaatttgca cngaaaggna aggaatttaa cctttatttt 1020
ttnntccttt antttgtnnn ccccctttta cccaggcgaa cngccatcnt ttaanaaaaa 1080
aaanagaang tttatttttc cttngaacca tcccaatana aancacccgc nggggaacgg 1140
ggnggnaggc cnctcacccc ctttntgtng gngggnc 1177

<210> 9
<211> 1146
<212> DNA
<213> Homo sapien

<220>
<221> misc_feature
<222> (1)...(1146)
<223> n = A,T,C or G

<400> 9
nccnnttnnt gatgttgtct ttttggcctc tctttggata ctttccctct cttcagaggt 60
gaaaagggtc aaaaggagct gttgacagtc atcccaggtg ggccaatgtg tccagagtac 120
agactccatc agtgaggtca aagcctgggg cttttcagag aagggaggat tatgggtttt 180
ccaattatac aagtcagaag tagaaagaag ggacataaac caggaagggg gtggagcact 240
catcacccag agggacttgt gcctctctca gtggtagtag aggggctact tcctcccacc 300
acggttgcaa ccaagaggca atgggtgatg agcctacagg ggacatancc gaggagacat 360
gggatgaccc taagggagta ggctggtttt aaggcggtgg gactgggtga gggaaactct 420
cctcttcttc agagagaagc agtacagggc gagctgaacc ggctgaaggt cgaggcgaaa 480
acacggtctg gctcaggaag accttggaag taaaattatg aatggtgcat gaatggagcc 540
atggaagggg tgctcctgac caaactcagc cattgatcaa tgttagggaa actgatcagg 600
gaagccggga atttcattaa caacccgcca cacagcttga acattgtgag gttcagtgac 660
ccttcaaggg gccactccac tccaactttg gccattctac tttgcnaaat ttccaaaact 720
tcctttttta aggccgaatc cntantccct naaaaacnaa aaaaaatctg cncctattct 780
ggaaaaggcc cancccttac caggctggaa gaaattttnc cttttttttt tttttgaagg 840
cntttnttaa attgaacctn aattcncccc cccaaaaaaa aacccnccng gggggcggat 900
ttccaaaaac naattccctt accaaaaaac aaaaacccnc ccttnttccc ttccnccctn 960
ttcttttaat tagggagaga tnaagccccc caatttccng gnctngatnn gtttcccccc 1020
cccccatttt ccnaaacttt ttcccancna ggaanccncc ctttttttng gtcngattna 1080
ncaaccttcc aaaccatttt tccnnaaaaa ntttgntngg ngggaaaaan acctnntttt 1140
atagan 1146

<210> 10
<211> 545
<212> DNA
<213> Homo sapien

<400> 10
cttcattggg tacgggcccc ctcgaggtcg acggtatcga taagcttgat atcgaattcc 60
tgcagcccgg gggatccact agttctagag tcaggaagaa ccaccaacct tcctgatttt 120
tattggctct gagttctgag gccagttttc ttcttctgtt gagtatgcgg gattgtcagg 180
cagatctggc tgtggaaagg agactgtggg cagcaagttt agaggcgtga ctgaaagtca 240

cactgcatct tgagctgctg aatcagcttt ctggttacca cgggcaacag ccgtgttttc 300
cttttgatgt cctttacagt ggattacagc cacctgctga ggtgagtagc ccacgctcct 360
ggtagatggc tccacgtaca tgcacagtag caaaggcgta cctgctgtca gtgttaacgt 420
taatatcctt accccatcgg agagcctgag tgagggcgat caattcagcc cttttgtgct 480
gaggtgtttg ctggttaagc cctgaaccca caacacatct gtctccatgg taacagctgc 540
accgg 545

<210> 11
<211> 196
<212> DNA
<213> Homo sapien

<400> 11
tctcctaggc tgggcacagt ggctcatacc tgtaatcctg accgtttcag aggctcaggt 60
ggggggatcg cttgagccca agatttcaag actagtctgg gtaacatagt gagaccctat 120
ctctacgaaa aaataaaaaa atgagcctgg tgtagtggca cacaccagct gaggagggag 180
aatcgagcct aggaga 196

<210> 12
<211> 388
<212> DNA
<213> Homo sapien

<220>
<221> misc_feature
<222> (1)...(388)
<223> n = A,T,C or G

<400> 12
tctcctaggc ttgggggctc tgactagaaa ttcaaggaac ctgggattca agtccaactg 60
tgacaccaac ttacactgtg gnctccaata aactgcttct ttcctattcc ctctctatta 120
aataaaataa ggaaaacgat gtctgtgtat agccaagtca gntatcctaa aaggagatac 180
taagtgacat taaatatcag aatgtaaaac ctgggaacca ggttcccagc ctgggattaa 240
actgacagca agaagactga acagtactac tgtgaaaagc ccgaagnggc aatatgttca 300
ctctaccgtt gaaggatggc tgggagaatg aatgctctgt cccccagtcc caagctcact 360
tactatacct cctttatagc ctaggaga 388

<210> 13
<211> 337
<212> DNA
<213> Homo sapien

<400> 13
tagtagttgc ctataatcat gtttctcatt attttcacat tttattaacc aatttctgtt 60
taccctgaaa aatatgaggg aaatatatga aacagggagg caatgttcag ataattgatc 120
acaagatatg atttctacat cagatgctct ttcctttcct gtttatttcc tttttatttc 180
ggttgtgggg tcgaatgtaa tagctttgtt tcaagagaga gttttggcag tttctgtagc 240
ttctgacact gctcatgtct ccaggcatct atttgcactt taggaggtgt cgtgggagac 300
tgagaggtct attttttcca tatttgggca actacta 337

<210> 14
<211> 571
<212> DNA
<213> Homo sapien

<220>
<221> misc_feature
<222> (1)...(571)

<223> n = A,T,C or G

<400> 14
tagtagttgc catacagtgc ctttccattt atttaacccc cacctgaacg gcataaactg 60
agtgttcagc tggtgttttt tactgtaaac aataaggaga ctttgctctt catttaaacc 120
aaaatcatat ttcatatttt acgctcgagg gtttttaccg gttccttttt acactcctta 180
aaacagtttt taagtcgttt ggaacaagat attttttctt tcctggcagc ttttaacatt 240
atagcaaatt tgtgtctggg ggactgctgg tcactgtttc tcacagttgc aaatcaaggc 300
atttgcaacc aagaaaaaaa aatttttttg ttttatttga aactggaccg gataaacggt 360
gtttggagcg gctgctgtat atagttttaa atggtttatt gcacctcctt aagttgcact 420
tatgtggggg ggggnttttg natagaaagt ntttantcac anagtcacag ggacttttnt 480
cttttggnna ctgagctaaa aagggctgnt tttcgggtgg gggcagatga aggctcacag 540
gaggcctttc tcttagaggg gggaactnct a 571

<210> 15
<211> 548
<212> DNA
<213> Homo sapien

<220>
<221> misc_feature
<222> (1)...(548)
<223> n = A,T,C or G

<400> 15
tatatattta ataacttaaa tatattttga tcacccactg gggtgataag acaatagata 60
taaaagtatt tccaaaaagc ataaaaccaa agtatcatac caaaccaaat tcatactgct 120
tcccccaccc gcactgaaac ttcaccttct aactgtctac ctaaccaaat tctacccttc 180
aagtctttgg tgcgtgctca ctactctttt tttttttttt tttnttttgg agatggagtc 240
tggctgtgca gcccaggggt ggagtacaat ggcacaacct cagctcactg naacctccgc 300
ctcccaggtt catgagattc tcctgnttca gccttcccag tagctgggac tacaggtgtg 360
catcaccatg cctggntaat cttttttngt tttngggtag agatgggggt tttacatgtt 420
ggccaggntg gtntcgaact cctgacctca agtgatccac ccacctcagg ctcccaaagt 480
gctaggatta cagacatgag ccactgngcc cagncctggt gcatgctcac ttctctaggc 540
aactacta 548

<210> 16
<211> 638
<212> DNA
<213> Homo sapien

<220>
<221> misc_feature
<222> (1)...(638)
<223> n = A,T,C or G

<400> 16
ttccgttatg cacatgcaga atattctatc ggtacttcag ctattactca ttttgatggc 60
gcaatccgag cctatcctca agatgagtat ttagaaagaa ttgatttagc gatagaccaa 120
gctggtaagc actctgacta cacgaaattg ttcagatgtg atggatttat gacagttgat 180
ctttggaaga gattattaag tgattatttt aaagggaatc cattaattcc agaatatctt 240
ggtttagctc aagatgatat agaaatagaa cagaaagaga ctacaaatga agatgtatca 300
ccaactgata ttgaagagcc tatagtagaa aatgaattag ctgcatttat tagccttaca 360
catagcgatt ttcctgatga atcttatatt cagccatcga catagcatta cctgatgggc 420
aaccttacga ataatagaaa ctgggtgcgg ggctattgat gaattcatcc ncagtaaatt 480
tggatatnac aaaatataac tcgattgcat ttggatgatg gaatactaaa tctggcaaaa 540
gtaactttgg agctactagt aacctctctt tttgagatgc aaaattttct tttagggttt 600
cttattctct actttacgga tattggagca taacggga 638

<210> 17
<211> 286
<212> DNA
<213> Homo sapien

<400> 17
actgatggat gtcgccggag gcgaggggcc ttatctgatg ctcggctgcc tgttcgtgat 60
gtgcgcggcg attgggctgt ttatctcaaa caccgccacg gcggtgctga tggcgcctat 120
tgccttagcg gcggcgaagt caatgggcgt ctcaccctat ccttttgcca tggtggtggc 180
gatggcggct tcggcggcgt ttatgacccc ggtctcctcg ccggttaaca ccctggtgct 240
tggccctggc aagtactcat ttagcgattt tgtcaaaata ggcgtg 286

<210> 18
<211> 262
<212> DNA
<213> Homo sapien

<220>
<221> misc_feature
<222> (1)...(262)
<223> n = A,T,C or G

<400> 18
tcggtcatag cagccccttc ttctcaattt catctgtcac taccctggtg tagtatctca 60
tagccttaca tttttatagc ctcctccctg gtctgtcttt tgattttcct gcctgtaatc 120
catatcacac ataactgcaa gtaaacattt ctaaagtgtg gttatgctca tgtcactcct 180
gtgncaagaa atagtttcca ttaccgtctt aataaaattc ggatttgttc tttnctattn 240
tcactcttca cctatgaccg aa 262

<210> 19
<211> 261
<212> DNA
<213> Homo sapien

<400> 19
tcggtcatag caaagccagt ggtttgagct ctctactgtg taaactccta aaccaaggcc 60
atttatgata aatggtggca ggatttttat tataaacatg tacccatgca aatttcctat 120
aactctgaga tatattcttc tacatttaaa caataaaaat aatctatttt taaaagccta 180
atttgcgtag ttaggtaaga gtgtttaatg agagggtata aggtataaat caccagtcaa 240
cgtttctctg cctatgaccg a 261

<210> 20
<211> 294
<212> DNA
<213> Homo sapien

<220>
<221> misc_feature
<222> (1)...(294)
<223> n = A,T,C or G

<400> 20
tacaacgagg cgacgtcggt aaaatcggac atgaagccac cgctggtctt ttcgtccgag 60
cgataggcgc cggccagcca gcggaacggt tgcccggatg gcgaagcgag ccggagttct 120
tcggactgag tatgaatctt gttgtgaaaa tactcgccgc cttcgttcga cgacgtcgcg 180
tcgaaatctt cganctcctt acgatcgaag tcttcgtggg cgacgatcgc ggtcagttcc 240
gccccaccga aatcatggtt gagccggatg ctgnccccga agncctcgtt tgtn 294

<210> 21
<211> 208
<212> DNA
<213> Homo sapien

<220>
<221> misc_feature
<222> (1)...(208)
<223> n = A,T,C or G

<400> 21
ttggtaaagg gcatggacgc agacgcctga cgtttggctg aaaatctttc attgattcgt 60
atcaatgaat aggaaaattc ccaaagaggg aatgtcctgt tgctcgccag tttttntgtt 120
gttctcatgg anaaggcaan gagctcttca gactattggn attntcgttc ggtcttctgc 180
caactagtcg ncttgcnang atcttcat 208

<210> 22
<211> 287
<212> DNA
<213> Homo sapien

<220>
<221> misc_feature
<222> (1)...(287)
<223> n = A,T,C or G

<400> 22
nccnttgagc tgagtgattg agatntgtaa tggttgtaag ggtgattcag gcggattagg 60
gtggcgggtc acccggcagt gggtctcccg acaggccagc aggatttggg gcaggtacgg 120
ngtgcgcatc gctcgactat atgctatggc aggcgagccg tggaaggngg atcaggtcac 180
ggcgctggag ctttccacgg tccatgnatt gngatggctg ttctaggcgg ctgttgccaa 240
gcgtgatggt acgctggctg gagcattgat ttctggtgcc aaggtgg 287

<210> 23
<211> 204
<212> DNA
<213> Homo sapien

<220>
<221> misc_feature
<222> (1)...(204)
<223> n = A,T,C or G

<400> 23
ttgggtaaag ggagcaagga gaaggcatgg agaggctcan gctggtcctg gcctacgact 60
gggccaagct gtcgccgggg atggtggaga actgaagcgg gacctcctcg aggtcctccg 120
ncgttacttc nccgtccagg aggagggtct ttccgtggtc tnggaggagc ggggggagaa 180
gatnctcctc atggtcnaca tccc 204

<210> 24
<211> 264
<212> DNA
<213> Homo sapien

<220>
<221> misc_feature
<222> (1)...(264)

<223> n = A,T,C or G

<400> 24
tggattggtc aggagcgggt agagtggcac cattgagggg atattcaaaa atattatttt 60
gtcctaaatg atagttgctg agtttttctt tgacccatga gttatattgg agtttatttt 120
ttaactttcc aatcgcatgg acatgttaga cttattttct gttaatgatt nctattttta 180
ttaaattgga tttgagaaat tggttnttat tatatcaatt tttggtattt gttgagtttg 240
acattatagc ttagtatgtg acca 264

<210> 25
<211> 376
<212> DNA
<213> Homo sapien

<220>
<221> misc_feature
<222> (1)...(376)
<223> n = A,T,C or G

<400> 25
ttacaacgag gggaaactcc gtctctacaa aaattaaaaa attagccagg tgtggtggtg 60
tgcacccgca atcccagcta cttgggaggt tgagacacaa gantcaccta natgtgggag 120
gtcaaggttg catgagtcat gattgtgcca ctgcactcca gcctgggtga cagaccgaga 180
ccctgcctca anaganaang aataggaagt tcagaaatcn tggntgtggn gcccagcaat 240
ctgcatctat ncaacccctg caggcaangc tgatgcagcc tangttcaag agctgctgtt 300
tctggaggca gcagttnggg cttccatcca gtatcacggc cacactcgca cnagccatct 360
gtcctccgtn tgtnac 376

<210> 26
<211> 372
<212> DNA
<213> Homo sapien

<220>
<221> misc_feature
<222> (1)...(372)
<223> n = A,T,C or G

<400> 26
ttacaacgag gggaaactcc gtctctacaa aaattaaaaa attagccagg tgtggtggtg 60
tgcacctgta atcccagcta cttgggcggc tgagacacaa gaaccaccta aatgtgggag 120
ggtcaaggtt gcatgagtca tgatcgcgcc actgcactcc agcctgggtg acagactgag 180
accctgcctc aaaagaaaaa gaataggaag ttcagaaacc ctgggtgtgg ngcccagcaa 240
tctgcattta aacaatccct gcaggcaatg ctgatgcagc ctaagttcaa gagctgctgt 300
tctggaggca gnagtaaggg cttccatcca gcatcacggn caacactgca aaagcacctg 360
tcctcgttgg ta 372

<210> 27
<211> 477
<212> DNA
<213> Homo sapien

<400> 27
   ttctgtccac atctacaagt tttatttatt ttgtgggttt tcagggtgac taagtttttc 60
   cctacattga aaagagaagt tgctaaaagg tgcacaggaa atcatttttt taagtgaata 120
   tgataatatg ggtccgtgct taatacaact gagacatatt tgttctctgt ttttttagag 180
   tcacctctta aagtccaatc ccacaatggt gaaaaaaaaa tagaaagtat ttgttctacc 240
   tttaaggaga ctgcagggat tctccttgaa aacggagtat ggaatcaatc ttaaataaat 300
   atgaaattgg ttggtcttct gggataagaa attcccaact cagtgtgctg aaattcacct 360
   gacttttttt gggaaaaaat agtcgaaaat gtcaatttgg tccataaaat acatgttact 420
   attaaaagat atttaaagac aaattctttc agagctctaa gattggtgtg gacagaa 477

<210> 28
<211> 438
<212> DNA
<213> Homo sapien

<220>
<221> misc_feature
<222> (1)...(438)
<223> n = A,T,C or G

<400> 28
tctncaacct cttgantgtc aaaaaccttn taggctatct ctaaaagctg actggtattc 60
attccagcaa aatccctcta gtttttggag tttcctttta ctatctgggg ctgcctgagc 120
cacaaatgcc aaattaagag catggctatt ttcgggggct gacaggtcaa aaggggtgta 180
aatccgataa gcctcctgga ggtgctctaa aaacactcct ggtgactcat catgcccctg 240
gacgacttca atcgncttag acaagtttat aggtttctgg gcagctccct gaatacccac 300
gaggagatac cggtggaaat cgtcaaaagt tctccctcca cttgagaaat ttgggtccca 360
attaggtccc aattgggtct ctaatcacta ttcctctagc ttcctcctcc ggnctattgg 420
ttgatgtgag gttgaaga 438

<210> 29
<211> 620
<212> DNA
<213> Homo sapien

<220>
<221> misc_feature
<222> (1)...(620)
<223> n = A,T,C or G

<400> 29
aagagggtac cagccccaag ccttgacaac ttccataggg tgtcaagcct gtgggtgcac 60
agaagtcaaa aattgagttt tgggatcctc agcctagatt tcagaggata taaagaaaca 120
cctaacacct agatattcag acaaaagttt actacaggga tgaagctttc acggaaaacc 180
tctactagga aagtacagaa gagaaatgtg ggtttggagc ccccaaacag aatcccctct 240
agaacactgc ctaatgaaac tgtgagaaga tggccactgt catccagaca ccagaatgat 300
agacccacca aaaacttatg ccatattgcc tataaaacct acagacactc aatgccagcc 360
ccatgaaaaa aaaactgaga agaagactgt nccctacaat gccaccggag cagaactgcc 420
ccaggccatg gaagcacagc tcttatatca atgtgacctg gatgttgaga catggaatcc 480
nangaaatcn ttttaanact tccacggttn aatgactgcc ctattanatt cngaacttan 540
atccnggcct gtgacctctt tgctttggcc attccccctt tttggaatgg ctnttttttt 600
cccatgcctg tnccctctta 620

<210> 30
<211> 100
<212> DNA
<213> Homo sapien

<400> 30
ttacaacgag ggggtcaatg tcataaatgt cacaataaaa caatctcttc tttttttttt 60
tttttttttt tttttttttt tttttttttt tttttttttt 100

<210> 31
<211> 762

<212> DNA
<213> Homo sapien

<220>
<221> misc_feature
<222> (1)...(762)
<223> n = A,T,C or G

<400> 31
tagtctatgc gccggacaga gcagaattaa attggaagtt gccctccgga ctttctaccc 60
acactcttcc tgaaaagaga aagaaaagag gcaggaaaga ggttaggatt tcattttcaa 120
gagtcagcta attaggagag cagagtttag acagcagtag gcaccccatg atacaaacca 180
tggacaaagt ccctgtttag taactgccag acatgatcct gctcaggttt tgaaatctct 240
ctgcccataa aagatggaga gcaggagtgc catccacatc aacacgtgtc caagaaagag 300
tctcagggag acaagggtat caaaaaacaa gattcttaat gggaaggaaa tcaaaccaaa 360
aaattagatt tttctctaca tatatataat atacagatat ttaacacatt attccagagg 420
tggctccagt ccttggggct tgagagatgg tgaaaacttt tgttccacat taacttctgc 480
tctcaaattc tgaagtatat cagaatggga caggcaatgt tttgctccac actggggcac 540
agacccaaat ggttctgtgc ccgaagaaga gaagcccgaa agacatgaag gatgcttaag 600
gggggttggg aaagccaaat tggtantatc ttttcctcct gcctgtgttc cngaagtctc 660
cnctgaagga attcttaaaa ccctttgtga ggaaatgccc ccttaccatg acaantggtc 720
ccattgcttt tagggngatg gaaacaccaa gggttttgat cc 762

<210> 32
<211> 276
<212> DNA
<213> Homo sapien

<400> 32
tagtctatgc gtgtattaac ctcccctccc tcagtaacaa ccaaagaggc aggagctgtt 60
attaccaacc ccattttaca gatgcatcaa taatgacaga gaagtgaagt gacttgcgca 120
cacaaccagt aaattggcag agtcagattt gaatccatgg agtctggtct gcactttcaa 180
tcaccgaata ccctttctaa gaaacgtgtg ctgaatgagt gcatggataa atcagtgtct 240
actcaacatc tttgcctaga tatcccgcat agacta 276

<210> 33
<211> 477
<212> DNA
<213> Homo sapien

<400> 33
tagtagttgc caaatatttg aaaatttacc cagaagtgat tgaaaacttt ttggaaacaa 60
aaacaaataa agccaaaagg taaaataaaa atatctttgc actctcgtta ttacctatcc 120
ataacttttt caccgtaagc tctcctgctt gttagtgtag tgtggttata ttaaactttt 180
tagttattat tttttattca cttttccact agaaagtcat tattgattta gcacacatgt 240
tgatctcatt tcattttttc tttttatagg caaaatttga tgctatgcaa caaaaatact 300
caagcccatt atcttttttc cccccgaaat ctgaaaattg caggggacag agggaagtta 360
tcccattaaa aaattgtaaa tatgttcagt ttatgtttaa aaatgcacaa aacataagaa 420
aattgtgttt acttgagctg ctgattgtaa gcagttttat ctcaggggca actacta 477

<210> 34
<211> 631
<212> DNA
<213> Homo sapien

<400> 34
tagtagttgc caattcagat gatcagaaat gctgctttcc tcagcattgt cttgttaaac 60
cgcatgccat ttggaacttt ggcagtgaga agccaaaagg aagaggtgaa tgacatatat 120

atatatatat attcaatgaa agtaaaatgt atatgctcat atactttcta gttatcagaa 180
tgagttaagc tttatgccat tgggctgctg catattttaa tcagaagata aaagaaaatc 240
tgggcatttt tagaatgtga tacatgtttt tttaaaactg ttaaatatta tttcgatatt 300
tgtctaagaa ccggaatgtt cttaaaattt actaaaacag tattgtttga ggaagagaaa 360
actgtactgt ttgccattat tacagtcgta caagtgcatg tcaagtcacc cactctctca 420
ggcatcagta tccacctcat agctttacac attttgacgg ggaatattgc agcatcctca 480
ggcctgacat ctgggaaagg ctcagatcca cctactgctc cttgctcgtt gatttgtttt 540
aaaatattgt gcctggtgtc acttttaagc cacagccctg cctaaaagcc agcagagaac 600
agaacccgca ccattctata ggcaactact a 631

<210> 35
<211> 578
<212> DNA
<213> Homo sapien

<400> 35
tagtagttgc catcccatat tacagaaggc tctgtataca tgacttattt ggaagtgatc 60
tgttttctct ccaaacccat ttatcgtaat ttcaccagtc ttggatcaat cttggtttcc 120
actgatacca tgaaacctac ttggagcaga cattgcacag ttttctgtgg taaaaactaa 180
aggtttattt gctaagctgt catcttatgc ttagtatttt ttttttacag tggggaattg 240
ctgagattac attttgttat tcattagata ctttgggata acttgacact gtcttctttt 300
tttcgctttt aattgctatc atcatgcttt tgaaacaaga acacattagt cctcaagtat 360
tacataagct tgcttgttac gcctggtggt ttaaaggact atctttggcc tcaggttcac 420
aagaatgggc aaagtgtttc cttatgttct gtagttctca ataaaagatt gccaggggcc 480
gggtactgtg gctcgcactg taatcccagc actttgggaa gctgaggctg gcggatcatg 540
ttagggcagg tgttcgaaac cagcctgggc aactacta 578

<210> 36
<211> 583
<212> DNA
<213> Homo sapien

<400> 36
tagtagttgc ctgtaatccc agcaactcag gaggctgggg caggagaatc agttgaacct 60
gggaggcaga agttgtaatt agcaaagatc gcaccattgc acttcagcct gggcaacaag 120
agtgagattc catctcaaaa acaaaaaaaa gaaaaagaaa agaaaaggaa aaaacgtata 180
aacccagcca aaacaaaatg atcattcttt taataagcaa gactaattta atgtgtttat 240
ttaatcaaag cagttgaatc ttctgagtta ttggtgaaaa tacccatgta gttaatttag 300
ggttcttact tgggtgaacg tttgatgttc acaggttata aaatggttaa caaggaaaat 360
gatgcataaa gaatcttata aactactaaa aataaataaa atataaatgg ataggtgcta 420
tggatggagt ttttgtgtaa tttaaaatct tgaagtcatt ttggatgctc attggttgtc 480
tggtaatttc cattaggaaa aggttatgat atggggaaac tgtttctgga aattgcggaa 540
tgtttctcat ctgtaaaatg ctagtatctc agggcaacta cta 583

<210> 37
<211> 716
<212> DNA
<213> Homo sapien

<220>
<221> misc_feature
<222> (1)...(716)
<223> n = A,T,C or G

<400> 37
gatctactag tcatntggat tctatccatg gcagctaagc ctttctgaat ggattctact 60
gctttcttgt tctttaatcc agacccttat atatgtttat gttcacaggc agggcaatgt 120
ttagtgaaaa caattctaaa ttttttattt tgcattttca tgctaatttc cgtcacactc 180

cagcaggctt cctgggagaa taaggagaaa tacagctaaa gacattgtcc ctgcttactt 240
acagcctaat ggtatgcaaa accacttcaa taaagtaaca ggaaaagtac taaccaggta 300
gaatggacca aaactgatat agaaaaatca gaggaagaga ggaacaaata tttactgagt 360
cctagaatgt acaaggcttt ttaattacat attttatgta aggcctgcaa aaaacaggtg 420
agtaatcaac atttgtccca ttttacatat aaggaaactg aagcttaaat tgaataattt 480
aatgcataga ttttatagtt agaccatgtt caggtcccta tgttatactt actagctgta 540
tgaatatgag aaaataattt tgttattttc ttggcatcag tattttcatc tgcaaaataa 600
agctaaagtt atttagcaaa cagtcagcat agtgcctgat acatagtagg tgctccaaac 660
atgattacnc tantattngg tattanaaaa atccaatata ggcntggata aaaccg 716

<210> 38
<211> 688
<212> DNA
<213> Homo sapien

<220>
<221> misc_feature
<222> (1)...(688)
<223> n = A,T,C or G

<400> 38
   ttctgtccac atatcatccc actttaattg ttaatcagca aaactttcaa tgaaaaatca 60
   tccattttaa ccaggatcac accaggaaac tgaaggtgta ttttttttta ccttaaaaaa 120
   aaaaaaaaaa accaaacaaa ccaaaacaga ttaacagcaa agagttctaa aaaatttaca 180
   tttctcttac aactgtcatt cagagaacaa tagttcttaa gtctgttaaa tcttggcatt 240
   aacagagaaa cttgatgaan agttgtactt ggaatattgt ggattttttt ttttgtctaa 300
   tctcccccta ttgttttgcc aacagtaatt taagtttgtg tggaacatcc ccgtagttga 360
   agtgtaaaca atgtatagga aggaatatat gataagatga tgcatcacat atgcattaca 420
   tgtagggacc ttcacaactt catgcactca gaaaacatgc ttgaagagga ggagaggacg 480
   gcccagggtc accatccagg tgccttgagg acagagaatg cagaagtggc actgttgaaa 540
   tttagaagac catgtgtgaa tggtttcagg cctgggatgt ttgccaccaa gaagtgcctc 600
   cgagaaattt ctttcccatt tggaatacag ggtggcttga tgggtacggt gggtgaccca 660
   acgaagaaaa tgaaattctg ccctttcc 688

<210> 39
<211> 585
<212> DNA
<213> Homo sapien

<220>
<221> misc_feature
<222> (1)...(585)
<223> n = A,T,C or G

<400> 39
   tagtagttgc cgcnnaccta aaanttggaa agcatgatgt ctaggaaaca tantaaaata 60
   gggtatgcct atgtgctaca gagagatgtt agcatttaaa gtgcatantt ttatgtattt 120
   tgacaaatgc atatncctct ataatccaca actgattacg aagctattac aattaaaaag 180
   tttggccggg cgtggtgggc ggtggctgac gcctgtaatc ccagcacttt gggaggccga 240
   ggcacgcgga tcacgaggtc gggagttcaa gaccatcctg gctaacacgg tgaaagtcca 300
   tctctactaa aaatacgaaa aaattacccc ggcgtggtgg cgggcgcctg tagtcccagc 360
   tactccggag gctgaggcag gagaatggcg tgaacccagg acacggagct tgcagtgtgc 420
   caacatcacg tcactgccct ccagcctggg ggacaggaac aagantcccg tcctcanaaa 480
   agaaaaatac tactnatant ttcnacttta ttttaantta cacagaactn cctcttggta 540
   cccccttacc attcatctca cccacctcct atagggcacn nctaa 585

<210> 40
<211> 475

<212> DNA
<213> Homo sapien

<400> 40
tctgtccaca ccaatcttag aagctctgaa aagaatttgt ctttaaatat cttttaatag 60
taacatgtat tttatggacc aaattgacat tttcgactgt tttttccaaa aaagtcaggt 120
gaatttcagc acactgagtt gggaatttct tatcccagaa gaccaaccaa tttcatattt 180
atttaagatt gattccatac tccgttttca aggagaatcc ctgcagtctc cttaaaggta 240
gaacaaatac ttcctatttt tttttcacca ttgtgggatt ggactttaag aggtgactct 300
aaaaaaacag agaacaaata tgtctcagtt gtattaagca cggacccata ttatcatatt 360
cacttaaaaa aatgatttcc tgtgcacctt ttggcaactt ctcttttcaa tgtagggaaa 420
aacttagtca ccctgaaaac ccacaaaata aataaaactt gtagatgtgg acaga 475

<210> 41
<211> 423
<212> DNA
<213> Homo sapien

<400> 41
taagagggta catcgggtaa gaacgtaggc acatctagag cttagagaag tctggggtag 60
gaaaaaaatc taagtattta taagggtata ggtaacattt aaaagtaggg ctagctgaca 120
ttatttagaa agaacacata cggagagata agggcaaagg actaagacca gaggaacact 180
aatatttagt gatcacttcc attcttggta aaaatagtaa cttttaagtt agcttcaagg 240
aagatttttg gccatgatta gttgtcaaaa gttagttctc ttgggtttat attactaatt 300
ttgttttaag atccttgtta gtgctttaat aaagtcatgt tatatcaaac gctctaaaac 360
attgtagcat gttaaatgtc acaatatact taccatttgt tgtatatggc tgtaccctct 420
cta 423

<210> 42
<211> 527
<212> DNA
<213> Homo sapien

<220>
<221> misc_feature
<222> (1)...(527)
<223> n = A,T,C or G

<400> 42
tctcctaggc taatgtgtgt gtttctgtaa aagtaaaaag ttaaaaattt taaaaataga 60
aaaaagctta tagaataaga atatgaagaa agaaaatatt tttgtacatt tgcacaatga 120
gtttatgttt taagctaagt gttattacaa aagagccaaa aaggttttaa aaattaaaac 180
gtttgtaaag ttacagtacc cttatgttaa tttataattg aagaaagaaa aacttttttt 240
tataaatgta gtgtagccta agcatacagt atttataaag tctggcagtg ttcaataatg 300
tcctaggcct tcacattcac tcactgactc acccagagca acttccagtc ctgtaagctc 360
cattcgtggt aagtgcccta tacaggtgca ccatttattt tacagtattt ttactgtacc 420
ttctctatgt ttccatatgt ttcgatatac aaataccact ggttactatn gcccnacagg 480
taattccagt aacacggcct gtatacgtct ggtancccta gngaaga 527

<210> 43
<211> 331
<212> DNA
<213> Homo sapien

<400> 43
tcttcaacct cgtaggacaa ctctcatatg cctgggcact atttttaggt tactaccttg 60
gctgcccttc tttaagaaaa aaaaaagaag aaaaaagaac ttttccacaa gtttctcttc 120
ctctagttgg aaaattagag aaatcatgtt tttaattttg tgttatttca gatcacaaat 180

tcaaacactt gtaaacatta agcttctgtt caatcccctg ggaagaggat tcattctgat 240
atttacggtt caaaagaagt tgtaatattg tgcttggaac acagagaacc agttattaac 300
ttcctactac tattatataa taaataataa c 331

<210> 44
<211> 592
<212> DNA
<213> Homo sapien

<220>
<221> misc_feature
<222> (1)...(592)
<223> n = A,T,C or G

<400> 44
ggcttagtag ttgccaggca aaatarcgtt gattctcctc aggagccacc cccaacaccc 60
ctgtttgctt ctagacctat acctagacta aagtcccagc agacccctag aggtgaggtt 120
cagagtgacc cttgaggaga tgtgctacac tagaaaagaa ctgcttgagt tttctaattt 180
atataagcag aaatctggag aagagtcata ggaatggata ttaagggtgt gagataatgg 240
cggaaggaat atagagttgg atcaggctgg acttattgat ttgaacccac taagtagaga 300
ttctgctttt gatgttgcag ctcagggagt taaaaaaggt tttaatggtt ctaatagttt 360
atttgcttgg ttagctgaaa tatggataaa agatggccca ctgtgagcaa gctggaaatg 420
cctgatctct ctcagtttaa tgtagaggaa gggatccaaa agtttaggga ganttggatg 480
ctggraktgg attggtcact ttgrgaccta cccwtcccag ctgggagggt ccagaagata 540
cacccttgac caacgctttg cgaaatggat ttgtgatggc ggcaactact aa 592

<210> 45
<211> 567
<212> DNA
<213> Homo sapien

<220>
<221> misc_feature
<222> (1)...(567)
<223> n = A,T,C or G

<400> 45
ggcttagtag ttgccattgc gagtgcttgc tcaacgagcg ttgaacatgg cggattgtct 60
agattcaacg gatttgagtt ttaccagcaa agcgaaccaa gcgcggccca gagaattatg 120
ggttggttgg ctttgaaaag atggaaatcc tgtaggccta gtcagaaaag ccttcttgca 180
gaacagttgg ttctcgggcg aacgctcatc aagatgccca ttggaaaggc tagcgtgtat 240
ttgggagagc ctgatagcgt gtcttctgat gatgtttgtg cttggacagt gacaaaagat 300
atgcaaagca agtccgaact agacgtcaag cttcgtgagc aaattattgt agactcctac 360
ttatactgtg aggaatgata gccaagggtg gggactttaa gactaaggtg gtttgtactt 420
gcgccgatga tcccaggcag aaagamctga tcgctagttt tatacggcaa actactaagc 480
cgaattccag cacactggcg gccgttacta attggatccg anctcggtac cagcttgatg 540
catascttga gttwtctata ntgtcnc 567

<210> 46
<211> 908
<212> DNA
<213> Homo sapien

<220>
<221> misc_feature
<222> (1)...(908)
<223> n = A,T,C or G

<400> 46
gagcgaaaga ccgagggcag ngnntangng cgangaagcg gagagggcca aaaagcaacc 60
gctttccccg gggggtgccg attcattaag gcaggtggag gacaggtttc ccgatggaag 120
gcggcagggg cgcaagcaat taatgtgagt aggccattca ttagcacccg ggcttaacat 180
ttaagcttcg ggttggtatg tggtgggaat tgtgagcgga taacaatttc acacaggaaa 240
cagctatgac catgattacg ccaagctatt taggtgacat tatagaataa ctcaagttat 300
gcatcaagct tggtaccgag ttcggatcca ctagtaacgg ccgccagtgt gtggaattcg 360
gcttagtagt tgccgaccat ggagtgctac ctaggctaga atacctgagy tcctccctag 420
cctcactcac attaaattgt atcttttcta cattagatgt cctcagcgcc ttatttctgc 480
tggacwatcg ataaattaat cctgatagga tgatagcagc agattaatta ctgagagtat 540
gttaatgtgt catccctcct atataacgta tttgcatttt aatggagcaa ttctggagat 600
aatccctgaa ggcaaaggaa tgaatcttga gggtgagaaa gccagaatca gtgtccagct 660
gcagttgtgg gagaaggtga tattatgtat gtctcagaag tgacaccata tgggcaacta 720
ctaagcccga attccagcac actggcgggc gttactaatg gatccgagct cggtaccaag 780
cttgatgcat agcttgagta tctatagtgt cactaaatag cctggcgtta tcatggtcat 840
agctgtttcc tgtgtgaaat tgttatccgc tcccaattcc ccccaccata cgagccggaa 900
cataaagt 908

<210> 47
<211> 480
<212> DNA
<213> Homo sapien

<220>
<221> misc_feature
<222> (1)...(480)
<223> n = A,T,C or G

<400> 47
tgccaacaag gaaagtttta aatttcccct tgaggattct tggtgatcat caaattcagt 60
ggtttttaag gttgttttct gtcaaataac tctaacttta agccaaacag tatatggaag 120
cacagataka atattacaca gataaaagag gagttgatct aaagtaraga tagttggggg 180
ctttaatttc tggaacctag gtctccccat cttcttctgt gctgaggaac ttcttggaag 240
cggggattct aaagttcttt ggaagacagt ttgaaaacca ccatgttgtt ctcagtacct 300
ttatttttaa aaagtaggtg aacattttga gagagaaaag ggcttggttg agatgaagtc 360
cccccccccc cttttttttt ttttagctga aatagatacc ctatgttnaa rgaarggatt 420
attatttacc atgccaytar scacatgctc tttgatgggc nyctccstac cctccttaag 480

<210> 48
<211> 591
<212> DNA
<213> Homo sapien

<400> 48
aagagggtac cgagtggaat ttccgcttca ctagtctggt gtggctagtc ggtttcgtgg 60
tggccaacat tacgaacttc caactcaacc gttcttggac gttcaagcgg gagtaccggc 120
gaggatggtg gcgtgaattc tggcctttct ttgccgtggg atcggtagcc gccatcatcg 180
gtatgtttat caagatcttc tttactaacc cgacctctcc gatttacctg cccgagccgt 240
ggtttaacga ggggaggggg atccagtcac gcgagtactg gtcccagatc ttcgccatcg 300
tcgtgacaat gcctatcaac ttcgtcgtca ataagttgtg gaccttccga acggtgaagc 360
actccgaaaa cgtccggtgg ctgctgtgcg gtgactccca aaatcttgat aacaacaagg 420
taaccgaatc gcgctaagga accccggcat ctcgggtact ctgcatatgc gtacccctta 480
agccgaattc cagcacactg gcggccgtta ctaattggat ccgaactccg taaccaagcc 540
tgatgcgtaa cttgagttat tctatagtgt ccctaaaata acctggcgtt a 591

<210> 49
<211> 454
<212> DNA

<213> Homo sapien

<400> 49
aagagggtac ctgccttgaa atttaaatgt ctaaggaaar tgggagatga ttaagagttg 60
gtgtggcyta gtcacaccaa aatgtattta ttacatcctg ctcctttcta gttgacagga 120
aagaaagctg ctgtggggaa aggagggata aatactgaag ggatttacta aacaaatgtc 180
catcacagag ttttcctttt tttttttttg agacagagtc ttgctctgtc acccaggctg 240
gaatgaagwg gtatgatctc agttgaatgc aacctctacc tcctaggttc aagcgattct 300
catgcctcag cctcctgagc agctgggact ataggcgcat gctaccatgc caggctaatt 360
tttatatttt tattagagac ggggtgttgc catgttggcc aggcaggtct cgaactcctg 420
ggcctcagat gatctgcccc accgtaccct ctta 454

<210> 50
<211> 463
<212> DNA
<213> Homo sapien

<400> 50
aagagggtac caaaaaaaag aaaaaggaaa aaaagaaaaa caacttgtat aaggctttct 60
gctgcataca gctttttttt tttaaataaa tggtgccaac aaatgttttt gcattcacac 120
caattgctgg ttttgaaatc gtactcttca aaggtatttg tgcagatcaa tccaatagtg 180
atgccccgta ggttttgtgg actgcccacg ttgtctacct tctcatgtag gagccattga 240
gagactgttt ggacatgcct gtgttcatgt agccgtgatg tccgggggcc gtgtacatca 300
tgttaccgtg gggtggggtc tgcattggct gctgggcata tggctgggtg cccatcatgc 360
ccatctgcat ctgcataggg tattggggcg tttgatccat atagccatga ttgctgtggt 420
agccactgtt catcattggc tgggacatgc tgttaccctc tta 463

<210> 51
<211> 399
<212> DNA
<213> Homo sapien

<400> 51
cttcaacctc ccaaagtgct gggattacag gactgagcca ccacgctcag cctaagcctc 60
tttttcacta ccctctaagc gatctaccac agtgatgagg ggctaaagag cagtgcaatt 120
tgattacaat aatggaactt agatttatta attaacaatt tttccttagc atgttggttc 180
cataattatt aagagtatgg acttacttag aaatgagctt tcattttaag aatttcatct 240
ttgaccttct ctattagtct gagcagtatg acactatacg tattttattt aactaaccta 300
ccttgagcta ttacttttta aaaggctata tacatgaatg tgtattgtca actgtaaagc 360
cccacagtat ttaattatat catgatgtct ttgaggttg 399

<210> 52
<211> 392
<212> DNA
<213> Homo sapien

<400> 52
cttcaacctc aatcaacctt ggtaattgat aaaatcatca cttaactttc tgatataatg 60
gcaataatta tctgagaaaa aaaagtggtg aaagattaaa cttgcatttc tctcagaatc 120
ttgaaggata tttgaataat tcaaaagcgg aatcagtagt atcagccgaa gaaactcact 180
tagctagaac gttggaccca tggatctaag tccctgccct tccactaacc agctgattgg 240
ttttgtgtaa acctcctaca cgcttgggct tggtcgcctc atttgtcaaa gtaaaggctg 300
aaataggaag ataatgaacc gtgtcttttt ggtctctttt ccatccatta ctctgatttt 360
acaaagaggc ctgtattccc ctggtgaggt tg 392

<210> 53
<211> 179
<212> DNA

<213> Homo sapien

<220>
<221> misc_feature
<222> (1)...(179)
<223> n = A,T,C or G

<400> 53
ttcgggtgat gcctcctcag gctacagtga agactggatt acagaaaggt gccagcgaga 60
tttcagattc ctgtaaacct ctaaagaaaa ggagtcgcgc ctcaactgat gtagaaatga 120
ctagttcagc atacngagac acntctgact ccgattctag aggactgagt gacctgcan 179

<210> 54
<211> 112
<212> DNA
<213> Homo sapien

<220>
<221> misc_feature
<222> (1)...(112)
<223> n = A,T,C or G

<400> 54
ttcgggtgat gcctcctcag gctacatcat natagaagca aagtagaana atcnngtttg 60
tgcattttcc cacanacaaa attcaaatga ntggaagaaa ttgggaragt at 112

<210> 55
<211> 225
<212> DNA
<213> Homo sapien

<400> 55
tgagcttccg cttctgacaa ctcaatagat aatcaaagga caactttaac agggattcac 60
aaaggagtat atccaaatgc caataaacat ataaaaagga attcagcttc atcatcatca 120
gaagwatgca aattaaaacc ataatgagaa accactatgt cccactagaa tagataaaat 180
cttaaaagac tggtaaaacc aagtgttggt aaggcaagag gagca 225

<210> 56
<211> 175
<212> DNA
<213> Homo sapien

<400> 56
gctcctcttg ccttaccaac acattctcaa aaacctgtta gagtcctaag cattctcctg 60
ttagtattgg gattttaccc ctgtcctata aagatgttat gtaccaaaaa tgaagtggag 120
ggccataccc tgagggaggg gagggatctc tagtgttgtc agaagcggaa gctca 175

<210> 57
<211> 223
<212> DNA
<213> Homo sapien

<400> 57
agccatttac cacccatgga tgaatggatt ttgtaattct agctgttgta ttttgtgaat 60
ttgttaattt tgttgttttt ctgtgaaaca catacattgg atatgggagg taaaggagtg 120
tcccagttgc tcctggtcac tccctttata gccattactg tcttgtttct tgtaactcag 180
gttaggtttt ggtctctctt gctccactgc aaaaaaaaaa aaa 223

<210> 58
<211> 211
<212> DNA
<213> Homo sapien

<400> 58
gttcgaaggt gaacgtgtag gtagcggatc tcacaactgg ggaactgtca aagacgaatt 60
aactgacttg gatcaatcaa atgtgactga ggaaacacct gaaggtgaag aacatcatcc 120
agtggcagac actgaaaata aggagaatga agttgaagag gtaaaagagg agggtccaaa 180
agagatgact ttggatgggt ggtaaatggc t 211

<210> 59
<211> 208
<212> DNA
<213> Homo sapien

<400> 59
gctcctcttg ccttaccaac tttgcaccca tcatcaacca tgtggccagg tttgcagccc 60
aggctgcaca tcaggggact gcctcgcaat acttcatgct gttgctgctg actgatggtg 120
ctgtgacgga tgtggaagcc acacgtgagg ctgtggtgcg tgcctcgaac ctgcccatgt 180
cagtgatcat tatgggtggt aaatggct 208

<210> 60
<211> 171
<212> DNA
<213> Homo sapien

<400> 60
agccatttac cacccatact aaattctagt tcaaactcca acttcttcca taaaacatct 60
aaccactgac accagttggc aatagcttct tccttcttta acctcttaga gtatttatgg 120
tcaatgccac acatttctgc aactgaataa agttggtaag gcaagaggag c 171

<210> 61
<211> 134
<212> DNA
<213> Homo sapien

<220>
<221> misc_feature
<222> (1)...(134)
<223> n = A,T,C or G

<400> 61
cgggtgatgc ctcctcaggc tttggtgtgt ccactcnact cactggcctc ttctccagca 60
actggtgaan atgtcctcan gaaaancncc acacgcngct cagggtgggg tgggaancat 120
canaatcatc nggc 134

<210> 62
<211> 145
<212> DNA
<213> Homo sapien

<400> 62
agagggtaca tatgcaacag tatataaagg aagaagtgca ctgagaggaa cttcatcaag 60
gccatttaat caataagtga tagagtcaag gctcaaccca ggtgtgacgg attccaggtc 120
ccaagctcct tactggtacc ctctt 145

<210> 63

<211> 297
<212> DNA
<213> Homo sapien

<400> 63
tgcactgaga ggaattcaaa gggtttatgc caaagaacaa accagtcctc tgcagcctaa 60
ctcatttgtt tttgggctgc gaagccatgt agagggcgat caggcagtag atggtccctc 120
ccacagtcag cgccatggtg gtccggtaaa gcatttggtc aggcaggcct cgtttcaggt 180
agacgggcac acatcagctt tctggaaaaa cttttgtagc tctggagctt tgtttttccc 240
agcataatca tacactgtgg aatcggaggt cagtttagtt ggtaaggcaa gaggagc 297

<210> 64
<211> 300
<212> DNA
<213> Homo sapien

<400> 64
gcactgagag gaacttccaa tactatgttg aataggagtg gtgagagagg gcatccttgt 60
cttgtgccgg ttttcaaagg gaatgcttcc agcttttgcc cattcagtat aatattaaag 120
aatgttttac cattttctgt cttgcctgtt tttctgtgtt tttgttggtc tcttcattct 180
ccatttttag gcctttacat gttaggaata tatttctttt aatgatactt cacctttggt 240
atcttttgtg agactctact catagtgtga taagcactgg gttggtaagg caagaggagc 300

<210> 65
<211> 203
<212> DNA
<213> Homo sapien

<400> 65
gctcctcttg ccttaccaac tcacccagta tgtcagcaat tttatcrgct ttacctacga 60
aacagcctgt atccaaacac ttaacacact cacctgaaaa gttcaggcaa caatcgcctt 120
ctcatgggtc tctctgctcc agttctgaac ctttctcttt tcctagaaca tgcatttarg 180
tcgatagaag ttcctctcag tgc 203

<210> 66
<211> 344
<212> DNA
<213> Homo sapien

<400> 66
tacggggacc cctgcattga gaaagcgaga ctcactctga agctgaaatg ctgttgccct 60
tgcagtgctg gtagcaggag ttctgtgctt tgtgggctaa ggctcctgga tgacccctga 120
catggagaag gcagagttgt gtgccccttc tcatggcctc gtcaaggcat catggactgc 180
cacacacaaa atgccgtttt tattaacgac atgaaattga aggagagaac acaattcact 240
gatgtggctc gtaaccatgg atatggtcac atacagaggt gtgattatgt aaaggttaat 300
tccacccacc tcatgtggaa actagcctca atgcaggggt ccca 344

<210> 67
<211> 157
<212> DNA
<213> Homo sapien

<400> 67
gcactgagag gaacttcgta gggaggttga actggctgct gaggaggggg aacaacaggg 60
taaccagact gatagccatt ggatggataa tatggtggtt gaggagggac actacttata 120
gcagagggtt gtgtatagcc tgaggaggca tcacccg 157

<210> 68

<211> 137
<212> DNA
<213> Homo sapien

<400> 68
gcactgagag gaacttctag aaagtgaaag tctagacata aaataaaata aaaatttaaa 60
actcaggaga gacagcccag cacggtggct cacgcctgta atcccagaac tttgggagcc 120
tgaggaggca tcacccg 137

<210> 69
<211> 137
<212> DNA
<213> Homo sapien

<400> 69
cgggtgatgc ctcctcaggc tgtattttga agactatcga ctggacttct tatcaactga 60
agaatccgtt aaaaatacca gttgtattat ttctacctgt caaaatccat ttcaaatgtt 120
gaagttcctc tcagtgc 137

<210> 70
<211> 220
<212> DNA
<213> Homo sapien

<220>
<221> misc_feature
<222> (1)...(220)
<223> n = A,T,C or G

<400> 70
agcatgttga gcccagacac gcaatctgaa tgagtgtgca cctcaagtaa atgtctacac 60
gctgcctggt ctgacatggc acaccatcnc gtggagggca casctctgct cngcctacwa 120
cgagggcant ctcatwgaca ggttccaccc accaaactgc aagaggctca nnaagtactr 180
ccagggtmya sggacmasgg tgggaytyca ycacwcatct 220

<210> 71
<211> 353
<212> DNA
<213> Homo sapien

<220>
<221> misc_feature
<222> (1)...(353)
<223> n = A,T,C or G

<400> 71
cgttagggtc tctatccact gctaaaccat acacctgggt aaacagggac catttaacat 60
tcccanctaa atatgccaag tgacttcaca tgtttatctt aaagatgtcc aaaacgcaac 120
tgattttctc ccctaaacct gtgatggtgg gatgattaan cctgagtggt ctacagcaag 180
ttaagtgcaa ggtgctaaat gaangtgacc tgagatacag catctacaag gcagtacctc 240
tcaacncagg gcaactttgc ttctcanagg gcatttagca gtgtctgaag taatttctgt 300
attacaactc acggggcggg gggtgaatat ctantggana gnagacccta acg 353

<210> 72
<211> 343
<212> DNA
<213> Homo sapien

<400> 72
gcactgagag gaacttccaa tacyatkatc agagtgaaca rgcarccyac agaacaggag 60
aaaatgttyg caatctctcc atctgacaaa aggctaatat ccagawtcta awaggaactt 120
aaacaaattt atgagaaaag aacaracaac ctcawcaaaa agtgggtgaa ggawatgcts 180
aaargaagac atytattcag ccagtaaaca yatgaaaaaa aggctcatsa tcactgawca 240
ttagagaaat gcaaatcaaa accacaatga gataccatct yayrccagtt agaayggtga 300
tcattaaaar stcaggaaac aacagatgct ggacaaggtg tca 343

<210> 73
<211> 321
<212> DNA
<213> Homo sapien

<220>
<221> misc_feature
<222> (1)...(321)
<223> n = A,T,C or G

<400> 73
gcactgagag gaacttcaga gagagagaga gagttccacc ctgtacttgg ggagagaaac 60
agaaggtgag aaagtctttg gttctgaagc agcttctaag atcttttcat ttgcttcatt 120
tcaaagttcc catgctgcca aagtgccatc ctttggggta ctgttttctg agctccagtg 180
ataactcatt tatacaaggg agatacccag aaaaaaagtg agcaaatctt aaaaaggtgg 240
cttgagttca gccttaaata ccatcttgaa atgacacaga gaaagaanga tgttgggtgg 300
gagtggatag agaccctaac g 321

<210> 74
<211> 321
<212> DNA
<213> Homo sapien

<400> 74
gcactgagag gaacttcaga gagagagaga gagttccacc ctgtacttgg ggagagaaac 60
agaaggtgag aaagtctttg gttctgaagc agcttctaag atcttttcat ttgcttcatt 120
tcaaagttcc catgctgcca aagtgccatc ctttggggta ctgttttctg agctccagtg 180
ataactcatt tatacaaggg agatacccag aaaaaaagtg agcaaatctt aaaaaggtgg 240
cttgagttca gycttaaata ccatcttgaa atgamacaga gaaagaagga tgttgggtgg 300
gagtggatag agaccctaac g 321

<210> 75
<211> 317
<212> DNA
<213> Homo sapien

<400> 75
gcactgagag gaacttccac atgcactgag aaatgcatgt tcacaaggac tgaagtctgg 60
aactcagttt ctcagttcca atcctgattc aggtgtttac cagctacaca accttaagca 120
agtcagataa ccttagcttc ctcatatgca aaatgagaat gaaaagtact catcgctgaa 180
ttgttttgag gattagaaaa acatctggca tgcagtagaa attcaattag tattcatttt 240
cattcttcta aattaaacaa ataggatttt tagtggtgga acttcagaca ccagaaatgg 300
gagtggatag agaccct 317

<210> 76
<211> 244
<212> DNA
<213> Homo sapien

<400> 76

cgttagggtc tctatccact cccactactg atcaaactct atttatttaa ttatttttat 60
catactttaa gttctgggat acacgtgcag catgcgcagg tttgttgcat aggtatacac 120
ttgccatggt ggtttgctgc acccatcagt ccatcatcta cattaggtat ttctcctaat 180
gctatccctc ccctagcccc ttacaccccc aacaggctct agtgtgtgaa gttcctctca 240
gtgc 244

<210> 77
<211> 254
<212> DNA
<213> Homo sapien

<400> 77
   cgttagggtc tctatccact gaaatctgaa gcacaggagg aagagaagca gtyctagtga 60
   gatggcaagt tcwtttacca cactctttaa catttygttt agttttaacc tttatttatg 120
   gataataaag gttaatatta ataatgattt attttaaggc attcccraat ttgcataatt 180
   ctccttttgg agataccctt ttatctccag tgcaagtctg gatcaaagtg atasamagaa 240
   gttcctctca gtgc 254

<210> 78
<211> 355
<212> DNA
<213> Homo sapien

<220>
<221> misc_feature
<222> (1)...(355)
<223> n = A,T,C or G

<400> 78
ttcgatacag gcaaacatga actgcaggag ggtggtgacg atcatgatgt tgccgatggt 60
ccggatggnc acgaagacgc actggancac gtgcttacgt ccttttgctc tgttgatggc 120
cctgagggga cgcaggaccc ttatgaccct cagaatcttc acaacgggag atggcactgg 180
attgantccc antgacacca gagacacccc aaccaccagn atatcantat attgatgtag 240
ttcctgtaga nggccccctt gtggaggaaa gctccatnag ttggtcatct tcaacaggat 300
ctcaacagtt tccgatggct gtgatgggca tagtcatant taaccntgtn tcgaa 355

<210> 79
<211> 406
<212> DNA
<213> Homo sapien

<400> 79
   taagagggta ccagcagaaa ggttagtatc atcagatagc atcttatacg agtaatatgc 60
   ctgctatttg aagtgtaatt gagaaggaaa attttagcgt gctcactgac ctgcctgtag 120
   ccccagtgac agctaggatg tgcattctcc agccatcaag agactgagtc aagttgttcc 180
   ttaagtcaga acagcagact cagctctgac attctgattc gaatgacact gttcaggaat 240
   cggaatcctg tcgattagac tggacagctt gtggcaagtg aatttgcctg taacaagcca 300
   gattttttaa aatttatatt gtaaataatg tgtgtgtgtg tgtgtgtata tatatatata 360
   tgtacagtta tctaagttaa tttaaaagtt gtttggtacc ctctta 406

<210> 80
<211> 327
<212> DNA
<213> Homo sapien

<400> 80
tttttttttt tttactcggc tcagtctaat cctttttgta gtcactcata ggccagactt 60
agggctagga tgatgattaa taagagggat gacataacta ttagtggcag gttagttgtt 120

tgtagggctc atggtagggg taaaaggagg gcaatttcta gatcaaataa taagaaggta 180
atagctacta agaagaattt tatggagaaa gggacgcggg cgggggatat agggtcgaag 240
ccgcactcgt aaggggtgga tttttctatg tagccgttga gttgtggtag tcaaaatgta 300
ataattatta gtagtaagcc taggaga 327

<210> 81
<211> 318
<212> DNA
<213> Homo sapien

<400> 81
tagtctatgc ggttgattcg gcaatccatt atttgctgga ttttgtcatg tgttttgcca 60
attgcattca taatttatta tgcatttatg cttgtatctc ctaagtcatg gtatataatc 120
catgcttttt atgttttgtc tgacataaac tcttatcaga gccctttgca cacagggatt 180
caataaatat taacacagtc tacatttatt tggtgaatat tgcatatctg ctgtactgaa 240
agcacattaa gtaacaaagg caagtgagaa gaatgaaaag cactactcac aacagttatc 300
atgattgcgc atagacta 318

<210> 82
<211> 338
<212> DNA
<213> Homo sapien

<400> 82
tcttcaacct ctactcccac taatagcttt ttgatgactt ctagcaagcc tcgctaacct 60
cgccttaccc cccactatta acctactggg agaactctct gtgctagtaa ccacgttctc 120
ctgatcaaat atcactctcc tacttacagg actcaacata ctagtcacag ccctatactc 180
cctctacata tttaccacaa cacaatgggg ctcactcacc caccacatta acaacataaa 240
accctcattc acacgagaaa acaccctcat gttcatacac ctatccccca ttctcctcct 300
atccctcaac cccgacatca ttaccgggtt ttcctctt 338

<210> 83
<211> 111
<212> DNA
<213> Homo sapien

<400> 83
agccatttac cacccatcca caaaaaaaaa aaaaaaaaag aaaaatatca aggaataaaa 60
atagactttg aacaaaaagg aacatttgct ggcctgagga ggcatcaccc g 111

<210> 84
<211> 224
<212> DNA
<213> Homo sapien

<400> 84
tcgggtgatg cctcctcagg ccaagaagat aaagcttcag acccctaaca catttccaaa 60
aaggaagaaa ggagaaaaaa gggcatcatc cccgttccga agggtcaggg aggaggaaat 120
tgaggtggat tcacgagttg cggacaactc ctttgatgcc aagcgaggtg cagccggaga 180
ctggggagag cgagccaatc aggttttgaa gttcctctca gtgc 224

<210> 85
<211> 348
<212> DNA
<213> Homo sapien

<400> 85
gcactgagag gaacttcgtt ggaaacgggt ttttttcatg taaggctaga cagaagaatt 60

ctcagtaact tccttgtgtt gtgtgtattc aactcacasa gttgaacgat cctttacaca 120
gagcagactt gtaacactct twttgtggaa tttgcaagtg gagatttcag scgctttgaa 180
gtsaaaggta gaaaaggaaa tatcttccta taaaaactag acagaatgat tctcagaaac 240
tcctttgtga tgtgtgcgtt caactcacag agtttaacct ttcwtttcat agaagcagtt 300
aggaaacact ctgtttgtaa agtctgcaag tggatagaga ccctaacg 348

<210> 86
<211> 293
<212> DNA
<213> Homo sapien

<400> 86
gcactgagag gaacttcytt gtgwtgtktg yattcaactc acagagttga asswtsmttt 60
acabagwkca ggcttkcaaa cactcttttt gtmgaatytg caagwggaka tttsrrccrc 120
tttgwggycw wysktmgaaw mggrwatatc ttcwyatmra amctagacag aaksattctc 180
akaawstyyy ytgtgawgws tgcrttcaac tcacagagkt kaacmwtyct kytsatrgag 240
cagttwkgaa actctmtttc tttggattct gcaagtggat agagacccta acg 293

<210> 87
<211> 10
<212> DNA
<213> Artificial Sequence

<220>
<223> Primer for amplification from breast tumor cDNA

<400> 87
ctcctaggct 10

<210> 88
<211> 10
<212> DNA
<213> Artificial Sequence

<220>
<223> Primer for amplification from breast tumor cDNA

<400> 88
agtagttgcc 10

<210> 89
<211> 11
<212> DNA
<213> Artificial Sequence

<220>
<223> Primer for amplification from breast tumor cDNA

<400> 89
ttccgttatg c 11

<210> 90
<211> 10
<212> DNA
<213> Artificial Sequence

<220>
<223> Primer for amplification from breast tumor cDNA

<400> 90
tggtaaaggg                                                              10

<210> 91
<211> 10
<212> DNA
<213> Artificial Sequence

<220>
<223> Primer for amplification from breast tumor cDNA

<400> 91
tcggtcatag                                                              10

<210> 92
<211> 10
<212> DNA
<213> Artificial Sequence

<220>
<223> Primer for amplification from breast tumor cDNA

<400> 92
tacaacgagg                                                              10

<210> 93
<211> 10
<212> DNA
<213> Artificial Sequence

<220>
<223> Primer for amplification from breast tumor cDNA

<400> 93
tggattggtc                                                              10

<210> 94
<211> 10
<212> DNA
<213> Artificial Sequence

<220>
<223> Primer for amplification from breast tumor cDNA

<400> 94
ctttctaccc                                                              10

<210> 95
<211> 10
<212> DNA
<213> Artificial Sequence

<220>
<223> Primer for amplification from breast tumor cDNA

<400> 95
ttttggctcc                                                              10

<210> 96
<211> 10
<212> DNA
<213> Artificial Sequence

<220>
<223> Primer for amplification from breast tumor cDNA

<400> 96
ggaaccaatc                                                              10

<210> 97
<211> 10
<212> DNA
<213> Artificial Sequence

<220>
<223> Primer for amplification from breast tumor cDNA

<400> 97
tcgatacagg                                                              10

<210> 98
<211> 10
<212> DNA
<213> Artificial Sequence

<220>
<223> Primer for amplification from breast tumor cDNA

<400> 98
ggtactaagg                                                              10

<210> 99
<211> 10
<212> DNA
<213> Artificial Sequence

<220>
<223> Primer for amplification from breast tumor cDNA

<400> 99
agtctatgcg                                                              10

<210> 100
<211> 10
<212> DNA
<213> Artificial Sequence

<220>
<223> Primer for amplification from breast tumor cDNA

<400> 100
ctatccatgg                                                              10

<210> 101
<211> 10

<212> DNA
<213> Artificial Sequence

<220>
<223> Primer for amplification from breast tumor cDNA

<400> 101
tctgtccaca                                                              10

<210> 102
<211> 10
<212> DNA
<213> Artificial Sequence

<220>
<223> Primer for amplification from breast tumor cDNA

<400> 102
aagagggtac                                                              10

<210> 103
<211> 10
<212> DNA
<213> Artificial Sequence

<220>
<223> Primer for amplification from breast tumor cDNA

<400> 103
cttcaacctc                                                              10

<210> 104
<211> 20
<212> DNA
<213> Artificial Sequence

<220>
<223> Primer for amplification from breast tumor cDNA

<400> 104
gctcctcttg ccttaccaac                                                   20

<210> 105
<211> 20
<212> DNA
<213> Artificial Sequence

<220>
<223> Primer for amplification from breast tumor cDNA

<400> 105
gtaagtcgag cagtgtgatg                                                   20

<210> 106
<211> 20
<212> DNA
<213> Artificial Sequence

<220>
<223> Primer for amplification from breast tumor cDNA

<400> 106
gtaagtcgag cagtctgatg                                                    20

<210> 107
<211> 20
<212> DNA
<213> Artificial Sequence

<220>
<223> Primer for amplification from breast tumor cDNA

<400> 107
gacttagtgg aaagaatgta                                                    20

<210> 108
<211> 20
<212> DNA
<213> Artificial Sequence

<220>
<223> Primer for amplification from breast tumor cDNA

<400> 108
gtaattccgc caaccgtagt                                                    20

<210> 109
<211> 20
<212> DNA
<213> Artificial Sequence

<220>
<223> Primer for amplification from breast tumor cDNA

<400> 109
atggttgatc gatagtggaa                                                    20

<210> 110
<211> 20
<212> DNA
<213> Artificial Sequence

<220>
<223> Primer for amplification from breast tumor cDNA

<400> 110
acggggaccc ctgcattgag                                                    20

<210> 111
<211> 20
<212> DNA
<213> Artificial Sequence

<220>
<223> Primer for amplification from breast tumor cDNA

<400> 111
tattctagac cattcgctac                                                    20

<210> 112
<211> 20
<212> DNA
<213> Artificial Sequence

<220>
<223> Primer for amplification from breast tumor cDNA

<400> 112
acataaccac tttagcgttc                                                    20

<210> 113
<211> 20
<212> DNA
<213> Artificial Sequence

<220>
<223> Primer for amplification from breast tumor cDNA

<400> 113
cgggtgatgc ctcctcaggc                                                    20

<210> 114
<211> 20
<212> DNA
<213> Artificial Sequence

<220>
<223> Primer for amplification from breast tumor cDNA

<400> 114
agcatgttga gcccagacac                                                    20

<210> 115
<211> 20
<212> DNA
<213> Artificial Sequence

<220>
<223> Primer for amplification from breast tumor cDNA

<400> 115
gacaccttgt ccagcatctg                                                    20

<210> 116
<211> 20
<212> DNA
<213> Artificial Sequence

<220>
<223> Primer for amplification from breast tumor cDNA

<400> 116
tacgctgcaa cactgtggag                                                    20

<210> 117
<211> 20
<212> DNA
<213> Artificial Sequence

<220>
<223> Primer for amplification from breast tumor cDNA

<400> 117
cgttagggtc tctatccact                                                    20

<210> 118
<211> 20
<212> DNA
<213> Artificial Sequence

<220>
<223> Primer for amplification from breast tumor cDNA

<400> 118
agactgactc atgtccccta                                                    20

<210> 119
<211> 20
<212> DNA
<213> Artificial Sequence

<220>
<223> Primer for amplification from breast tumor cDNA

<400> 119
tcatcgctcg gtgactcaag                                                    20

<210> 120
<211> 20
<212> DNA
<213> Artificial Sequence

<220>
<223> Primer for amplification from breast tumor cDNA

<400> 120
caagattcca taggctgacc                                                    20

<210> 121
<211> 20
<212> DNA
<213> Artificial Sequence

<220>
<223> Primer for amplification from breast tumor cDNA

<400> 121
acgtactggt cttgaaggtc                                                    20

<210> 122
<211> 20
<212> DNA

<213> Artificial Sequence

<220>
<223> Primer for amplification from breast tumor cDNA

<400> 122
gacgcttggc cacttgacac                                                    20

<210> 123
<211> 20
<212> DNA
<213> Artificial Sequence

<220>
<223> Primer for amplification from breast tumor cDNA

<400> 123
gtatcgacgt agtggtctcc                                                    20

<210> 124
<211> 20
<212> DNA
<213> Artificial Sequence

<220>
<223> Primer for amplification from breast tumor cDNA

<400> 124
tagtgacatt acgacgctgg                                                    20

<210> 125
<211> 20
<212> DNA
<213> Artificial Sequence

<220>
<223> Primer for amplification from breast tumor cDNA

<400> 125
cgggtgatgc ctcctcaggc                                                    20

<210> 126
<211> 23
<212> DNA
<213> Artificial Sequence

<220>
<223> Primer for amplification from breast tumor cDNA

<400> 126
atggctattt tcgggggctg aca                                                23

<210> 127
<211> 22
<212> DNA
<213> Artificial Sequence

<220>

<223> Primer for amplification from breast tumor cDNA

<400> 127
ccggtatctc ctcgtgggta tt                                                22

<210> 128
<211> 18
<212> DNA
<213> Artificial Sequence

<220>
<223> Primer for amplification from breast tumor cDNA

<400> 128
ctgcctgagc cacaaatg                                                     18

<210> 129
<211> 24
<212> DNA
<213> Artificial Sequence

<220>
<223> Primer for amplification from breast tumor cDNA

<400> 129
ccggaggagg aagctagagg aata                                              24

<210> 130
<211> 14
<212> DNA
<213> Artificial Sequence

<220>
<223> Primer

<400> 130
tttttttttt ttag                                                         14

<210> 131
<211> 18
<212> PRT
<213> Artificial Sequence

<220>
<223> Predicited Th Motifs (B-cell epitopes)

<400> 131
Ser Ser Gly Gly Arg Thr Phe Asp Asp Phe His Arg Tyr Leu Leu Val
 1               5                  10                  15
Gly Ile

<210> 132
<211> 22
<212> PRT
<213> Artificial Sequence

<220>

<223> Predicited Th Motifs (B-cell epitopes)

<221> VARIANT
<222> (1)...(22)
<223> Xaa = Any Amino Acid

<400> 132
Gln Gly Ala Ala Gln Lys Pro Ile Asn Leu Ser Lys Xaa Ile Glu Val
1               5                   10                  15
Val Gln Gly His Asp Glu
            20

<210> 133
<211> 23
<212> PRT
<213> Artificial Sequence

<220>
<223> Predicited Th Motifs (B-cell epitopes)

<400> 133
Ser Pro Gly Val Phe Leu Glu His Leu Gln Glu Ala Tyr Arg Ile Tyr
1               5                   10                  15
Thr Pro Phe Asp Leu Ser Ala
            20

<210> 134
<211> 9
<212> PRT
<213> Artificial Sequence

<220>
<223> Predicited HLA A2.1 Motifs (T-cell epitopes)

<400> 134
Tyr Leu Leu Val Gly Ile Gln Gly Ala
1               5

<210> 135
<211> 9
<212> PRT
<213> Artificial Sequence

<220>
<223> Predicited HLA A2.1 Motifs (T-cell epitopes)

<400> 135
Gly Ala Ala Gln Lys Pro Ile Asn Leu
1               5

<210> 136
<211> 9
<212> PRT
<213> Artificial Sequence

<220>
<223> Predicited HLA A2.1 Motifs (T-cell epitopes)

<221> VARIANT
<222> (1)...(9)
<223> Xaa = Any Amino Acid

<400> 136
Asn Leu Ser Lys Xaa Ile Glu Val Val
1               5

<210> 137
<211> 9
<212> PRT
<213> Artificial Sequence

<220>
<223> Predicited HLA A2.1 Motifs (T-cell epitopes)

<400> 137
Glu Val Val Gln Gly His Asp Glu Ser
1               5

<210> 138
<211> 9
<212> PRT
<213> Artificial Sequence

<220>
<223> Predicited HLA A2.1 Motifs (T-cell epitopes)

<400> 138
His Leu Gln Glu Ala Tyr Arg Ile Tyr
1               5

<210> 139
<211> 9
<212> PRT
<213> Artificial Sequence

<220>
<223> Predicited HLA A2.1 Motifs (T-cell epitopes)

<400> 139
Asn Leu Ala Phe Val Ala Gln Ala Ala
1               5

<210> 140
<211> 9
<212> PRT
<213> Artificial Sequence

<220>
<223> Predicited HLA A2.1 Motifs (T-cell epitopes)

<400> 140
Phe Val Ala Gln Ala Ala Pro Asp Ser
1               5

<210> 141
<211> 9388

<212> DNA
<213> Homo sapien

<400> 141
gctcgcggcc gcgagctcaa ttaaccctca ctaaagggag tcgactcgat cagactgtta 60
ctgtgtctat gtagaaagaa gtagacataa gagattccat tttgttctgt actaagaaaa 120
attcttctgc cttgagatgc tgttaatctg taaccctagc cccaaccctg tgctcacaga 180
gacatgtgct gtgttgactc aaggttcaat ggatttaggg ctatgctttg ttaaaaaagt 240
gcttgaagat aatatgcttg ttaaaagtca tcaccattct ctaatctcaa gtacccaggg 300
acacaataca ctgcggaagg ccgcagggac ctctgtctag gaaagccagg tattgtccaa 360
gatttctccc catgtgatag cctgagatat ggcctcatgg gaagggtaag acctgactgt 420
cccccagccc gacatccccc agcccgacat cccccagccc gacacccgaa aagggtctgt 480
gctgaggagg attagtaaaa gaggaaggcc tctttgcagt tgaggtaaga ggaaggcatc 540
tgtctcctgc tcgtccctgg gcaatagaat gtcttggtgt aaaacccgat tgtatgttct 600
acttactgag ataggagaaa acatccttag ggctggaggt gagacacgct ggcggcaata 660
ctgctcttta atgcaccgag atgtttgtat aagtgcacat caaggcacag cacctttcct 720
taaacttatt tatgacacag agacctttgt tcacgttttc ctgctgaccc tctccccact 780
attaccctat tggcctgcca catccccctc tccgagatgg tagagataat gatcaataaa 840
tactgaggga actcagagac cagtgtccct gtaggtcctc cgtgtgctga gcgccggtcc 900
cttgggctca cttttctttc tctatacttt gtctctgtgt ctctttcttt tctcagtctc 960
tcgttccacc tgacgagaaa tacccacagg tgtggagggg caggccaccc cttcaataat 1020
ttactagcct gttcgctgac aacaagactg gtggtgcaga aggttgggtc ttggtgttca 1080
ccgggtggca ggcatgggcc aggtgggagg gtctccagcg cctggtgcaa atctccaaga 1140
aagtgcagga aacagcacca agggtgattg taaattttga tttggcgcgg caggtagcca 1200
ttccagcgca aaaatgcgca ggaaagcttt tgctgtgctt gtaggcaggt aggccccaag 1260
cacttcttat tggctaatgt ggagggaacc tgcacatcca ttggctgaaa tctccgtcta 1320
tttgaggctg actgagcgcg ttcctttctt ctgtgttgcc tggaaacgga ctgtctgcct 1380
agtaacatct gatcacgttt cccattggcc gccgtttccg gaagcccgcc ctcccatttc 1440
cggaagcctg gcgcaaggtt ggtctgcagg tggcctccag gtgcaaagtg ggaagtgtga 1500
gtcctcagtc ttgggctatt cggccacgtg cctgccggac atgggacgct ggagggtcag 1560
cagcgtggag tcctggcctt ttgcgtccac gggtgggaaa ttggccattg ccacggcggg 1620
aactgggact caggctgccc cccggccgtt tctcatccgt ccaccggact cgtgggcgct 1680
cgcactggcg ctgatgtagt ttcctgacct ctgacccgta ttgtctccag attaaaggta 1740
aaaacggggc tttttcagcc cactcgggta aaacgccttt tgatttctag gcaggtgttt 1800
tgttgcacgc ctgggaggga gtgacccgca ggttgaggtt tattaaaata cattcctggt 1860
ttatgttatg tttataataa agcaccccaa cctttacaaa atctcacttt ttgccagttg 1920
tattatttag tggactgtct ctgataagga cagccagtta aaatggaatt ttgttgttgc 1980
taattaaacc aatttttagt tttggtgttt gtcctaatag caacaacttc tcaggcttta 2040
taaaaccata tttcttgggg gaaatttctg tgtaaggcac agcgagttag tttggaattg 2100
ttttaaagga agtaagttcc tggttttgat atcttagtag tgtaatgccc aacctggttt 2160
ttactaaccc tgtttttaga ctctcccttt ccttaaatca cctagccttg tttccacctg 2220
aattgactct cccttagcta agagcgccag atggactcca tcttggctct ttcactggca 2280
gccccttcct caaggactta acttgtgcaa gctgactccc agcacatcca agaatgcaat 2340
taactgttaa gatactgtgg caagctatat ccgcagttcc gaggaattca tccgattgat 2400
tatgcccaaa agccccgcgt ctatcacctt gtaataatct taaagcccct gcacctggaa 2460
ctattaactt tcctgtaacc atttatcctt ttaacttttt tgcttacttt atttctgtaa 2520
aattgtttta actagacctc ccctcccctt tctaaaccaa agtataaaag aagatctagc 2580
cccttcttca gagcggagag aattttgagc attagccatc tcttggcggc cagctaaata 2640
aatggacttt taatttgtct caaagtgtgg cgttttctct aactcgctca ggtacgacat 2700
ttggaggccc cagcgagaaa cgtcaccggg agaaacgtca ccgggcgaga gccgggcccg 2760
ctgtgtgctc ccccggaagg acagccagct tgtagggggg agtgccacct gaaaaaaaaa 2820
tttccaggtc cccaaagggt gaccgtcttc cggaggacag cggatcgact accatgcggg 2880
tgcccaccaa aattccacct ctgagtcctc aactgctgac cccggggtca ggtaggtcag 2940
atttgacttt ggttctggca gagggaagcg accctgatga gggtgtccct cttttgactc 3000
tgcccatttc tctaggatgc tagagggtag agccctggtt ttctgttaga cgcctctgtg 3060
tctctgtctg ggagggaagt ggccctgaca ggggccatcc cttgagtcag tccacatccc 3120
aggatgctgg gggactgagt cctggtttct ggcagactgg tctctctctc tctctttttc 3180
tatctctaat ctttccttgt tcaggtttct tggagaatct ctgggaaaga aaaaagaaaa 3240

actgttataa actctgtgtg aatggtgaat gaatggggga ggacaagggc ttgcgcttgt 3300
cctccagttt gtagctccac ggcgaaagct acggagttca agtgggccct cacctgcggt 3360
tccgtggcga cctcataagg cttaaggcag catccggcat agctcgatcc gagccggggg 3420
tttataccgg cctgtcaatg ctaagaggag cccaagtccc ctaaggggga gcggccaggc 3480
gggcatctga ctgatcccat cacgggaccc cctccccttg tttgtctaaa aaaaaaaaaa 3540
gaagaaactg tcataactgt ttacatgccc tagggtcaac tgtttgtttt atgtttattg 3600
ttctgttcgg tgtctattgt cttgtttagt ggttgtcaag gttttgcatg tcaggacgtc 3660
gatattgccc aagacgtctg ggtaagaact tctgcaaggt ccttagtgct gattttttgt 3720
cacaggaggt taaatttctc atcaatcatt taggctggcc accacagtcc tgtcttttct 3780
gccagaagca agtcaggtgt tgttacggga atgagtgtaa aaaaacattc gcctgattgg 3840
gatttctggc accatgatgg ttgtatttag attgtcatac cccacatcca ggttgattgg 3900
acctcctcta aactaaactg gtggtgggtt caaaacagcc accctgcaga tttccttgct 3960
cacctctttg gtcattctgt aacttttcct gtgcccttaa atagcacact gtgtagggaa 4020
acctaccctc gtactgcttt acttcgttta gattcttact ctgttcctct gtggctactc 4080
tcccatctta aaaacgatcc aagtggtcct tttcctcctc cctgccccct accccacaca 4140
tctcgttttc cagtgcgaca gcaagttcag cgtctccagg acttggctct gctctcactc 4200
cttgaaccct taaaagaaaa agctgggttt gagctatttg cctttgagtc atggagacac 4260
aaaaggtatt tagggtacag atctagaaga agagagagaa cacctagatc caactgaccc 4320
aggagatctc gggctggcct ctagtcctcc tccctcaatc ttaaagctac agtgatgtgg 4380
caagtggtat ttagctgttg tggtttttct gctctttctg gtcatgttga ttctgttctt 4440
tcgatactcc agccccccag ggagtgagtt tctctgtctg tgctgggttt gatatctatg 4500
ttcaaatctt attaaattgc cttcaaaaaa aaaaaaaaaa gggaaacact tcctcccagc 4560
cttgtaaggg ttggagccct ctccagtata tgctgcagaa tttttctctc ggtttctcag 4620
aggattatgg agtccgcctt aaaaaaggca agctctggac actctgcaaa gtagaatggc 4680
caaagtttgg agttgagtgg ccccttgaag ggtcactgaa cctcacaatt gttcaagctg 4740
tgtggcgggt tgttactgaa actcccggcc tccctgatca gtttccctac attgatcaat 4800
ggctgagttt ggtcaggagc accccttcca tggctccact catgcaccat tcataatttt 4860
acctccaagg tcctcctgag ccagaccgtg ttttcgcctc gaccctcagc cggttcagct 4920
cgccctgtac tgcctctctc tgaagaagag gagagtctcc ctcacccagt cccaccgcct 4980
taaaaccagc ctactccctt agggtcatcc catgtctcct cggctatgtc ccctgtaggc 5040
tcatcaccca ttgcctcttg gttgcaaccg tggtgggagg aagtagcccc tctactacca 5100
ctgagagagg cacaagtccc tctgggtgat gagtgctcca cccccttcct ggtttatgtc 5160
ccttctttct acttctgact tgtataattg gaaaacccat aatcctccct tctctgaaaa 5220
gccccaggct ttgacctcac tgatggagtc tgtactctgg acacattggc ccacctggga 5280
tgactgtcaa cagctccttt tgaccctttt cacctctgaa gagagggaaa gtatccaaag 5340
agaggccaaa aagtacaacc tcacatcaac caataggccg gaggaggaag ctagaggaat 5400
agtgattaga gacccaattg ggacctaatt gggacccaaa tttctcaagt ggagggagaa 5460
cttttgacga tttccaccgg tatctcctcg tgggtattca gggagctgct cagaaaccta 5520
taaacttgtc taaggcgact gaagtcgtcc aggggcatga tgagtcacca ggagtgtttt 5580
tagagcacct ccaggaggct tatcggattt acaccccttt tgacctggca gcccccgaaa 5640
atagccatgc tcttaatttg gcatttgtgg ctcaggcagc cccagatagt aaaaggaaac 5700
tccaaaaact agagggattt tgctggaatg aataccagtc agcttttaga gatagcctaa 5760
aaggtttttg acagtcaaga ggttgaaaaa caaaaacaag cagctcaggc agctgaaaaa 5820
agccactgat aaagcatcct ggagtatcag agtttactgt tagatcagcc tcatttgact 5880
tcccctccca catggtgttt aaatccagct acactacttc ctgactcaaa ctccactatt 5940
cctgttcatg actgtcagga actgttggaa actactgaaa ctggccgacc tgatcttcaa 6000
aatgtgcccc taggaaaggt ggatgccacc gtgttcacag acagtagcag cttcctcgag 6060
aagggactac gaaaggccgg tgcagctgtt accatggaga cagatgtgtt gtgggctcag 6120
gctttaccag caaacacctc agcacaaaag gctgaattga tcgccctcac tcaggctctc 6180
cgatggggta aggatattaa cgttaacact gacagcaggt acgcctttgc tactgtgcat 6240
gtacgtggag ccatctacca ggagcgtggg ctactcacct cagcaggtgg ctgtaatcca 6300
ctgtaaagga catcaaaagg aaaacacggc tgttgcccgt ggtaaccaga aagctgattc 6360
agcagctcaa gatgcagtgt gactttcagt cacgcctcta aacttgctgc ccacagtctc 6420
ctttccacag ccagatctgc ctgacaatcc cgcatactca acagaagaag aaaactggcc 6480
tcagaactca gagccaataa aaatcaggaa ggttggtgga ttcttcctga ctctagaatc 6540
ttcatacccc gaactcttgg gaaaacttta atcagtcacc tacagtctac cacccattta 6600
ggaggagcaa agctacctca gctcctccgg agccgtttta agatccccca tcttcaaagc 6660
ctaacagatc aagcagctct ccggtgcaca acctgcgccc aggtaaatgc caaaaaaggt 6720

cctaaaccca gcccaggcca ccgtctccaa gaaaactcac caggagaaaa gtgggaaatt 6780
gactttacag aagtaaaacc acaccgggct gggtacaaat accttctagt actggtagac 6840
accttctctg gatggactga agcatttgct accaaaaacg aaactgtcaa tatggtagtt 6900
aagtttttac tcaatgaaat catccctcga cgtgggctgc ctgttgccat agggtctgat 6960
aatggaccgg ccttcgcctt gtctatagtt tagtcagtca gtaaggcgtt aaacattcaa 7020
tggaagctcc attgtgccta tcgaccccag agctctgggc aagtagaacg catgaactgc 7080
accctaaaaa acactcttac aaaattaatc ttagaaaccg gtgtaaattg tgtaagtctc 7140
cttcctttag ccctacttag agtaaggtgc accccttact gggctgggtt cttacctttt 7200
gaaatcatgt atgggagggc gctgcctatc ttgcctaagc taagagatgc ccaattggca 7260
aaaatatcac aaactaattt attacagtac ctacagtctc cccaacaggt acaagatatc 7320
atcctgccac ttgttcgagg aacccatccc aatccaattc ctgaacagac agggccctgc 7380
cattcattcc cgccaggtga cctgttgttt gttaaaaagt tccagagaga aggactccct 7440
cctgcttgga agagacctca caccgtcatc acgatgccaa cggctctgaa ggtggatggc 7500
attcctgcgt ggattcatca ctcccgcatc aaaaaggcca acggagccca actagaaaca 7560
tgggtcccca gggctgggtc aggcccctta aaactgcacc taagttgggt gaagccatta 7620
gattaattct ttttcttaat tttgtaaaac aatgcatagc ttctgtcaaa cttatgtatc 7680
ttaagactca atataacccc cttgttataa ctgaggaatc aatgatttga ttccccaaaa 7740
acacaagtgg ggaatgtagt gtccaacctg gtttttacta accctgtttt tagactctcc 7800
ctttccttta atcactcagc cttgtttcca cctgaattga ctctccctta gctaagagcg 7860
ccagatggac tccatcttgg ctctttcact ggcagccgct tcctcaagga cttaacttgt 7920
gcaagctgac tcccagcaca tccaagaatg caattaactg ataagatact gtggcaagct 7980
atatccgcag ttcccaggaa ttcgtccaat tgattacacc caaaagcccc gcgtctatca 8040
ccttgtaata atcttaaagc ccctgcacct ggaactatta acgttcctgt aaccatttat 8100
ccttttaact tttttgccta ctttatttct gtaaaattgt tttaactaga ccccccctct 8160
cctttctaaa ccaaagtata aaagcaaatc tagccccttc ttcaggccga gagaatttcg 8220
agcgttagcc gtctcttggc caccagctaa ataaacggat tcttcatgtg tctcaaagtg 8280
tggcgttttc tctaactcgc tcaggtacga ccgtggtagt attttcccca acgtcttatt 8340
tttagggcac gtatgtagag taacttttat gaaagaaacc agttaaggag gttttgggat 8400
ttcctttatc aactgtaata ctggttttga ttatttattt atttatttat tttttttgag 8460
aaggagtttc actcttgttg cccaggctgg agtgcaatgg tgcgatcttg gctcactgca 8520
acttccgcct cccaggttca agcgattctc ctgcctcagc ctcgagagta gctgggatta 8580
taggcatgcg ccaccacacc cagctaattt tgtattttta gtaaagatgg ggtttcttca 8640
tgttggtcaa gctggtctgg aactccccgc ctcgggtgat ctgcccgcct cggcctccga 8700
aagtgctggg attacaggtg tgatccacca cacccagccg atttatatgt atataaatca 8760
cattcctcta accaaaatgt agtgtttcct tccatcttga atataggctg tagaccccgt 8820
gggtatggga cattgttaac agtgagacca cagcagtttt tatgtcatct gacagcatct 8880
ccaaatagcc ttcatggttg tcactgcttc ccaagacaat tccaaataac acttcccagt 8940
gatgacttgc tacttgctat tgttacttaa tgtgttaagg tggctgttac agacactatt 9000
agtatgtcag gaattacacc aaaatttagt ggctcaaaca atcattttat tatgtatgtg 9060
gattctcatg gtcaggtcag gatttcagac agggcacaag ggtagcccac ttgtctctgt 9120
ctatgatgtc tggcctcagc acaggagact caacagctgg ggtctgggac catttggagg 9180
cttgttccct cacatctgat acctggcttg ggatgttgga agagggggtg agctgagact 9240
gagtgcctat atgtagtgtt tccatatggc cttgacttcc ttacagcctg gcagcctcag 9300
ggtagtcaga attcttagga ggcacagggc tccagggcag atgctgaggg gtcttttatg 9360
aggtagcaca gcaaatccac ccaggatc 9388

<210> 142
<211> 419
<212> DNA
<213> Homo sapien

<400> 142
tgtaagtcga gcagtgtgat ggaaggaatg gtctttggag agagcatatc catctcctcc 60
tcactgcctc ctaatgtcat gaggtacact gagcagaatt aaacagggta gtcttaacca 120
cactattttt agctaccttg tcaagctaat ggttaaagaa cacttttggt ttacacttgt 180
tgggtcatag aagttgcttt ccgccatcac gcaataagtt tgtgtgtaat cagaaggagt 240
taccttatgg tttcagtgtc attctttagt taacttggga gctgtgtaat ttaggctttg 300
cgtattattt cacttctgtt ctccacttat gaagtgattg tgtgttcgcg tgtgtgtgcg 360

tgcgcatgtg cttccggcag ttaacataag caaataccca acatcacact gctcgactt 419

<210> 143
<211> 402
<212> DNA
<213> Homo sapien

<400> 143
tgtaagtcga gcagtgtgat gtccactgca gtgtgttgct gggaacagtt aatgagcaaa 60
ttgtatacaa tggctagtac attgaccggg atttgttgaa gctggtgagt gttatgactt 120
agcctgttag actagtctat gcacatggct ctggtcaact accgctctct catttctcca 180
gataaatccc ccatgcttta tattctcttc caaacatact atcctcatca ccacatagtt 240
cctttgttaa tgctttgttc tagactttcc cttttctgtt ttcttattca aacctatatc 300
tctttgcata gattgtaaat tcaaatgccc tcagggtgca ggcagttcat gtaagggagg 360
gaggctagcc agtgagatct gcatcacact gctcgactta ca 402

<210> 144
<211> 224
<212> DNA
<213> Homo sapien

<400> 144
tcgggtgatg cctcctcagg ccaagaagat aaagcttcag acccctaaca catttccaaa 60
aaggaagaaa ggagaaaaaa gggcatcatc cccgttccga agggtcaggg aggaggaaat 120
tgaggtggat tcacgagttg cggacaactc ctttgatgcc aagcgaggtg cagccggaga 180
ctggggagag cgagccaatc aggttttgaa gttcctctca gtgc 224

<210> 145
<211> 111
<212> DNA
<213> Homo sapien

<400> 145
agccatttac cacccatcca caaaaaaaaa aaaaaaaaag aaaaatatca aggaataaaa 60
atagactttg aacaaaaagg aacatttgct ggcctgagga ggcatcaccc g 111

<210> 146
<211> 585
<212> DNA
<213> Homo sapien

<400> 146
tagcatgttg agcccagaca cttgtagaga gaggaggaca gttagaagaa gaagaaaagt 60
ttttaaatgc tgaaagttac tataagaaag ctttggcttt ggatgagact tttaaagatg 120
cagaggatgc tttgcagaaa cttcataaat atatgcaggt gattccttat ttcctcctag 180
aaatttagtg atatttgaaa taatgcccaa acttaatttt ctcctgagga aaactattct 240
acattactta agtaaggcat tatgaaaagt ttctttttag gtatagtttt tcctaattgg 300
gtttgacatt gcttcatagt gcctctgttt ttgtccataa tcgaaagtaa agatagctgt 360
gagaaaacta ttacctaaat ttggtatgtt gttttgagaa atgtccttat agggagctca 420
cctggtggtt tttaaattat tgttgctact ataattgagc taattataaa aacctttttg 480
agacatattt taaattgtct tttcctgtaa tactgatgat gatgttttct catgcatttt 540
cttctgaatt gggaccattg ctgctgtgtc tgggctcaca tgcta 585

<210> 147
<211> 579
<212> DNA
<213> Homo sapien

<220>
<221> misc_feature
<222> (1)...(579)
<223> n = A,T,C or G

<400> 147
tagcatgttg agcccagaca ctgggcagcg ggggtggcca cggcagctcc tgccgagccc 60
aagcgtgttt gtctgtgaag gaccctgacg tcacctgcca ggctagggag gggtcaatgt 120
ggagtgaatg ttcaccgact ttcgcaggag tgtgcagaag ccaggtgcaa cttggtttgc 180
ttgtgttcat cacccctcaa gatatgcaca ctgctttcca aataaagcat caactgtcat 240
ctccagatgg ggaagacttt ttctccaacc agcaggcagg tccccatcca ctcagacacc 300
agcacgtcca ccttctcggg cagcaccacg tcctccacct tctgctggta cacggtgatg 360
atgtcagcaa agccgttctg cangaccagc tgccccgtgt gctgtgccat ctcactggcc 420
tccaccgcgt acaccgctct aggccgcgca tantgtgcac agaanaaatg atgatccagt 480
cccacagccc acgtccaaga ngactttatc cgtcagggat tctttattct gcaggatgac 540
ctgtggtatt aattgttcgt gtctgggctc aacatgcta 579

<210> 148
<211> 249
<212> DNA
<213> Homo sapien

<400> 148
tgacaccttg tccagcatct gcaagccagg aagagagtcc tcaccaagat ccccaccccg 60
ttggcaccag gatcttggac ttccaatctc cagaactgtg agaaataagt atttgtcgct 120
aaataaatct ttgtggtttc agatatttag ctatagcaga tcaggctgac taagagaaac 180
cccataagag ttacatactc attaatctcc gtctctatcc ccaggtctca gatgctggac 240
aaggtgtca 249

<210> 149
<211> 255
<212> DNA
<213> Homo sapien

<400> 149
   tgacaccttg tccagcatct gctattttgt gactttttaa taatagccat tctgactggt 60
   gtgagatggt aactcattgt gggtttggtc tgcatttctc taatgatcag tgatattaag 120
   ctttttttaa atatgcttgt tgaccacatg tatatcatct tttgagaagt gtctgttcat 180
   atcctttgcc cactttttaa tttttttatc ttgtaaattt gtttaatttc cttacagatg 240
   ctggacaagg tgtca 255

<210> 150
<211> 318
<212> DNA
<213> Homo sapien

<400> 150
ttacgctgca acactgtgga ggccaagctg ggatcacttc ttcattctaa ctggagagga 60
gggaagttca agtccagcag agggtgggtg ggtagacagt ggcactcaga aatgtcagct 120
ggacccctgt ccccgcatag gcaggacagc aaggctgtgg ctctccaggg ccagctgaag 180
aacaggacac tgtctccgct gccacaaagc gtcagagact cccatctttg aagcacggcc 240
ttcttggtct tcctgcactt ccctgttctg ttagagacct ggttatagac aaggcttctc 300
cacagtgttg cagcgtaa 318

<210> 151
<211> 323
<212> DNA
<213> Homo sapien

<220>
<221> misc_feature
<222> (1)...(323)
<223> n = A,T,C or G

<400> 151
tnacgcngcn acnntgtaga ganggnaagg cnttccccac attncccctt catnanagaa 60
ttattcnacc aagnntgacc natgccnttt atgacttaca tgcnnactnc ntaatctgtn 120
tcnngcctta aaagcnnntc cactacatgc ntcancactg tntgtgtnac ntcatnaact 180
gtcngnaata ggggcncata actacagaaa tgcanttcat actgcttcca ntgccatcng 240
cgtgtggcct tncctactct tcttntattc caagtagcat ctctggantg cttccccact 300
ctccacattg ttgcagcnat aat 323

<210> 152
<211> 311
<212> DNA
<213> Homo sapien

<400> 152
tcaagattcc ataggctgac cagtccaagg agagttgaaa tcatgaagga gagtctatct 60
ggagagagct gtagttttga gggttgcaaa gacttaggat ggagttggtg ggtgtggtta 120
gtctctaagg ttgattttgt tcataaattt catgccctga atgccttgct tgcctcaccc 180
tggtccaagc cttagtgaac acctaaaagt ctctgtcttc ttgctctcca aacttctcct 240
gaggatttcc tcagattgtc tacattcaga tcgaagccag ttggcaaaca agatgcagtc 300
cagagggtca g 311

<210> 153
<211> 332
<212> DNA
<213> Homo sapien

<400> 153
caagattcca taggctgacc aggaggctat tcaagatctc tggcagttga ggaagtctct 60
ttaagaaaat agtttaaaca atttgttaaa atttttctgt cttacttcat ttctgtagca 120
gttgatatct ggctgtcctt tttataatgc agagtgggaa ctttccctac catgtttgat 180
aaatgttgtc caggctccat tgccaataat gtgttgtcca aaatgcctgt ttagttttta 240
aagacggaac tccacccttt gcttggtctt aagtatgtat ggaatgttat gataggacat 300
agtagtagcg gtggtcagcc tatggaatct tg 332

<210> 154
<211> 345
<212> DNA
<213> Homo sapien

<220>
<221> misc_feature
<222> (1)...(345)
<223> n = A,T,C or G

<400> 154
tcaagattcc ataggctgac ctggacagag atctcctggg tctggcccag gacagcaggc 60
tcaagctcag tggagaaggt ttccatgacc ctcagattcc cccaaacctt ggattgggtg 120
acattgcatc tcctcagaga gggaggagat gtangtctgg gcttccacag ggacctggta 180
ttttaggatc agggtaccgc tggcctgagg cttggatcat tcanagcctg ggggtggaat 240
ggctggcagc ctgtggcccc attgaaatag gctctggggc actccctctg ttcctanttg 300
aacttgggta aggaacagga atgtggtcan cctatggaat cttga 345

<210> 155
<211> 295
<212> DNA
<213> Homo sapien

<220>
<221> misc_feature
<222> (1)...(295)
<223> n = A,T,C or G

<400> 155
gacgcttggc cacttgacac attaaacagt tttgcataat cactancatg tatttctagt 60
ttgctgtctg ctgtgatgcc ctgccctgat tctctggcgt taatgatggc aagcataatc 120
aaacgctgtt ctgttaattc caagttataa ctggcattga ttaaagcatt atctttcaca 180
actaaactgt tcttcatana acagcccata ttattatcaa attaagagac aatgtattcc 240
aatatccttt anggccaata tatttnatgt cccttaatta agagctactg tccgt 295

<210> 156
<211> 406
<212> DNA
<213> Homo sapien

<220>
<221> misc_feature
<222> (1)...(406)
<223> n = A,T,C or G

<400> 156
gacgcttggc cacttgacac tgcagtggga aaaccagcat gagccgctgc ccccaaggaa 60
cctcgaagcc caggcagagg accagccatc ccagcctgca ggtaaagtgt gtcacctgtc 120
aggtgggctt ggggtgagtg ggtgggggaa gtgtgtgtgc aaagggggtg tnaatgtnta 180
tgcgtgtgag catgagtgat ggctagtgtg actgcatgtc agggagtgtg aacaagcgtg 240
cgggggtgtg tgtgcaagtg cgtatgcata tgagaatatg tgtctgtgga tgagtgcatt 300
tgaaagtctg tgtgtgtgcg tgtggtcatg anggtaantt antgactgcg caggatgtgt 360
gagtgtgcat ggaacactca ntgtgtgtgt caagtggccn ancgtc 406

<210> 157
<211> 208
<212> DNA
<213> Homo sapien

<220>
<221> misc_feature
<222> (1)...(208)
<223> n = A,T,C or G

<400> 157
   tgacgcttgg ccacttgaca cactaaaggg tgttactcat cactttcttc tctcctcggt 60
   ggcatgtgag tgcatctatt cacttggcac tcatttgttt ggcagtgact gtaanccana 120
   tctgatgcat acaccagctt gtaaattgaa taaatgtctc taatactatg tgctcacaat 180
   anggtanggg tgaggagaag gggagaga 208

<210> 158
<211> 547
<212> DNA
<213> Homo sapien

<220>

<221> misc_feature
<222> (1)...(547)
<223> n = A,T,C or G

<400> 158
cttcaacctc cttcaacctc cttcaacctc ctggattcaa acaatcatcc cacctcagac 60
tccttagtag ctgagactac agactcacgc cactacatct ggctaaattt ttgtagagat 120
agggtttcat catgttgccc tggctggtct caaactcctg acctcaagca atgtgcccac 180
ctcagcctcc caaagtgctg ggattacagg cataagccac catgcccagt ccatntttaa 240
tctttcctac cacattctta ccacactttc ttttatgttt agatacataa atgcttacca 300
ttatgataca attgcccaca gtattaagac agtaacatgc tgcacaggtt tgtagcctag 360
gaacagtagg caataccaca tagcttaggt gtgtggtaga ctataccatc taggtttgtg 420
taagttacac tttatgctgt ttacacaatg acaaaaccat ctaatgatgc atttctcaga 480
atgtatcctt gtcagtaagc tatgatgtac agggaacact gcccaaggac acagatattg 540
tacctgt 547

<210> 159
<211> 203
<212> DNA
<213> Homo sapien

<400> 159
gctcctcttg ccttaccaac tcacccagta tgtcagcaat tttatcrgct ttacctacga 60
aacagcctgt atccaaacac ttaacacact cacctgaaaa gttcaggcaa caatcgcctt 120
ctcatgggtc tctctgctcc agttctgaac ctttctcttt tcctagaaca tgcatttarg 180
tcgatagaag ttcctctcag tgc 203

<210> 160
<211> 402
<212> DNA
<213> Homo sapien

<400> 160
tgtaagtcga gcagtgtgat gggtggaaca gggttgtaag cagtaattgc aaactgtatt 60
taaacaataa taataatatt tagcatttat agagcacttt atatcttcaa agtacttgca 120
aacattayct aattaaatac cctctctgat tataatctgg atacaaatgc acttaaactc 180
aggacagggt catgagaraa gtatgcattt gaaagttggt gctagctatg ctttaaaaac 240
ctatacaatg atgggraagt tagagttcag attctgttgg actgtttttg tgcatttcag 300
ttcagcctga tggcagaatt agatcatatc tgcactcgat gactytgctt gataacttat 360
cactgaaatc tgagtgttga tcatcacact gctcgactta ca 402

<210> 161
<211> 193
<212> DNA
<213> Homo sapien

<400> 161
agcatgttga gcccagacac tgaccaggag aaaaaccaac caatagaaac acgcccagac 60
actgaccagg agaaaaacca accaataaaa acaggcccgg acataagaca aataataaaa 120
ttagcggaca aggacatgaa aacagctatt gtaagagcgg atatagtggt gtgtgtctgg 180
gctcaacatg cta 193

<210> 162
<211> 147
<212> DNA
<213> Homo sapien

<400> 162

tgttgagccc agacactgac caggagaaaa accaaccaat aaaaacaggc ccggacataa 60
gacaaataat aaaattagcg gacaaggaca tgaaaacagc tattgtaaga gcggatatag 120
tggtgtgtgt ctgggctcaa catgcta 147

<210> 163
<211> 294
<212> DNA
<213> Homo sapien

<400> 163
tagcatgttg agcccagaca caaatctttc cttaagcaat aaatcatttc tgcatatgtt 60
tttaaaacca cagctaagcc atgattattc aaaaggacta ttgtattggg tattttgatt 120
tgggttctta tctccctcac attatcttca tttctatcat tgacctctta tcccagagac 180
tctcaaactt ttatgttata caaatcacat tctgtctcaa aaaatatctc acccacttct 240
cttctgtttc tgcgtgtgta tgtgtgtgtg tgtgtgtctg ggctcaacat gcta 294

<210> 164
<211> 412
<212> DNA
<213> Homo sapien

<220>
<221> misc_feature
<222> (1)...(412)
<223> n = A,T,C or G

<400> 164
cgggattggc tttgagctgc agatgctgcc tgtgaccgca cccggcgtgg aacagaaagc 60
cacctggctg caagtgcgcc agagccgccc tgactacgtg ctgctgtggg gctggggcgt 120
gatgaactcc accgccctga aggaagccca ggccaccgga tacccccgcg acaagatgta 180
cggcgtgtgg tgggccggtg cggagcccga tgtgcgtgac gtgggcgaag gcgccaaggg 240
ctacaacgcg ctggctctga acggctacgg cacgcagtcc aaggtgatcc angacatcct 300
gaaacacgtg cacgacaagg gccagggcac ggggcccaaa gacgaagtgg gctcggtgct 360
gtacacccgc ggcgtgatca tccagatgct ggacaaggtg tcaatcacta at 412

<210> 165
<211> 361
<212> DNA
<213> Homo sapien

<400> 165
ttgacacctt gtccagcatc tgcatctgat gagagcctca gatggctacc actaatggca 60
gaaggcaaag gagaacaggc attgtatggc aagaaaggaa gaaagagaga ggggagaaag 120
gtgctaggtt cttttcaaca accagttctt gatggaactg agagtaagag ctcaaggcca 180
ggtgtggtga ctccaaccag taatcccaac attttaggag gctgaggcag gcagatgtct 240
tgaccccatg agtttgtgac cagcctgaac aacatcatga gactccatct ctacaataat 300
tacaaaaatt aatcaggcat tgtggtatgc cctgtagtcc cagatgctgg acaaggtgtc 360
a 361

<210> 166
<211> 427
<212> DNA
<213> Homo sapien

<400> 166
twgactgact catgtcccct acacccaact atcttctcca ggtggccagg catgatagaa 60
tctgatcctg acttagggga atattttctt tttacttccc atcttgattc cctgccggtg 120
agtttcctgg ttcagggtaa gaaaggagct caggccaaag taatgaacaa atccatcctc 180

acagacgtac agaataagag aacwtggacw tagccagcag aacmcaaktg aaamcagaac 240
mcttamctag gatracaamc mcrraratar ktgcycmcmc wtataataga aaccaaactt 300
gtatctaatt aaatatttat ccacygtcag ggcattagtg gttttgataa atacgctttg 360
gctaggattc ctgaggttag aatggaaraa caattgcamc gagggtaggg gacatgagtc 420
aktctaa 427

<210> 167
<211> 500
<212> DNA
<213> Homo sapien

<220>
<221> misc_feature
<222> (1)...(500)
<223> n = A,T,C or G

<400> 167
aacgtcgcat gctcccggcc gccatggccg cgggatagac tgactcatgt cccctaagat 60
agaggagaca cctgctaggt gtaaggagaa gatggttagg tctacggagg ctccagggtg 120
ggagtagttc cctgctaagg gagggtagac tgttcaacct gttcctgctc cggcctccac 180
tatagcagat gcgagcagga gtaggagaga gggaggtaag agtcagaagc ttatgttgtt 240
tatgcgggga aacgccrtat cgggggcagc cragttatta ggggacantr tagwyartcw 300
agntagcatc caaagcgngg gagttntccc atatggttgg acctgcaggc ggccgcatta 360
gtgattagca tgtgagcccc agacacgcat agcaacaagg acctaaactc agatcctgtg 420
ctgattactt aacatgaatt attgtattta tttaacaact ttgagttatg aggcatatta 480
ttaggtccat attacctgga 500

<210> 168
<211> 358
<212> DNA
<213> Homo sapien

<400> 168
ttcatcgctc ggtgactcaa gcctgtaatc ccagaacttt gggaggccga ggggagcaga 60
tcacctgagg ttgggagttt gagaccagcc tggccaacat ggtgacaacc cgtctctgct 120
aaaaatacaa aaattagcca agcatggtgg catgcacttg taatcccagc tactcgggag 180
gctgaggcag gagaatcact tgaggccagg aggcagaggt tgcagtgagg cagaggttga 240
gatcatgcca ctgcactcca gcctgggcaa cagagtaaga ctccatctca aaaaaaaaaa 300
aaaaaaagaa tgatcagagc cacaaataca gaaaaccttg agtcaccgag cgatgaaa 358

<210> 169
<211> 1265
<212> DNA
<213> Homo sapien

<400> 169
ttctgtccac accaatctta gagctctgaa agaatttgtc tttaaatatc ttttaatagt 60
aacatgtatt ttatggacca aattgacatt ttcgactatt ttttcccaaa aaaagtcagg 120
tgaatttcag cacactgagt tgggaatttc ttatcccaga agwcggcacg agcaatttca 180
tatttattta agattgattc catactccgt tttcaaggag aatccctgca gtctccttaa 240
aggtagaaca aatactttct attttttttt caccattgtg ggattggact ttaagaggtg 300
actctaaaaa aacagagaac aaatatgtct cagttgtatt aagcacggac ccatattatc 360
atattcactt aaaaaaatga tttcctgtgc accttttggc aacttctctt ttcaatgtag 420
ggaaaaactt agtcaccctg aaaacccaca aaataaataa aacttgtaga tgtgggcaga 480
argtttgggg gtggacattg tatgtgttta aattaaaccc tgtatcactg agaagctgtt 540
gtatgggtca gagaaaatga atgcttagaa gctgttcaca tcttcaagag cagaagcaaa 600
ccacatgtct cagctatatt attatttatt ttttatgcat aaagtgaatc atttcttctg 660
tattaatttc caaagggttt taccctctat ttaaatgctt tgaaaaacag tgcattgaca 720

atgggttgat atttttcttt aaaagaaaaa tataattatg aaagccaaga taatctgaag 780
cctgttttat tttaaaactt tttatgttct gtggttgatg ttgtttgttt gtttgtttct 840
attttgttgg ttttttactt tgttttttgt tttgttttgt tttggtttdg catactacat 900
gcagtttctt taaccaatgt ctgtttggct aatgtaatta aagttgttaa tttatatgag 960
tgcatttcaa ctatgtcaat ggtttcttaa tatttattgt gtagaagtac tggtaatttt 1020
tttatttaca atatgtttaa agagataaca gtttgatatg ttttcatgtg tttatagcag 1080
aagttattta tttctatggc attccagcgg atattttggt gtttgcgagg catgcagtca 1140
atattttgta cagttagtgg acagtattca gcaacgcctg atagcttctt tggccttatg 1200
ttaaataaaa agacctgttt gggatgtaaa aaaaaaaaaa aaaaaaaaaa aaaaaaaaaa 1260
aaaaa 1265

<210> 170
<211> 383
<212> DNA
<213> Homo sapien

<400> 170
tgtaagtcga gcagtgtgat gacgatattc ttcttattaa tgtggtaatt gaacaaatga 60
tctgtgatac tgatcctgag ctaggaggcg ctgttcagtt aatgggactt cttcgtactc 120
taattgatcc agagaacatg ctggctacaa ctaataaaac cgaaaaaagt gaatttctaa 180
attttttcta caaccattgt atgcatgttc tcacagcacc acttttgacc aatacttcag 240
aagacaaatg tgaaaaggat aatatagttg gatcaaacaa aaacaacaca atttgtcccg 300
ataattatca aacagcacag ctacttgcct taattttaga gttactcaca ttttgtgtgg 360
aacatcacac tgctcgactt aca 383

<210> 171
<211> 383
<212> DNA
<213> Homo sapien

<400> 171
tgggcacctt caatatcgca agttaaaaat aatgttgagt ttattatact tttgacctgt 60
ttagctcaac agggtgaagg catgtaaaga atgtggactt ctgaggaatt ttcttttaaa 120
aagaacataa tgaagtaaca ttttaattac tcaaggacta cttttggttg aagtttataa 180
tctagatacc tctacttttt gtttttgctg ttcgacagtt cacaaagacc ttcagcaatt 240
tacagggtaa aatcgttgaa gtagtggagg tgaaactgaa atttaaaatt attctgtaaa 300
tactataggg aaagaggctg agcttagaat cttttggttg ttcatgtgtt ctgtgctctt 360
atcatcacac tgctcgactt aca 383

<210> 172
<211> 699
<212> DNA
<213> Homo sapien

<220>
<221> misc_feature
<222> (1)...(699)
<223> n = A,T,C or G

<400> 172
tcgggtgatg cctcctcagg cttgtcgtta gtgtacacag agctgctcat gaagcgacag 60
cggctgcccc tggcacttca gaacctcttc ctctacactt ttggtgcgct tctgaatcta 120
ggtctgcatg ctggcggcgg ctctggccca ggcctcctgg aaagtttctc aggatgggca 180
gcactcgtgg tgctgagcca ggcactaaat ggactgctca tgtctgctgt catggagcat 240
ggcagcagca tcacacgcct ctttgtggtg tcctgctcgc tggtggtcaa cgccgtgctc 300
tcagcagtcc tgctacggct gcagctcaca gccgccttct tcctggccac attgctcatt 360
ggcctggcca tgcgcctgta ctatggcagc cgctagtccc tgacaacttc caccctgatt 420
ccggaccctg tagattgggc gccaccacca gatccccctc ccaggccttc ctccctctcc 480

catcagcggc cctgtaacaa gtgccttgtg agaaaagctg gagaagtgag ggcagccagg 540
ttattctctg gaggttggtg gatgaagggg tacccctagg agatgtgaag tgtgggtttg 600
gttaaggaaa tgcttaccat cccccacccc caaccaagtt nttccagact aaagaattaa 660
ggtaacatca atacctaggc ctgaggaggc atcacccga 699

<210> 173
<211> 701
<212> DNA
<213> Homo sapien

<400> 173
tcgggtgatg cctcctcagg ccagatcaaa cttggggttg aaaactgtgc aaagaaatca 60
atgtcggaga aagaattttg caaaagaaaa atgcctaatc agtactaatt taataggtca 120
cattagcagt ggaagaagaa atgttgatat tttatgtcag ctattttata atcaccagag 180
tgcttagctt catgtaagcc atctcgtatt cattagaaat aagaacaatt ttattcgtcg 240
gaaagaactt ttcaatttat agcatcttaa ttgctcagga ttttaaattt tgataaagaa 300
agctccactt ttggcaggag tagggggcag ggagagagga ggctccatcc acaaggacag 360
agacaccagg gccagtaggg tagctggtgg ctggatcagt cacaacggac tgacttatgc 420
catgagaaga aacaacctcc aaatctcagt tgcttaatac aacacaagct catttcttgc 480
tcacgttaca tgtcctatgt agatcaacag caggtgactc agggacccag gctccatctc 540
catatgagct tccatagtca ccaggacacg ggctctgaaa gtgtcctcca tgcagggaca 600
catgcctctt cctttcattg ggcagagcaa gtcacttatg gccagaagtc acactgcagg 660
gcagtgccat cctgctgtat gcctgaggag gcatcacccg a 701

<210> 174
<211> 700
<212> DNA
<213> Homo sapien

<220>
<221> misc_feature
<222> (1)...(700)
<223> n = A,T,C or G

<400> 174
tcgggtgatg cctcctcang cccctaaatc agagtccagg gtcagagcca caggagacag 60
ggaaagacat agattttaac cggccccctt caggagattc tgaggctcag ttcactttgt 120
tgcagtttga acagaggcag caaggctagt ggttaggggc acggtctcta aagctgcact 180
gcctggatct gcctcccagc tctgccagga accagctgcg tggccttgag ctgctgacac 240
gcagaaagcc ccctgtggac ccagtctcct cgtctgtaag atgaggacag gactctagga 300
accctttccc ttggtttggc ctcactttca caggctccca tcttgaactc tatctactct 360
tttcctgaaa ccttgtaaaa gaaaaaagtg ctagcctggg caacatggca aaaccctgtc 420
tctacaaaaa atacaaaaat tagttgggtg tggtggcatg tgcctgtagt cccagccact 480
tgggaggtgc tgaggtggga ggatcacttg agcccgggag gtggaggttg cagtgagcca 540
agatcatgcc actgcactcc agcctgagta atagagtaag actctgtctc aaaaacaaca 600
acaacaacag tgagtgtgcc tctgtttccg ggttggatgg ggcaccacat ttatgcatct 660
ctcagatttg gacgctgcag cctgaggagg catcacccga 700

<210> 175
<211> 484
<212> DNA
<213> Homo sapien

<220>
<221> misc_feature
<222> (1)...(484)
<223> n = A,T,C or G

<400> 175
tatagggcga attgggcccg agttgcatgn tcccggccgc catggccgcg ggattcgggt 60
gatgcctcct caggcttgtc tgccacaagc tacttctctg agctcagaaa gtgccccttg 120
atgagggaaa atgtcctact gcactgcgaa tttctcagtt ccattttacc tcccagtcct 180
ccttctaaac cagttaataa attcattcca caagtattta ctgattacct gcttgtgcca 240
gggactattc tcaggctgaa gaaggtggga ggggagggcg gaacctgagg agccacctga 300
gccagcttta tatttcaacc atggctggcc catctgagag catctcccca ctctcgccaa 360
cctatcgggg catagcccag ggatgccccc aggcggccca ggttagatgc gtccctttgg 420
cttgtcagtg atgacataca ccttagctgc ttagctggtg ctggcctgag gaggcatcac 480
ccga 484

<210> 176
<211> 432
<212> DNA
<213> Homo sapien

<400> 176
tcgggtgatg cctcctcagg gctcaaggga tgagaagtga cttctttctg gagggaccgt 60
tcatgccacc caggatgaaa atggataggg acccacttgg aggacttgct gatatgtttg 120
gacaaatgcc aggtagcgga attggtactg gtccaggagt tatccaggat agattttcac 180
ccaccatggg acgtcatcgt tcaaatcaac tcttcaatgg ccatggggga cacatcatgc 240
ctcccacaca atcgcagttt ggagagatgg gaggcaagtt tatgaaaagc caggggctaa 300
gccagctcta ccataaccag agtcagggac tcttatccca gctgcaagga cagtcgaagg 360
atatgccacc tcggttttct aagaaaggac agcttaatgc agatgagatt agcctgagga 420
ggcatcaccc ga 432

<210> 177
<211> 788
<212> DNA
<213> Homo sapien

<400> 177
tagcatgttg agcccagaca cagtagcatt tgtgccaatt tctggttgga atggtgacaa 60
catgctggag ccaagtgcta acatgccttg gttcaaggga tggaaagtca cccgtaagga 120
tggcaatgcc agtggaacca cgctgcttga ggctctggac tgcatcctac caccaactcg 180
cccaactgac aagcccttgc gcctgcctct ccaggatgtc tacaaaattg gtggtattgg 240
tactgttcct gttggccgag tggagactgg tgttctcaaa cccggtatgg tggtcacctt 300
tgctccagtc aacgttacaa cggaagtaaa atctgtcgaa atgcaccatg aagctttgag 360
tgaagctctt cctggggaca atgtgggctt caatgtcaag aatgtgtctg tcaaggatgt 420
tcgtcgtggc aacgttgctg gtgacagcaa aaatgaccca ccaatggaag cagctggctt 480
cactgctcag gtgattatcc tgaaccatcc aggccaaata agtgccggct atgcccctgt 540
attggattgc cacacggctc acattgcatg caagtttgct gagctgaagg aaaagattga 600
tcgccgttct ggtaaaaagc tggaagatgg ccctaaattc ttgaagtctg gtgatgctgc 660
cattgttgat atggttcctg gcaagcccat gtgtgttgag agcttctcag actatccacc 720
tttgggtcgc tttgctgttc gtgatatgag acagacagtt gcggtgggtg tctgggctca 780
acatgcta 788

<210> 178
<211> 786
<212> DNA
<213> Homo sapien

<400> 178
tagcatgttg agcccagaca cctgtgtttc tgggagctct ggcagtggcg gattcatagg 60
cacttgggct gcactttgaa tgacacactt ggctttatta gattcactag tttttaaaaa 120
attgttgttc gtttcttttc attaaaggtt taatcagaca gatcagacag cataattttg 180
tatttaatga cagaaacgtt ggtacatttc ttcatgaatg agcttgcatt ctgaagcaag 240
agcctacaaa aggcacttgt tataaatgaa agttctggct ctagaggcca gtactctgga 300

gtttcagagc agccagtgat tgttccagtc agtgatgcct agttatatag aggaggagta 360
cactgtgcac tcttctaggt gtaagggtat gcaactttgg atcttaaaat tctgtacaca 420
tacacacttt atatatatgt atgtatgtat gaaaacatga aattagtttg tcaaatatgt 480
gtgtgtttag tattttagct tagtgcaact atttccacat tatttattaa attgatctaa 540
gacactttct tgttgacacc ttgaatatta atgttcaagg gtgcaatgtg tattccttta 600
gattgttaaa gcttaattac tatgatttgt agtaaattaa cttttaaaat gtatttgagc 660
ccttctgtag tgtcgtaggg ctcttacagg gtgggaaaga ttttaatttt ccagttgcta 720
attgaacagt atggcctcat tatatatttt gatttatagg agtttgtgtc tgggctcaac 780
atgcta 786

<210> 179
<211> 796
<212> DNA
<213> Homo sapien

<400> 179
tagcatgttg agcccagaca ctggttacaa gaccagacct gcttcctcca tatgtaaaca 60
gcttttaaaa agccagtgaa cctttttaat actttggcaa ccttctttca caggcaaaga 120
acacccccat ccgccccttg tttggagtgc agagtttggc tttggttctt tgccttgcct 180
ggagtatact tctaattcct gttgtcctgc acaagctgaa taccgagcta cccaccgcca 240
cccaggccag gtttccactc atttattact ttatgtttct gttccattgc tggtccacag 300
aaataagttt tcctttggag gaatgtgatt ataccccttt aatttcctcc ttttgctttt 360
ttttaatatc attggtatgt gtttggccca gaggaaactg aaattcacca tcatcttgac 420
tggcaatccc attaccatgc tttttttaaa aaacgtaatt tttcttgcct tacattggca 480
gagtagccct tcctggctac tggcttaatg tagtcactca gtttctaggt ggcattaggc 540
atgagacctg aagcacagac tgtcttacca caaaaggtga caagatctca aaccttagcc 600
aaagggctat gtcaggtttc aatgctatct gcttctgttc ctgctcactg ttctggattt 660
tgtccttctt catccctagc accagaattt cccagtctcc ctccctacct tcccttgttt 720
taattctaat ctatcagcaa aataactttt caaatgtttt aaccggtatc tccatgtgtc 780
tgggctcaac atgcta 796

<210> 180
<211> 488
<212> DNA
<213> Homo sapien

<400> 180
ggatgtgctg caaggcgatt aagttgggta acgccagggt tttcccagtc acgacgttgt 60
aaaacgacgg ccagtgaatt gtaatacgac tcactatagg gcgaattggg cccgacgtcg 120
catgctcccg gccgccatgg ccgcgggata gcatgttgag cccagacacc tgcaggtcat 180
ttggagagat ttttcacgtt accagcttga tggtcttttt caggaggaga gacactgagc 240
actcccaagg tgaggttgaa gatttcctct agatagccgg ataagaagac taggagggat 300
gcctagaaaa tgattagcat gcaaatttct acctgccatt tcagaactgt gtgtcagccc 360
acattcagct gcttcttgtg aactgaaaag agagaggtat tgagactttt ctgatggccg 420
ctctaacatt gtaacacagt aatctgtgtg tgtgtgggtg tgtgtgtgtg tctgggctca 480
acatgcta 488

<210> 181
<211> 317
<212> DNA
<213> Homo sapien

<400> 181
tagcatgttg agcccagaca cggcgacggt acctgatgag tggggtgatg gcacctgtga 60
aaaggaggaa cgtcatcccc catgatattg gggacccaga tgatgaacca tggctccgcg 120
tcaatgcata tttaatccat gatactgctg attggaagga cctgaacctg aagtttgtgc 180
tgcaggttta tcgggactat tacctcacgg gtgatcaaaa cttcctgaag gacatgtggc 240
ctgtgtgtct agtaagggat gcacatgcag tggccagtgt gccaggggta tggttggtgt 300

ctgggctcaa catgcta                                                    317

<210> 182
<211> 507
<212> DNA
<213> Homo sapien

<220>
<221> misc_feature
<222> (1)...(507)
<223> n = A,T,C or G

<400> 182
tagcatgttg agcccagaca ctggctgtta gccaaatcct ctctcagctg ctccctgtgg      60
tttggtgact caggattaca gaggcatcct gtttcaggga acaaaaagat tttagctgcc     120
agcagagagc accacataca ttagaatggt aaggactgcc acctccttca agaacaggag     180
tgagggtggt ggtgaatggg aatggaagcc tgcattccct gatgcatttg tgctctctca     240
aatcctgtct tagtcttagg aaaggaagta aagtttcaag gacggttccg aactgctttt     300
tgtgtctggg ctcaacatgc tatcccgcgg ccatggcggc cgggagcatg cgacgtcggg     360
cccaattcgc cctatagtga gtcgtattac aattcactgg ccgtcgtttt acaacgtcgt     420
gactgggaaa accctggcgt tacccaactt aatcgccttg cagcacatcc ccctttccca     480
gctggcgtaa tancgaaaag gcccgca                                         507

<210> 183
<211> 227
<212> DNA
<213> Homo sapien

<400> 183
gatttacgct gcaacactgt ggaggtagcc ctggagcaag gcaggcatgg atgcttctgc      60
aatccccaaa tggagcctgg tatttcagcc aggaatctga gcagagcccc ctctaattgt     120
agcaatgata agttattctc tttgttcttc aaccttccaa tagccttgag cttccagggg     180
agtgtcgtta atcattacag cctggtctcc acagtgttgc agcgtaa                   227

<210> 184
<211> 225
<212> DNA
<213> Homo sapien

<400> 184
ttacgctgca acactgtgga gcagattaac atcagacttt tctatcaaca tgactggggt      60
tactaaaaag acaacaaatc aatggcttca aaagtctaag gaataatttc gatacttcaa     120
ctttataaaa cctgacaaaa ctatcaatca agcataaaga cagatgaaga acatttccag     180
attttggcca atcagatatt ttacctccac agtgttgcag cgtaa                     225

<210> 185
<211> 597
<212> DNA
<213> Homo sapien

<400> 185
ggcccgacgt cgcatgctcc cggccgccat ggccgcggga ttcgttaggg tctctatcca      60
ctgggaccca taggctagtc agagtattta gagttgagtt cctttctgct tcccagaatt     120
tgaaagaaaa ggagtgaggt gatagagctg agagatcaga tttgcctctg aagcctgttc     180
aagatgtatg tgctcagacc ccaccactgg ggcctgtggg tgaggtcctg ggcatctatt     240
tgaatgaatt gctgaagggg agcactatgc caaggaaggg gaacccatcc tggcactggc     300
acaggggtca ccttatccag tgctcagtgc ttctttgctg ctacctggtt ttctctcata     360
tgtgaggggc aggtaagaag aagtgcccrg tgttgtgcga gttttagaac atctaccagt     420

aagtggggaa gtttcacaaa gcagcagctt tgttttgtgt attttcacct tcagttagaa 480
gaggaaggct gtgagatgaa tgttagttga gtggaaaaga cgggtaagct tagtggatag 540
agaccctaac gaatcactag tgcggccgcc ttgcaggtcg accatatggg agagctc 597

<210> 186
<211> 597
<212> DNA
<213> Homo sapien

<400> 186
ggcccgaagt tgcatgttcc cggccgccat ggccgcggga ttcgttaggg tctctatcca 60
ctacctaaaa aatcccaaac atataactga actcctcaca cccaattgga ccaatccatc 120
accccagagg cctacagatc ctcctttgat acataagaaa atttccccaa actacctaac 180
tatatcattt tgcaagattt gttttaccaa attttgatgg cctttctgag cttgtcagtg 240
tgaaccacta ttacgaacga tcggatatta actgcccctc accgtccagg tgtagctggc 300
aacatcaagt gcagtaaata ttcattaagt tttcacctac taaggtgctt aaacacccta 360
gggtgccatg tcggtagcag atcttttgat ttgtttttat ttcccataag ggtcctgttc 420
aaggtcaatc atacatgtag tgtgagcagc tagtcactat cgcatgactt ggagggtgat 480
aatagaggcc tcctttgctg ttaaagaact cttgtcccag cctgtcaaag tggatagaga 540
ccctaacgaa tcactagtgc ggccgcctgc aggtcgacca tatgggagag ctcccaa 597

<210> 187
<211> 324
<212> DNA
<213> Homo sapien

<400> 187
tcgttagggt ctctatccac ttgcaggtaa aatccaatcc tgtgtatatc ttatagtctt 60
ccatatgtag tggttcaaga gactgcagtt ccagaaagac tagccgagcc catccatgtc 120
ttccacttaa ccctgctttg ggttacacat cttaactttt ctgttcaagt ttctctgtgt 180
agtttatagc atgagtattg ggawaatgcc ctgaaacctg acatgagatc tgggaaacac 240
aaacttactc aataagaatt tctcccatat ttttatgatg gaaaaatttc acatgcacag 300
aggagtggat agagacccta acga 324

<210> 188
<211> 178
<212> DNA
<213> Homo sapien

<220>
<221> misc_feature
<222> (1)...(178)
<223> n = A,T,C or G

<400> 188
gcgcggggat tcggggtgat acctcctcat gccaaaatac aacgtntaat ttcacaactt 60
gccttccaat ttacgcattt tcaatttgct ctccccattt gttgagtcac aacaaacacc 120
attgcccaga aacatgtatt acctaacatg cacatactct taaaactact catccctt 178

<210> 189
<211> 367
<212> DNA
<213> Homo sapien

<400> 189
tgacaccttg tccagcatct gacacagtct tggctcttgg aaaatattgg ataaatgaaa 60
atgaatttct ttagcaagtg gtataagctg agaatatacg tatcacatat cctcattcta 120
agacacattc agtgtccctg aaattagaat aggacttaca ataagtgtgt tcactttctc 180

aatagctgtt attcaattga tggtaggcct taaaagtcaa agaaatgaga gggcatgtga 240
aaaaaagctc aacatcactg atcattagaa aacttccatt caaaccccca atgagatacc 300
atctcatacc agtcagaatg gctattatta aaaagtcaaa aaataacaga tgctggacaa 360
ggtgtca 367

<210> 190
<211> 369
<212> DNA
<213> Homo sapien

<220>
<221> misc_feature
<222> (1)...(369)
<223> n = A,T,C or G

<400> 190
gacaccttgt ccagcatctg acaacgctaa cagcctgagg agatctttat ttatttattt 60
agtttttact ctggctaggc agatggtggc taaaacattc atttacccat ttattcattt 120
aattgttcct gcaaggccta tggatagagt attgtccagc actgctctgg aagctaggag 180
catggggatg aacaagatag gctacatcct gttcccacag aacttccact ttagtctggg 240
aaacagatga tatatacaaa tatataaatg aattcaggta gttttaagta cgaaaagaat 300
aagaaagcag agtcatgatt tanaatgctg gaaacagggg ctattgcttg agatattgaa 360
ggtgcccaa 369

<210> 191
<211> 369
<212> DNA
<213> Homo sapien

<400> 191
tgacaccttg tccagcatct gcacagggaa aagaaactat tatcagagtg aacaggcaac 60
ctacagaatg ggagaaaatt tttgcaatct atccatctga caaagggcta atatccagaa 120
tctacaaaga acttatacaa atttacaaga aacaaacaaa caaacaactc ctcaaaaagt 180
gggtgaagga tgtgaacaga cacttctcaa aagaagacat ttatggggcc aacaaacata 240
tgaaaaaaag ctcatcatca ctggtcacta gataaatgca aatcaaaacc acaatgagat 300
accatctcat tccagttaga atggcaatca ttaaaaagtc aggaaacaac agatgctgga 360
caaggtgtc 369

<210> 192
<211> 449
<212> DNA
<213> Homo sapien

<400> 192
tgacgcttgg ccacttgaca cttcatcttt gcacagaaaa acttctttac agatttaatt 60
caagactggt ctagtgacag tcctccagac attttttcat ttgttccata tacgtggaat 120
tttaaaatca tgtttcatca gtttgaaatg atttgggctg ctaatcaaca caattggatc 180
gactgttcta ctaaacaaca ggaaaatgtg tatctggcag cctgtggaga aacactaaac 240
attgattttt ctttgccttt tacggacttt gttccagcta catgtaatac caagttctct 300
ttaagaggag aagatgttga tcttcatttg tttctaccag actgccaccc tagtaaatat 360
tctttattta tgctggtaaa aaattgccat ccaaataaga tgattcatga tactggtatt 420
cctgctgagt gtcaagtggc caagcgtca 449

<210> 193
<211> 372
<212> DNA
<213> Homo sapien

<400> 193
tgacgcttgg ccacttgaca ccagggatgt akcagttgaa tataatcctg caattgtaca 60
tattggcaat ttcccatcaa acattctaga aagagacaac caggattgct aggccataaa 120
agctgcaata aataactggt aattgcagta atcatttcag gccaattcaa tccagtttgg 180
ctcagaggtg cctttggctg agagaagagg tgagatataa tgtgttttct tgcaacttct 240
tggaagaata actccacaat agtctgagga ctagatacaa acctatttgc cattaaagca 300
ccagagtctg ttaattccag tactgataag tgttggagat tagactccag tgtgtcaagt 360
ggccaagcgt ca 372

<210> 194
<211> 309
<212> DNA
<213> Homo sapien

<220>
<221> misc_feature
<222> (1)...(309)
<223> n = A,T,C or G

<400> 194
tgacgcttgg ccacttgaca cttatgtaga atccatcgtg ggctgatgca agccctttat 60
ttaggcttag tgttgtgggc accttcaata tcacactaga gacaaacgcc acaagatctg 120
cagaaacatt cagttctgan cactcgaatg gcaggataac tttttgtgtt gtaatccttc 180
acatatacaa aaacaaactc tgcantctca cgttacaaaa aaacgtactg ctgtaaaata 240
ttaagaaggg gtaaaggata ccatctataa caaagtaact tacaactagt gtcaagtggc 300
caagcgtca 309

<210> 195
<211> 312
<212> DNA
<213> Homo sapien

<220>
<221> misc_feature
<222> (1)...(312)
<223> n = A,T,C or G

<400> 195
tgacgcttgg ccacttgaca cccaatctcg cacttcatcc tcccagcacc tgatgaagta 60
ggactgcaac tatccccact tcccagatga ggggaccaan gtacacatta ggacccggat 120
gggagcacag atttgtccga tcccagactc caagcactca gcgtcactcc aggacagcgg 180
ctttcagata aggtcacaaa catgaatggc tccgacaacc ggagtcagtc cgtgctgagt 240
taaggcaatg gtgacacgga tgcacgtgtn acctgtaatg gttcatcgta agtgtcaagt 300
ggccaagcgt ca 312

<210> 196
<211> 288
<212> DNA
<213> Homo sapien

<400> 196
tgtatcgacg tagtggtctc ctcagccatg cagaactgtg actcaattaa acctctttcc 60
tttatgaatt acccaatctc gggtagtgtc tttatagtag tgtgagaatg gactaataca 120
agtacatttt acttagtaat aataataaac aaatatatta catttttgtg tatttactac 180
accatatttt ttattgttat tgtagtgtac accttctact tattaaaaga aataggcccg 240
aggcgggcag atcacgaggt caggagatgg agaccactac gtcgatac 288

<210> 197

<211> 289
<212> DNA
<213> Homo sapien

<400> 197
ttgggcacct tcaatatcat gacaggtgat gtgataacca agaaggctac taagtgatta 60
atgggtgggt aatgtataca gagtaggtac actggacaga ggggtaattc atagccaagg 120
caggagaagc agaatggcaa aacatttcat cacactactc aggatagcat gcagtttaaa 180
acctataagt agtttatttt tggaattttc cacttaatat tttcagactg caggtaacta 240
aactgtggaa cacaagaaca tagataaggg gagaccacta cgtcgatac 289

<210> 198
<211> 288
<212> DNA
<213> Homo sapien

<400> 198
gtatcgacgt agtggtctcc caagcagtgg gaagaaaacg tgaaccaatt aaaatgtatc 60
agatacccca aagaaaggcg cttgagtaaa gattccaagt gggtcacaat ctcagatctt 120
aaaattcagg ctgtcaaaga gatttgctat gaggttgctc tcaatgactt caggcacagt 180
cggcaggaga ttgaagccct ggccattgtc aagatgaagg agctttgtgc catgtatggc 240
aagaaagacc ccaatgagcg ggactcctgg agaccactac gtcgatac 288

<210> 199
<211> 1027
<212> DNA
<213> Homo sapien

<220>
<221> misc_feature
<222> (1)...(1027)
<223> n = A,T,C or G

<400> 199
gctttttggg aaaaacncaa ntgggggaaa gggggnttnn tngcaagggg ataaaggggg 60
aancccaggg tttccccatt cagggaggtg taaaaagncg gccaggggat tgtaanagga 120
ttcaataata gggggaatgg gcccngaagt tgcaaggttc cngcccgcca tgnccgcggg 180
atttagtgac attacgacgs tggtaataaa gtgggsccaa waaatatttg tgatgtgatt 240
tttsgaccag tgaacccatt gwacaggacc tcatttccty tgagatgrta gccataatca 300
gataaaagrt tagaagtytt tctgcacgtt aacagcatca ttaaatggag tggcatcacc 360
aatttcaccc tttgttagcc gataccttcc ccttgaaggc attcaattaa gtgaccaatc 420
gtcatacgag aggggatggc atggggattg atgatgatat caggggtgat accttcacag 480
gtgaaaggca tatcctcttg tctatactga ataccacaag tacccttttg accatgtcga 540
ctagcaaatt tgtctccaat ctgtgtwatc cctaacagag cgtaccctta ttttacaaaa 600
tttatatcct tcctgattga gagttaccat aacctgatcc acaatgcccg tctcgctwgt 660
tctgagaaaa gtgctacagt ctctcttggt atagcgtcta ttggtgctct ccaattcatc 720
ttcatttttc aggcaaggtg aactgttttg cctataataa cmtcatctcc tgatacmcga 780
aacccckgga rctatcaaac catcatcatc cagcgttckt watgtymcta aatccctatt 840
gcggccgcct gcaggtcaac atatnggaaa accccccacc ccttnggagc ntaccttgaa 900
ttttccatat gtcccntaaa ttanctngnc ttancctggc cntaacctnt tccggtttaa 960
attgtttccg cccccnttcc ccnccttnna accggaaacc ttaattttna accnggggtt 1020
cctatcc 1027

<210> 200
<211> 207
<212> DNA
<213> Homo sapien

<400> 200
agtgacatta cgacgctggc catcttgaat cctagggcat gaagttgccc caaagttcag 60
cacttggtta agcctgatcc ctctggttta tcacaaagaa taggatggga taaagaaagt 120
ggacacttaa ataagctata aattatatgg tccttgtcta gcaggagaca actgcacagg 180
tatactacca gcgtcgtaat gtcacta 207

<210> 201
<211> 209
<212> DNA
<213> Homo sapien

<400> 201
tgggcacctt caatatctat taaaagcaca aatactgaag aacacaccaa gactatcaat 60
gaggttacat ctggagtcct cgatatatca ggaaaaaatg aagtgaacat tcacagagtt 120
ttacttcttt gggaactcaa atgctagaaa agaaaagggt gccctctttc tctggcttcc 180
tggtcctatc cagcgtcgta atgtcacta 209

<210> 202
<211> 349
<212> DNA
<213> Homo sapien

<220>
<221> misc_feature
<222> (1)...(349)
<223> n = A,T,C or G

<400> 202
ntacgctgca acactgtgga gccactggtt tttattcccg gcaggttatc cagcaaacag 60
tcactgaaca caccgaagac cgtggtatgg taaccgttca cagtaatcgt tccagtcgtc 120
tgcgggaccc cgacgagcgt cactgggtac agaccagatt cagccggaag agaaagcgcc 180
gcagggagag actcgaactc cactccgctg gtgagcagcc ccatgttttc aactcgaagt 240
tcaaacggca ttgggttata taccatcagc tgaacttcac acacatctcc ttgaacccac 300
tggaaatcta ttttcttgtt ccgctcttct ccacagtgtt gcagcgtaa 349

<210> 203
<211> 241
<212> DNA
<213> Homo sapien

<400> 203
tgctcctctt gccttaccaa cccaaagccc actgtgaaat atgaagtgaa tgacaaaatt 60
cagttttcaa cgcaatatag tatagtttat ctgattcttt tgatctccag gacactttaa 120
acaactgcta ccaccaccac caacctaggg atttaggatt ctccacagac cagaaattat 180
ttctcctttg agtttcaggc tcctctggga ctcctgttca tcaatgggtg gtaaatggct 240
a 241

<210> 204
<211> 248
<212> DNA
<213> Homo sapien

<400> 204
tagccattta ccacccatct gcaaaccswg acmwwcargr cywgwackya ggcgatttga 60
agtactggta atgctctgat catgttagtt acataagtgt ggtcagttta caaaaattca 120
cagaactaaa tactcaatgc tatgtgttca tgtctgtgtt tatgtgtgtg taatgtttca 180
attaagtttt tttaaaaaaa agagatgatt tccaaataag aaagccgtgt tggtaaggca 240
agaggagc 248

<210> 205
<211> 505
<212> DNA
<213> Homo sapien

<220>
<221> misc_feature
<222> (1)...(505)
<223> n = A,T,C or G

<400> 205
tacgctgcaa cactgtggag ccattcatac aggtccctaa ttaaggaaca agtgattatg 60
ctacctttgc acggttaggg taccgcggcc gttaaacatg tgtcactggg caggcggtgc 120
ctctaatact ggtgatgcta gaggtgatgt ttttggtaaa caggcggggt aagatttgcc 180
gagttccttt tacttttttt aacctttcct tatgagcatg cctgtgttgg gttgacagtg 240
ggggtaataa tgacttgttg gttgattgta gatattgggc tgttaattgt cagttcagtg 300
ttttaatctg acgcaggctt atgcggagga gaatgttttc atgttactta tactaacatt 360
agttcttcta tagggtgata gattggtcca attgggtgtg aggagttcag ttatatgttt 420
gggatttttt aggtagtggg tgttganctt gaacgctttc ttaattggtg gctgctttta 480
rgcctactat gggtggtaaa tggct 505

<210> 206
<211> 179
<212> DNA
<213> Homo sapien

<400> 206
tagactgact catgtcccct accaaagccc atgtaaggag ctgagttctt aaagactgaa 60
gacagactat tctctggaga aaaataaaat ggaaattgta ctttaaaaaa aaaaaaaatc 120
ggccgggcat ggtagcacac acctgtaatc ccagctacta ggggacatga gtcagtcta 179

<210> 207
<211> 176
<212> DNA
<213> Homo sapien

<400> 207
agactgactc atgtccccta ccccaccttc tgctgtgctg ccgtgttcct aacaggtcac 60
agactggtac tggtcagtgg cctgggggtt ggggacctct attatatggg atacaaattt 120
aggagttgga attgacacga tttagtgact gatgggatat gggtggtaaa tggcta 176

<210> 208
<211> 196
<212> DNA
<213> Homo sapien

<400> 208
agactgactc atgtccccta tttaacaggg tctctagtgc tgtgaaaaaa aaaaatgctg 60
aacattgcat ataacttata ttgtaagaaa tactgtacaa tgactttatt gcatctgggt 120
agctgtaagg catgaaggat gccaagaagt ttaaggaata tgggtggtaa atggctaggg 180
gacatgagtc agtcta 196

<210> 209
<211> 345
<212> DNA
<213> Homo sapien

<220>
<221> misc_feature
<222> (1)...(345)
<223> n = A,T,C or G

<400> 209
gacgcttggc cacttgacac cttttatttt ttaaggattc ttaagtcatt tangtnactt 60
tgtaagtttt tcctgtgccc ccataagaat gatagcttta aaaattatgc tggggtagca 120
aagaagatac ttctagcttt agaatgtgta ggtatagcca ggattcttgt gaggaggggt 180
gatttagagc aaatttctta ttctccttgc ctcatctgta acatggggat aataatagaa 240
ctggcttgac aaggttggaa ttagtattac atggtaaata catgtaaaat gtttagaatg 300
gtgccaagta tctaggaagt acttgggcat gggtggtaaa tggct 345

<210> 210
<211> 178
<212> DNA
<213> Homo sapien

<400> 210
gacgcttggc cacttgacac tagagtaggg tttggccaac tttttctata aaggaccaga 60
gagtaaatat ttcaggcttt gtgggttgtg cagtctctct tgcaactact cagctctgcc 120
attgtagcat agaaatcagc catagacagg acagaaatga atgggtggta aatggcta 178

<210> 211
<211> 454
<212> DNA
<213> Homo sapien

<400> 211
tgggcacctt caatatctat ccagcgcatc taaattcgct tttttcttga ttaaaaattt 60
caccacttgc tgtttttgct catgtatacc aagtagcagt ggtgtgaggc catgcttgtt 120
ttttgattcg atatcagcac cgtataagag cagtgctttg gccattaatt tatcttcatt 180
gtagacagca tagtgtagag tggtatctcc atactcatct ggaatatttg gatcagtgcc 240
atgttccagc aacattaacg cacattcatc ttcctggcat tgtacggcct ttgtcagagc 300
tgtcctcttt ttgttgtcaa ggacattaag ttgacatcgt ctgtccagca cgagttttac 360
tacttctgaa ttcccattgg cagaggccag atgtagagca gtcctctttt gcttgtccct 420
cttgttcaca tcagtgtccc tgagcataac ggaa 454

<210> 212
<211> 337
<212> DNA
<213> Homo sapien

<400> 212
tccgttatgc cacccagaaa acctactgga gttacttatt aacatcaagg ctggaaccta 60
tttgcctcag tcctatctga ttcatgagca catggttatt actgatcgca ttgaaaacat 120
tgatcacctg ggtttcttta tttatcgact gtgtcatgac aaggaaactt acaaactgca 180
acgcagagaa actattaaag gtattcagaa acgtgaagcc agcaattgtt tcgcaattcg 240
gcattttgaa aacaaatttg ccgtggaaac tttaatttgt tcttgaacag tcaagaaaaa 300
cattattgag gaaaattaat atcacagcat aacggaa 337

<210> 213
<211> 715
<212> DNA
<213> Homo sapien

<220>
<221> misc_feature

<222> (1)...(715)
<223> n = A,T,C or G

<400> 213
tcgggtgatg cctcctcagg catcttccat ccatctcttc aagattagct gtcccaaatg 60
tttttccttc tcttctttac tgataaattt ggactccttc ttgacactga tgacagcttt 120
agtatccttc ttgtcacctt gcagacttta aacataaaaa tactcattgg ttttaaaagg 180
aaaaaagtat acattagcac tattaagctt ggccttgaaa cattttctat cttttattaa 240
atgtcggtta gctgaacaga attcatttta caatgcagag tgagaaaaga agggagctat 300
atgcatttga gaatgcaagc attgtcaaat aaacatttta aatgctttct taaagtgagc 360
acatacagaa atacattaag atattagaaa gtgtttttgc ttgtgtacta ctaattaggg 420
aagcaccttg tatagttcct cttctaaaat tgaagtagat tttaaaaacc catgtaattt 480
aattgagctc tcagttcaga ttttaggaga attttaacag ggatttggtt ttgtctaaat 540
tttgtcaatt tntttagtta atctgtataa ttttataaat gtcaaactgt atttagtccg 600
ttttcatgct gctatgaaag aaatacccan gacagggtta tttataaang gaaagangtt 660
aatttgactc ccagttcaca ggcctgagga ngnatcnccc gaaatcctta ttgcg 715

<210> 214
<211> 345
<212> DNA
<213> Homo sapien

<220>
<221> misc_feature
<222> (1)...(345)
<223> n = A,T,C or G

<400> 214
ggtaangngc atacntcggt gctccggccg ccggagtcgg gggattcggg tgatgcctcc 60
tcaggcccac ttgggcctgc ttttcccaaa tggcagctcc tctggacatg ccattccttc 120
tcccacctgc ctgattcttc atatgttggg tgtccctgtt tttctggtgc tatttcctga 180
ctgctgttca gctgccactg tcctgcaaag cctgcctttt taaatgcctc accattcctt 240
catttgtttc ttaaatatgg gaagtgaaag tgccacctga ggccgggcac agtggctcac 300
gcctgtaatc ccagcacttt gggagcctga ggaggcatca cccga 345

<210> 215
<211> 429
<212> DNA
<213> Homo sapien

<400> 215
ggtgatgcct cctcaggcga agctcaggga ggacagaaac ctcccgtgga gcagaagggc 60
aaaagctcgc ttgatcttga ttttcagtac gaatacagac cgtgaaagcg gggcctcacg 120
atccttctga ccttttgggt tttaagcagg aggtgtcaga aaagttacca cagggataac 180
tggcttgtgg cggccaagcg ttcatagcga cgtcgctttt tgatccttcg atgtcggctc 240
ttcctatcat tgtgaagcag aattcaccaa gcgttggatt gttcacccac taatagggaa 300
cgtgagctgg gtttagaccg tcgtgagaca ggttagtttt accctactga tgatgtgtkg 360
ttgccatggt aatcctgctc agtacgagag gaaccgcagg ttcasacatt tggtgtatgt 420
gcttgcctt 429

<210> 216
<211> 593
<212> DNA
<213> Homo sapien

<220>
<221> misc_feature
<222> (1)...(593)

<223> n = A,T,C or G

<400> 216
tgacacctat gtccngcatc tgttcacagt ttccacaaat agccagcctt tggccacctc 60
tctgtcctga ggtatacaag tatatcagga ggtgtatacc ttctcttctc ttccccacca 120
aagagaacat gcaggctctg gaagctgtct taggagcctt tgggctcaga atttcagagt 180
cttgggtacc ttggatgtgg tctggaagga gaaacattgg ctctggataa ggagtacagc 240
cggaggaggg tcacagagcc ctcagctcaa gcccctgtgc cttagtctaa aagcagcttt 300
ggatgaggaa gcaggttaag taacatacgt aagcgtacac aggtagaaag tgctgggagt 360
cagaattgca cagtgtgtag gagtagtacc tcaatcaatg agggcaaatc aactgaaaga 420
agaagaccna ttaatgaatt gcttangggg aaggatcaag gctatcatgg agatctttct 480
aggaagatta ttgtttanaa ttatgaaagg antagggcag ggacagggcc agaagtanaa 540
ganaacattg cctatanccc ttgtcttgca cccagatgct ggacaaggtg tca 593

<210> 217
<211> 335
<212> DNA
<213> Homo sapien

<400> 217
tgacaccttg tccagcatct gacgtgaaga tgagcagctc agaggaggtg tcctggattt 60
cctggttctg tgggctccgt ggcaatgaat tcttctgtga agtggatgaa gactacatcc 120
aggacaaatt taatcttact ggactcaatg agcaggtccc tcactatcga caagctctag 180
acatgatctt ggacctggag cctgatgaag aactggaaga caaccccaac cagagtgacc 240
tgattgagca ggcagccgag atgctttatg gattgatcca cgcccgctac atccttacca 300
accgtggcat cgcccagatg ctggacaagg tgtca 335

<210> 218
<211> 248
<212> DNA
<213> Homo sapien

<400> 218
tacgtactgg tcttgaaggt cttaggtaga gaaaaaatgt gaatatttaa tcaaagacta 60
tgtatgaaat gggactgtaa gtacagaggg aagggtggcc cttatcgcca gaagttggta 120
gatgcgtccc cgtcatgaaa tgttgtgtca ctgcccgaca tttgccgaat tactgaaatt 180
ccgtagaatt agtgcaaatt ctaacgttgt tcatctaaga ttatggttcc atgtttctag 240
tactttta 248

<210> 219
<211> 530
<212> DNA
<213> Homo sapien

<220>
<221> misc_feature
<222> (1)...(530)
<223> n = A,T,C or G

<400> 219
tgacgcttgg ccacttgaca caagtagggg ataaggacaa agacccatna ggtggcctgt 60
cagccttttg ttactgttgc ttccctgtca ccacggcccc ctctgtaggg gtgtgctgtg 120
ctctgtggac attggtgcat tttcacacat accattctct ttctgcttca cagcagtcct 180
gaggcgggag cacacaggac taccttgtca gatgangata atgatgtctg gccaactcac 240
cccccaacct tctcactagt tatangaaga gccangccta naaccttcta tcctgncccc 300
ttgccctatg acctcatccc tgttccatgc cctattctga tttctggtga actttggagc 360
agcctggttt ntcctcctca ctccagcctc tctccatacc atggtanggg ggtgctgttc 420
cacncaaang gtcaggtgtg tctggggaat cctnananct gccnggagtt tccnangcat 480

tcttaaaaac cttcttgcct aatcanatng tgtccagtgg ccaaccntcn 530

<210> 220
<211> 531
<212> DNA
<213> Homo sapien

<400> 220
tgacgcttgg ccacttgaca ctaaatagca tcttctaaag gcctgattca gagttgtgga 60
aaattctccc agtgtcaggg attgtcagga acagggctgc tcctgtgctc actttacctg 120
ctgtgtttct gctggaaaag gagggaagag gaatggctga tttttaccta atgtctccca 180
gtttttcata ttcttcttgg atcctcttct ctgacaactg ttcccttttg gtcttcttct 240
tcttgctcag agagcaggtc tctttaaaac tgagaaggga gaatgagcaa atgattaaag 300
aaaacacact tctgaggccc agagatcaaa tattaggtaa atactaaacc gcttgcctgc 360
tgtggtcact tttctcctct ttcacatgct ctatccctct atcccccacc tattcatatg 420
gcttttatct gccaagttat ccggcctctc atcaaccttc tcccctagcc tactggggga 480
tatccatctg ggtctgtctc tggtgtattg gtgtcaagtg gccaagcgtc a 531

<210> 221
<211> 530
<212> DNA
<213> Homo sapien

<400> 221
attgacgctt ggccacttga cacccgcctg cctgcaatac tggggcaagg gccttcactg 60
ctttcctgcc accagctgcc actgcacaca gagatcagaa atgctaccaa ccaagactgt 120
tggtcctcag cctctctgag gagaaagagc agaagcctgg aagtcagaag agaagctaga 180
tcggctacgg ccttggcagc cagcttcccc acctgtggca ataaagtcgt gcatggctta 240
acaatggggg cacctcctga gaaacacatt gttaggcaat tcggcgtgtg ttcatcagag 300
catatttaca caaacctcga tagtgcagcc tactatccac tattgctcct acgctgcaaa 360
cctgaacagc atgggactgt actgaatact ggaagcagct ggtgatggta cttatttgtg 420
tatctaaaca cagagaaggt acagtaagaa tatggtatca taaacttaca gggaccgcca 480
tcctatatgc agtctgttgt gaccaaaatg tgtcaagtgg ccaagcgtca 530

<210> 222
<211> 578
<212> DNA
<213> Homo sapien

<220>
<221> misc_feature
<222> (1)...(578)
<223> n = A,T,C or G

<400> 222
tgtatcgacg tagtggtctc cgggctacta ggccgttgtg tgctggtagt acctggttca 60
ctgaaaggcg catctccctc cccgcgtcgc cctgaagcag gggaggactt tcgcccagcc 120
aaggcagttg tatgagtttt agctgcggca cttcgagacc tctgagccca cctccttcag 180
gagccttccc cgattaagga agccagggta aggattcctt cctcccccag acaccacgaa 240
caaaccacca ccccccctat tctggcagcc catatacatc agaacgaaac aaaaataaca 300
aataaacnaa aaccaaaaaa aaaagagaag gggaaatgta tatgtctgtc catcctgttg 360
ctttagcctg tcagctccta nagggcaggg accgtgtctt ccgaatggtc tgtgcagcgc 420
cgactgcggg aagtatcgga ggaggaagca gagtcagcag aagttgaacg gtgggcccgg 480
cggctcttgg gggctggtgt tgtacttcga gaccgctttc gctttttgtc ttagatttac 540
gtttgctctt tggagtggga naccactacn tcnataca 578

<210> 223
<211> 578

<212> DNA
<213> Homo sapien

<400> 223
tgtatcgacg tagtggtctc ctcttgcaaa ggactggctg gtgaatggtt tccctgaatt 60
atggacttac cctaaacata tcttatcatc attaccagtt gcaaaatatt agaatgtgtt 120
gtcactgttt catttgattc ctagaaggtt agtcttagat atgttacttt aacctgtatg 180
ctgtagtgct ttgaatgcat tttttgtttg catttttgtt tgcccaacct gtcaattata 240
gctgcttagg tctggactgt cctggataaa gctgttaaaa tattcaccag tccagccatc 300
ttacaagcta attaagtcaa ctaaatgctt ccttgttttg ccagacttgt tatgtcaatc 360
ctcaatttct gggttcattt tgggtgccct aaatcttagg gtgtgacttt cttagcatcc 420
tgtaacatcc attcccaagc aagcacaact tcacataata ctttccagaa gttcattgct 480
gaagcctttc cttcacccag cggagcaact tgattttcta caacttccct catcagagcc 540
acaagagtat gggatatgga gaccactacg tcgataca 578

<210> 224
<211> 345
<212> DNA
<213> Homo sapien

<220>
<221> misc_feature
<222> (1)...(345)
<223> n = A,T,C or G

<400> 224
tgtatcgacg tantggtctc ccaaggtgct gggattgcag gcatgagcca ccactcccag 60
gtggatcttt ttctttatac ttacttcatt aggtttctgt tattcaagaa gtgtagtggt 120
aaaagtcttt tcaatctaca tggttaaata atgatagcct gggaaataaa tagaaatttt 180
ttctttcatc tttaggttga ataaagaaac agaaaaaata gaacatactg aaaataatct 240
aagttccaac catagaagaa ctgcagaaga aatgaagaaa gtgatgatga tttagatttt 300
gatattgatt tagaagacac aggaggagac cactacgtcg ataca 345

<210> 225
<211> 347
<212> DNA
<213> Homo sapien

<400> 225
tgtatcgacg tagtggtctc caaactgagg tatgtgtgcc actagcacac aaagccttcc 60
aacagggacg caggcacagg cagtttaaag ggaatctgtt tctaaattaa tttccacctt 120
ctctaagtat tctttcctaa aactgatcaa ggtgtgaagc ctgtgctctt tcccaactcc 180
cctttgacaa cagccttcaa ctaacacaag aaaaggcatg tctgacactc ttcctgagtc 240
tgactctgat acgttgttct gatgtctaaa gagctccaga acaccaaagg gacaattcag 300
aatgctggtg tataacagac tccaatggag accactacgt cgataca 347

<210> 226
<211> 281
<212> DNA
<213> Homo sapien

<220>
<221> misc_feature
<222> (1)...(281)
<223> n = A,T,C or G

<400> 226
aggngnggga ntgtatcgac gtagtggtct cccaacagtc tgtcattcag tctgcaggtg 60

tcagtgtttt ggacaatgag gcaccattgt cacttattga ctcctcagct ctaaatgctg 120
aaattaaatc ttgtcatgac aagtctggaa ttcctgatga ggttttacaa agtattttgg 180
atcaatactc caacaaatca gaaagccaga aagaggatcc tttcaatatt gcagaaccac 240
gagtggattt acacacctca ggagaccact acgtcgatac a 281

<210> 227
<211> 3646
<212> DNA
<213> Homo sapien

<400> 227
gggaaacact tcctcccagc cttgtaaggg ttggagccct ctccagtata tgctgcagaa 60
tttttctctc ggtttctcag aggattatgg agtccgcctt aaaaaaggca agctctggac 120
actctgcaaa gtagaatggc caaagtttgg agttgagtgg ccccttgaag ggtcactgaa 180
cctcacaatt gttcaagctg tgtggcgggt tgttactgaa actcccggcc tccctgatca 240
gtttccctac attgatcaat ggctgagttt ggtcaggagc accccttccg tggctccact 300
catgcaccat tcataatttt acctccaagg tcctcctgag ccagaccgtg ttttcgcctc 360
gaccctcagc cggttcggct cgccctgtac tgcctctctc tgaagaagag gagagtctcc 420
ctcacccagt cccaccgcct taaaaccagc ctactccctt agggtcatcc catgtctcct 480
cggctatgtc ccctgtaggc tcatcaccca ttgcctcttg gttgcaaccg tggtgggagg 540
aagtagcccc tctactacca ctgagagagg cacaagtccc tctgggtgat gagtgctcca 600
cccccttcct ggtttatgtc ccttctttct acttctgact tgtataattg gaaaacccat 660
aatcctccct tctctgaaaa gccccaggct ttgacctcac tgatggagtc tgtactctgg 720
acacattggc ccacctggga tgactgtcaa cagctccttt tgaccctttt cacctctgaa 780
gagagggaaa gtatccaaag agaggccaaa aagtacaacc tcacatcaac caataggccg 840
gaggaggaag ctagaggaat agtgattaga gacccaattg ggacctaatt gggacccaaa 900
tttctcaagt ggagggagaa cttttgacga tttccaccgg tatctcctcg tgggtattca 960
gggagctgct cagaaaccta taaacttgtc taaggcgact gaagtcgtcc aggggcatga 1020
tgagtcacca ggagtgtttt tagagcacct ccaggaggct tatcagattt acaccccttt 1080
tgacctggca gcccccgaaa atagccatgc tcttaatttg gcatttgtgg ctcaggcagc 1140
cccagatagt aaaaggaaac tccaaaaact agagggattt tgctggaatg aataccagtc 1200
agcttttaga gatagcctaa aaggtttttg acagtcaaga ggttgaaaaa caaaaacaag 1260
cagctcaggc agctgaaaaa agccactgat aaagcatcct ggagtatcag agtttactgt 1320
tagatcagcc tcatttgact tcccctccca catggtgttt aaatccagct acactacttc 1380
ctgactcaaa ctccactatt cctgttcatg actgtcagga actgttggaa actactgaaa 1440
ctggccgacc tgatcttcaa aatgtgcccc taggaaaggt ggatgccacc atgttcacag 1500
acagtagcag cttcctcgag aagggactac gaaaggccgg tgcagctgtt accatggaga 1560
cagatgtgtt gtgggctcag gctttaccag caaacacctc agcacaaaag gctgaattga 1620
tcgccctcac tcaggctctc cgatggggta aggatattaa cgttaacact gacagcaggt 1680
acgcctttgc tactgtgcat gtacgtggag ccatctacca ggagcgtggg ctactcacct 1740
cagcaggtgg ctgtaatcca ctgtaaagga catcaaaagg aaaacacggc tgttgcccgt 1800
ggtaaccaga aagctgattc agcagctcaa gatgcagtgt gactttcagt cacgcctcta 1860
aacttgctgc ccacagtctc ctttccacag ccagatctgc ctgacaatcc cgcatactca 1920
acagaagaag aaaactggcc tcagaactca gagccaataa aaatcaggaa ggttggtgga 1980
ttcttcctga ctctagaatc ttcatacccc gaactcttgg gaaaacttta atcagtcacc 2040
tacagtctac cacccattta ggaggagcaa agctacctca gctcctccgg agccgtttta 2100
agatccccca tcttcaaagc ctaacagatc aagcagctct ccggtgcaca acctgcgccc 2160
aggtaaatgc caaaaaaggt cctaaaccca gcccaggcca ccgtctccaa gaaaactcac 2220
caggagaaaa gtgggaaatt gactttacag aagtaaaacc acaccgggct gggtacaaat 2280
accttctagt actggtagac accttctctg gatggactga agcatttgct accaaaaacg 2340
aaactgtcaa tatggtagtt aagtttttac tcaatgaaat catccctcga catgggctgc 2400
ctgtttgcca tagggtctga taatggaccg gccttcgcct tgtctatagt ttagtcagtc 2460
agtaaggcgt taaacattca atggaagctc cattgtgcct atcgacccca gagctctggg 2520
caagtagaac gcatgaactg caccctaaaa aacactctta caaaattaat cttagaaacc 2580
ggtgtaaatt gtgtaagtct ccttccttta gccctactta gagtaaggtg caccccttac 2640
tgggctgggt tcttaccttt tgaaatcatg tatgggaggg tgctgcctat cttgcctaag 2700
ctaagagatg cccaattggc aaaaatatca caaactaatt tattacagta cctacagtct 2760
ccccaacagg tacaagatat catcctgcca cttgttcgag gaacccatcc caatccaatt 2820

cctgaacaga cagggccctg ccattcattc ccgccaggtg acctgttgtt tgttaaaaag 2880
ttccagagag aaggactccc tcctgcttgg aagagacctc acaccgtcat cacgatgcca 2940
acggctctga aggtggatgg cattcctgcg tggattcatc actcccgcat caaaaaggcc 3000
aacagagccc aactagaaac atgggtcccc agggctgggt caggcccctt aaaactgcac 3060
ctaagttggg tgaagccatt agattaattc tttttcttaa ttttgtaaaa caatgcatag 3120
cttctgtcaa acttatgtat cttaagactc aatataaccc ccttgttata actgaggaat 3180
caatgatttg attcccccaa aaacacaagt ggggaatgta gtgtccaacc tggtttttac 3240
taaccctgtt tttagactct ccctttcctt taatcactca gcttgtttcc acctgaattg 3300
actctccctt agctaagagc gccagatgga ctccatcttg gctctttcac tggcagccgc 3360
ttcctcaagg acttaacttg tgcaagctga ctcccagcac atccaagaat gcaattaact 3420
gataagatac tgtggcaagc tatatccgca gttcccagga attcgtccaa ttgatcacag 3480
cccctctacc cttcagcaac caccaccctg atcagtcagc agccatcagc accgaggcaa 3540
ggccctccac cagcaaaaag attctgactc actgaagact tggatgatca ttagtatttt 3600
tagcagtaaa gttttttttt ctttttcttt cttttttctt cgtgcc 3646

<210> 228
<211> 419
<212> DNA
<213> Homo sapien

<220>
<221> misc_feature
<222> (1)...(419)
<223> n = A,T,C or G

<400> 228
taagagggta caagatctaa gcacagccgt caatgcagaa cacagaacgt agcctggtaa 60
gtgtgttaag agtgggaatt tttggagtac agagtaaggc acctaaccct agctggggtt 120
tggtgacggt cccagatggc ttacagaaga aagtgtcctg agatgagttt ttaagaatga 180
ataaggatag acacaagtga ggactgactt ggcagtggtg aatggtgggt ggcaaaaaac 240
ttcgcatgta tggaaactgc acgtacagga atgaagaatg agactgtgtg gtgtttaatg 300
agctgcaaat actaatttta tcctgaaagt tttgaagagt taactaaaaa gtatttttta 360
gtaaggaaat aaccctacat ttcagggtta ttgtttgttt anatattgaa ggtgcccaa 419

<210> 229
<211> 148
<212> DNA
<213> Homo sapien

<400> 229
aagagggtac ctgtatgtag ccatggtggc aatgagagac tgattactac ctgctggaga 60
ttgtttaagt gagttaatat attaaggata aagggagcca ggttttttga ctgttggaga 120
aggaaattac agatattgaa ggtcccaa 148

<210> 230
<211> 257
<212> DNA
<213> Homo sapien

<400> 230
taagagggta cmaaaaaaaa aaaatagaac gaatgagtaa gacctactat ttgatagtac 60
aacagggtga ctatagtcaa tgataactta attatacatt taacatagag tgtaattgga 120
ttgtttgtaa ctcgaaggat aaatgcttga gaggatggat accccattct ccatgatgta 180
cttatttcac attacatgcc tgtatcaaag catctcatat accctataaa tatgtacacc 240
tactatgtac cctctta 257

<210> 231
<211> 260

<212> DNA
<213> Homo sapien

<400> 231
taagagggta cgggtatttg ctgatgggat ttttttttct ttctttttct ttggaaaaca 60
aaatgaaagc cagaacaaaa ttattgaaca aaagacaggg actaaatctg gagaaatgaa 120
gtcccctcac ctgactgcca tttcattcta tctgaccttc cagtctaggt taggagaata 180
gggggtggag gggattaatc tgatacaggt atatttaaag caactctgca tgtgtgccag 240
aagtccatgg taccctctta 260

<210> 232
<211> 596
<212> DNA
<213> Homo sapien

<220>
<221> misc_feature
<222> (1)...(596)
<223> n = A,T,C or G

<400> 232
tgctcctctt gccttaccaa ccacaaatta gaaccataat gagatgtcac ctcatacctg 60
gtgggattaa cattatttaa aaaatcagaa gtattgacaa ggatgtgaag aaattagaac 120
atctgtgcac tgttggtggg aatgtaaaaa aggtgtggcc actatgggta acagcatgaa 180
ggttcctcaa aaaaaatttt ttttaatcta ctctatgatc gatcttgagg ttgtttatgc 240
aaaagaactg aaatcaggat tttgaggaaa tattcacatt cccacatcca tttctgcttt 300
attcataata ctcaagagat ggaaacaacc taaatgtcca tcccgggatg aatggataaa 360
cacagtgtgg tatatgcata caatggaata ttatttagtc tttaaaaaga aaaattctat 420
catatactac aacttanatn aaccttgagg acacaatgct nagtgaaata agccacggaa 480
ggacgaatac tgcattattc ccttatatga agtatctaaa gtggtcaaac tcttanagca 540
naaagtaaaa atgggtggtt gccanacagt tggttaggcn agaaganaan cctant 596

<210> 233
<211> 96
<212> DNA
<213> Homo sapien

<400> 233
tcttctgaag acctttcgcg actcttaagc tcgtggttgg taaggcaaga ggagcgttgg 60
taaggcaaga ggagcgttgg taaggcaaga ggagca 96

<210> 234
<211> 313
<212> DNA
<213> Homo sapien

<400> 234
tgtaagtcga gcagtgtgat gataaaactt gaatggatca atagttgctt cttatggatg 60
agcaaagaaa gtagtttctt gtgatggaat ctgctcctgg caaaaatgct gtgaacgttg 120
ttgaaaagac aacaaagagt ttagagtagt acataaattt agaatagtac ataaacttag 180
aatagtacat aaacttagta cataaataat gcacgaagca ggggcagggc ttgagagaat 240
tgacttcaat ttggaaagag tatctactgt aggttagatg ctctcaaaca gcatcacact 300
gctcgactta caa 313

<210> 235
<211> 550
<212> DNA
<213> Homo sapien

<400> 235
aacgaggaca gatccttaaa aagaatgttg agtgaaaaaa gtagaaaata agataatctc 60
caaagtccag tagcattatt taaacatttt taaaaaatac actgataaaa attttgtaca 120
tttcccaaaa atacatatgg aagcacagca gcatgaatgc ctatgggrtt gaggataggg 180
gttgggagta gggatgggga taaaggggga aaataaaacc agagaggagt cttacacatt 240
tcatgaacca aggagtataa ttatttcaac tatttgtacc wgaagtccag aaagagtgga 300
ggcagaaggg ggagaagagg gcgaagaaac gtttttggga gaggggtccc asaagagaga 360
ttttcgcgat gtggcgctac atacgttttt ccaggatgcc ttaagctctg caccctattt 420
ttctcatcac taatattaga ttaaaccctt tgaagacagc gtctgtggtt tctctacttc 480
agctttccct ccgtgtcttg cacacagtag ctgttttaca agggttgaac tgactgaagt 540
gagattattc 550

<210> 236
<211> 325
<212> DNA
<213> Homo sapien

<400> 236
tagactgact catgtcccct accagagtag ctagaattaa tagcacaagc ctctacaccc 60
aggaactcac tattgaatac ataaatggaa tttattcagc cttaaaaagt ttggaaggaa 120
attctgacat atgctaaaac atggatgaac cttgaagact ttatgataag taaaagaagc 180
cagtcataaa aggaaaaata ttgcatgatt ccacttatat gaggtaccta gagtagtcaa 240
tttcatagaa acacaaaata gaatggtgtt tgccagggct tttgaggaaa agggaatgac 300
aagttagggg acatgagtca gtcta 325

<210> 237
<211> 373
<212> DNA
<213> Homo sapien

<220>
<221> misc_feature
<222> (1)...(373)
<223> n = A,T,C or G

<400> 237
tagactgact catgtcccct atctactcaa catttccact tgaagtctga taggcatctc 60
agacttatct tgtcccaaag caaactcttt atttcttttc atcctagtct ttatttcttg 120
tgctgtctta cccatctcaa aagagtgcca aaatccacca agttgctgaa acagaaatct 180
aagaaatatc cttgattctt ctttttccca tctacttcac ttctaattca ttagtaaata 240
atctgtttca gaaaaccaaa cacctcatgt tctcactcat aagggggagt tgaacaatga 300
gaacacacag acacagggag gggaacatca cacaccacgg cccgtcaggg agtangggac 360
atgagtcagt cta 373

<210> 238
<211> 492
<212> DNA
<213> Homo sapien

<220>
<221> misc_feature
<222> (1)...(492)
<223> n = A,T,C or G

<400> 238
tagactgact catgtcccct ataatgctcc caggcatcag aaagcatctc aaactggagc 60
tgacaccatg gcagaggttt caggtaagtc acaaaagggg tcctaaagaa tttgccctca 120

atatcagagt gattagaaga agtggacaga gctacccaag ttaaacatat gcgagataaa 180
aaaaatatgg cacttgtgaa cacacactac aggaggaaaa taaggaacat aatagcatat 240
tgtgctatta tgatgatgaa gaacctctct anaagaaaac ataaccaaag aaacaaagaa 300
aattcctgcn aatgtttaat gctatagaag aaattaacaa aaacatatat tcaatgaatt 360
cagaaaagtt agcaggtcan aagaaaacaa atcaaagacc agaataatcc cattttagat 420
tgtcgagtaa actanaacag aaagaatacc actggaaatt gaattcctac gtangggaca 480
tgantcantc ta 492

<210> 239
<211> 482
<212> DNA
<213> Homo sapien

<220>
<221> misc_feature
<222> (1)...(482)
<223> n = A,T,C or G

<400> 239
tggaaagtat ttaatgatgg gcaacttgct gtttacttcc tacatatccc atcatcttct 60
gtattttttt aaataacttt tttttggatt tttaaagtaa ccttattctg agaggtaaca 120
tggattacat acttctaagc cattaggaga ctctatgtta aaccaaaagg aaatgttact 180
agatcttcat ttgatcaata ggatgtgata atcatcatct ttctgctcta atggaaaagt 240
actanaaaca tggaaccata atcttagatg aacaacgtta gaatttgcac taattctacg 300
gaatttcagt aattcggcaa atgtcgggca gtgacacaac atttcatgac ggggacgcat 360
ctaccaactt ctggcgataa gggccaccct tccctctgta cttacagtcc catttcatac 420
acagtctttg attaaatatt cacatttttt ctctacctaa agaccttcaa gaccagtacg 480
ta 482

<210> 240
<211> 519
<212> DNA
<213> Homo sapien

<220>
<221> misc_feature
<222> (1)...(519)
<223> n = A,T,C or G

<400> 240
tgtatcgacg tagtggtctc cccatgtgat agtctgaaat atagcctcat gggatgagag 60
gctgtgcccc agcccgacac ccgtaaaggg tctgtgctga ggtggattag taaaagagga 120
aagccttgca gttgagatag aggaagggca ctgtctcctg cctgcccctg ggaactgaat 180
gtctcggtat aaaacccgat tgtacatttg ttcaattctg agataggaga aaaaccaccc 240
tatggcggga ggcgagacat gttggcagca atgctgcctt gttatgcttt actccacaga 300
tgtttgggcg gagggaaaca taaatctggc ctacgtgcac atccaggcat agtacctccc 360
tttgaactta attatgacac agattccttt gctcacatgt ttttttgctg accttctcct 420
tattatcacc ctgctctcct accgcattcc ttgtgctgag ataatgaaaa taatatcaat 480
aaaaacttga nggaactcgg agaccactac gtcgataca 519

<210> 241
<211> 771
<212> DNA
<213> Homo sapien

<220>
<221> misc_feature
<222> (1)...(771)

<223> n = A,T,C or G

<400> 241
tgtatcgacg tagtggtctc cactcccgcc ttgacggggc tgctatctgc cttccaggcc 60
actgtcacgg ctcccgggta gaagtcactt atgagacaca ccagtgtggc cttgttggct 120
tgaagctcct cagaggaggg tgggaacaga gtgaccgagg gggcagcctt gggctgacct 180
aggacggtca gcttggtccc tccgccaaac acgagagtgc tgctgcttgt atatgagctg 240
cagtaataat cagcctcgtc ctcagcctgg agcccagaga tggtcaggga ggccgtgttg 300
ccanacttgg agccagagaa gcgattagaa acccctgagg gccgattacc gacctcataa 360
atcatgaatt tgggggcttt gcctgggtgc tgttggtacc angagacatt attataacca 420
ccaacgtcac tgctggttcc antgcaggga aaatggttga tcnaactgtc caagaaaacc 480
actacgtcca taccaatcca ctaattgccn gccgcctgca ggttcaacca tattggggaa 540
naactccccn ccgccgtttg ggattgncat naacctttga aattttttcc tattanttgt 600
ccccctaaaa taaaccnttg ggcnttaatc cattgggtcc atancttntt tncccggttt 660
ttaaaanttg tttatcccgc cncccnattt cccccccaac tttccaaaac ccgaaaccnt 720
tnaaatttnt tnaaaccctg gggggttccc nnaattnnan ttnaanctnc c 771

<210> 242
<211> 167
<212> DNA
<213> Homo sapien

<400> 242
tgggcacctt caatatcggg ctcatcgata acatcacgct gctgatgctg ctgttgctgg 60
tcctctctag gaacctctgg attttcaaat tctttgagga attcatccaa attatctgcc 120
tctcctcctt tcctcctttt tctaaggtct tctggtacaa gcggtca 167

<210> 243
<211> 338
<212> DNA
<213> Homo sapien

<400> 243
ttgggcacct tcaatatcta ctgatctaaa tagtgtggtt tgaggcctct tgttcctggc 60
taaaaatcct tggcaagagt caatctccac tttacaatag aggtaaaaat cttacaatgg 120
atattcttga caaagctagc atagagacag caattttaca caaggtattt ttcacctgtt 180
taataacagt ggttttccta cacccatagg gtgccaccaa gggaggagtg cacagttgca 240
gaaacaaatt aagatactga agacaacact acttaccatt tcccgtatag ctaaccacca 300
gttcaactgt acatgtatgt tcttatgggc aatcaaga 338

<210> 244
<211> 346
<212> DNA
<213> Homo sapien

<400> 244
tttttggctc ccatacagca cactctcatg ggaaatgtct gttctaaggt caacccataa 60
tgcaaaaatc atcaatatac ttgaagatcc ccgtgtaagg tacaatgtat ttaatattat 120
cactgataca attgatccaa taccagtttt agtctggcat tgaatcaaat cactgttttt 180
gttgtataaa aagagaaata tttagcttat atttaagtac catattgtaa gaaaaaagat 240
gcttatcttt acatgctaaa atcatgatct gtacattggt gcagtgaata ttactgtaaa 300
agggaagaag gaatgaagac gagctaagga tattgaaggt gcccaa 346

<210> 245
<211> 521
<212> DNA
<213> Homo sapien

<220>
<221> misc_feature
<222> (1)...(521)
<223> n = A,T,C or G

<400> 245
accaatccca cacggatact gagggacaag tatatcatcc catttcatcc ctacagcagc 60
aacttcatga ggcaggagtt attagtccca ttttacagaa gaggaaactg agacttaggg 120
agatcaagta atttgcccag gtcgcacaat tagtgataga gccagggctt gaagcgacgt 180
ctgtcttaag ccaatgaccc ctgcagatta ttagagcaac tgttctccac aacagtgtaa 240
gcctcttgct anaagctcag gtccacaagg gcagagattt ttgtctgttt tgctcattgc 300
tccttcccca ttgcttagag cagggtctgc cacgaancag gttctcaatg catagttatt 360
aaatgtatat aagagcaaac atatgttaca gagaactttc tgtatgcttg tcacttacat 420
gaatcacctg tganatgggt atgcttgttc cccantgttg cagatnaaga tattgaangt 480
gcccaaatca ctanttgcgg gcgcctgcan gtccancata t 521

<210> 246
<211> 482
<212> DNA
<213> Homo sapien

<220>
<221> misc_feature
<222> (1)...(482)
<223> n = A,T,C or G

<400> 246
tggaaccaat ccaaataccc atcaatgata gactggataa agaaaatttg gcacatgttc 60
accatgaaat actatgcagc cataaaaaag gatgagttca tatcctttgc agggacatgg 120
atgaagctgg agaccatcat tctcagcaaa ctaacaaggg aacagaaaac caaacactgc 180
atgttctcac tcttaagtgg gagctgaaca atgagaacac atggacacag ggaggggaac 240
atcacacagt ggggcctgct ggtgggtagg ggtctagggg agggatagca ttaggagaaa 300
tacctaatgt agatgacggg ttgatgggtg cagcaaacca ccatgacacg tgtataccta 360
tgtaacaaac ctgcatgttc tgcacatgta ccccagaact taaagtgtta ataaaaaaat 420
taagaaaaaa gttaagtatg tcatagatac ataaaatatt gtanatattg aaggtgccca 480
aa 482

<210> 247
<211> 474
<212> DNA
<213> Homo sapien

<220>
<221> misc_feature
<222> (1)...(474)
<223> n = A,T,C or G

<400> 247
ttcgatacag gcacagagta agcagaaaaa tggctgtggt ttaaccaagt gagtacagtt 60
aagtgagaga ggggcagaga agacaagggc atatgcaggg ggtgattata acaggtggtt 120
gtgctgggaa gtgagggtac tcggggatga ggaacagtga aaaagtggca aaaagtggta 180
agatcagtga attgtacttc tccagaattt gatttctggn ggagtcaaat aactatccag 240
tttggggtat catanggcaa cagttgaggt ataggaggta gaagtcncag tgggataatt 300
gaggttatga anggtttggt actgactggt actgacaang tctgggttat gaccatggga 360
atgaatgact gtanaagcgt anaggatgaa actattccac ganaaagggg tccnaaaact 420
aaaaannnaa gnnnnngggg aatattattt atgtggatat tgaangtgcc caaa 474

<210> 248

<211> 355
<212> DNA
<213> Homo sapien

<220>
<221> misc_feature
<222> (1)...(355)
<223> n = A,T,C or G

<400> 248
ttcgatacag gcaaacatga actgcaggag ggtggtgacg atcatgatgt tgccgatggt 60
ccggatggnc acgaagacgc actggancac gtgcttacgt ccttttgctc tgttgatggc 120
cctgagggga cgcaggaccc ttatgaccct cagaatcttc acaacgggag atggcactgg 180
attgantccc antgacacca gagacacccc aaccaccagn atatcantat attgatgtag 240
ttcctgtaga nggccccctt gtggaggaaa gctccatnag ttggtcatct tcaacaggat 300
ctcaacagtt tccgatggct gtgatgggca tagtcatant taaccntgtn tcgaa 355

<210> 249
<211> 434
<212> DNA
<213> Homo sapien

<400> 249
ttggattggt cctccaggag aacaagggga aaaaggtgac cgagggctcc ctggaactca 60
aggatctcca ggagcaaaag gggatggggg aattcctggt cctgctggtc ccttaggtcc 120
acctggtcct ccaggcttac caggtcctca aggcccaaag ggtaacaaag gctctactgg 180
acccgctggc cagaaaggtg acagtggtct tccagggcct cctgggcctc caggtccacc 240
tggtgaagtc attcagcctt taccaatctt gtcctccaaa aaaacgagaa gacatactga 300
aggcatgcaa gcagatgcag atgataatat tcttgattac tcggatggaa tggaagaaat 360
atttggttcc ctcaattccc tgaaacaaga catcgagcat atgaaatttc caatgggtac 420
tcagaccaat ccaa 434

<210> 250
<211> 430
<212> DNA
<213> Homo sapien

<220>
<221> misc_feature
<222> (1)...(430)
<223> n = A,T,C or G

<400> 250
tggattggtc acatggcaga gacaggattc caaggcagtg agaggaggat acaatgcttc 60
tcactagtta ttattattta ttttattttt gagatgaagt ctcgctttgt ctcccaggct 120
ggagagcggt ggtgcgatct tggctctctg caacccccgc ctcaagcaat tctcctgtct 180
tagcctcgcg ggtagatgga attacaggcg cccaccgcca tgcccaacta atttttttgt 240
gtcttcagta gagacagggt ttcgccatgt tgggcaggct ggtcttgaac tcctgacctc 300
nagtgatctg ccctcctcgg cctcacaaag tgctggaatt acaggcatgg gctgctgcac 360
ccagtcaact tctcactagt tatggcctta tcattttcac cacattctat tggcccaaaa 420
aaaaaaaaan 430

<210> 251
<211> 329
<212> DNA
<213> Homo sapien

<400> 251

tggtactcca ccatyatggg gtcaaccgcc atcctcgccc tcctcctggc tgttctccaa 60
ggagtctgtg ccgaggtgca gctgrtgcag tctggagcag aggtgaaaaa gtccggggag 120
tctctgaaga tctcctgtaa gggttctgga tacaccttta agatctactg gatcgcctgg 180
gtgcgccagt tgcccgggaa aggcctggag tggatggggc tcatctttcc tgatgactct 240
gataccagat acagcccgtc cttccaaggc caggtcacca tctcagtcga taagtccatc 300
agcaccgcct atctgcagtg gagtaccaa 329

<210> 252
<211> 536
<212> DNA
<213> Homo sapien

<400> 252
tggtactcca ctcagcccaa ccttaattaa gaattaagag ggaacctatt actattctcc 60
caggctcctc tgctctaacc aggcttctgg gacagtatta gaaaaggatg tctcaacaag 120
tatgtagatc ctgtactggc ctaagaagtt aaactgagaa tagcataaat cagaccaaac 180
ttaatggtcg ttgagacttg tgtcctggag cagctgggat aggaaaactt ttgggcagca 240
agaggaagaa ctgcctggaa gggggcatca tgttaaaaat tacaagggga acccacacca 300
ggcccccttc ccagctctca gcctagagta ttagcatttc tcagctagag actcacaact 360
tccttgctta gaatgtgcca ccggggggag tccctgtggg tgatgaggct ctcaagagtg 420
agagtggcat cctatcttct gtgtgcccac aggagcctgg cccgagactt agcaggtgaa 480
gtttctggtc caggctttgc ccttgactca ctatgtgacc tctggtggag taccaa 536

<210> 253
<211> 507
<212> DNA
<213> Homo sapien

<220>
<221> misc_feature
<222> (1)...(507)
<223> n = A,T,C or G

<400> 253
ntgttgcgat cccagtaact cgggaagctg aggcgggagg atcacctgag ctcaggaggt 60
tgaggccgca gtgagccggg accacgccac tacactccag cctggggcat agagtgagac 120
cctccaagac agaaaagaaa agaaaggaag ggaaagggaa agggaaaagg aaaaggaaaa 180
ggaaaaggaa aaggaaaaga caagacaaaa caagacttga atttggatct cctgacttca 240
attttatgtt ctttctacac cacaattcct ctgcttacta agatgataat ttagaaaccc 300
ctcgttccat tctttacagc aagctggaag tttggtcaag taattacaat aatagtaaca 360
aatttgaata ttatatgcca ggtgtttttc attcctgctc tcacttaatt ctcaccactc 420
tgatataaat acaattgctg ccgggtgtgg tggctcatgc ctgtaatccc ggcactttgg 480
gagaccgagg tgggcggats gcaacaa 507

<210> 254
<211> 222
<212> DNA
<213> Homo sapien

<220>
<221> misc_feature
<222> (1)...(222)
<223> n = A,T,C or G

<400> 254
ttggattggt cactgtgagg aagccaaatc ggatccgaga gtctttttct aaaggccagt 60
actggccaca ctttctcctg ccgccttcct caaagctgaa gacacacaga gcaaggcgct 120
tctgttttac tccccaatgg taactccaaa ccatagatgg ttagctnccc tgctcatctt 180

tccacatccc tgctattcag tatagtccgt ggaccaatcc aa 222

<210> 255
<211> 463
<212> DNA
<213> Homo sapien

<400> 255
tgttgcgatc cataaatgct gaaatggaaa taaacaacat gatgagggag gattaagttg 60
gggagggagc acattaaggt ggccatgaag tttgttggaa gaagtgactt ttgaacaagg 120
ccttggtgtt aagagctgat gagagtgtcc cagacagagg ggccactggt acaatagacg 180
agatgggaga gggcttggaa ggtgtgcgaa ataggaagga gtttgttctg gtatgagtct 240
agtgaacaca gaggcgagag gccctggtgg gtgcagctgg agagttatgc agaataacat 300
taggccctgt gggggactgt agactgtcag caataatcca cagtttggat tttattctaa 360
gagtgatggg aagccgtgga aagggggtta agcaaggagt gaaattatca gatttacagt 420
gataaaaata aattggtctg gctactgggg aaaaaaaaaa aaa 463

<210> 256
<211> 262
<212> DNA
<213> Homo sapien

<400> 256
ttggattggt caacctgctc aactctacyt ttcctccttc ttcctaaaaa attaatgaat 60
ccaatacatt aatgccaaaa cccttgggtt ttatcaatat ttctgttaaa aagtattatc 120
cagaactgga cataatacta cataataata cataacaacc ccttcatctg gatgcaaaca 180
tctattaata tagcttaaga tcactttcac tttacagaag caacatcctg ttgatgttat 240
tttgatgttt ggaccaatcc aa 262

<210> 257
<211> 461
<212> DNA
<213> Homo sapien

<220>
<221> misc_feature
<222> (1)...(461)
<223> n = A,T,C or G

<400> 257
gnggnnnnnn nnncaattcg actcngttcc cntggtancc ggtcgacatg gccgcgggat 60
taccgcttgt nnctgggggt gtatggggga ctatgaccgc ttgtagctgg gggtgtatgg 120
gggactatga ccgcttgtag mtggkggtgt atgggggact atgaccgctt gtcgggtggt 180
cggataaacc gacgcaaggg acgtgatcga agctgcgttc ccgctctttc gcatcggtag 240
ggatcatgga cagcaatatc cgcattcgyc tgaaggcgtt cgaccatcgc gtgctcgatc 300
aggcgaccgg cgacatcgcc gacaccgcac gccgtaccgg cgcgctcatc cgcggtccga 360
tcccgcttcc cacgcgcatc gagaagttca cggtcaaccg tggcccgcac gtcgacaaga 420
agtcgcgcga gcagttcgag gtgcgtacct acaagcggtc a 461

<210> 258
<211> 332
<212> DNA
<213> Homo sapien

<220>
<221> misc_feature
<222> (1)...(332)
<223> n = A,T,C or G

<400> 258
tgaccgcttg tagctggggg tgtatggggg actacgaccg cttgtagctg ggggtgtatg 60
ggggactatg accgcttgta gctgggggtg tatgggggac tatgaccgct tgtagctggg 120
ggtgtatggg ggactaggac cgcttgtagc tgggggtgta tgggggacta tgaccgcttg 180
tagctggggg tgtatggggg actacgaccg cttgtagctg ggggtgtatg ggggactatg 240
accgcttgta nctgggggtg tatgggggac tatgaccgct tgtgctgcct gggggatggg 300
aggagagttg tggttgggga aaaaaaaaaa aa 332

<210> 259
<211> 291
<212> DNA
<213> Homo sapien

<220>
<221> misc_feature
<222> (1)...(291)
<223> n = A,T,C or G

<400> 259
taccgcttgt gaccgcttgt gaccgcttgt gaccgcttgt gaccgcttgt gaccgcttgt 60
gaccgcttgt gaccgcttgt gaccgcttgt gaccgcttgt gaccgcttgt gaccgcttgt 120
gaccgcttgt gaccgcttgt nacngggggt gtctggggga ctatgannga ntgtnactgg 180
gggtgtctgg gggnctatga nngantgtna cngggggtgt ctgggggact atganngact 240
gtgcnncctg ggggatcnga ggagantngn ggntagngat ggttngggan a 291

<210> 260
<211> 238
<212> DNA
<213> Homo sapien

<400> 260
taagagggta ctggttaaaa tacaggaaat ctggggtaat gaggcagaga accaggatac 60
tttgaggtca gggatgaaaa ctagaatttt tttctttttt tttgcctgag aaacttgctg 120
ctctgaagag gcccatgtat taattgcttt gatcttcctt ttcttacagc cctttcaagg 180
gcagagccct ccttatcctg aaggaatctt atccttagct atagtatgta ccctctta 238

<210> 261
<211> 746
<212> DNA
<213> Homo sapien

<220>
<221> misc_feature
<222> (1)...(746)
<223> n = A,T,C or G

<400> 261
ttgggcacct tcaatatcaa tagctaacat ttattgagtg tttatcgtat cataaaacac 60
tgttctaagc ctttaaacgt actaattcat ttaatgctca taatcacttt agaaggtggg 120
tactagtatt agtctcattt acagatgcaa catgcaggca cagagaggtt aattaacttg 180
cccaaggtaa cacagctaag aaatagaaaa aatattgaat ctggaaagtt gggcttctgg 240
gtaacccaca gagtcttcaa tgagcctggg gcctcactca gtttgctttt acaaagcgaa 300
tgagtaacat cacttaattc agtgagtagg ccaaatggag gtcagctacg agtttctgct 360
gttcttgcag tggactgaca gatgtttaca acgtctggcc atcagtwaat ggactgatta 420
tcattgggaw gtgggtgggc tgaatgttgg ccagtgaagt ttattcawgc catattttta 480
tgtttaggat gacttttggc tggtcctagg gcaagctctg tctgscacgg aacacagaat 540
wacacaggga ccccctcaat ttctggtgtg gctagaacca tgaaccactg gttgggggaa 600

caagcggtca aaacctaagt gcggccggct ggcagggtcc acccatatgg ggaaaactcc 660
cnacgcgttt ggaatgcctn agctngaatt attctaanag ttgtccncnt aaaattagcc 720
tgggcgttaa tcangggtcn naagcc 746

<210> 262
<211> 588
<212> DNA
<213> Homo sapien

<220>
<221> misc_feature
<222> (1)...(588)
<223> n = A,T,C or G

<400> 262
   tgaccgcttg tcatctcaca tggggtcctg cacgcttttg cctttgtagg aaacctgaca 60
   tttgtctgtt tcttctttct cttttccttc ccatatcctc ctaatttacg tttgacttgt 120
   ttgctgagga ggcaggagct agagactgct gtgagctcat aggggtggga agtttatcct 180
   tcaagtcccg cccactcatc actgcttctc accttcccct gaccaggctt acaagtgggt 240
   tcttgcctgc tttccctttg gacccaacaa gcccctgtaa tgagtgtgca tgactctgac 300
   agctgtggac tcagggtcct tggctacagc tgccatgtaa aatatctcat ccagttctcg 360
   caaattgtta aaataaccac atttcttaga ttccagtacc caaatcatgt ctttacgaac 420
   tgctcctcac acccagaagt ggcacaataa ttcttgggga attattactt ttttttttct 480
   ctctnttnnc gnnngnnnng gnnngnccag gaattaccac nttggaagac ctggccngaa 540
   tttattatan aggggagccg attntttttc ctaacacaaa gcgggtca 588

<210> 263
<211> 730
<212> DNA
<213> Homo sapien

<220>
<221> misc_feature
<222> (1)...(730)
<223> n = A,T,C or G

<400> 263
tttttttttt tttggcctga gcaactgaaa ttatgaaatt tccatatact caaaagagta 60
agactgcaaa aagattaaat gtaaaagttg tcttgtatac agtaatgttt aagataccta 120
ttanatttat aaatggaaaa ttagggcatt tggatataca agttgaaaat tcaggagtga 180
ggttgggctg gctgggtata tactgaaaac tgtcagtaca cagatgacat ctaaaaccac 240
aaatctggtt ttattttagc agtgatatgt gtcactccca caaaagcctt cccaattggc 300
ctcagcatac acaacaagtc acctccccac agccctctac acataaacaa attccttagt 360
ttagttcagg aggaaatgcg cccttttcct tccgctctag gtgaccgcaa ggcccagttc 420
tcgtcaccaa gatgttaagg gaagtctgcc aaagaggcat ctgaaaggaa ataaggggaa 480
tgggagtgac cacaaaggaa agccaaggan aaactttgga gaccgtttct aganccctgg 540
catttcacaa caaaactcng gaacaaacct tgtctcatca atcatttaag cccttcgttt 600
ggannagact ttctgaactg ggcgctgaac ataancctca ttgaatgtct tcacagtctc 660
ccagctgaag gcacaccttg ggccagaagg ggaatcttcc aggtcctcaa nacagggctc 720
gccctttgnc 730

<210> 264
<211> 715
<212> DNA
<213> Homo sapien

<220>
<221> misc_feature

<222> (1)...(715)
<223> n = A,T,C or G

<400> 264
   tttttttttt tttggccagt atgatagtct ctaccactat attgaagctc ttaggtcatt 60
   tacacttaat gtggttatag atgctgttga gcttacttct accaccttgc tatttctccc 120
   gtctcttttt tgttcctttt ctcttctttt cctcccttat tttataattg aattttttag 180
   gattctattt tatatagatt tatcagctat aacactttgt attcttttgt tttgtggttc 240
   ttctgtcatt tcaatgtgca tcttaaactc atcacaatct attttcaaat aatatcatat 300
   aaccttacat ataatgtaag aatctaccac catatatttc catttctccc ttccatccta 360
   tgtntgtcat attttttcct ttatatatgt tttaaagaca taatagtata tgggaggttt 420
   ttgcttaaaa tgtgatcaat attccttcaa ngaaacgtaa aaattcaaaa taaatntctg 480
   tttattctca aatnnaccta atatttccta ccatntctna tacntttcaa gaatctgaag 540
   gcattggttt tttccggctt aagaacctcc tctaaagcac tctaagcaga attaagtctt 600
   ctgggagagg aattctccca agcttgggcc ttnanntgta ctccntnang gttaaanttt 660
   ggccgggaaa tagaaattcc aagttaacag gntanttttt ntttttnttn tcncc 715

<210> 265
<211> 152
<212> DNA
<213> Homo sapien

<400> 265
tttttttttt tttcccaaca caaagcacca ttatctttcc tcacaatttt caacatagtt 60
tgattcccat gaagaggtta tgatttctaa agaaaacatg gctactatac tatcaatcag 120
ggttaaatct tttttttttg agacggagtt ta 152

<210> 266
<211> 193
<212> DNA
<213> Homo sapien

<220>
<221> misc_feature
<222> (1)...(193)
<223> n = A,T,C or G

<400> 266
taaactccgt ccccttctta atcaatatgg aggctaccca ctccacatta ccttcttttc 60
aagggactgt ttccgtaact gttgtgggta ttcacgacca ggcttctaaa cctcttaaaa 120
ctccccaatt ctggtgccaa cttggacaac atgctttttt tttttttttt tttttttttn 180
gagacggagt tta 193

<210> 267
<211> 460
<212> DNA
<213> Homo sapien

<400> 267
tgttgcgatc ccttaagcat gggtgctatt aaaaaaatgg tggagaagaa aatacctgga 60
atttacgtct tatctttaga gattgggaag accctgatgg aggacgtgga gaacagcttc 120
ttcttgaatg tcaattccca agtaacaaca gtgtgtcagg cacttgctaa ggatcctaaa 180
ttgcagcaag gctacaatgc tatgggattc tcccagggag gccaatttct gagggcagtg 240
gctcagagat gcccttcacc tcccatgatc aatctgatct cggttggggg acaacatcaa 300
ggtgtttttg gactccctcg atgcccagga gagagctctc acatctgtga cttcatccga 360
aaaacactga atgctggggc gtactccaaa gttgttcagg aacgcctcgt gcaagccgaa 420
tactggcatg acccataaaa ggaggatgtg gatcgcaaca 460

<210> 268
<211> 533
<212> DNA
<213> Homo sapien

<220>
<221> misc_feature
<222> (1)...(533)
<223> n = A,T,C or G

<400> 268
tgttgcgatc cgttgataga atagcgacgt ggtaatgagt gcatggcacg cctccgactt 60
accttcgccc gtggggaccc cgagtacgtc tacggcgtcg tcacttagag taccctctgg 120
acgcccgggc gcgttcgatt taccggaagc gcgagctgca gtgggcttgc gcccccggcc 180
aaattctttg gggggtttaa ggccgcgggg aatttgaggt atctctatca gtatgtagcc 240
aagttggaac agtcgccatt cccgaaatcg ctttctttga atccgcaccg cctccagcat 300
tgcctcattc atcaacctga aggcacgcat aagtgacggt tgtgtcttca gcagctccac 360
tccataacta gcgcgctcga cctcgtcttc gtacgcgcca ggtccgtgcg tgcgaattcc 420
caactccggt gagttgcgca tttcaagttn cgaaactgtt cgcctccacn atttggcatg 480
ttcacgcatg acacggaata aactcgtcca gtaccgggaa tgggatcgca aca 533

<210> 269
<211> 50
<212> DNA
<213> Homo sapien

<400> 269
tttttttttt ttcgcctgaa ttagctacag atcctcctca caagcggtca 50

<210> 270
<211> 519
<212> DNA
<213> Homo sapien

<400> 270
tgttgcgatc caaataaccc accagcttct tgcacacttc gcagaagcca ccgtcctttg 60
gctgagtcac gtgaacggtc agtgcaagca gccgcgtgcc agagcagagg tgcagcatgc 120
tgcacaccag ctcagggctg acctcctcca gcaggatgga caggatggag ctgccgtacg 180
tgtccaccac ctcctggcac tcttccgaca gggacttcgg cagcttcgag cacattttgt 240
caaaagcgtc gagtatttct ttctcagtct tgttgttgtc aatcagcttg gtcacctcct 300
tcaccaggaa ttcacacacc tcacagtaaa catcagactt tgctgggacc tcgtgcttct 360
taatgggctc caccagttcc agggcaggga tgacattctt ggaggccact ttggcgggga 420
ccagagtctg catgggcatc tctttcacct catcacagaa cccaaccagc gcacagatct 480
ccttgggttg catgtgcatc atcatctggg atcgcaaca 519

<210> 271
<211> 457
<212> DNA
<213> Homo sapien

<400> 271
tttttttttt ttcgggcggc gaccggacgt gcactcctcc agtagcggct gcacgtcgtg 60
ccaatggccc gctatgagga ggtgagcgtg tccggcttcg aggagttcca ccgggccgtg 120
gaacagcaca atggcaagac cattttcgcc tactttacgg gttctaagga cgccgggggg 180
aaaagctggt gccccgactg cgtgcaggct gaaccagtcg tacgagaggg gctgaagcac 240
attagtgaag gatgtgtgtt catctactgc caagtaggag aagagcctta ttggaaagat 300
ccaaataatg acttcagaaa aaacttgaaa gtaacagcag tgcctacact acttaagtat 360
ggaacacctc aaaaactggt agaatctgag tgtcttcagg ccaacctggt ggaaatgttg 420

ttctctgaag attaagattt taggatggca atcaaga 457

<210> 272
<211> 102
<212> DNA
<213> Homo sapien

<400> 272
tttttttttt ttgggcaaca acctgaatac cttttcaagg ctctggcttg ggctcaagcc 60
cgcaggggaa atgcaactgg ccaggtcaca gggcaatcaa ga 102

<210> 273
<211> 455
<212> DNA
<213> Homo sapien

<220>
<221> misc_feature
<222> (1)...(455)
<223> n = A,T,C or G

<400> 273
tttttttttt ttggcaatca acaggtttaa gtcttcggcc gaagttaatc tcgtgttttt 60
ggcaatcaac aggtttaagt cttcggccga agttaatctc gtgtttttgg caatcaacag 120
gtttaagtct tcggccgaag ttaatctcgt gtttttggca atcaacaggt ttaagtcttc 180
ggccgaagtt aatctcgtgt ttttggcaat caacaggttt aagtcttcgg ccgaagttaa 240
tctcgtgttt ttggcaatca acaggtttaa gtcttcggcc gaagttaatc tcgtgttttt 300
ggcaatcaag aggtttaagt cttcggccga agttaatctc gtgtttttgg caatcaacag 360
gtttaagtct tcggccgaan ttaatctcgt gtttttggca atcaacaggt ttaantcttc 420
ggccgaagtt aatctcgtgt ttttggcaat caana 455

<210> 274
<211> 461
<212> DNA
<213> Homo sapien

<400> 274
tttttttttt ttggccaata cccttgatga acatcaatgt gaaaatcctc ggtaaaatac 60
tggcaaacca aatccagcag cacatcaaaa agcttatcca ccatgatcaa gtgggcttca 120
tccctgggat gcaaggctgg ttcaacataa gaaaatcaat aaatgtaatc catcacataa 180
acagaaccaa agacaaaaac cacatgatta tctcaataga tgcagaaaag gccttggaca 240
aattcaacag cccttcatgc taaacactct taataaacta gatattgatg gaatgtatct 300
caaaataata agagctattt atgacaaacc cacagccaat atcatactga atgggcaaag 360
actggaagca ttccctttga aaactggcac aagacaagga tgccctctct caccgctcct 420
attcaacata gtattggaag ttctggccag ggcaatcaag a 461

<210> 275
<211> 729
<212> DNA
<213> Homo sapien

<220>
<221> misc_feature
<222> (1)...(729)
<223> n = A,T,C or G

<400> 275
tttttttttt ttggccaaca ccaagtcttc cacgtgggag gttttattat gttttacaac 60

catgaaaaca taggaaggtg gctgttacag caaacatttc agatagacga atcggccaag 120
ctccccaaac cccaccttca cagcctcttc cacacgtctc ccanagattg ttgtccttca 180
cttgcaaatt canggatgtt ggaagtngac atttnnagtn gcnggaaccc catcagtgaa 240
ncantaagca gaantacgat gactttgana nacanctgat gaagaacacn ctacnganaa 300
ccctttctnt cgtgttanga tctcnngtcc ntcactaatg cggccccctg cnggtccacc 360
atttgggaga actccccccn cgttggatcc ccccttgagt ntcccattct ngtcccccan 420
accngncttg ngngncantn cnncctcnca ccntgtttcc ctgnngtnaa aatnngtttt 480
nccgccnccc naattcccac ccnaatcaca gcgaanccng aaggccttcn naagtgttta 540
angcccngng gtttcctcnt ntanttgcag cctaccctcc cncttnnnnt tncgngttgg 600
tcgcgccctg gncncgcctn gttcctcttt nnggnnacaa cctngntcnn nggcncntcn 660
nnnctnttcc tnnnactagc tngcctntcc ncnccgnggn ncanngcaca ttncncnnac 720
tntgtnncc 729

<210> 276
<211> 339
<212> DNA
<213> Homo sapien

<400> 276
tgacctgaca tgtagtagat acttaataaa tatttgtgga atgaatggat gaagtggagt 60
tacagagaaa aatagaaaag tacaaattgt tgtcagtgtt ttgaaggaaa attatgatct 120
ttcccaaagt tctgacttca ttctaagaca gggttagtat ctccatacat aattttactt 180
gcttttgaaa atcaaatgag ataatctatt tagattgata atttatttag actggctata 240
aactattaag tgctagcaaa tatacatttt aatctcattt tccacctctt gtgatatagc 300
tatgtaggtg ttgactttaa tggatgtcag gtcaatccc 339

<210> 277
<211> 664
<212> DNA
<213> Homo sapien

<220>
<221> misc_feature
<222> (1)...(664)
<223> n = A,T,C or G

<400> 277
tgacctgaca tccataacaa aatctttctc cattatattc ttctagggga atttcttgaa 60
aagcatccaa aggaaacaaa tgatggtaag accgtgccaa gtggggagca gacaccaaag 120
taagaccaca gattttacat tcaacaggta gctcacagta ctttgcccga cactgtgggc 180
agaaatagcc tcctaatgta agccctggct cagtattgcc atccaaatgc gccatgctga 240
aagagggttt tgcatcctgg tcagatnaag aagcaatggt gtgctgagga aatcccatac 300
gaataagtga gcattcagaa cttgagctag caggaggagg actaagatga tgtgtgagca 360
actctttgta atggctttca tctaaaataa catggtacgt gccaccagtt tcacgagcaa 420
gtacagtgca aacgcgaact tctgcagaca atccaataac agatactcta attttagctg 480
cctttagggt cttgattaaa tcataaatat tagatggatc gcaagttgta aggntgctaa 540
aagatgatta gtacttctcg acttgtatgt ccaggcatgt tgttttaaan tctgccttag 600
nccctgctta ggggaatttt taaagaagat ggctctccat gttcanggtc aatcacnaat 660
tgcc 664

<210> 278
<211> 452
<212> DNA
<213> Homo sapien

<220>
<221> misc_feature
<222> (1)...(452)

<223> n = A,T,C or G

<400> 278
tgacctgaca ttgaggaaga gcacacacct ctgaaattcc ttaggttcag aagggcattt 60
gacacagagt gggcctctga taattcatga aatgcattct gaagtcatcc agaatggagg 120
ctgcaatctg ctgtgctttg ggggttgcct cactgtgctc ctggatatca cacaaaagct 180
gcaatccttc ttcttcaact aacattttgc agtatttgct gggattttta ctgcagacat 240
gatacatagc ccatagtgcc cagagctgaa cctctggttg agagaagttg ccaaggagcg 300
ggaaaaatgt cttgaaagat ctataggtca ccaatgctgt catcttacaa cttgaacttg 360
gccaattctg tatggttgca tgcagatctt ggagaagagt acgcctctgg aagtcacggg 420
atatccaaan ctgtctgtca gatgtcaggt ca 452

<210> 279
<211> 274
<212> DNA
<213> Homo sapien

<400> 279
tttttttttt ttcggcaagg caaatttact tctgcaaaag ggtgctgctt gcacttttgg 60
ccactgcgag agcacaccaa acaaagtagg gaagggtttt ttatccctaa cgcggttatt 120
ccctggttct gtgtcgtgtc cccattggct ggagtcagac tgcacaatct acactgaccc 180
aactggctac tgtttaaaat tgaatatgaa taattaggta ggaaggggga ggctgtttgt 240
tacggtacaa gacgtgtttg ggcatgtcag gtca 274

<210> 280
<211> 272
<212> DNA
<213> Homo sapien

<400> 280
tacctgacat ggagaaataa cttgtagtat tttgcgtgca atggaatact atatgagggt 60
gaaaatgaat gaactagcaa tgcgtgtatc aacatgaata aatccccaaa acataataat 120
gttgaatgga aaaggtgagt ttcagaagga tatatatgcc ctctaaatcc atttatgtaa 180
acctttaaaa aactacatta tttatggtca taagtccatc cagaaaatat ttaaaaacct 240
acatgggatt gataactact gatgtcaggt ca 272

<210> 281
<211> 431
<212> DNA
<213> Homo sapien

<220>
<221> misc_feature
<222> (1)...(431)
<223> n = A,T,C or G

<400> 281
tttttttttt ttggccaata gcatgattta aacattggaa aaagtcaaat gagcaatgcg 60
aatttttatg ttctcttgaa taatcaaaag agtaggcaac attggttcct cattcttgaa 120
tagcattaat cagaaaatat tgcatagcct ctagcctcct tagagtaggt gtgctctctc 180
aaatatatca tagtcccaca gtttatttca tgtatatttt ctgcctgaat cacatagaca 240
tttgaatttg caacgcctga tgtaaatata taaattctta ccaatcagaa acatagcaag 300
aaattcaggg acttggtcat yatcagggta tgacagcana tccctgtara aacactgata 360
cacactcaca cacgtatgca acgtggagat gtcgcyttww kkktwywcwm rmrycrwcgn 420
aatcacttan n 431

<210> 282
<211> 98

<212> DNA
<213> Homo sapien

<400> 282
attcgattcg atgcttgagc ccaggagttc aagactgcag tgagccactg cacttcaggc 60
tggacaacag agcgagtccc tgtgccaaaa aaaaaaaa 98

<210> 283
<211> 764
<212> DNA
<213> Homo sapien

<220>
<221> misc_feature
<222> (1)...(764)
<223> n = A,T,C or G

<400> 283
tttttttttt ttcgcaagca cgtgcacttt attgaatgac actgtagaca ggtgtgtggg 60
tataaactgc tgtatctagg ggcaggacca agggggcagg ggcaacagcc ccagcgtgca 120
gggccascat tgcacagtgg astgcaaagg ttgcaggcta tgggcggcta ctavtaaccc 180
cgtttttcct gtattatctg taacataata tggtagactg tcacagagcc gaatwccart 240
hacasgatga atccaawggt caygaggatg cccasaatca gggcccasat sttcaggcac 300
ttggcggtgg gggcatasgc ctgkgccccg gtcacgtcsc caaccwtcty cctgtcccta 360
cmcttgawtc cncnccttnn nntnccntna tntgcccgcc cncctcctng ngtcaaccng 420
natctgcact anctccctcn ccccttntgg antctcntcc ttcaantaan nttatccttn 480
acncccccct cncctttccc ctnccncccn tnatcccngn nccnctatca ntcntnccct 540
cnctntnctn cnnatcgttc cncctnntaa ctacnctttn nacnanncct cactnatncc 600
ngnnanttct ttccttccct cccnacgcnn tgcgtgcgcc cgtctngcct nnnctncgna 660
cccnnacttt atttaccttt ncaccctagc nctctacttn acccanccnc tcctacctcc 720
nggnccaccc nnccctnatc nctnnctctn tcnnctcntt cccc 764

<210> 284
<211> 157
<212> DNA
<213> Homo sapien

<400> 284
caagtgtagg cacagtgatg aaagcctgga gcaaacacaa tctgtgggta attaacgttt 60
atttctcccc ttccaggaac gtcttgcatg gatgatcaaa gatcagctcc tggtcaacat 120
aaataagcta gtttaagata cgttccccta cacttga 157

<210> 285
<211> 150
<212> DNA
<213> Homo sapien

<400> 285
attcgattgt actcagacaa caatatgcta agtggaagaa gtcagtcaca aaagaccaca 60
tactgtatga cttcatttac attaagtgtc cagaataggc aaatccgtag agacagaaag 120
tagatgagca gctgcctagg tctgagtaca 150

<210> 286
<211> 219
<212> DNA
<213> Homo sapien

<400> 286

attcgatttt tttttttttg gccatgatga aattcttact ccctcagatt ttttgtctgg 60
ataaatgcaa gtctcaccac cagatgtgaa attacagtaa actttgaagg aatctcctga 120
gcaaccttgg ttaggatcaa tccaatattc accatctggg aagtcaggat ggctgagttg 180
caggtcttta caagttcggg ctggattggt ctgagtaca 219

<210> 287
<211> 196
<212> DNA
<213> Homo sapien

<400> 287
attcgattct tgaggctacc aggagctagg agaagaggca tggaacaaat tttccctcat 60
atccatactc agaaggaacc aaccctgctg acaccttaat ttcagcttct ggcctctaga 120
actgtgagag agtacatttc tcttggttta agccaagaga atctgtcttt tggtacttta 180
tatcatagcc tcaaga 196

<210> 288
<211> 199
<212> DNA
<213> Homo sapien

<400> 288
attcgatttc agtccagtcc cagaacccac attgtcaatt actactctgt araagattca 60
tttgttgaaa ttcattgagt aaaacattta tgatccctta atatatgcca attaccatgc 120
taggtactga agattcaagt gaccgagatg ctagcccttg ggttcaagtg atccctctcc 180
cagagtgcac tggactgaa 199

<210> 289
<211> 182
<212> DNA
<213> Homo sapien

<400> 289
attcgattct tgaggctaca aacctgtaca gtatgttact ctactgaata ctgtaggcaa 60
tagtaataca gaagcaagta tctgtatatg taaacattaa aaaggtacag tgaaacttca 120
gtattataat cttagggacc accattatat atgtggtcca tcattggcca aaaaaaaaaa 180
aa 182

<210> 290
<211> 1646
<212> DNA
<213> Homo sapien

<400> 290
ggcacgagga gaaatgtaat tccatatttt atttgaaact tattccatat tttaattgga 60
tattgagtga ttgggttatc aaacacccac aaactttaat tttgttaaat ttatatggct 120
ttgaaataga agtataagtt gctaccattt tttgataaca ttgaaagata gtattttacc 180
atctttaatc atcttggaaa atacaagtcc tgtgaacaac cactctttca cctagcagca 240
tgaggccaaa agtaaaggct ttaaattata acatatggga ttcttagtag tatgtttttt 300
tcttgaaact cagtggctct atctaacctt actatctcct cactctttct ctaagactaa 360
actctaggct cttaaaaatc tgcccacacc aatcttagaa gctctgaaaa gaatttgtct 420
ttaaatatct tttaatagta acatgtattt tatggaccaa attgacattt tcgactattt 480
tttccaaaaa agtcaggtga atttcagcac actgagttgg gaatttctta tcccagaaga 540
ccaaccaatt tcatatttat ttaagattga ttccatactc cgttttcaag gagaatccct 600
gcagtctcct taaaggtaga acaaatactt tctatttttt tttcaccatt gtgggattgg 660
actttaagag gtgactctaa aaaaacagag aacaaatatg tctcagttgt attaagcacg 720
gacccatatt atcatattca cttaaaaaaa tgatttcctg tgcacctttt ggcaacttct 780
cttttcaatg tagggaaaaa cttagtcacc ctgaaaaccc acaaaataaa taaaacttgt 840

agatgtgggc agaaggtttg ggggtggaca ttgtatgtgt ttaaattaaa ccctgtatca 900
ctgagaagct gttgtatggg tcagagaaaa tgaatgctta gaagctgttc acatcttcaa 960
gagcagaagc aaaccacatg tctcagctat attattattt attttttatg cataaagtga 1020
atcatttctt ctgtattaat ttccaaaggg ttttaccctc tatttaaatg ctttgaaaaa 1080
cagtgcattg acaatgggtt gatatttttc tttaaaagaa aaatataatt atgaaagcca 1140
agataatctg aagcctgttt tattttaaaa ctttttatgt tctgtggttg atgttgtttg 1200
tttgtttgtt tctattttgt tggtttttta ctttgttttt tgttttgttt tgttttgttt 1260
kgcatactac atgcagttct ttaaccaatg tctgtttggc taatgtaatt aaagttgtta 1320
atttatatga gtgcatttca actatgtcaa tggtttctta atatttattg tgtagaagta 1380
ctggtaattt ttttatttac aatatgttta aagagataac agtttgatat gttttcatgt 1440
gtttatagca gaagttattt atttctatgg cattccagcg gatattttgg tgtttgcgag 1500
gcatgcagtc aatattttgt acagttagtg gacagtattc agcaacgcct gatagcttct 1560
ttggccttat gttaaataaa aagacctgtt tgggatgtat tttttatttt taaaaaaaaa 1620
aaaaaaaaaa aaaaaaaaaa aaaaaa 1646

<210> 291
<211> 1851
<212> DNA
<213> Homo sapien

<400> 291
tcatcaccat tgccagcagc ggcaccgtta gtcaggtttt ctgggaatcc cacatgagta 60
cttccgtgtt cttcattctt cttcaatagc cataaatctt ctagctctgg ctggctgttt 120
tcacttcctt taagcctttg tgactcttcc tctgatgtca gctttaagtc ttgttctgga 180
ttgctgtttt cagaagagat ttttaacatc tgtttttctt tgtagtcaga aagtaactgg 240
caaattacat gatgatgact agaaacagca tactctctgg ccgtctttcc agatcttgag 300
aagatacatc aacattttgc tcaagtagag ggctgactat acttgctgat ccacaacata 360
cagcaagtat gagagcagtt cttccatatc tatccagcgc atttaaattc gcttttttct 420
tgattaaaaa tttcaccact tgctgttttt gctcatgtat accaagtagc agtggtgtga 480
ggccatgctt gttttttgat tcgatatcag caccgtataa gagcagtgct ttggccatta 540
atttatcttc attgtagaca gcatagtgta gagtggtatt tccatactca tctggaatat 600
ttggatcagt gccatgttcc agcaacatta acgcacattc atcttcctgg cattgtacgg 660
cctttgtcag agctgtcctc tttttgttgt caaggacatt aagttgacat cgtctgtcca 720
gcacgagttt tactacttct gaattcccat tggcagaggc cagatgtaga gcagtcctct 780
tttgcttgtc cctcttgttc acatccgtgt ccctgagcat gacgatgaga tcctttctgg 840
ggactttacc ccaccaggca gctctgtgga gcttgtccag atcttctcca tggacgtggt 900
acctgggatc catgaaggcg ctgtcatcgt agtctcccca agcgaccacg ttgctcttgc 960
cgctcccctg cagcagggga agcagtggca gcaccacttg cacctcttgc tcccaagcgt 1020
cttcacagag gagtcgttgt ggtctccaga agtgcccacg ttgctcttgc cgctccccct 1080
gtccatccag ggaggaagaa atgcaggaaa tgaaagatgc atgcacgatg gtatactcct 1140
cagccatcaa acttctggac agcaggtcac ttccagcaag gtggagaaag ctgtccaccc 1200
acagaggatg agatccagaa accacaatat ccattcacaa acaaacactt ttcagccaga 1260
cacaggtact gaaatcatgt catctgcggc aacatggtgg aacctaccca atcacacatc 1320
aagagatgaa gacactgcag tatatctgca caacgtaata ctcttcatcc ataacaaaat 1380
aatataattt tcctctggag ccatatggat gaactatgaa ggaagaactc cccgaagaag 1440
ccagtcgcag agaagccaca ctgaagctct gtcctcagcc atcagcgcca cggacaggar 1500
tgtgtttctt ccccagtgat gcagcctcaa gttatcccga agctgccgca gcacacggtg 1560
gctcctgaga aacaccccag ctcttccggt ctaacacagg caagtcaata aatgtgataa 1620
tcacataaac agaattaaaa gcaaagtcac ataagcatct caacagacac agaaaaggca 1680
tttgacaaaa tccagcatcc ttgtatttat tgttgcagtt ctcagaggaa atgcttctaa 1740
cttttcccca tttagtatta tgttggctgt gggcttgtca taggtggttt ttattacttt 1800
aaggtatgtc ccttctatgc ctgttttgct gagggtttta attctcgtgc c 1851

<210> 292
<211> 1851
<212> DNA
<213> Homo sapien

<400> 292
tcatcaccat tgccagcagc ggcaccgtta gtcaggtttt ctgggaatcc cacatgagta 60
cttccgtgtt cttcattctt cttcaatagc cataaatctt ctagctctgg ctggctgttt 120
tcacttcctt taagcctttg tgactcttcc tctgatgtca gctttaagtc ttgttctgga 180
ttgctgtttt cagaagagat ttttaacatc tgtttttctt tgtagtcaga aagtaactgg 240
caaattacat gatgatgact agaaacagca tactctctgg ccgtctttcc agatcttgag 300
aagatacatc aacattttgc tcaagtagag ggctgactat acttgctgat ccacaacata 360
cagcaagtat gagagcagtt cttccatatc tatccagcgc atttaaattc gcttttttct 420
tgattaaaaa tttcaccact tgctgttttt gctcatgtat accaagtagc agtggtgtga 480
ggccatgctt gttttttgat tcgatatcag caccgtataa gagcagtgct ttggccatta 540
atttatcttc attgtagaca gcatagtgta gagtggtatt tccatactca tctggaatat 600
ttggatcagt gccatgttcc agcaacatta acgcacattc atcttcctgg cattgtacgg 660
cctttgtcag agctgtcctc tttttgttgt caaggacatt aagttgacat cgtctgtcca 720
gcacgagttt tactacttct gaattcccat tggcagaggc cagatgtaga gcagtcctct 780
tttgcttgtc cctcttgttc acatccgtgt ccctgagcat gacgatgaga tcctttctgg 840
ggactttacc ccaccaggca gctctgtgga gcttgtccag atcttctcca tggacgtggt 900
acctgggatc catgaaggcg ctgtcatcgt agtctcccca agcgaccacg ttgctcttgc 960
cgctcccctg cagcagggga agcagtggca gcaccacttg cacctcttgc tcccaagcgt 1020
cttcacagag gagtcgttgt ggtctccaga agtgcccacg ttgctcttgc cgctccccct 1080
gtccatccag ggaggaagaa atgcaggaaa tgaaagatgc atgcacgatg gtatactcct 1140
cagccatcaa acttctggac agcaggtcac ttccagcaag gtggagaaag ctgtccaccc 1200
acagaggatg agatccagaa accacaatat ccattcacaa acaaacactt ttcagccaga 1260
cacaggtact gaaatcatgt catctgcggc aacatggtgg aacctaccca atcacacatc 1320
aagagatgaa gacactgcag tatatctgca caacgtaata ctcttcatcc ataacaaaat 1380
aatataattt tcctctggag ccatatggat gaactatgaa ggaagaactc cccgaagaag 1440
ccagtcgcag agaagccaca ctgaagctct gtcctcagcc atcagcgcca cggacaggar 1500
tgtgtttctt ccccagtgat gcagcctcaa gttatcccga agctgccgca gcacacggtg 1560
gctcctgaga aacaccccag ctcttccggt ctaacacagg caagtcaata aatgtgataa 1620
tcacataaac agaattaaaa gcaaagtcac ataagcatct caacagacac agaaaaggca 1680
tttgacaaaa tccagcatcc ttgtatttat tgttgcagtt ctcagaggaa atgcttctaa 1740
cttttcccca tttagtatta tgttggctgt gggcttgtca taggtggttt ttattacttt 1800
aaggtatgtc ccttctatgc ctgttttgct gagggtttta attctcgtgc c 1851

<210> 293
<211> 668
<212> DNA
<213> Homo sapien

<400> 293
cttgagcttc caaataygga agactggccc ttacacasgt caatgttaaa atgaatgcat 60
ttcagtattt tgaagataaa attrgtagat ctataccttg ttttttgatt cgatatcagc 120
accrtataag agcagtgctt tggccattaa tttatctttc attrtagaca gcrtagtgya 180
gagtggtatt tccatactca tctggaatat ttggatcagt gccatgttcc agcaacatta 240
acgcacattc atcttcctgg cattgtacgg cctgtcagta ttagacccaa aaacaaatta 300
catatcttag gaattcaaaa taacattcca cagctttcac caactagtta tatttaaagg 360
agaaaactca tttttatgcc atgtattgaa atcaaaccca cctcatgctg atatagttgg 420
ctactgcata cctttatcag agctgtcctc tttttgttgt caaggacatt aagttgacat 480
cgtctgtcca gcaggagttt tactacttct gaattcccat tggcagaggc cagatgtaga 540
gcagtcctat gagagtgaga agacttttta ggaaattgta gtgcactagc tacagccata 600
gcaatgattc atgtaactgc aaacactgaa tagcctgcta ttactctgcc ttcaaaaaaa 660
aaaaaaaa 668

<210> 294
<211> 1512
<212> DNA
<213> Homo sapien

<400> 294

gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagt 600
gccttcatgg agcccaggta ccacgtccgt ggagaagatc tggacaagct ccacagagct 660
gcctggtggg gtaaagtccc cagaaaggat ctcatcgtca tgctcaggga cactgacgtg 720
aacaagaagg acaagcaaaa gaggactgct ctacatctgg cctctgccaa tgggaattca 780
gaagtagtaa aactcstgct ggacagacga tgtcaactta atgtccttga caacaaaaag 840
aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca aggtatagat ctactaattt tatcttcaaa atactgaaat gcattcattt 1080
taacattgac gtgtgtaagg gccagtcttc cgtatttgga agctcaagca taacttgaat 1140
gaaaatattt tgaaatgacc taattatctm agactttatt ttaaatattg ttattttcaa 1200
agaagcatta gagggtacag tttttttttt ttaaatgcac ttctggtaaa tacttttgtt 1260
gaaaacactg aatttgtaaa aggtaatact tactattttt caatttttcc ctcctaggat 1320
ttttttcccc taatgaatgt aagatggcaa aatttgccct gaaataggtt ttacatgaaa 1380
actccaagaa aagttaaaca tgtttcagtg aatagagatc ctgctccttt ggcaagttcc 1440
taaaaaacag taatagatac gaggtgatgc gcctgtcagt ggcaaggttt aagatatttc 1500
tgatctcgtg cc 1512

<210> 295
<211> 1853
<212> DNA
<213> Homo sapien

<400> 295
gggtcgccca gggggsgcgt gggctttcct cgggtgggtg tgggttttcc ctgggtgggg 60
tgggctgggc trgaatcccc tgctggggtt ggcaggtttt ggctgggatt gacttttytc 120
ttcaaacaga ttggaaaccc ggagttacct gctagttggt gaaactggtt ggtagacgcg 180
atctgttggc tactactggc ttctcctggc tgttaaaagc agatggtggt tgaggttgat 240
tccatgccgg ctgcttcttc tgtgaagaag ccatttggtc tcaggagcaa gatgggcaag 300
tggtgctgcc gttgcttccc ctgctgcagg gagagcggca agagcaacgt gggcacttct 360
ggagaccacg acgactctgc tatgaagaca ctcaggagca agatgggcaa gtggtgccgc 420
cactgcttcc cctgctgcag ggggagtggc aagagcaacg tgggcgcttc tggagaccac 480
gacgaytctg ctatgaagac actcaggaac aagatgggca agtggtgctg ccactgcttc 540
ccctgctgca gggggagcrg caagagcaag gtgggcgctt ggggagacta cgatgacagy 600
gccttcatgg akcccaggta ccacgtccrt ggagaagatc tggacaagct ccacagagct 660
gcctggtggg gtaaagtccc cagaaaggat ctcatcgtca tgctcaggga cackgaygtg 720
aacaagargg acaagcaaaa gaggactgct ctacatctgg cctctgccaa tgggaattca 780
gaagtagtaa aactcstgct ggacagacga tgtcaactta atgtccttga caacaaaaag 840
aggacagctc tgayaaaggc cgtacaatgc caggaagatg aatgtgcgtt aatgttgctg 900
gaacatggca ctgatccaaa tattccagat gagtatggaa ataccactct rcactaygct 960
rtctayaatg aagataaatt aatggccaaa gcactgctct tatayggtgc tgatatcgaa 1020
tcaaaaaaca agcatggcct cacaccactg ytacttggtr tacatgagca aaaacagcaa 1080
gtsgtgaaat ttttaatyaa gaaaaaagcg aatttaaaat gcrctggata gatatggaag 1140
ractgctctc atacttgctg tatgttgtgg atcagcaagt atagtcagcc ytctacttga 1200
gcaaaatrtt gatgtatctt ctcaagatct ggaaagacgg ccagagagta tgctgtttct 1260
agtcatcatc atgtaatttg ccagttactt tctgactaca aagaaaaaca gatgttaaaa 1320
atctcttctg aaaacagcaa tccagaacaa gacttaaagc tgacatcaga ggaagagtca 1380
caaaggctta aaggaagtga aaacagccag ccagaggcat ggaaactttt aaatttaaac 1440
ttttggttta atgttttttt tttttgcctt aataatatta gatagtccca aatgaaatwa 1500

cctatgagac taggctttga gaatcaatag attctttttt taagaatctt ttggctagga 1560
gcggtgtctc acgcctgtaa ttccagcacc ttgagaggct gaggtgggca gatcacgaga 1620
tcaggagatc gagaccatcc tggctaacac ggtgaaaccc catctctact aaaaatacaa 1680
aaacttagct gggtgtggtg gcgggtgcct gtagtcccag ctactcagga rgctgaggca 1740
ggagaatggc atgaacccgg gaggtggagg ttgcagtgag ccgagatccg ccactacact 1800
ccagcctggg tgacagagca agactctgtc tcaaaaaaaa aaaaaaaaaa aaa 1853

<210> 296
<211> 2184
<212> DNA
<213> Homo sapien

<400> 296
ggcacgagaa ttaaaaccct cagcaaaaca ggcatagaag ggacatacct taaagtaata 60
aaaaccacct atgacaagcc cacagccaac ataatactaa atggggaaaa gttagaagca 120
tttcctctga gaactgcaac aataaataca aggatgctgg attttgtcaa atgccttttc 180
tgtgtctgtt gagatgctta tgtgactttg cttttaattc tgtttatgtg attatcacat 240
ttattgactt gcctgtgtta gaccggaaga gctggggtgt ttctcaggag ccaccgtgtg 300
ctgcggcagc ttcgggataa cttgaggctg catcactggg gaagaaacac aytcctgtcc 360
gtggcgctga tggctgagga cagagcttca gtgtggcttc tctgcgactg gcttcttcgg 420
ggagttcttc cttcatagtt catccatatg gctccagagg aaaattatat tattttgtta 480
tggatgaaga gtattacgtt gtgcagatat actgcagtgt cttcatctct tgatgtgtga 540
ttgggtaggt tccaccatgt tgccgcagat gacatgattt cagtacctgt gtctggctga 600
aaagtgtttg tttgtgaatg gatattgtgg tttctggatc tcatcctctg tgggtggaca 660
gctttctcca ccttgctgga agtgacctgc tgtccagaag tttgatggct gaggagtata 720
ccatcgtgca tgcatctttc atttcctgca tttcttcctc cctggatgga cagggggagc 780
ggcaagagca acgtgggcac ttctggagac cacaacgact cctctgtgaa gacgcttggg 840
agcaagaggt gcaagtggtg ctgccactgc ttcccctgct gcaggggagc ggcaagagca 900
acgtggtcgc ttggggagac tacgatgaca gcgccttcat ggatcccagg taccacgtcc 960
atggagaaga tctggacaag ctccacagag ctgcctggtg gggtaaagtc cccagaaagg 1020
atctcatcgt catgctcagg gacacggatg tgaacaagag ggacaagcaa aagaggactg 1080
ctctacatct ggcctctgcc aatgggaatt cagaagtagt aaaactcgtg ctggacagac 1140
gatgtcaact taatgtcctt gacaacaaaa agaggacagc tctgacaaag gccgtacaat 1200
gccaggaaga tgaatgtgcg ttaatgttgc tggaacatgg cactgatcca aatattccag 1260
atgagtatgg aaataccact ctacactatg ctgtctacaa tgaagataaa ttaatggcca 1320
aagcactgct cttatacggt gctgatatcg aatcaaaaaa caagcatggc ctcacaccac 1380
tgctacttgg tatacatgag caaaaacagc aagtggtgaa atttttaatc aagaaaaaag 1440
cgaatttaaa tgcgctggat agatatggaa gaactgctct catacttgct gtatgttgtg 1500
gatcagcaag tatagtcagc cctctacttg agcaaaatgt tgatgtatct tctcaagatc 1560
tggaaagacg gccagagagt atgctgtttc tagtcatcat catgtaattt gccagttact 1620
ttctgactac aaagaaaaac agatgttaaa aatctcttct gaaaacagca atccagaaca 1680
agacttaaag ctgacatcag aggaagagtc acaaaggctt aaaggaagtg aaaacagcca 1740
gccagaggca tggaaacttt taaatttaaa cttttggttt aatgtttttt ttttttgcct 1800
taataatatt agatagtccc aaatgaaatw acctatgaga ctaggctttg agaatcaata 1860
gattcttttt ttaagaatct tttggctagg agcggtgtct cacgcctgta attccagcac 1920
cttgagaggc tgaggtgggc agatcacgag atcaggagat cgagaccatc ctggctaaca 1980
cggtgaaacc ccatctctac taaaaataca aaaacttagc tgggtgtggt ggcgggtgcc 2040
tgtagtccca gctactcagg argctgaggc aggagaatgg catgaacccg ggaggtggag 2100
gttgcagtga gccgagatcc gccactacac tccagcctgg gtgacagagc aagactctgt 2160
ctcaaaaaaa aaaaaaaaaa aaaa 2184

<210> 297
<211> 1855
<212> DNA
<213> Homo sapien

<220>
<221> misc_feature

<222> (1)...(1855)
<223> n = A,T,C or G

<400> 297
tgcacgcatc ggccagtgtc tgtgccacgt acactgacgc cccctgagat gtgcacgccg 60
cacgcgcacg ttgcacgcgc ggcagcggct tggctggctt gtaacggctt gcacgcgcac 120
gccgcccccg cataaccgtc agactggcct gtaacggctt gcaggcgcac gccgcacgcg 180
cgtaacggct tggctgccct gtaacggctt gcacgtgcat gctgcacgcg cgttaacggc 240
ttggctggca tgtagccgct tggcttggct ttgcattytt tgctkggctk ggcgttgkty 300
tcttggattg acgcttcctc cttggatkga cgtttcctcc ttggatkgac gtttcytyty 360
tcgcgttcct ttgctggact tgacctttty tctgctgggt ttggcattcc tttggggtgg 420
gctgggtgtt ttctccgggg gggktkgccc ttcctggggt gggcgtgggk cgcccccagg 480
gggcgtgggc tttccccggg tgggtgtggg ttttcctggg gtggggtggg ctgtgctggg 540
atccccctgc tggggttggc agggattgac ttttttcttc aaacagattg gaaacccgga 600
gtaacntgct agttggtgaa actggttggt agacgcgatc tgctggtact actgtttctc 660
ctggctgtta aaagcagatg gtggctgagg ttgattcaat gccggctgct tcttctgtga 720
agaagccatt tggtctcagg agcaagatgg gcaagtggtg cgccactgct tcccctgctg 780
cagggggagc ggcaagagca acgtgggcac ttctggagac cacaacgact cctctgtgaa 840
gacgcttggg agcaagaggt gcaagtggtg ctgcccactg cttcccctgc tgcaggggag 900
cggcaagagc aacgtggkcg cttggggaga ctacgatgac agcgccttca tggakcccag 960
gtaccacgtc crtggagaag atctggacaa gctccacaga gctgcctggt ggggtaaagt 1020
ccccagaaag gatctcatcg tcatgctcag ggacactgay gtgaacaaga rggacaagca 1080
aaagaggact gctctacatc tggcctctgc caatgggaat tcagaagtag taaaactcgt 1140
gctggacaga cgatgtcaac ttaatgtcct tgacaacaaa aagaggacag ctctgacaaa 1200
ggccgtacaa tgccaggaag atgaatgtgc gttaatgttg ctggaacatg gcactgatcc 1260
aaatattcca gatgagtatg gaaataccac tctacactat gctgtctaca atgaagataa 1320
attaatggcc aaagcactgc tcttatacgg tgctgatatc gaatcaaaaa acaaggtata 1380
gatctactaa ttttatcttc aaaatactga aatgcattca ttttaacatt gacgtgtgta 1440
agggccagtc ttccgtattt ggaagctcaa gcataacttg aatgaaaata ttttgaaatg 1500
acctaattat ctaagacttt attttaaata ttgttatttt caaagaagca ttagagggta 1560
cagttttttt tttttaaatg cacttctggt aaatactttt gttgaaaaca ctgaatttgt 1620
aaaaggtaat acttactatt tttcaatttt tccctcctag gatttttttc ccctaatgaa 1680
tgtaagatgg caaaatttgc cctgaaatag gttttacatg aaaactccaa gaaaagttaa 1740
acatgtttca gtgaatagag atcctgctcc tttggcaagt tcctaaaaaa cagtaataga 1800
tacgaggtga tgcgcctgtc agtggcaagg tttaagatat ttctgatctc gtgcc 1855

<210> 298
<211> 1059
<212> DNA
<213> Homo sapien

<400> 298
gcaacgtggg cacttctgga gaccacaacg actcctctgt gaagacgctt gggagcaaga 60
ggtgcaagtg gtgctgccca ctgcttcccc tgctgcaggg gagcggcaag agcaacgtgg 120
gcgcttgrgg agactmcgat gacagygcct tcatggagcc caggtaccac gtccgtggag 180
aagatctgga caagctccac agagctgccc tggtggggta aagtccccag aaaggatctc 240
atcgtcatgc tcagggacac tgaygtgaac aagarggaca agcaaaagag gactgctcta 300
catctggcct ctgccaatgg gaattcagaa gtagtaaaac tcstgctgga cagacgatgt 360
caacttaatg tccttgacaa caaaaagagg acagctctga yaaaggccgt acaatgccag 420
gaagatgaat gtgcgttaat gttgctggaa catggcactg atccaaatat tccagatgag 480
tatggaaata ccactctrca ctaygctrtc tayaatgaag ataaattaat ggccaaagca 540
ctgctcttat ayggtgctga tatcgaatca aaaaacaagg tatagatcta ctaattttat 600
cttcaaaata ctgaaatgca ttcattttaa cattgacgtg tgtaagggcc agtcttccgt 660
atttggaagc tcaagcataa cttgaatgaa aatattttga aatgacctaa ttatctaaga 720
ctttatttta aatattgtta ttttcaaaga agcattagag ggtacagttt ttttttttta 780
aatgcacttc tggtaaatac ttttgttgaa aacactgaat ttgtaaaagg taatacttac 840
tatttttcaa tttttccctc ctaggatttt tttcccctaa tgaatgtaag atggcaaaat 900
ttgccctgaa ataggtttta catgaaaact ccaagaaaag ttaaacatgt ttcagtgaat 960

agagatcctg ctcctttggc aagttcctaa aaaacagtaa tagatacgag gtgatgcgcc 1020
tgtcagtggc aaggtttaag atatttctga tctcgtgcc 1059

<210> 299
<211> 329
<212> PRT
<213> Homo sapien

<400> 299
Met Asp Ile Val Val Ser Gly Ser His Pro Leu Trp Val Asp Ser Phe
1               5                   10                  15
Leu His Leu Ala Gly Ser Asp Leu Leu Ser Arg Ser Leu Met Ala Glu
            20                  25                  30
Glu Tyr Thr Ile Val His Ala Ser Phe Ile Ser Cys Ile Ser Ser Ser
        35                  40                  45
Leu Asp Gly Gln Gly Glu Arg Gln Glu Gln Arg Gly His Phe Trp Arg
    50                  55                  60
Pro Gln Arg Leu Leu Cys Glu Asp Ala Trp Glu Gln Glu Val Gln Val
65                  70                  75                  80
Val Leu Pro Leu Leu Pro Leu Leu Gln Gly Ser Gly Lys Ser Asn Val
                85                  90                  95
Val Ala Trp Gly Asp Tyr Asp Asp Ser Ala Phe Met Asp Pro Arg Tyr
            100                 105                 110
His Val His Gly Glu Asp Leu Asp Lys Leu His Arg Ala Ala Trp Trp
        115                 120                 125
Gly Lys Val Pro Arg Lys Asp Leu Ile Val Met Leu Arg Asp Thr Asp
    130                 135                 140
Val Asn Lys Arg Asp Lys Gln Lys Arg Thr Ala Leu His Leu Ala Ser
145                 150                 155                 160
Ala Asn Gly Asn Ser Glu Val Val Lys Leu Val Leu Asp Arg Arg Cys
                165                 170                 175
Gln Leu Asn Val Leu Asp Asn Lys Lys Arg Thr Ala Leu Thr Lys Ala
            180                 185                 190
Val Gln Cys Gln Glu Asp Glu Cys Ala Leu Met Leu Leu Glu His Gly
        195                 200                 205
Thr Asp Pro Asn Ile Pro Asp Glu Tyr Gly Asn Thr Thr Leu His Tyr
    210                 215                 220
Ala Val Tyr Asn Glu Asp Lys Leu Met Ala Lys Ala Leu Leu Leu Tyr
225                 230                 235                 240
Gly Ala Asp Ile Glu Ser Lys Asn Lys His Gly Leu Thr Pro Leu Leu
                245                 250                 255
Leu Gly Ile His Glu Gln Lys Gln Gln Val Val Lys Phe Leu Ile Lys
            260                 265                 270
Lys Lys Ala Asn Leu Asn Ala Leu Asp Arg Tyr Gly Arg Thr Ala Leu
        275                 280                 285
Ile Leu Ala Val Cys Cys Gly Ser Ala Ser Ile Val Ser Pro Leu Leu
    290                 295                 300
Glu Gln Asn Val Asp Val Ser Ser Gln Asp Leu Glu Arg Arg Pro Glu
305                 310                 315                 320
Ser Met Leu Phe Leu Val Ile Ile Met
                325

<210> 300
<211> 148
<212> PRT
<213> Homo sapien

<220>

<221> VARIANT
<222> (1)...(148)
<223> Xaa = Any Amino Acid

<400> 300
Met Thr Xaa Pro Ser Trp Ser Pro Gly Thr Thr Ser Val Glu Lys Ile
1               5                   10                  15
Trp Thr Ser Ser Thr Glu Leu Pro Trp Trp Gly Lys Val Pro Arg Lys
            20                  25                  30
Asp Leu Ile Val Met Leu Arg Asp Thr Asp Val Asn Lys Xaa Asp Lys
        35                  40                  45
Gln Lys Arg Thr Ala Leu His Leu Ala Ser Ala Asn Gly Asn Ser Glu
    50                  55                  60
Val Val Lys Leu Xaa Leu Asp Arg Arg Cys Gln Leu Asn Val Leu Asp
65                  70                  75                  80
Asn Lys Lys Arg Thr Ala Leu Xaa Lys Ala Val Gln Cys Gln Glu Asp
                85                  90                  95
Glu Cys Ala Leu Met Leu Leu Glu His Gly Thr Asp Pro Asn Ile Pro
            100                 105                 110
Asp Glu Tyr Gly Asn Thr Thr Leu His Tyr Ala Xaa Tyr Asn Glu Asp
        115                 120                 125
Lys Leu Met Ala Lys Ala Leu Leu Leu Tyr Gly Ala Asp Ile Glu Ser
    130                 135                 140
Lys Asn Lys Val
145

<210> 301
<211> 1155
<212> DNA
<213> Homo sapien

<400> 301
atggtggttg aggttgattc catgccggct gcctcttctg tgaagaagcc atttggtctc      60
aggagcaaga tgggcaagtg gtgctgccgt tgcttcccct gctgcaggga gagcggcaag     120
agcaacgtgg gcacttctgg agaccacgac gactctgcta tgaagacact caggagcaag     180
atgggcaagt ggtgccgcca ctgcttcccc tgctgcaggg ggagtggcaa gagcaacgtg     240
ggcgcttctg gagaccacga cgactctgct atgaagacac tcaggaacaa gatgggcaag     300
tggtgctgcc actgcttccc ctgctgcagg gggagcggca agagcaaggt gggcgcttgg     360
ggagactacg atgacagtgc cttcatggag cccaggtacc acgtccgtgg agaagatctg     420
gacaagctcc acagagctgc ctggtggggt aaagtcccca gaaaggatct catcgtcatg     480
ctcagggaca ctgacgtgaa caagaaggac aagcaaaaga ggactgctct acatctggcc     540
tctgccaatg ggaattcaga agtagtaaaa ctcctgctgg acagacgatg tcaacttaat     600
gtccttgaca acaaaaagag gacagctctg ataaaggccg tacaatgcca ggaagatgaa     660
tgtgcgttaa tgttgctgga acatggcact gatccaaata ttccagatga gtatggaaat     720
accactctgc actacgctat ctataatgaa gataaattaa tggccaaagc actgctctta     780
tatggtgctg atatcgaatc aaaaaacaag catggcctca caccactgtt acttggtgta     840
catgagcaaa aacagcaagt cgtgaaattt ttaatcaaga aaaaagcgaa tttaaatgca     900
ctggatagat atggaaggac tgctctcata cttgctgtat gttgtggatc agcaagtata     960
gtcagccttc tacttgagca aaatattgat gtatcttctc aagatctatc tggacagacg    1020
gccagagagt atgctgtttc tagtcatcat catgtaattt gccagttact ttctgactac    1080
aaagaaaaac agatgctaaa aatctcttct gaaaacagca atccagaaaa tgtctcaaga    1140
accagaaata aataa                                                     1155

<210> 302
<211> 2000
<212> DNA
<213> Homo sapien

<400> 302
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gctagaaaat 1560
tttatggcta tcgaagaaat gaagaagcac ggaagtactc atgtcggatt cccagaaaac 1620
ctgactaatg gtgccactgc tggcaatggt gatgatggat taattcctcc aaggaagagc 1680
agaacacctg aaagccagca atttcctgac actgagaatg aagagtatca cagtgacgaa 1740
caaaatgata ctcagaagca attttgtgaa gaacagaaca ctggaatatt acacgatgag 1800
attctgattc atgaagaaaa gcagatagaa gtggttgaaa aaatgaattc tgagctttct 1860
cttagttgta agaaagaaaa agacatcttg catgaaaata gtacgttgcg ggaagaaatt 1920
gccatgctaa gactggagct agacacaatg aaacatcaga gccagctaaa aaaaaaaaaa 1980
aaaaaaaaaa aaaaaaaaaa 2000

<210> 303
<211> 2040
<212> DNA
<213> Homo sapien

<400> 303
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcggca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagaaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaatcaaga aaaaagcgaa tttaaatgca 900
ctggatagat atggaaggac tgctctcata cttgctgtat gttgtggatc agcaagtata 960

gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaca agacttaaag 1140
ctgacatcag aggaagagtc acaaaggttc aaaggcagtg aaaatagcca gccagagaaa 1200
atgtctcaag aaccagaaat aaataaggat ggtgatagag aggttgaaga agaaatgaag 1260
aagcatgaaa gtaataatgt gggattacta gaaaacctga ctaatggtgt cactgctggc 1320
aatggtgata atggattaat tcctcaaagg aagagcagaa cacctgaaaa tcagcaattt 1380
cctgacaacg aaagtgaaga gtatcacaga atttgcgaat tagtttctga ctacaaagaa 1440
aaacagatgc caaaatactc ttctgaaaac agcaacccag aacaagactt aaagctgaca 1500
tcagaggaag agtcacaaag gcttgagggc agtgaaaatg gccagccaga gaaaagatct 1560
caagaaccag aaataaataa ggatggtgat agagagctag aaaattttat ggctatcgaa 1620
gaaatgaaga agcacggaag tactcatgtc ggattcccag aaaacctgac taatggtgcc 1680
actgctggca atggtgatga tggattaatt cctccaagga agagcagaac acctgaaagc 1740
cagcaatttc ctgacactga gaatgaagag tatcacagtg acgaacaaaa tgatactcag 1800
aagcaatttt gtgaagaaca gaacactgga atattacacg atgagattct gattcatgaa 1860
gaaaagcaga tagaagtggt tgaaaaaatg aattctgagc tttctcttag ttgtaagaaa 1920
gaaaaagaca tcttgcatga aaatagtacg ttgcgggaag aaattgccat gctaagactg 1980
gagctagaca caatgaaaca tcagagccag ctaaaaaaaa aaaaaaaaaa aaaaaaaaaa 2040

<210> 304
<211> 384
<212> PRT
<213> Homo sapien

<400> 304
Met Val Val Glu Val Asp Ser Met Pro Ala Ala Ser Ser Val Lys Lys
1               5                   10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly

260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Asn Val Ser Arg Thr Arg Asn Lys
    370                 375                 380

<210> 305
<211> 656
<212> PRT
<213> Homo sapien

<400> 305
Met Val Val Glu Val Asp Ser Met Pro Ala Ala Ser Ser Val Lys Lys
 1               5                  10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp
        35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285

Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460
Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu
            500                 505                 510
Asn Gly Gln Pro Glu Leu Glu Asn Phe Met Ala Ile Glu Glu Met Lys
        515                 520                 525
Lys His Gly Ser Thr His Val Gly Phe Pro Glu Asn Leu Thr Asn Gly
    530                 535                 540
Ala Thr Ala Gly Asn Gly Asp Asp Gly Leu Ile Pro Pro Arg Lys Ser
545                 550                 555                 560
Arg Thr Pro Glu Ser Gln Gln Phe Pro Asp Thr Glu Asn Glu Glu Tyr
                565                 570                 575
His Ser Asp Glu Gln Asn Asp Thr Gln Lys Gln Phe Cys Glu Glu Gln
            580                 585                 590
Asn Thr Gly Ile Leu His Asp Glu Ile Leu Ile His Glu Glu Lys Gln
        595                 600                 605
Ile Glu Val Val Glu Lys Met Asn Ser Glu Leu Ser Leu Ser Cys Lys
    610                 615                 620
Lys Glu Lys Asp Ile Leu His Glu Asn Ser Thr Leu Arg Glu Glu Ile
625                 630                 635                 640
Ala Met Leu Arg Leu Glu Leu Asp Thr Met Lys His Gln Ser Gln Leu
                645                 650                 655

<210> 306
<211> 671
<212> PRT
<213> Homo sapien

<400> 306
Met Val Val Glu Val Asp Ser Met Pro Ala Ala Ser Ser Val Lys Lys
1               5                   10                  15
Pro Phe Gly Leu Arg Ser Lys Met Gly Lys Trp Cys Cys Arg Cys Phe
            20                  25                  30
Pro Cys Cys Arg Glu Ser Gly Lys Ser Asn Val Gly Thr Ser Gly Asp

35                  40                  45
His Asp Asp Ser Ala Met Lys Thr Leu Arg Ser Lys Met Gly Lys Trp
    50                  55                  60
Cys Arg His Cys Phe Pro Cys Cys Arg Gly Ser Gly Lys Ser Asn Val
65                  70                  75                  80
Gly Ala Ser Gly Asp His Asp Asp Ser Ala Met Lys Thr Leu Arg Asn
                85                  90                  95
Lys Met Gly Lys Trp Cys Cys His Cys Phe Pro Cys Cys Arg Gly Ser
            100                 105                 110
Gly Lys Ser Lys Val Gly Ala Trp Gly Asp Tyr Asp Asp Ser Ala Phe
        115                 120                 125
Met Glu Pro Arg Tyr His Val Arg Gly Glu Asp Leu Asp Lys Leu His
    130                 135                 140
Arg Ala Ala Trp Trp Gly Lys Val Pro Arg Lys Asp Leu Ile Val Met
145                 150                 155                 160
Leu Arg Asp Thr Asp Val Asn Lys Lys Asp Lys Gln Lys Arg Thr Ala
                165                 170                 175
Leu His Leu Ala Ser Ala Asn Gly Asn Ser Glu Val Val Lys Leu Leu
            180                 185                 190
Leu Asp Arg Arg Cys Gln Leu Asn Val Leu Asp Asn Lys Lys Arg Thr
        195                 200                 205
Ala Leu Ile Lys Ala Val Gln Cys Gln Glu Asp Glu Cys Ala Leu Met
    210                 215                 220
Leu Leu Glu His Gly Thr Asp Pro Asn Ile Pro Asp Glu Tyr Gly Asn
225                 230                 235                 240
Thr Thr Leu His Tyr Ala Ile Tyr Asn Glu Asp Lys Leu Met Ala Lys
                245                 250                 255
Ala Leu Leu Leu Tyr Gly Ala Asp Ile Glu Ser Lys Asn Lys His Gly
            260                 265                 270
Leu Thr Pro Leu Leu Leu Gly Val His Glu Gln Lys Gln Gln Val Val
        275                 280                 285
Lys Phe Leu Ile Lys Lys Lys Ala Asn Leu Asn Ala Leu Asp Arg Tyr
    290                 295                 300
Gly Arg Thr Ala Leu Ile Leu Ala Val Cys Cys Gly Ser Ala Ser Ile
305                 310                 315                 320
Val Ser Leu Leu Leu Glu Gln Asn Ile Asp Val Ser Ser Gln Asp Leu
                325                 330                 335
Ser Gly Gln Thr Ala Arg Glu Tyr Ala Val Ser Ser His His His Val
            340                 345                 350
Ile Cys Gln Leu Leu Ser Asp Tyr Lys Glu Lys Gln Met Leu Lys Ile
        355                 360                 365
Ser Ser Glu Asn Ser Asn Pro Glu Gln Asp Leu Lys Leu Thr Ser Glu
    370                 375                 380
Glu Glu Ser Gln Arg Phe Lys Gly Ser Glu Asn Ser Gln Pro Glu Lys
385                 390                 395                 400
Met Ser Gln Glu Pro Glu Ile Asn Lys Asp Gly Asp Arg Glu Val Glu
                405                 410                 415
Glu Glu Met Lys Lys His Glu Ser Asn Asn Val Gly Leu Leu Glu Asn
            420                 425                 430
Leu Thr Asn Gly Val Thr Ala Gly Asn Gly Asp Asn Gly Leu Ile Pro
        435                 440                 445
Gln Arg Lys Ser Arg Thr Pro Glu Asn Gln Gln Phe Pro Asp Asn Glu
    450                 455                 460
Ser Glu Glu Tyr His Arg Ile Cys Glu Leu Val Ser Asp Tyr Lys Glu
465                 470                 475                 480
Lys Gln Met Pro Lys Tyr Ser Ser Glu Asn Ser Asn Pro Glu Gln Asp
                485                 490                 495
Leu Lys Leu Thr Ser Glu Glu Glu Ser Gln Arg Leu Glu Gly Ser Glu

500                 505                 510
Asn Gly Gln Pro Glu Lys Arg Ser Gln Glu Pro Glu Ile Asn Lys Asp
        515                 520                 525
Gly Asp Arg Glu Leu Glu Asn Phe Met Ala Ile Glu Glu Met Lys Lys
    530                 535                 540
His Gly Ser Thr His Val Gly Phe Pro Glu Asn Leu Thr Asn Gly Ala
545                 550                 555                 560
Thr Ala Gly Asn Gly Asp Asp Gly Leu Ile Pro Pro Arg Lys Ser Arg
                565                 570                 575
Thr Pro Glu Ser Gln Gln Phe Pro Asp Thr Glu Asn Glu Glu Tyr His
            580                 585                 590
Ser Asp Glu Gln Asn Asp Thr Gln Lys Gln Phe Cys Glu Glu Gln Asn
        595                 600                 605
Thr Gly Ile Leu His Asp Glu Ile Leu Ile His Glu Glu Lys Gln Ile
    610                 615                 620
Glu Val Val Glu Lys Met Asn Ser Glu Leu Ser Leu Ser Cys Lys Lys
625                 630                 635                 640
Glu Lys Asp Ile Leu His Glu Asn Ser Thr Leu Arg Glu Glu Ile Ala
                645                 650                 655
Met Leu Arg Leu Glu Leu Asp Thr Met Lys His Gln Ser Gln Leu
            660                 665                 670

<210> 307
<211> 800
<212> DNA
<213> Homo sapien

<400> 307
atkagcttcc gcttctgaca acactagaga tccctcccct ccctcagggt atggccctcc      60
acttcatttt tggtacataa catctttata ggacaggggt aaaatcccaa tactaacagg     120
agaatgctta ggactctaac aggtttttga gaatgtgttg gtaagggcca ctcaatccaa     180
tttttcttgg tcctccttgt ggtctaggag gacaggcaag ggtgcagatt ttcaagaatg     240
catcagtaag ggccactaaa tccgaccttc ctcgttcctc cttgtggtct gggaggaaaa     300
ctagtgtttc tgttgctgtg tcagtgagca caactattcc gatcagcagg gtccagggac     360
cactgcaggt tcttgggcag ggggagaaac aaaacaaacc aaaaccatgg gcrgttttgt     420
ctttcagatg ggaaacactc aggcatcaac aggctcacct ttgaaatgca tcctaagcca     480
atgggacaaa tttgacccac aaaccctgga aaaagaggtg gctcattttt tttgcactat     540
ggcttggccc caacattctc tctctgatgg ggaaaaatgg ccacctgagg gaagtacaga     600
ttacaatact atcctgcagc ttgacctttt ctgtaagagg gaaggcaaat ggagtgaaat     660
accttatgtc caagctttct tttcattgaa ggagaataca ctatgcaaag cttgaaattt     720
acatcccaca ggaggacctc tcagcttacc cccatatcct agcctcccta tagctcccct     780
tcctattagt gataagcctc                                                 800

<210> 308
<211> 102
<212> PRT
<213> Homo sapien

<220>
<221> VARIANT
<222> (1)...(102)
<223> Xaa = Any Amino Acid

<400> 308
Met Gly Xaa Phe Val Phe Gln Met Gly Asn Thr Gln Ala Ser Thr Gly
 1               5                  10                  15
Ser Pro Leu Lys Cys Ile Leu Ser Gln Trp Asp Lys Phe Asp Pro Gln
            20                  25                  30

Thr Leu Glu Lys Glu Val Ala His Phe Phe Cys Thr Met Ala Trp Pro
        35                  40                  45
Gln His Ser Leu Ser Asp Gly Glu Lys Trp Pro Pro Glu Gly Ser Thr
    50                  55                  60
Asp Tyr Asn Thr Ile Leu Gln Leu Asp Leu Phe Cys Lys Arg Glu Gly
65                  70                  75                  80
Lys Trp Ser Glu Ile Pro Tyr Val Gln Ala Phe Phe Ser Leu Lys Glu
                85                  90                  95
Asn Thr Leu Cys Lys Ala
            100

<210> 309
<211> 9
<212> PRT
<213> Artificial Sequence

<220>
<223> Made in the lab

<400> 309
Leu Met Ala Glu Glu Tyr Thr Ile Val
1               5

<210> 310
<211> 9
<212> PRT
<213> Artificial Sequence

<220>
<223> Made in the lab

<400> 310
Lys Leu Met Ala Lys Ala Leu Leu Leu
1               5

<210> 311
<211> 9
<212> PRT
<213> Artificial Sequence

<220>
<223> Made in the lab

<400> 311
Gly Leu Thr Pro Leu Leu Leu Gly Ile
1               5

<210> 312
<211> 10
<212> PRT
<213> Artificial Sequence

<220>
<223> Made in the lab

<400> 312
Lys Leu Val Leu Asp Arg Arg Cys Gln Leu
1               5                   10

<210> 313
<211> 1852
<212> DNA
<213> Homo sapiens

<400> 313
ggcacgagaa ttaaaaccct cagcaaaaca ggcatagaag ggacatacct taaagtaata 60
aaaaccacct atgacaagcc cacagccaac ataatactaa atggggaaaa gttagaagca 120
tttcctctga gaactgcaac aataaataca aggatgctgg attttgtcaa atgccttttc 180
tgtgtctgtt gagatgctta tgtgactttg cttttaattc tgtttatgtg attatcacat 240
ttattgactt gcctgtgtta gaccggaaga gctggggtgt ttctcaggag ccaccgtgtg 300
ctgcggcagc ttcgggataa cttgaggctg catcactggg gaagaaacac aytcctgtcc 360
gtggcgctga tggctgagga cagagcttca gtgtggcttc tctgcgactg gcttcttcgg 420
ggagttcttc cttcatagtt catccatatg gctccagagg aaaattatat tattttgtta 480
tggatgaaga gtattacgtt gtgcagatat actgcagtgt cttcatctct tgatgtgtga 540
ttgggtaggt tccaccatgt tgccgcagat gacatgattt cagtacctgt gtctggctga 600
aaagtgtttg tttgtgaatg gatattgtgg tttctggatc tcatcctctg tgggtggaca 660
gctttctcca ccttgctgga agtgacctgc tgtccagaag tttgatggct gaggagtata 720
ccatcgtgca tgcatctttc atttcctgca tttcttcctc cctggatgga cagggggagc 780
ggcaagagca acgtgggcac ttctggagac cacaacgact cctctgtgaa gacgcttggg 840
agcaagaggt gcaagtggtg ctgccactgc ttcccctgct gcagggggag cggcaagagc 900
aacgtggtcg cttggggaga ctacgatgac agcgccttca tggatcccag gtaccacgtc 960
catggagaag atctggacaa gctccacaga gctgcctggt ggggtaaagt ccccagaaag 1020
gatctcatcg tcatgctcag ggacacggat gtgaacaaga gggacaagca aaagaggact 1080
gctctacatc tggcctctgc caatgggaat tcagaagtag taaaactcgt gctggacaga 1140
cgatgtcaac ttaatgtcct tgacaacaaa aagaggacag ctctgacaaa ggccgtacaa 1200
tgccaggaag atgaatgtgc gttaatgttg ctggaacatg gcactgatcc aaatattcca 1260
gatgagtatg gaaataccac tctacactat gctgtctaca atgaagataa attaatggcc 1320
aaagcactgc tcttatacgg tgctgatatc gaatcaaaaa acaagcatgg cctcacacca 1380
ctgctacttg gtatacatga gcaaaaacag caagtggtga aatttttaat caagaaaaaa 1440
gcgaatttaa atgcgctgga tagatatgga agaactgctc tcatacttgc tgtatgttgt 1500
ggatcagcaa gtatagtcag ccctctactt gagcaaaatg ttgatgtatc ttctcaagat 1560
ctggaaagac ggccagagag tatgctgttt ctagtcatca tcatgtaatt tgccagttac 1620
tttctgacta caaagaaaaa cagatgttaa aaatctcttc tgaaaacagc aatccagaac 1680
aagacttaaa gctgacatca gaggaagagt cacaaaggct taaaggaagt gaaaacagcc 1740
agccagagct agaagattta tggctattga agaagaatga agaacacgga agtactcatg 1800
tgggattccc agaaaacctg actaacggtg ccgctgctgg caatggtgat ga 1852

<210> 314
<211> 879
<212> DNA
<213> Homo sapiens

<400> 314
atgcatcttt catttcctgc atttcttcct ccctggatgg acagggggag cggcaagagc 60
aacgtgggca cttctggaga ccacaacgac tcctctgtga agacgcttgg gagcaagagg 120
tgcaagtggt gctgccactg cttcccctgc tgcaggggga gcggcaagag caacgtggtc 180
gcttggggag actacgatga cagcgccttc atggatccca ggtaccacgt ccatggagaa 240
gatctggaca agctccacag agctgcctgg tggggtaaag tccccagaaa ggatctcatc 300
gtcatgctca gggacacgga tgtgaacaag agggacaagc aaaagaggac tgctctacat 360
ctggcctctg ccaatgggaa ttcagaagta gtaaaactcg tgctggacag acgatgtcaa 420
cttaatgtcc ttgacaacaa aaagaggaca gctctgacaa aggccgtaca atgccaggaa 480
gatgaatgtg cgttaatgtt gctggaacat ggcactgatc caaatattcc agatgagtat 540
ggaaatacca ctctacacta tgctgtctac aatgaagata aattaatggc caaagcactg 600
ctcttatacg gtgctgatat cgaatcaaaa aacaagcatg gcctcacacc actgctactt 660
ggtatacatg agcaaaaaca gcaagtggtg aaatttttaa tcaagaaaaa agcgaattta 720
aatgcgctgg atagatatgg aagaactgct ctcatacttg ctgtatgttg tggatcagca 780

agtatagtca gccctctact tgagcaaaat gttgatgtat cttctcaaga tctggaaaga 840
cggccagaga gtatgctgtt tctagtcatc atcatgtaa 879

<210> 315
<211> 292
<212> PRT
<213> Homo sapiens

<400> 315
Met His Leu Ser Phe Pro Ala Phe Leu Pro Pro Trp Met Asp Arg Gly
                5                   10                  15

Ser Gly Lys Ser Asn Val Gly Thr Ser Gly Asp His Asn Asp Ser Ser
            20                  25                  30

Val Lys Thr Leu Gly Ser Lys Arg Cys Lys Trp Cys Cys His Cys Phe
        35                  40                  45

Pro Cys Cys Arg Gly Ser Gly Lys Ser Asn Val Val Ala Trp Gly Asp
    50                  55                  60

Tyr Asp Asp Ser Ala Phe Met Asp Pro Arg Tyr His Val His Gly Glu
65                  70                  75                  80

Asp Leu Asp Lys Leu His Arg Ala Ala Trp Trp Gly Lys Val Pro Arg
                85                  90                  95

Lys Asp Leu Ile Val Met Leu Arg Asp Thr Asp Val Asn Lys Arg Asp
            100                 105                 110

Lys Gln Lys Arg Thr Ala Leu His Leu Ala Ser Ala Asn Gly Asn Ser
        115                 120                 125

Glu Val Val Lys Leu Val Leu Asp Arg Arg Cys Gln Leu Asn Val Leu
    130                 135                 140

Asp Asn Lys Lys Arg Thr Ala Leu Thr Lys Ala Val Gln Cys Gln Glu
145                 150                 155                 160

Asp Glu Cys Ala Leu Met Leu Leu Glu His Gly Thr Asp Pro Asn Ile
                165                 170                 175

Pro Asp Glu Tyr Gly Asn Thr Thr Leu His Tyr Ala Val Tyr Asn Glu
            180                 185                 190

Asp Lys Leu Met Ala Lys Ala Leu Leu Leu Tyr Gly Ala Asp Ile Glu
        195                 200                 205

Ser Lys Asn Lys His Gly Leu Thr Pro Leu Leu Leu Gly Ile His Glu
    210                 215                 220

Gln Lys Gln Gln Val Val Lys Phe Leu Ile Lys Lys Lys Ala Asn Leu
225                 230                 235                 240

Asn Ala Leu Asp Arg Tyr Gly Arg Thr Ala Leu Ile Leu Ala Val Cys
                245                 250                 255

Cys Gly Ser Ala Ser Ile Val Ser Pro Leu Leu Glu Gln Asn Val Asp

260                 265                 270

Val Ser Ser Gln Asp Leu Glu Arg Arg Pro Glu Ser Met Leu Phe Leu
        275                 280                 285

Val Ile Ile Met
    290

<210> 316
<211> 584
<212> DNA
<213> Homo sapiens

<400> 316
agttgggcca aattcccctc cccctacagc ttgaagggga cataaccaat agcctggggt 60
ttttttgtgg tcctttggag atttctttgc ttattttctt ctgggtgggg gtgattagag 120
gaggcttatc actaatagga aggggagcta tagggaggct aggatatggg ggtaagctga 180
gaggtcctcc tgtgggatgt aaatttcaag ctttgcatag tgtattctcc ttcaatgaaa 240
agaaagcttg gacataaggt atttcactcc atttgccttc cctcttacag aaaaggtcaa 300
gctgcaggat agtattgtaa tctgtacttc cctcaggtgg ccatttttcc ccatcagaga 360
gagaatgttg gggccaagcc atagtgcaga aaaaaaaatg agccacctct tttccagggg 420
tttgtgggtc aaatttgtcc cattggctta ggatgcattt caaaggtgag cctgttgatg 480
cctgagtgtt tcccatctga aagacaaaac tgcccatggt tttggtttgt tttgtttctc 540
cccctgccca agaactatca aactcctgag ccaacaacta aaaa 584

<210> 317
<211> 829
<212> DNA
<213> Homo sapiens

<400> 317
attagcttcc gcttctgaca acactagaga tccctcccct ccctcagggt atggccctcc 60
acttcatttt tggtacataa catctttata ggacaggggt aaaatcccaa tactaacagg 120
agaatgctta ggactctaac aggtttttga gaatgtgttg gtaagggcca ctcaatccaa 180
tttttcttgg tcctccttgt ggtctaggag gacaggcaag ggtgcagatt ttcaagaatg 240
catcagtaag ggccactaaa tccgaccttc ctcgttcctc cttgtggtct gggaggaaaa 300
ctagtgtttc tgttgctgtg tcagtgagca caactattcc gatcagcagg gtccagggac 360
cactgcaggt tcttgggcag ggggagaaac aaaacaaacc aaaaccatgg gcagttttgt 420
ctttcagatg ggaaacactc aggcatcaac aggctcacct ttgaaatgca tcctaagcca 480
atgggacaaa tttgacccac aaaccctgga aaaagaggtg gctcattttt tttgcactat 540
ggcttggccc caacattctc tctctgatgg ggaaaaatgg ccacctgagg gaagtacaga 600
ttacaatact atcctgcagc ttgacctttt ctgtaagagg gaaggcaaat ggagtgaaat 660
accttatgtc caagctttct tttcattgaa ggagaataca ctatgcaaag cttgaaattt 720
acatcccaca ggaggacctc tcagcttacc cccatatcct agcctcccta tagctcccct 780
tcctattagt gataagcctc ctctaatcac ccccacccag aagaaaata 829

<210> 318
<211> 30
<212> PRT
<213> Homo sapien

<400> 318
Thr Ala Ala Ser Asp Asn Phe Gln Leu Ser Gln Gly Gly Gln Gly Phe
1               5                   10                  15

Ala Ile Pro Ile Gly Gln Ala Met Ala Ile Ala Gly Gln Ile
            20                  25                  30

<210> 319
<211> 41
<212> DNA
<213> Artificial Sequence

<220>
<223> PCR primer

<400> 319

ggcctctgcc aatgggaact cagaagtagt aaaactcctg c                41


<210> 320
<211> 41
<212> DNA
<213> Artificial Sequence

<220>
<223> PCR primer

<400> 320

gcaggagttt tactacttct gagttcccat tggcagaggc c                41


<210> 321
<211> 60
<212> DNA
<213> Artificial Sequence

<220>
<223> PCR primer

<400> 321

ggggaattcc cgctggtgcc gcgcggcagc cctatggtgg ttgaggttga        50
ttccatgccg                                                    60


<210> 322
<211> 42
<212> DNA
<213> Artificial Sequnce

<220>
<223> PCR primer

<400> 322

cccgaattct tatttatttc tggttcttga gacattttct gg                42

<210> 323
<211> 1590
<212> DNA
<213> Homo sapiens

<400> 323
atgcatcacc atcaccatca cacggccgcg tccgataact tccagctgtc ccagggtggg 60
cagggattcg ccattccgat cgggcaggcg atggcgatcg cgggccagat caagcttccc 120
accgttcata tcgggcctac cgccttcctc ggcttgggtg ttgtcgacaa caacggcaac 180
ggcgcacgag tccaacgcgt ggtcgggagc gctccggcgg caagtctcgg catctccacc 240
ggcgacgtga tcaccgcggt cgacggcgct ccgatcaact cggccaccgc gatggcggac 300
gcgcttaacg ggcatcatcc cggtgacgtc atctcggtga cctggcaaac caagtcgggc 360
ggcacgcgta cagggaacgt gacattggcc gagggacccc cggccgaatt cccgctggtg 420
ccgcgcggca gccctatggt ggttgaggtt gattccatgc cggctgcttc ttctgtgaag 480
aagccatttg gtctcaggag caagatgggc aagtggtgct gccgttgctt cccctgctgc 540
agggagagcg gcaagagcaa cgtgggcact tctggagacc acgacgactc tgctatgaag 600
acactcagga gcaagatggg caagtggtgc cgccactgct tcccctgctg cagggggagt 660
ggcaagagca acgtgggcgc ttctggagac cacgacgact ctgctatgaa gacactcagg 720
aacaagatgg gcaagtggtg ctgccactgc ttcccctgct gcagggggag cggcaagagc 780
aaggtgggcg cttggggaga ctacgatgac agygccttca tggagcccag gtaccacgtc 840
cgtggagaag atctggacaa gctccacaga gctgcctggt ggggtaaagt ccccagaaag 900
gatctcatcg tcatgctcag ggacactgac gtgaacaaga aggacaagca aaagaggact 960
gctctacatc tggcctctgc caatgggaat tcagaagtag taaaactcct gctggacaga 1020
cgatgtcaac ttaatgtcct tgacaacaaa aagaggacag ctctgataaa ggccgtacaa 1080
tgccaggaag atgaatgtgc gttaatgttg ctggaacatg gcactgatcc aaatattcca 1140
gatgagtatg gaaataccac tctgcactac gctatctata atgaagataa attaatggcc 1200
aaagcactgc tcttatatgg tgctgatatc gaatcaaaaa acaagcatgg cctcacacca 1260
ctgttacttg gtgtacatga gcaaaaacag caagtcgtga aatttttaat caagaaaaaa 1320
gcgaatttaa atgcactgga tagatatgga aggactgctc tcatacttgc tgtatgttgt 1380
ggatcagcaa gtatagtcag ccttctactt gagcaaaata ttgatgtatc ttctcaagat 1440
ctatctggac agacggccag agagtatgct gtttctagtc atcatcatgt aatttgccag 1500
ttactttctg actacaaaga aaaacagatg ctaaaaatct cttctgaaaa cagcaatcca 1560
gaaaatgtct caagaaccag aaataaataa 1590

<210> 324
<211> 529
<212> PRT
<213> Homo sapiens

<400> 324
Met His His His His His His Thr Ala Ala Ser Asp Asn Phe Gln Leu
                5                   10                  15

Ser Gln Gly Gly Gln Gly Phe Ala Ile Pro Ile Gly Gln Ala Met Ala
            20                  25                  30

Ile Ala Gly Gln Ile Lys Leu Pro Thr Val His Ile Gly Pro Thr Ala
        35                  40                  45

Phe Leu Gly Leu Gly Val Val Asp Asn Asn Gly Asn Gly Ala Arg Val
    50                  55                  60

Gln Arg Val Val Gly Ser Ala Pro Ala Ala Ser Leu Gly Ile Ser Thr
65                  70                  75                  80

Gly Asp Val Ile Thr Ala Val Asp Gly Ala Pro Ile Asn Ser Ala Thr
                85                  90                  95

Ala Met Ala Asp Ala Leu Asn Gly His His Pro Gly Asp Val Ile Ser
            100                 105                 110

Val Thr Trp Gln Thr Lys Ser Gly Gly Thr Arg Thr Gly Asn Val Thr
        115                 120                 125

Leu Ala Glu Gly Pro Pro Ala Glu Phe Pro Leu Val Pro Arg Gly Ser
    130                 135                 140

Pro Met Val Val Glu Val Asp Ser Met Pro Ala Ala Ser Ser Val Lys
145                 150                 155                 160

Lys Pro Phe Gly Leu Arg Ser Lys Met Gly Lys Trp Cys Cys Arg Cys
                165                 170                 175

Phe Pro Cys Cys Arg Glu Ser Gly Lys Ser Asn Val Gly Thr Ser Gly
            180                 185                 190

Asp His Asp Asp Ser Ala Met Lys Thr Leu Arg Ser Lys Met Gly Lys
        195                 200                 205

Trp Cys Arg His Cys Phe Pro Cys Cys Arg Gly Ser Gly Lys Ser Asn
    210                 215                 220

Val Gly Ala Ser Gly Asp His Asp Asp Ser Ala Met Lys Thr Leu Arg
225                 230                 235                 240

Asn Lys Met Gly Lys Trp Cys Cys His Cys Phe Pro Cys Cys Arg Gly
                245                 250                 255

Ser Gly Lys Ser Lys Val Gly Ala Trp Gly Asp Tyr Asp Asp Ser Ala
            260                 265                 270

Phe Met Glu Pro Arg Tyr His Val Arg Gly Glu Asp Leu Asp Lys Leu
        275                 280                 285

His Arg Ala Ala Trp Trp Gly Lys Val Pro Arg Lys Asp Leu Ile Val
    290                 295                 300

Met Leu Arg Asp Thr Asp Val Asn Lys Lys Asp Lys Gln Lys Arg Thr
305                 310                 315                 320

Ala Leu His Leu Ala Ser Ala Asn Gly Asn Ser Glu Val Val Lys Leu
                325                 330                 335

Leu Leu Asp Arg Arg Cys Gln Leu Asn Val Leu Asp Asn Lys Lys Arg
            340                 345                 350

Thr Ala Leu Ile Lys Ala Val Gln Cys Gln Glu Asp Glu Cys Ala Leu
        355                 360                 365

Met Leu Leu Glu His Gly Thr Asp Pro Asn Ile Pro Asp Glu Tyr Gly
    370                 375                 380

Asn Thr Thr Leu His Tyr Ala Ile Tyr Asn Glu Asp Lys Leu Met Ala
385                 390                 395                 400

Lys Ala Leu Leu Leu Tyr Gly Ala Asp Ile Glu Ser Lys Asn Lys His
                405                 410                 415

Gly Leu Thr Pro Leu Leu Leu Gly Val His Glu Gln Lys Gln Gln Val

420                 425                 430

Val Lys Phe Leu Ile Lys Lys Lys Ala Asn Leu Asn Ala Leu Asp Arg
        435                 440                 445

Tyr Gly Arg Thr Ala Leu Ile Leu Ala Val Cys Cys Gly Ser Ala Ser
    450                 455                 460

Ile Val Ser Leu Leu Leu Glu Gln Asn Ile Asp Val Ser Ser Gln Asp
465                 470                 475                 480

Leu Ser Gly Gln Thr Ala Arg Glu Tyr Ala Val Ser Ser His His His
                485                 490                 495

Val Ile Cys Gln Leu Leu Ser Asp Tyr Lys Glu Lys Gln Met Leu Lys
            500                 505                 510

Ile Ser Ser Glu Asn Ser Asn Pro Glu Asn Val Ser Arg Thr Arg Asn
        515                 520                 525

Lys


<210> 325
<211> 1155
<212> DNA
<213> Homo sapiens

<400> 325
atggtggctg aggtttgttc aatgcccact gcctctactg tgaagaagcc atttgatctc 60
aggagcaaga tgggcaagtg gtgccaccac cgcttcccct gctgcagggg gagcggcaag 120
agcaacatgg gcacttctgg agaccacgac gactccttta tgaagatgct caggagcaag 180
atgggcaagt gttgccgcca ctgcttcccc tgctgcaggg ggagcggcac gagcaacgtg 240
ggcacttctg gagaccatga aaactccttt atgaagatgc tcaggagcaa gatgggcaag 300
tggtgctgtc actgcttccc ctgctgcagg gggagcggca agagcaacgt gggcgcttgg 360
ggagactacg accacagcgc cttcatggag ccgaggtacc acatccgtcg agaagatctg 420
gacaagctcc acagagctgc ctggtggggt aaagtcccca gaaaggatct catcgtcatg 480
ctcagggaca ctgacatgaa caagagggac aaggaaaaga ggactgctct acatttggcc 540
tctgccaatg gaaattcaga agtagtacaa ctcctgctgg acagacgatg tcaacttaat 600
gtccttgaca acaaaaaaag gacagctctg ataaaggcca tacaatgcca ggaagatgaa 660
tgtgtgttaa tgttgctgga acatggcgct gatcgaaata ttccagatga gtatggaaat 720
accgctctac actatgctat ctacaatgaa gataaattaa tggccaaagc actgctctta 780
tatggtgctg atattgaatc aaaaaacaag gttggcctca caccactttt gcttggcgta 840
catgaacaaa aacagcaagt ggtgaaattt ttaatcaaga aaaaagctaa tttaaatgta 900
cttgatagat atggaaggac tgccctcata cttgctgtat gttgtggatc agcaagtata 960
gtcaatcttc tacttgagca aaatgttgat gtatcttctc aagatctatc tggacagacg 1020
gccagagagt atgctgtttc tagtcatcat catgtaattt gtgaattact ttctgactat 1080
aaagaaaaac agatgctaaa aatctcttct gaaaacagca atccagaaaa tgtctcaaga 1140
accagaaata aataa                                                   1155

<210> 326
<211> 384
<212> PRT
<213> Homo sapiens

<400> 326
Met Val Ala Glu Val Cys Ser Met Pro Thr Ala Ser Thr Val Lys Lys
                5                   10                  15

Pro Phe Asp Leu Arg Ser Lys Met Gly Lys Trp Cys His His Arg Phe
            20                  25                  30

Pro Cys Cys Arg Gly Ser Gly Lys Ser Asn Met Gly Thr Ser Gly Asp
        35                  40                  45

His Asp Asp Ser Phe Met Lys Met Leu Arg Ser Lys Met Gly Lys Cys
    50                  55                  60

Cys Arg His Cys Phe Pro Cys Cys Arg Gly Ser Gly Thr Ser Asn Val
65                  70                  75                  80

Gly Thr Ser Gly Asp His Glu Asn Ser Phe Met Lys Met Leu Arg Ser
                85                  90                  95

Lys Met Gly Lys Trp Cys Cys His Cys Phe Pro Cys Cys Arg Gly Ser
            100                 105                 110

Gly Lys Ser Asn Val Gly Ala Trp Gly Asp Tyr Asp His Ser Ala Phe
        115                 120                 125

Met Glu Pro Arg Tyr His Ile Arg Arg Glu Asp Leu Asp Lys Leu His
    130                 135                 140

Arg Ala Ala Trp Trp Gly Lys Val Pro Arg Lys Asp Leu Ile Val Met
145                 150                 155                 160

Leu Arg Asp Thr Asp Met Asn Lys Arg Asp Lys Glu Lys Arg Thr Ala
                165                 170                 175

Leu His Leu Ala Ser Ala Asn Gly Asn Ser Glu Val Val Gln Leu Leu
            180                 185                 190

Leu Asp Arg Arg Cys Gln Leu Asn Val Leu Asp Asn Lys Lys Arg Thr
        195                 200                 205

Ala Leu Ile Lys Ala Ile Gln Cys Gln Glu Asp Glu Cys Val Leu Met
    210                 215                 220

Leu Leu Glu His Gly Ala Asp Arg Asn Ile Pro Asp Glu Tyr Gly Asn
225                 230                 235                 240

Thr Ala Leu His Tyr Ala Ile Tyr Asn Glu Asp Lys Leu Met Ala Lys
                245                 250                 255

Ala Leu Leu Leu Tyr Gly Ala Asp Ile Glu Ser Lys Asn Lys Val Gly
            260                 265                 270

Leu Thr Pro Leu Leu Leu Gly Val His Glu Gln Lys Gln Gln Val Val
        275                 280                 285

Lys Phe Leu Ile Lys Lys Lys Ala Asn Leu Asn Val Leu Asp Arg Tyr
    290                 295                 300

Gly Arg Thr Ala Leu Ile Leu Ala Val Cys Cys Gly Ser Ala Ser Ile
305                 310                 315                 320

Val Asn Leu Leu Leu Glu Gln Asn Val Asp Val Ser Ser Gln Asp Leu
                325                 330                 335

Ser Gly Gln Thr Ala Arg Glu Tyr Ala Val Ser Ser His His His Val
            340                 345                 350

Ile Cys Glu Leu Leu Ser Asp Tyr Lys Glu Lys Gln Met Leu Lys Ile
        355                 360                 365

Ser Ser Glu Asn Ser Asn Pro Glu Asn Val Ser Arg Thr Arg Asn Lys
    370                 375                 380

<210> 327
<211> 634
<212> DNA
<213> Homo sapiens

<400> 327
gactgctcta catctggcct ctgccaatgg aaattcagaa gtagtaaaac tcctgctgga 60
cagacgatgt caacttaata tccttgacaa caaaaagagg acagctctga caaaggccgt 120
acaatgccag gaagatgaat gtgcgttaat gttgctggaa catggcactg atccgaatat 180
tccagatgag tatggaaata ccgctctaca ctatgctatc tacaatgaag ataaattaat 240
ggccaaagca ctgctcttat acggtgctga tatcgaatca aaaaacaagc atggcctcac 300
accactgtta cttggtgtac atgagcaaaa acagcaagtg gtgaaatttt taatcaagaa 360
aaaagcaaat ttaaatgcac tggatagata tggaagaact gctctcatac ttgctgtatg 420
ttgtggatcg gcaagtatag tcagccttct acttgagcaa aacattgatg tatcttctca 480
agatctatct ggacagacgg ccagagagta tgctgtttct agtcgtcata atgtaatttg 540
ccagttactt tctgactaca aagaaaaaca gatactaaaa gtctcttctg aaaacagcaa 600
tccaggaaat gtctcaagaa ccagaaataa ataa 634

<210> 328
<211> 1155
<212> DNA
<213> Homo sapiens

<400> 328
atggtggttg aggttgattc catgccggct gcctcttctg tgaagaagcc atttggtctc 60
aggagcaaga tgggcaagtg gtgctgccgt tgcttcccct gctgcaggga gagcggcaag 120
agcaacgtgg gcacttctgg agaccacgac gactctgcta tgaagacact caggagcaag 180
atgggcaagt ggtgccgcca ctgcttcccc tgctgcaggg ggagtggcaa gagcaacgtg 240
ggcgcttctg gagaccacga cgactctgct atgaagacac tcaggaacaa gatgggcaag 300
tggtgctgcc actgcttccc ctgctgcagg gggagcagca agagcaaggt gggcgcttgg 360
ggagactacg atgacagtgc cttcatggag cccaggtacc acgtccgtgg agaagatctg 420
gacaagctcc acagagctgc ctggtggggt aaagtcccca gaaaggatct catcgtcatg 480
ctcagggaca ctgacgtgaa caagcaggac aagcaaaaga ggactgctct acatctggcc 540
tctgccaatg ggaattcaga agtagtaaaa ctcctgctgg acagacgatg tcaacttaat 600
gtccttgaca acaaaaagag gacagctctg ataaaggccg tacaatgcca ggaagatgaa 660
tgtgcgttaa tgttgctgga acatggcact gatccaaata ttccagatga gtatggaaat 720
accactctgc actacgctat ctataatgaa gataaattaa tggccaaagc actgctctta 780
tatggtgctg atatcgaatc aaaaaacaag catggcctca caccactgtt acttggtgta 840
catgagcaaa aacagcaagt cgtgaaattt ttaattaaga aaaaagcgaa tttaaatgca 900
ctggatagat atggaaggac tgctctcata cttgctgtat gttgtggatc agcaagtata 960
gtcagccttc tacttgagca aaatattgat gtatcttctc aagatctatc tggacagacg 1020
gccagagagt atgctgtttc tagtcatcat catgtaattt gccagttact ttctgactac 1080
aaagaaaaac agatgctaaa aatctcttct gaaaacagca atccagaaaa tgtctcaaga 1140
accagaaata aataa 1155

<210> 329

<211> 1155
<212> DNA
<213> Homo sapiens

<400> 329
atggtggctg aggtttgttc aatgcccgct gcctctgctg tgaagaagcc atttgatctc 60
aggagcaaga tgggcaagtg gtgccaccac cgcttcccct gctgcagggg gagcggcaag 120
agcaacatgg gcacttctgg agaccacgac gactccttta tgaagacgct caggagcaag 180
atgggcaagt gttgccacca ctgcttcccc tgctgcaggg ggagcggcac gagcaatgtg 240
ggcacttctg gagaccatga caactccttt atgaagacac tcaggagcaa gatgggcaag 300
tggtgctgtc actgcttccc ctgctgcagg gggagcggca agagcaacgt gggcacttgg 360
ggagactacg acgacagcgc cttcatggag ccgaggtacc acgtccgtcg agaagatctg 420
gacaagctcc acagagctgc ctggtggggt aaagtcccca gaaaggatct catcgtcatg 480
ctcagggaca ctgacatgaa caagagggac aagcaaaaga ggactgctct acatttggcc 540
tctgccaatg gaaattcaga agtagtacaa ctcctgctgg acagacgatg tcaacttaac 600
gtccttgaca acaaaaaaag gacagctctg ataaaggccg tacaatgcca ggaagatgaa 660
tgtgtgttaa tgttgctgga acatggcgct gatggaaata ttcaagatga gtatggaaat 720
accgctctac actatgctat ctacaatgaa gataaattaa tggccaaagc actgctctta 780
tatggtgctg atattgaatc aaaaaacaag tgtggcctca caccactttt gcttggcgta 840
catgaacaaa aacagcaagt ggtgaaattt ttaatcaaga aaaaagctaa tttaaatgca 900
cttgatagat atggaagaac tgccctcata cttgctgtat gttgtggatc agcaagtata 960
gtcaatcttc tacttgagca aaatgttgat gtatcttctc aagatctatc tggacagacg 1020
gccagagagt atgctgtttc tagtcatcat catgtaattt gtgaattact ttctgactat 1080
aaagaaaaac agatgctaaa aatctcttct gaaaacagca atccagaaaa tgtctcaaga 1140
accagaaata aataa 1155

<210> 330
<211> 1155
<212> DNA
<213> Homo sapiens

<400> 330
atggtggctg aggtttgttc aatgcccact gcctctactg tgaagaagcc atttgatctc 60
aggagcaaga tgggcaagtg gtgccaccac cgcttcccct gctgcagggg gagcggcaag 120
agcaacatgg gcacttctgg agaccacgac gactccttta tgaagatgct caggagcaag 180
atgggcaagt gttgccgcca ctgcttcccc tgctgcaggg ggagcggcac gagcaacgtg 240
ggcacttctg gagaccatga aaactccttt atgaagatgc tcaggagcaa gatgggcaag 300
tggtgctgtc actgcttccc ctgctgcagg gggagcggca agagcaacgt gggcgcttgg 360
ggagactacg accacagcgc cttcatggag ccgaggtacc acatccgtcg agaagatctg 420
gacaagctcc acagagctgc ctggtggggt aaagtcccca gaaaggatct catcgtcatg 480
ctcagggaca ctgacatgaa caagagggac aaggaaaaga ggactgctct acatttggcc 540
tctgccaatg gaaattcaga agtagtacaa ctcctgctgg acagacgatg tcaacttaat 600
gtccttgaca acaaaaaaag gacagctctg ataaaggcca tacaatgcca ggaagatgaa 660
tgtgtgttaa tgttgctgga acatggcgct gatcgaaata ttccagatga gtatggaaat 720
accgctctac actatgctat ctacaatgaa gataaattaa tggccaaagc actgctctta 780
tatggtgctg atattgaatc aaaaaacaag tgtggcctca caccactttt gcttggcgta 840
catgaacaaa aacagcaagt ggtgaaattt ttaatcaaga aaaaagctaa tttaaatgta 900
cttgatagat atggaagaac tgccctcata cttgctgtat gttgtggatc agcaagtata 960
gtcaatcttc tacttgagca aaatgttgat gtatcttctc aagatctatc tggacagacg 1020
gccagagagt atgctgtttc tagtcatcat catgtaattt gtgaattact ttctgactat 1080
aaagaaaaac agatgctaaa aatctcttct gaaaacagca atccagaaaa tgtctcaaga 1140
accagaaata aataa 1155

<210> 331
<211> 210
<212> PRT
<213> Homo sapiens

<400> 331
Thr Ala Leu His Leu Ala Ser Ala Asn Gly Asn Ser Glu Val Val Lys
                5                   10                  15

Leu Leu Leu Asp Arg Arg Cys Gln Leu Asn Ile Leu Asp Asn Lys Lys
            20                  25                  30

Arg Thr Ala Leu Thr Lys Ala Val Gln Cys Gln Glu Asp Glu Cys Ala
        35                  40                  45

Leu Met Leu Leu Glu His Gly Thr Asp Pro Asn Ile Pro Asp Glu Tyr
    50                  55                  60

Gly Asn Thr Ala Leu His Tyr Ala Ile Tyr Asn Glu Asp Lys Leu Met
65                  70                  75                  80

Ala Lys Ala Leu Leu Leu Tyr Gly Ala Asp Ile Glu Ser Lys Asn Lys
                85                  90                  95

His Gly Leu Thr Pro Leu Leu Leu Gly Val His Glu Gln Lys Gln Gln
            100                 105                 110

Val Val Lys Phe Leu Ile Lys Lys Lys Ala Asn Leu Asn Ala Leu Asp
        115                 120                 125

Arg Tyr Gly Arg Thr Ala Leu Ile Leu Ala Val Cys Cys Gly Ser Ala
    130                 135                 140

Ser Ile Val Ser Leu Leu Leu Glu Gln Asn Ile Asp Val Ser Ser Gln
145                 150                 155                 160

Asp Leu Ser Gly Gln Thr Ala Arg Glu Tyr Ala Val Ser Ser Arg His
                165                 170                 175

Asn Val Ile Cys Gln Leu Leu Ser Asp Tyr Lys Glu Lys Gln Ile Leu
            180                 185                 190

Lys Val Ser Ser Glu Asn Ser Asn Pro Gly Asn Val Ser Arg Thr Arg
        195                 200                 205

Asn Lys
    210


<210> 332
<211> 384
<212> PRT
<213> Homo sapiens

<400> 332
Met Val Ala Glu Val Cys Ser Met Pro Thr Ala Ser Thr Val Lys Lys
                5                   10                  15

Pro Phe Asp Leu Arg Ser Lys Met Gly Lys Trp Cys His His Arg Phe
            20                  25                  30

Pro Cys Cys Arg Gly Ser Gly Lys Ser Asn Met Gly Thr Ser Gly Asp
        35                  40                  45

His Asp Asp Ser Phe Met Lys Met Leu Arg Ser Lys Met Gly Lys Cys
    50                  55                  60

Cys Arg His Cys Phe Pro Cys Cys Arg Gly Ser Gly Thr Ser Asn Val
65                  70                  75                  80

Gly Thr Ser Gly Asp His Glu Asn Ser Phe Met Lys Met Leu Arg Ser
                85                  90                  95

Lys Met Gly Lys Trp Cys Cys His Cys Phe Pro Cys Cys Arg Gly Ser
            100                 105                 110

Gly Lys Ser Asn Val Gly Ala Trp Gly Asp Tyr Asp His Ser Ala Phe
        115                 120                 125

Met Glu Pro Arg Tyr His Ile Arg Arg Glu Asp Leu Asp Lys Leu His
    130                 135                 140

Arg Ala Ala Trp Trp Gly Lys Val Pro Arg Lys Asp Leu Ile Val Met
145                 150                 155                 160

Leu Arg Asp Thr Asp Met Asn Lys Arg Asp Lys Glu Lys Arg Thr Ala
                165                 170                 175

Leu His Leu Ala Ser Ala Asn Gly Asn Ser Glu Val Val Gln Leu Leu
            180                 185                 190

Leu Asp Arg Arg Cys Gln Leu Asn Val Leu Asp Asn Lys Lys Arg Thr
        195                 200                 205

Ala Leu Ile Lys Ala Ile Gln Cys Gln Glu Asp Glu Cys Val Leu Met
    210                 215                 220

Leu Leu Glu His Gly Ala Asp Arg Asn Ile Pro Asp Glu Tyr Gly Asn
225                 230                 235                 240

Thr Ala Leu His Tyr Ala Ile Tyr Asn Glu Asp Lys Leu Met Ala Lys
                245                 250                 255

Ala Leu Leu Leu Tyr Gly Ala Asp Ile Glu Ser Lys Asn Lys Cys Gly
            260                 265                 270

Leu Thr Pro Leu Leu Leu Gly Val His Glu Gln Lys Gln Gln Val Val
        275                 280                 285

Lys Phe Leu Ile Lys Lys Lys Ala Asn Leu Asn Val Leu Asp Arg Tyr
    290                 295                 300

Gly Arg Thr Ala Leu Ile Leu Ala Val Cys Cys Gly Ser Ala Ser Ile
305                 310                 315                 320

Val Asn Leu Leu Leu Glu Gln Asn Val Asp Val Ser Ser Gln Asp Leu
                325                 330                 335

Ser Gly Gln Thr Ala Arg Glu Tyr Ala Val Ser Ser His His His Val
            340                 345                 350

Ile Cys Glu Leu Leu Ser Asp Tyr Lys Glu Lys Gln Met Leu Lys Ile
        355                 360                 365

Ser Ser Glu Asn Ser Asn Pro Glu Asn Val Ser Arg Thr Arg Asn Lys
    370                 375                 380


<210> 333
<211> 384
<212> PRT
<213> Homo sapiens

<400> 333
   Met Val Ala Glu Val Cys Ser Met Pro Ala Ala Ser Ala Val Lys Lys
                   5                   10                  15
   Pro Phe Asp Leu Arg Ser Lys Met Gly Lys Trp Cys His His Arg Phe
               20                  25                  30
   Pro Cys Cys Arg Gly Ser Gly Lys Ser Asn Met Gly Thr Ser Gly Asp
           35                  40                  45
   His Asp Asp Ser Phe Met Lys Thr Leu Arg Ser Lys Met Gly Lys Cys
       50                  55                  60
   Cys His His Cys Phe Pro Cys Cys Arg Gly Ser Gly Thr Ser Asn Val
   65                  70                  75                  80
   Gly Thr Ser Gly Asp His Asp Asn Ser Phe Met Lys Thr Leu Arg Ser
                   85                  90                  95
   Lys Met Gly Lys Trp Cys Cys His Cys Phe Pro Cys Cys Arg Gly Ser
               100                 105                 110
   Gly Lys Ser Asn Val Gly Thr Trp Gly Asp Tyr Asp Asp Ser Ala Phe
           115                 120                 125
   Met Glu Pro Arg Tyr His Val Arg Arg Glu Asp Leu Asp Lys Leu His
       130                 135                 140
   Arg Ala Ala Trp Trp Gly Lys Val Pro Arg Lys Asp Leu Ile Val Met
   145                 150                 155                 160
   Leu Arg Asp Thr Asp Met Asn Lys Arg Asp Lys Gln Lys Arg Thr Ala
                   165                 170                 175
   Leu His Leu Ala Ser Ala Asn Gly Asn Ser Glu Val Val Gln Leu Leu
               180                 185                 190
   Leu Asp Arg Arg Cys Gln Leu Asn Val Leu Asp Asn Lys Lys Arg Thr
           195                 200                 205
   Ala Leu Ile Lys Ala Val Gln Cys Gln Glu Asp Glu Cys Val Leu Met
       210                 215                 220
   Leu Leu Glu His Gly Ala Asp Gly Asn Ile Gln Asp Glu Tyr Gly Asn
   225                 230                 235                 240
   Thr Ala Leu His Tyr Ala Ile Tyr Asn Glu Asp Lys Leu Met Ala Lys
                   245                 250                 255
   Ala Leu Leu Leu Tyr Gly Ala Asp Ile Glu Ser Lys Asn Lys Cys Gly
               260                 265                 270
   Leu Thr Pro Leu Leu Leu Gly Val His Glu Gln Lys Gln Gln Val Val
           275                 280                 285
   Lys Phe Leu Ile Lys Lys Lys Ala Asn Leu Asn Ala Leu Asp Arg Tyr
       290                 295                 300
   Gly Arg Thr Ala Leu Ile Leu Ala Val Cys Cys Gly Ser Ala Ser Ile
   305                 310                 315                 320
   Val Asn Leu Leu Leu Glu Gln Asn Val Asp Val Ser Ser Gln Asp Leu
                   325                 330                 335
   Ser Gly Gln Thr Ala Arg Glu Tyr Ala Val Ser Ser His His His Val
               340                 345                 350
   Ile Cys Glu Leu Leu Ser Asp Tyr Lys Glu Lys Gln Met Leu Lys Ile
           355                 360                 365
   Ser Ser Glu Asn Ser Asn Pro Glu Asn Val Ser Arg Thr Arg Asn Lys
       370                 375                 380


<210> 334
<211> 384
<212> PRT
<213> Homo sapiens

<400> 334
Met Val Val Glu Val Asp Ser Met Pro Ala Ala Ser Ser Val Lys Lys
                5                   10                  15

Pro Phe Gly Leu Arg Ser Lys Met Gly Lys Trp Cys Cys Arg Cys Phe
            20                  25                  30

Pro Cys Cys Arg Glu Ser Gly Lys Ser Asn Val Gly Thr Ser Gly Asp
        35                  40                  45

His Asp Asp Ser Ala Met Lys Thr Leu Arg Ser Lys Met Gly Lys Trp
    50                  55                  60

Cys Arg His Cys Phe Pro Cys Cys Arg Gly Ser Gly Lys Ser Asn Val
65                  70                  75                  80

Gly Ala Ser Gly Asp His Asp Asp Ser Ala Met Lys Thr Leu Arg Asn
                85                  90                  95

Lys Met Gly Lys Trp Cys Cys His Cys Phe Pro Cys Cys Arg Gly Ser
            100                 105                 110

Ser Lys Ser Lys Val Gly Ala Trp Gly Asp Tyr Asp Asp Ser Ala Phe
        115                 120                 125

Met Glu Pro Arg Tyr His Val Arg Gly Glu Asp Leu Asp Lys Leu His
    130                 135                 140

Arg Ala Ala Trp Trp Gly Lys Val Pro Arg Lys Asp Leu Ile Val Met
145                 150                 155                 160

Leu Arg Asp Thr Asp Val Asn Lys Gln Asp Lys Gln Lys Arg Thr Ala
                165                 170                 175

Leu His Leu Ala Ser Ala Asn Gly Asn Ser Glu Val Val Lys Leu Leu
            180                 185                 190

Leu Asp Arg Arg Cys Gln Leu Asn Val Leu Asp Asn Lys Lys Arg Thr
        195                 200                 205

Ala Leu Ile Lys Ala Val Gln Cys Gln Glu Asp Glu Cys Ala Leu Met
    210                 215                 220

Leu Leu Glu His Gly Thr Asp Pro Asn Ile Pro Asp Glu Tyr Gly Asn
225                 230                 235                 240

Thr Thr Leu His Tyr Ala Ile Tyr Asn Glu Asp Lys Leu Met Ala Lys
                245                 250                 255

Ala Leu Leu Leu Tyr Gly Ala Asp Ile Glu Ser Lys Asn Lys His Gly
            260                 265                 270

Leu Thr Pro Leu Leu Leu Gly Val His Glu Gln Lys Gln Gln Val Val
        275                 280                 285

Lys Phe Leu Ile Lys Lys Lys Ala Asn Leu Asn Ala Leu Asp Arg Tyr
    290                 295                 300

Gly Arg Thr Ala Leu Ile Leu Ala Val Cys Cys Gly Ser Ala Ser Ile
305                 310                 315                 320

Val Ser Leu Leu Leu Glu Gln Asn Ile Asp Val Ser Ser Gln Asp Leu
                325                 330                 335

Ser Gly Gln Thr Ala Arg Glu Tyr Ala Val Ser Ser His His His Val
            340                 345                 350

Ile Cys Gln Leu Leu Ser Asp Tyr Lys Glu Lys Gln Met Leu Lys Ile
        355                 360                 365

Ser Ser Glu Asn Ser Asn Pro Glu Asn Val Ser Arg Thr Arg Asn Lys
    370                 375                 380